(19) Europäisches Patentamt
European Patent Office
Office européen des brevets

(11) EP 2 362 226 B1

(12) # EUROPEAN PATENT SPECIFICATION

(45) Date of publication and mention of the grant of the patent:
**17.02.2016 Bulletin 2016/07**

(51) Int Cl.:
*G01N 33/68* (2006.01) *C12Q 1/34* (2006.01)
*A61P 25/28* (2006.01)

(21) Application number: **10187871.8**

(22) Date of filing: **07.03.2006**

(54) **Diagnostic method for neurodegenerative diseases based on measurement of SIRT1 levels or activity**

Diagnostisches Verfahren für neurodegenerative Krankheiten basierend auf der Messung von SIRT1 Spiegel oder Aktivität

Méthode diagnostique pour maladies neurodégénératives basée sur la mesure des niveaux ou de l'activité de SIRT1

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.03.2005 US 74374**

(43) Date of publication of application:
**31.08.2011 Bulletin 2011/35**

(62) Document number(s) of the earlier application(s) in accordance with Art. 76 EPC:
**06737459.5 / 1 863 461**

(73) Proprietors:
- **The President and Fellows of Harvard College**
  **Cambridge, MA 02138 (US)**
- **Biomol International L.P.**
  **Plymouth Meeting, PA 19462-1202 (US)**

(72) Inventors:
- **Sinclair, David**
  **Chestnut Hill, MA 02467 (US)**
- **Tsai, Li-huei**
  **Cambridge, Massachusetts 02138 (US)**
- **Nguyen, Minh**
  **Calgary, Alberta T2P 5P6 (US)**
- **Howitz, Konrad**
  **Allentown, Pennsylvania 18104 (US)**
- **Zipkin, Robert**
  **Wynnewood, Pennsylvania 19096 (US)**
- **Bitterman, Kevin J.**
  **Boston, MA 02118 (US)**

(74) Representative: **Lee, Nicholas John et al**
**Kilburn & Strode LLP**
**20 Red Lion Street**
**London WC1R 4PJ (GB)**

(56) References cited:
**WO-A-2005/002555 WO-A2-2006/068656**

- **PORCU M ET AL: "The emerging therapeutic potential of sirtuin-interacting drugs: from cell death to lifespan extension", TRENDS IN PHARMACOLOGICAL SCIENCES, ELSEVIER, HAYWARTH, GB, vol. 26, no. 2, 1 February 2005 (2005-02-01), pages 94-103, XP004727629, ISSN: 0165-6147, DOI: DOI:10.1016/J.TIPS.2004.12.009**
- **ARAKI T ET AL: "Increased Nuclear NAD Biosynthesis and SIRT1 Activation Prevent Axonal Degeneration", SCIENCE, AMERICAN ASSOCIATION FOR THE ADVANCEMENT OF SCIENCE, WASHINGTON, DC; US, vol. 305, 13 August 2004 (2004-08-13), pages 1010-1013, XP003001697, ISSN: 0036-8075, DOI: DOI:10.1126/SCIENCE.1098014**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Background

There is now good evidence from model organisms that the pace of aging can be regulated[1]. Longevity regulatory genes have been identified in many eukaryotes, including rodents, flies, nematode worms and even single-celled organisms such as baker's yeast (reviewed in[2,3]). These genes appear to be part of an evolutionarily conserved longevity pathway that evolved to promote survival in response to deteriorating environmental conditions[1,4]. The yeast *S. cerevisiae* has proven a particularly useful model in which to study cell autonomous pathways of longevity regulation[2]. In this organism, replicative lifespan is defined as the number of daughter cells an individual mother cell produces before dying. Yeast lifespan extension is governed by *PNC1,* a calorie restriction (CR)-and stress-responsive gene that depletes nicotinamide, a potent inhibitor of the longevity protein Sir2. Both *PNC1* and *SIR2* are required for lifespan extension by CR or mild stress[5,6] and additional copies of these genes extend lifespan 30-70% [5-7]. Based on these results we proposed that CR may confer health benefits in a variety of species because it is a mild stress that induces a sirtuin-mediated organismal defense response[6].

Sir2, a histone deacetylase (HDAC), is the founding member of the sirtuin deacetylase family, which is characterized by a requirement for $NAD^+$ as a co-substrate[8-13]. *SIR2* was originally identified as a gene required for the formation of transcriptionally silent heterochromatin at yeast mating-type loci[14]. Subsequent studies have shown that Sir2 suppresses recombination between repetitive DNA sequences at ribosomal RNA genes (rDNA)[15-17]. Sir2 has also been implicated in the partitioning of carbonylated proteins to yeast mother cells during budding[18]. Studies in *C. elegans,* mammalian cells, and the single-celled parasite *Leishmania,* indicate that the survival and longevity functions of sirtuins are conserved[19-22]. In *C. elegans* additional copies of sir-2.1 extend lifespan by 50% via the insulin/IGF-1 signalling pathway, the same pathway recently shown to regulate lifespan in rodents[23-25].

### Summary

Thus, there is provided a method for determining whether a subject has a neurodegenerative disease comprising:

i) obtaining a biological sample from a subject; and
ii) determining the level or activity of a sirtuin protein in the biological sample, wherein a higher level or activity of the sirtuin protein in the biological sample of the subject relative to a control level indicates that the subject has a neurodegenerative disease, wherein the sirtuin protein is SIRT1.

### Brief description of the drawings

Figure 1 shows the effects of resveratrol on the kinetics of recombinant human SIRT1. a, Resveratrol dose-response of SIRT1 catalytic rate at 25 $\mu$M $NAD^+$, 25 $\mu$M p53-382 acetylated peptide. Relative initial rates are the mean of two determinations, each derived from the slopes of fluorescence (arbitrary fluorescence units, AFU) vs. time plots with data obtained at 0, 5, 10 and 20 min. of deacetylation. b, SIRT1 initial rate at 3 mM $NAD^+$, as a function of p 53-382 acetylated peptide concentration in the presence ($\Delta$) or absence (v) of 100 $\mu$M resveratrol. Lines represent non-linear least-squares fits to the Michaelis-Menten equation. Kinetic constants: $K_m$(control, v)= 64 $\mu$M, $K_m$ (+resveratrol, $\Delta$)=1.8 $\mu$M; $V_{max}$(control, v)=1107 AFU/min., $V_{max}$(+resveratrol, $\Delta$)=926 AFU/min. c, SIRT1 initial rate at 1 mM p53-382 acetylated peptide, as a function of $NAD^+$ concentration, in the presence $\Delta$) or absence (v) of 100 $\mu$M resveratrol. Lines represent non-linear least-squares fits to the Michaelis-Menten equation. Kinetic constants: $K_m$(control, v)= 558 $\mu$M, $K_m$(+resveratrol, $\Delta$)=101 $\mu$M; $V_{max}$(control, v)=1863 AFU/min., $V_{max}$(+resveratrol, $\Delta$)=1749 AFU/min. d, Effects of resveratrol on nicotinamide inhibition of SIRT1. Kinetic constants are shown relative to those of the control (no nicotinamide, no resveratrol) and represent the mean of two determinations. Error bars are standard errors of the mean. The variable substrate in each experiment (N = $NAD^+$, P = p53 acetylated peptide), the presence/absence of nicotinamide (+/-) and the resveratrol concentration ($\mu$M) are indicated beneath each pair of $K_m$-$V_{max}$ bars.

Figure 2 shows the effects of polyphenols on Sir2 and *S. cerevisiae* lifespan. a, Initial deacetylation rate of recombinant GST-Sir2 as a function of resveratrol concentration. Rates were determined at the indicated resveratrol concentrations, either with 100 $\mu$M 'Fluor de Lys' acetylated lysine substrate (FdL) plus 3 mM $NAD^+$ ($\Delta$) or with 200 $\mu$M p53-382 acetylated peptide substrate plus 200 $\mu$M $NAD^+$ (v). b, Lifespan analyses were determined by micro-manipulating

individual yeast cells as described[37] on complete 2% glucose medium with 10 μM of each compound, unless otherwise stated. Average lifespan for wild type, 22.9 generations, quercetin, 23.4; piceatannol. 24.0. c, Average lifespan for wild type, 22.9 generations; fisetin, 30.0; butein, 35.5; resveratrol, 36.8. d, Average lifespan for wild type untreated, 21.0 generations; growth on resveratrol, 10 μM, 35.7; 100 μM, 29.4; 500 μM, 29.3.

Figure 3 shows that resveratrol extends lifespan by mimicking CR and suppressing rDNA recombination. Yeast lifespans were determined as in Fig. 2. a, Average lifespan for wild type (wt) untreated, 19.0 generations; wild type + resveratrol (wt+R) 37.8; glucose-restricted + resveratrol (CR+R), 39.9. b, Average lifespans for wild type *sir2Δ,* 9.9; *sir2Δ* + resveratrol, 10.0; *pnc1Δ,* 19.2; *pnc1Δ*+ resveratrol, 33.1. c, Resveratrol suppresses the frequency of ribosomal DNA recombination in the presence and absence of nicotinamide (NAM). Frequencies were determined by loss of the *ADE2* marker gene from the rDNA locus (*RDN1*). d, Resveratrol does not suppress rDNA recombination in a *sir2* strain e, Resveratrol and other sirtuin activators do not significantly increase rDNA silencing compared to a 2x*SIR2* strain. Pre-treated cells (*RDN1::URA3*) were harvested and spotted as 10-fold serial dilutions on either SC or SC with 5-fluororotic acid (5-FOA). In this assay, increased rDNA silencing results in increased survival on 5-FOA medium. f, Quantitation of the effect of resveratrol on rDNA silencing by counting numbers of surviving cells on FOA/total plated.

Figure 4 shows that resveratrol and other polyphenols stimulate *SIRT1* activity in human cells. a, Method for assaying intracellular deacetylase activity with a fluorogenic, cell-permeable substrate, FdL ('Fluor de Lys', BIOMOL). FdL (200 μM) is added to growth media and cells incubated for 1-3 hours to allow F dL to enter the cells and the lysine-deacetylated product (deAc-FdL) to accumulate intracellularly. Cells are lysed with detergent in the presence of 1 μM TSA, 1 mM nicotinamide. Addition of the non-cell-permeable Developer (BIOMOL) releases a fluorophor, specifically from deAc-FdL. b, SIRT1 activating polyphenols can stimulate TSA-insensitive, FdL deacetylation by HeLa S3 cells. Cells were grown adherently in DMEM/10% FCS and treated for 1 hour with 200 μM FdL, 1 μM TSA and either vehicle (0.5% final DMSO, Control) or 500 μM of the indicated compound. Intracellular accumulation of deAc-FdL was then determined as described briefly in a. The intracellular deAc-FdL level for each compound (mean of six replicates) are plotted against the ratios to the control rate obtained in the *in vitro* SIRT1 polyphenol screen (see Table 1, Supplementary Tables 1 and 3). c, U2OS osteosarcoma cells grown to ≥90% confluence in DMEM/10% FCS were exposed to 0 or 10 grays of gamma irradiation (IR). Whole cell lysates were prepared 4 hours post-irradiation and were probed by Western blotting with indicated antibodies. d, U2OS cells cultured as above were pre-treated with the indicated amounts of resveratrol or a 0.5% DMSO blank for 4 hours after which cells were exposed to 0 or 50 J/cm$^2$ of UV radiation. Lysates were prepared and analyzed by Western blot as in c. e, Human embryonic kidney cells (HEK 293) expressing wild type SIRT1 or dominant negative SIRT1-H363Y (SIRT1-HY) protein were cultured as above, pre-treated with the indicated amounts of resveratrol or a 0.5% DMSO blank for 4 hours and exposed to 50 J/cm$^2$ of UV radiation as above. Lysates were prepared and analyzed as above.

Figure 5 shows that intracellular deacetylation activity may be measured with a cell-permeable, fluorogenic HDAC and sirtuin substrate. HeLa S3 cells were grown to confluence in DMEM/10% FCS and then incubated with fresh medium containing 200 μM FdL for the indicated times, 37°C. Intracellular and medium levels of deacetylated substrate (deAc-FdL) were determined according to the manufacturer's instructions (HDAC assay kit, BIOMOL). All data points represent the mean of two determinations. a, Concentration ratio of intracellular ($[deAc\text{-}FdL]_i$) to medium ($[deAc\text{-}FdL]_o$) concentrations in the presence (Δ) or a bsence (ν) o f 1 μM trichostatin A (TSA). b, Total accumulation of deacetylated substrate (deAc-FdL) in the presence (Δ) or absence (v) of 1 μM TSA. c, Intracellular accumulation of deacetylated substrate (deAc-FdL) in the presence (Δ) or absence (ν) of 1 μMTSA.

Figure 6 shows that deacetylation site preferences of recombinant SIRT1. Initial rates of deacetylation were determined for a series of fluorogenic acetylated peptide substrates based on short stretches of human histone H3, H4 and p53 sequence (see key to substrate name and single letter peptide sequence below the bar graph). Recombinant human SIRT1 (1 μg, BIOMOL), was incubated 10 min, 37°C, with 25 μM of the indicated fluorogenic acetylated peptide substrate and 500 μM $NAD^+$. Reactions were stopped by the addition of 1 mM nicotinamide and the deacetylation-dependent fluorescent signal was determined.

Figure 7 is a graph representing SIRT2 activity as a function of resveratrol concentration.

Figure 8 shows an alignment of the amino acid sequences of hSIRT2, hSIRT1 and *S. cerevisiae* Sir2.

Figure 9A shows resveratrol and BML-230 dose responses of SIRT1 catalytic rate. Points represent the mean of three determinations and error bars are standard errors of the mean.

Figure 9B shows the ratio of BML-230-activated to resveratrol-activated SIRT1 rates as a function of activator concentration (the ratios were calculated from data of Figure 9A).

Figure 10 shows the effect of polyphenolic STACs on metazoan sirtuins. a, Schematic of Sir2 polypeptides from human, yeast, *C. elegans* and *D. melanogaster* aligned to show conserved regions. Amino acids forming the $NAD^+$-binding pocket (grey) and substrate binding groove (black) are indicated. Percentages refer to the homology to SIRT1. b, Effect of polyphenolic STACs (500 μM) on $NAD^+$-dependent, trichostatin A (TSA)-insensitive deacetylase activity in Drosophila S2 cells. c, Fold stimulation of recombinant SIR-2.1 by STACs (10 μM). d, Fold stimulation of recombinant dSir2 by STACs (10 μM). Values are the mean of at least three determinations (+/- standard error). e, Dose-dependent activation of *C. elegans* SIR-2.1 by resveratrol. Rates were determined using a fluorogenic acetylated lysine substrate (Fluor de Lys). f, Dose-dependent activation of Drosophila dSir2 by resveratrol. g, SIR-2.1 initial rate at 10 μM Fluor de Lys as a function of $NAD^+$ concentration, in the presence or absence of 100 μM resveratrol. AFU, arbitrary fluorescence units.

Figure 11 shows the *C. elegans* survival on resveratrol. a, Survivorship of adult wild-type N2 *C. elegans* treated with 100 μM resveratrol fed with heat-killed OP50 *E. coli.* Mean lifespan relative to control (triangles, n = 47) was increased by 14.5% (Log-Rank test, P <.0001) by 100 μM resveratrol (squares, n = 46). b, Survivorship of *sir-2.1* mutants treated with resveratrol fed with heat-killed OP50. Adult lifespan of *sir-2.1* animals does not differ significantly from N2 controls (Log-Rank, P = .68) and the effect on lifespan of 100 μM resveratrol on *sir-2.1* mutant animals was not statistically significant (5.2% extension, Log-Rank P = .058; n = 60 control, 58 treated). c, Survivorship of wild-type N2 *C. elegans* on 100 μM resveratrol fed with live OP50 (12.6% extension, P<.0001; n = 47 control, 67 treated). d, Survivorship of *sir-2.1* mutants on 100 μM resveratrol fed with live OP50 (3.3% extension, P=0.81; n = 57 control, 51 treated) e, Fecundity of adult hermaphrodites treated with 100 μM resveratrol. Controls: 106 eggs/5 worms/5 hours (s.d. 10.0); resveratrol-treated: 99 eggs/5 worms/5 hours (s.d. 13.0). f, Feeding rates of L4 larval and adult hermaphrodites treated with 100 μM resveratrol. L4 on live OP50: control 310+10.2 pumps/min, resveratrol 315±9.8; Adult on dead OP50: control 228±26.2, resveratrol 2833±31.9; Adult on live OP50: control 383±16.0, resveratrol 383±22.7.

Figure 12 shows wild-type female *D. melanogaster* survival with adults fed resveratrol or fisetin. a, Canton-S on 15% SY media. b, Canton-S on 5% SY media with resveratrol at two concentrations. c, Strain *yw* on 3% CSY media. d, Strain yw on 2% CSY media with resveratol at two concentrations. e, Strain *yw* on 3% CSY media with 100 μM resveratrol or fisetin. f, Strain yw on 2% CSY media with 100 μM resveratrol or fisetin. Life table statistics for this figure, for males and for additional trials are in Table 20. g, Mean daily fecundity per female (s.e.) estimated over 5-day intervals of Canton-S on 15% SY media with 0 or 10 μM resveratrol. h, Proportion (s.e.) of *yw* females feeding on diet with and without resveratrol in crop-filling assay. i, Mean (s.e.) body mass of Canton-S males and females feeding on diet without and with resveratrol (10μM).

Figure 13 shows the survivorship of *D. melanogaster* adults with mutant alleles of *dSir2* when fed resveratrol (100μM). Females (a) and males (b) with loss-of-function genotype $dSir2^{4.5}/dSir2^{5.26}$. Females (c) and males (d) with strong hypomorphic genotype $dSir2^{17}/dSir2^{KG00871}$.

Figure 14 shows the mortality rates of control and resveratrol treated adults. Mortality was estimated as $\ln(-\ln(p_x))$ where $p_x$ is the survival probability at day x to *x*+1. a, *C. elegans* wild-type N2 on heat-killed OP50 *E. coli.* b, *C. elegans* wild-type N2 on live OP50 *E. coli.* In a and b mortality is plotted only at days with observed mortality. c, *D. melanogaster* wildtype females of Trial 1 at effective doses of resveratrol on 15% SY diet. d, *D. melanogaster* wildtype males of Trial 1 at effective doses of resveratrol on 15% SY diet. In c and d mortality is smoothed from 3-day running average of $p_x$.

Figure 15 shows the stimulation of SIRT 1 catalytic rate by 100 μM plant polyphenols (Table 1).

Figure 16 shows the effect of 100 μM stilbenes and chalcones on SIRT 1 catalytic rate (Supplementary Table 1).

Figure 17 shows the effect of 100 μM flavones on SIRT 1 catalytic rate (Supplementary Table 2).

Figure 18 shows the effect of 100 μM flavones on SIRT 1 catalytic rate (Supplementary Table 3).

Figure 19 shows the effect of 100 μM isoflavones, flavanones and anthocyanidins on SIRT 1 catalytic rate (Supplementary Table 4).

Figure 20 shows the effect of 100 μM catechins (Flavan-3-ols) on SIRT 1 catalytic rate (Supplementary Table 5).

Figure 21 shows the effect of 100 μM free radical protective compounds on SIRT 1 catalytic rate (Supplementary Table 6).

Figure 22 shows the effect of 100 μM miscellaneous compounds on SIRT 1 catalytic rate (Supplementary Table 7).

Figure 23 shows the effect of 100 μM of various modulators on SIRT 1 catalytic rate (Supplementary Table 8).

Figure 24 shows the effect of 100 μM of new resveratrol analogs on SIRT 1 catalytic rate (Table 9).

Figure 25 shows the effect of 100 μM of new resveratrol analogs on SIRT 1 catalytic rate (Table 10).

Figure 26 shows the effect of 100 μM of new resveratrol analogs on SIRT 1 catalytic rate (Table 11).

Figure 27 shows the effect of 100 μM of new resveratrol analogs on SIRT 1 catalytic rate (Table 12).

Figure 28 shows the effect of 100 μM of new resveratrol analogs on SIRT 1 catalytic rate (Table 13).

Figure 29 shows synthetic intermediates of resveratrol analog synthesis (Table 14).

Figure 30 shows synthetic intermediates of resveratrol analog synthesis (Table 15).

Figure 31 shows synthetic intermediates of resvezatrol analog synthesis (Table 16).

Figure 32 shows synthetic intermediates of resveratrol analog synthesis (Table 17).

Figure 33 shows synthetic intermediates of resveratrol analog synthesis (Table 18).

Figure 34 shows the effect of resveratrol on *Drosophila melanogaster* (Table 20).

Figures 35A-G shows sirtuin activators and the fold activation of SIRT1 (Table 21).

Figure 36 shows sirtuin inhibitors and the fold inhibition of SIRT1 (Table 22).

Figure 37 shows the upregulation of SIRT1 in mouse models displaying progressive and severe neurodegeneration. (A) is an immunoblot showing the upregulation of SIRT1 in p25 transgenic (Tg) mice during progressive neurodegeneration (after 2-12 weeks of induction). (B) is a graph showing the quantification of levels of SIRT1 in p25 Tg mice. ** P(T<=t) two tails: 0.004. (C) is a graph showing mRNA levels of Sirtuin family members in p25 Tg mice revealed by microarrays. ** P(T<=t) two tails: 0.003. (D) is an immunoblot showing the progressive increase of SIRT1 in mutant SOD1G37R (line 29) peaking at stage of massive neurodegeneration (10-12 month). (E) is a graph showing the quantification of levels of SIRT1 in SOD1G37R mice. ** P(T<=t) two tails: 0.005. *** P(T<=t) two tails: 0.002. (F) in an immunoblot showing the treatment of primary cortical neurons with low concentrations of ionomycin (1 μm) and hydrogen peroxide (25 μm) induces the rapid upregulation of SIRT associated with generation of p25. Time expressed in minutes.

Figure 38 provides a western blot and a graph showing the levels of SIRT1 in forbrains of PDAPP-V717F mice. There is no increase of SIRT1 in brains of PDAPP-V717F mice.

Figure 39 shows primary neurons treated with resveratrol are protected against either p25 or mutant SOD1 toxicity. (A) is a series of immunofluorescence images showing n o toxicity observed in GFP-transfected neurons treated with resveratrol for 4 8 hours (50 to 500 nm). For all experiments (A to E), primary rat neurons were transfected at DIV1 with plasmids and resveratrol was added to the medium at 2-3 hours after transfection. Characterization of neuronal integrity was performed 24 to 48 hours after transfection. Bar: 40 μm. (B) shows representative confocal images of dying and healthy neurons transfected with p25-GFP and treated with DMSO (control) or resveratrol (250 nm) for 24 hours, respectively. Bar: 20 μm. (C) is a graph showing the quantification of surviving p25-GFP expressing neurons after treatment with DMSO (control) or 250 nm of resveratrol for 24 hours expressed in % (49 +/- 2 % vs 72 +/- 8 %; ** P(T<=t) two tails: 0.01). (D) shows representative confocal images of neurons transfected with

SOD1G93A-FLAG and treated with DMSO (Control) or 500 nm of resveratrol for 48 hours. Bar: 25 μm. (E) is a graph showing the quantification of surviving SOD1G93A-FLAG-expressing neurons after treatment with control (DMSO) or resveratrol (500 nm) for 48 hours expressed in %. (28 +/- 3 % vs 41 +/- 3 %; ** P(T<=t) two tails: 0.01).

Figure 40 shows that overexpression of SIRT1 protects against p25 and mutant SOD1 toxicity. (A) is a series of immunofluorescence images of primary neurons transfected with p 25-GFP and the effects of overexpression of SIRT1 or SIRT1 lacking deacetylase activity (H363Y) on p25 GFP toxicity. Arrows point to neurons with ectopic expression of SIRT1. Bar: 20 μm. p25-GFP is shown in green and SIRT1 in red. (B) is a graph showing the quantification of surviving p25-GFP expressing neurons with or without ectopic expression of SIRT1 or H363Y. ** P(T<=t) two tails: 0.001. Non-significant (NS); P(T<=t) two tails: 0.27. (C) is an immunoblot showing unchanged levels of p25-GFP following expression of SIRT1 or H363Y. h: human; m: mouse. Comparison of HEK and CAD cells for SIRT1 expression. (D) is a series of immunofluorescence images showing primary neurons transfected with wild type hSOD1 or mutant hSOD1G93A and the effects of overexpression of SIRT1 or H363Y on SOD1G93A toxicity. Arrows point to SOD1 aggregates as detected with FLAG Ab. WTSOD1 is not toxic. Bar: 25 μm. hSOD1 is in red; Flag is in green; DAPI in blue. (E) is a graph showing the quantification of surviving SOD193A and WT SOD1-expressing neurons with or without ectopic expression of SIRT1 or H363Y. ** P(T<=t) two tails: 0.001. Non-significant (NS); P(T<=t) two tails: 0.33.

Figure 41 is a series of immunofluorescence images showing the effects of SIRT1 overexpression on p25-GFP expressing neurons. Intact neuronal processes and nuclear morphology in primary neurons co-transfected with p25-GFP and SIRT1 (white arrow). No protection is observed in singly p25-transfected neurons (white arrowheads). Bar: 10 μm.

Figure 42 is a series of immunofluorescence images showing the subcellular localization of SIRT1 in CNS neurons. SIRT1 localizes in nucleus and cell bodies of spinal motor neurons in wild-type (WT) and SOD1G93A mice. Bar: 15 μm.

Figure 43 shows the upregulation of SIRT1 in prefontal cortex of post-mortem AD samples. (A) is an immunoblot and a graph showing the levels of SIRT1 in post-mortem prefontal cortex of control (n=9) and AD (n=11) patients scored by graph (average +/standard error). Two sets of samples were processed independently. P(T<=t) two tails: 0.004. (B) is a series of confocal images of prefontal cortex SIRT1-positive neurons from control #1 and AD patients #1 and 2 in 1$^{st}$ set. Bars: 120 μm and 40 μm. (C) shows the absence of correlation between levels of SIRT1-expressing neurons and proximity to β-amyloid plaques (stars). White arrows point to SIRT1-expressing neurons distant from a β-amyloid plaque stained with 4G8 Abs. White arrowheads point to SIRT1-expressing neurons in close proximity of β-amyloid plaques. AD samples 1 and 2 were used. Bar: 20 μm.

Figure 44 shows that resveratrol prevents neurodegeneration in p25 transgenic mice. (A) is a schematic diagram showing the experimental design for intracerebral ventricular (ICV) injection of resveratrol (Resv) or Vehicle (Veh) in p25 transgenic mice (n=5 for vehicle; n=9 for resveratrol). (B) is an immunoblot showing the downgulation of Bax, activated caspase-3 and GFAP, markers of apoptosis and astrogliosis, in the hippocampus of p25 transgenic mice treated with resveratrol (n=3) compared to p25 animals injected with vehicle (n=2) as revealed by western blots. Actin and FAK are used for loading controls. (C) is a pair of immunofluorescence images showing reduction of GFAP-expressing cells in CA1 of p25 transgenic mice treated with resveratrol (n=3) compared to p25 animals injected with vehicle (n=2) as revealed by immunofluorescence staining. (D) is a series of images showing immunofluorescence staining revealed reduced caspase-3 activation and higher number of p25-GFP -expressing cells in CA1 of p25 transgenic mice (n=2) injected with resveratrol versus vehicle (n=2). (E) is a pair of images showing preservation of the integrity of neuronal processes in resveratrol-treated p25 mice (n=4) but not in vehicle-injected p25 mice (n=3) as visualized by confocal microscopy. Bar: 15 μm. (F) is a pair graphs showing two weeks induced p25 mice were injected icv with either resveratrol (n=9) or vehicle (n=5) 2-3x/week for 3 weeks. Subsequently resveratrol and vehicle treated p25 and whild type mice (n=12) were subjected to contextual fear conditiong. Left: Vehicle treated p25 mice displayed reduced freezing behavior during the memory test when compared to wild type littermates ($t_{(1,15)}$ =3.028; P= 0.006). However, when compared to the vehicle group resveratrol-treated p25 mice showed significantly improved freezing behavoir during the memory test ($t_{(1,12)}$ =2.675; P=0.0202) that was indistinguishable from wild type littermates. Right: Resveratrol had no effect on the escape response to the electric foot shock during the training procedure, suggesting preserved a ssociative learning in resveratrol-treated p 25 mice. P<0.05; ES, electric foot shock.

Figure 45 shows the acetylation of p53, a SIRT1 substrate, in p25 transgenic mice reversed by resveratrol. (A) is

an immunoblot showing upregulation of p53 in p25 transgenic mice (n=4) detected by immunoprecipitation followed by western blot. (B) is an immunoblot showing acetylation of p53 at lysine 382 in p25 transgenic mice (n=3) detected by immunoprecipitation followed by western blot. (C) is an immunoblot and an graph showing efficient knock down of p53 by RNAi in cell line transfected with p53. P53 knock down in p25-expressing primary hippocampal neurons rescues p25 neurotoxicity by 25%. **P(T<=t) two tails: 0.001. (D) is an immunoblot showing reduced acetylation of p53 at lysine 382 and downregulation of p53 in p25 transgenic mice (n=3) treated with resveratrol.

Figure 46 shows SIRT1 expression prevents neurodegeneration in p25 transgenic mice. (A) is a series of confocal pictures of right side forebrain of p25 trangenic mouse 807 injected with a control lentivirus. A low number of CA1 hippocampal neurons in p25 transgenic mice caudal to the injection site are GFP-positive. The white arrow indicates the side of injection. Bar: 200 μm. (B) is a series of confocal pictures of left side forebrain of p25 trangenic mice animal 807 injected with a SIRT1 lentivirus. Numerous CA1 hippocampal neurons in p25 transgenic mice caudal to the injection site are GFP-positive. The white arrow indicates the side of injection. (C) and (D) are confocal pictures of CA1 hippocampal GFP-positive neurons in control and SIRT1-injected p25 mouse 806. Bar: 100 μm. (E) and (F) are high magnification confocal pictures of CA1 hippocampal GFP-positive neurons in control and SIRT1-injected p25 mouse 807. Neuronal integrity in SIRT1-injected p25 mice is better preserved than in contralateral control injected side. Bar: 15 μm.(G)-(I) are images of GFP-positive neurons expressing SIRT1 as revealed by co-staining with HA antibody. Bar: 15 μm.

Figure 47 shows the effects of resveratrol on neurodegeneration in hippocampus and CA3 of p25 mice. (A) is a graph showing the quantification of the downgulation of Bax, activated caspase-3 and GFAP, markers of apoptosis and astrogliosis, in the hippocampus of p25 transgenic mice treated with resveratrol (n=3) compared to p25 animals injected with vehicle (n=2). (B) is a pair of images showing that reduced number of activated caspase-3-expressing neurons in the CA3 of p25 mice treated with resveratrol compared to vehicle-injected mice (black arrows). Bar: 10 μm.

## Detailed description

Definitions

As used herein, the following terms and phrases shall have the meanings set forth below. Unless defined otherwise, all technical and scientific terms used herein have the same meaning as commonly understood to one of ordinary skill in the art

The singular forms "a," "an," and "the" include plural reference unless the context clearly dictates otherwise.

"Activating a sirtuin protein" refers to the action of producing an activated sirtuin protein, i.e., a sirtuin protein that is capable of performing at least one of its biological activities to at least some extent, e.g., with an increase of activity of at least about 10%, 50%, 2 fold or more. Biological activities of sirtuin proteins include deacetylation, e.g., of histones and p53; extending lifespan; increasing genomic stability; silencing transcription; and controlling the segregation of oxidized proteins between mother and daughter cells.

An "activating compound" or a "sirtuin activating compound" refers to a compound that activates a sirtuin protein or stimulates or increases at least one of its activities. Activating compounds may have a formula selected from the group of formulas **1-25,30** and **32-65.**

The term "agent" is used herein to denote a chemical compound, a mixture of chemical compounds, a biological macromolecule (such as a nucleic acid, an antibody, a protein or portion thereof, e.g., a peptide), or an extract made from biological materials such as bacteria, plants, fungi, or animal (particularly mammalian) cells or tissues. The activity of such agents may render it suitable as a "therapeutic agent" which is a biologically, physiologically, or pharmacologically active substance (or substances) that acts locally or systemically in a subject.

A "form that is naturally occurring" when referring to a compound means a compound that is in a form, e.g., a composition, in which it can be found naturally. For example, since resveratrol can be found in red wine, it is present in red wine in a form that is naturally occurring. A compound is not in a form that is naturally occurring if, e.g., the compound has been purified and separated from at least some of the other molecules that are found with the compound in nature.

"Inhibiting a sirtuin protein" refers to the action of reducing at I east one of the biological activities of a sirtuin protein to at least some extent, e.g., at least about 10%, 50%, 2 fold or more.

An "inhibitory compound" or "inhibiting compound" or "sirtuin inhibitory compound" refers to a compound that inhibits a sirtuin protein. Inhibitory compounds may have a formula selected from the group of formulas **26-29, 31** and **66-68.**

A "naturally occurring compound" refers to a compound that can be found in nature, i.e., a compound that has not been designed by man. A naturally occurring compound may have been made by man or by nature. For example, resveratrol is a naturally-occurring compound. A "non-naturally occurring compound" is a compound that is not known

to exist in nature or that does not occur in nature.

"Replicative lifespan" of a cell refers to the number of daughter cells produced by an individual "mother cell." " Chronological aging" or "chronological lifespan," on the other hand, refers to the length of time a population of non-dividing cells remains viable when deprived of nutrients. "Increasing the lifespan of a cell" or "extending the lifespan of a cell," as applied to cells or organisms, refers to increasing the number of daughter cells produced by one cell; increasing the ability of cells or organisms to cope with stresses and combat damage, e.g., to DNA, proteins; and/or increasing the ability of cells or organisms to survive and exist in a living state for longer under a particular condition, e.g., stress. Lifespan can be increased by at least about 20%, 30%, 40%, 50%, 60% or between 20% and 70%, 30% and 60%, 40% and 60% or more using methods described herein.

"Sirtuin deacetylase protein family members;" "Sir2 family members;" "Sir2 protein family members;" or "sirtuin proteins" includes yeast Sir2, Sir-2.1, and human SIRT1 and SIRT2 proteins. The nucleotide and amino acid sequences of the human sirtuin, SIRT1 (silent mating type information regulation 2 homolog), are set forth as SEQ ID NOs: 1 and 2, respectively (corresponding to GenBank Accession numbers NM_012238 and NP_036370, respectively). The mouse homolog of SIRT1 is Sirt2$\alpha$.. Human Sirt2 corresponds to Genbank Accession numbers NM_012237 and NP_036369 (for variant 1; SEQ ID NOs: 3 and 4, respectively) and NM_030593 and NP_085096 (for variant 2; SEQ ID NOs: 5 and 6, respectively). Other family members include the four additional yeast Sir2-like genes termed "*HST* genes" (homologues of Sir two) HOST1, HST2, HST3 and HST4, and the five other human homologues hSIRT3 (corresponding to Genbank Accession numbers NM_012239 and NP_036371; SEQ ID NOs: 7 and 8, respectively), hSIRT4 (corresponding to Genbank Accession numbers NM_012240 and NP_036372; SEQ ID NOs: 9 and 10, respectively), hSIRT5 (corresponding to Genbank Accession numbers NM_012241 and NP_036373 for variant 1 (SEQ ID NOs: 11 and 12, respectively) and NM_031244 and NP_112534 for variant 2 (SEQ ID NOs: 13 and 14, respectively)), hSIRT6 (corresponding to Genbank Accession numbers NM_016539 and NP_057623; SEQ ID NOs: 15 and 16, respectively) and hSIRT7 (corresponding to Genbank Accession numbers N_016538 and NP_057622; SEQ ID NOs: 17 and 18, respectively) (Brachmann et al. (1995) Genes Dev. 9:2888 and Frye et al. (1999) BBRC 260:273). Preferred sirtuins are those that share more similarities with SIRT1, i.e., hSIRT1, and/or Sir2 than with SIRT2, such as those members having at least part of the N-terminal sequence present in SIRT1 and absent in SIRT2 such as SIRT3 has.

"Biologically active portion of a sirtuin" refers to a portion of a sirtuin protein having a biological activity, such as the ability to deacetylate. Biologically active portions of sirtuins may comprise the core domain of sirtuins. For example, amino acids 62-293 of SIRT1 having SEQ ID NO: 2, which are encoded by nucleotides 237 to 932 of SEQ ID NO: 1, encompass the $NAD^+$ binding domain and the substrate binding domain. Therefore, this region is sometimes referred to as the core domain. Other biologically active portions of SIRT1, also sometimes referred to as core domains, include about amino acids 261 to 447 of SEQ ID NO: 2, which are encoded by nucleotides 834 to 1394 of SEQ ID NO: 1; about amino acids 242 to 493 of SEQ ID NO: 2, which are encoded by nucleotides 777 to 1532 of SEQ ID NO: 1; or about amino acids 254 to 495 of SEQ ID NO: 2, which are encoded by nucleotides 813 to 1538 of SEQ ID NO: 1.

The terms "comprise" and "comprising" are used in the inclusive, open sense, meaning that additional elements may be included.

A "direct activator" of a sirtuin is a molecule that activates a sirtuin by binding to it. A "direct inhibitor" of a sirtuin is a molecule that inhibits a sirtuin by binding to it

The term "including" is used to mean "including but not limited to". "Including" and "including but not limited to" are used interchangeably.

"Neurodegenerative disorder" or "neurodegenerative disease" or "neuropathology" refers to a wide range of diseases and/or disorders of the central and peripheral nervous system, such as Parkinson's disease, Alzheimer's disease (AD), amyotrophic lateral sclerosis (ALS), denervation atrophy, otosclerosis, stroke, dementia, multiple sclerosis, Huntington's disease, encephalopathy associated with acquired immunodeficiency disease (AIDS), and other diseases associated with neuronal cell toxicity and cell death.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino

acid gap is weighted as if it were a single amino acid or nucleotide mismatch between the two sequences.

Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

The term "cis" is art-recognized and refers to the arrangement of two atoms or groups around a double bond such that the atoms or groups are on the same side of the double bond. Cis configurations are often labeled as (Z) configurations.

The term "trans" is art-recognized and refers to the arrangement of two atoms or groups around a double bond such that the atoms or groups are on the opposite sides of a double bond. Trans configurations are often labeled as (E) configurations.

The term "covalent bond" is art-recognized and refers to a bond between two atoms where electrons are attracted electrostatically to both nuclei of the two atoms, and the net effect of increased electron density between the nuclei counterbalances the internuclear repulsion. The term covalent bond includes coordinate bonds when the bond is with a metal ion.

The term "therapeutic agent" is art-recognized and refers to any chemical moiety that is a biologically, physiologically, or pharmacologically active substance that acts locally or systemically in a subject The term thus means any substance intended for use in the diagnosis, cure, mitigation, treatment or prevention of disease or in the enhancement of desirable physical or mental development and/or conditions in an animal or human.

The term "therapeutic effect" is art-recognized and refers to a local or systemic effect in animals, particularly mammals, and more particularly humans caused by a pharmacologically active substance. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art. For example, certain compositions described herein may be administered in a sufficient amount to produce a at a reasonable benefit/risk ratio applicable to such treatment.

The term "synthetic" is art-recognized and refers to production by *in vitro* chemical or enzymatic synthesis.

The term "meso compound" is art-recognized and refers to a chemical compound which has at least two chiral centers but is achiral due to a plane or point of symmetry.

The term "chiral" is art-recognized and refers to molecules which have the property of non-superimposability of the mirror image partner, while the term "achiral" refers to molecules which are superimposable on their mirror image partner. A "prochiral molecule" is a molecule which has the potential to be converted to a chiral molecule in a particular process.

The term "stereoisomers" is art-recognized and refers to compounds which have identical chemical constitution, but differ with regard to the arrangement of the atoms or groups in space. In particular, "enantiomers" refer to two stereoisomers of a compound which are non-superimposable mirror images of one another. "Diastereomers", on the other hand, refers to stereoisomers with two or more centers of dissymmetry and whose molecules are not mirror images of one another.

Furthermore, a "stereoselective process" is one which produces a particular stereoisomer of a reaction product in preference to other possible stereoisomers of that product. An "enantioselective process" is one which favors production of one of the two possible enantiomers of a reaction product.

The term "regioisomers" is art-recognized and refers to compounds which have the same molecular formula but differ in the connectivity of the atoms. Accordingly, a "regioselective process" is one which favors the production of a particular regioisomer over others, e.g., the reaction produces a statistically significant increase in the yield of a certain regioisomer.

The term "epimers" is art-recognized and refers to molecules with identical chemical constitution and containing more than one stereocenter, but which differ in configuration at only one of these stereocenters.

The term "$ED_{50}$" is art-recognized. In certain embodiments, $ED_{50}$ means the dose of a drug which produces 50% of its maximum response or effect, or alternatively, the dose which produces a pre-determined response in 50% of test subjects or preparations. The term "$LD_{50}$" is art-recognized. In certain embodiments, $LD_{50}$ means the dose of a drug which is lethal in 50% of test subjects. The term "therapeutic index" is an art-recognized term which refers to the therapeutic index of a drug, defined as $LD_{50}/ED_{50}$.

The term "structure-activity relationship" or "(SAR)" is art-recognized and refers to the way in which altering the molecular structure of a drug or other compound alters its biological activity, e.g., its interaction with a receptor, enzyme, nucleic acid or other target and the like.

The term "aliphatic" is art-recognized and refers to a linear, branched, cyclic alkane, alkene, or alkyne. In certain embodiments, aliphatic groups in the present compounds are linear or branched and have from 1 to about 20 carbon atoms.

The term "alkyl" is art-recognized, and includes saturated aliphatic groups, including straight-chain alkyl groups, b ranched-chain alkyl groups, cycloalkyl (alicyclic) groups, alkyl substituted cycloalkyl groups, and cycloalkyl substituted alkyl groups. In certain embodiments, a straight chain or branched chain alkyl has about 30 or fewer carbon atoms in its backbone (e.g., $C_1$-$C_{30}$ for straight chain, $C_3$-$C_{30}$ for branched chain), and alternatively, about 20 or fewer. Likewise, cycloalkyls have from about 3 to about 10 carbon atoms in their ring structure, and alternatively about 5, 6 or 7 carbons in the ring structure. The term "alkyl" is also defined to include halosubstituted alkyls.

The term "aralkyl" is art-recognized and refers to an alkyl group substituted with an aryl group (e.g., an aromatic or heteroaromatic group).

The terms "alkenyl" and "alkynyl" are art-recognized and refer to unsaturated aliphatic groups analogous in length and possible substitution to the alkyls described above, but that contain at least one double or triple bond respectively.

Unless the number of carbons is otherwise specified, "lower alkyl" refers to an alkyl group, as defined above, but having from one to about ten carbons, alternatively from one to about six carbon atoms in its backbone structure. Likewise, "lower alkenyl" and "lower alkynyl" have similar chain lengths.

The term "heteroatom" is art-recognized and refers to an atom of any element other than carbon or hydrogen. Illustrative heteroatoms include boron, nitrogen, oxygen, phosphorus, sulfur and selenium.

The term "aryl" is art-recognized and refers to 5-, 6- and 7-membered single-ring aromatic groups that may include from zero to four heteroatoms, for example, benzene, naphtalene, anthracene, pyrene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics." The aromatic ring may be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, - $CF_3$, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings may be cycloalkyls, cycloalkenyls, cycloalkynyls, aryls and/or heterocyclyls.

The terms ortho, meta and para are art-recognized and refer to 1,2-, 1,3- and 1,4-disubstituted benzenes, respectively. For example, the names 1,2-dimethylbenzene and ortho-dimethylbenzene are synonymous.

The terms "heterocyclyl" or "heterocyclic group" are art-recognized and refer to 3-to about 10-membered ring structures, alternatively 3- to about 7-membered rings, whose ring structures include one to four heteroatoms. Heterocycles may also be polycycles. Heterocyclyl groups include, for example, thiophene, thianthrene, furan, pyran, isobenzofuran, chromene, xanthene, phenoxanthene, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring may be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carbonyl, silyl, ether, alkylthio, sulfonyl, ketone, aldehyde, ester, a heterocyclyl, an aromatic or heteroaromatic moiety, -$CF_3$, -CN, or the like.

The terms "polycyclyl" or "polycyclic group" are art-recognized and refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle may be substituted with such substituents as described above, as for example, halogen, alkyl, aralkyl, alkenyl, alkenyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -$CF_3$, -CN, or the like.

The term "carbocycle" is art-recognized and refers to an aromatic or non-aromatic ring in which each atom of the ring is carbon.

The term "nitro" is art-recognized and refers to -$NO_2$; the term "halogen" is art-recognized and refers to -F, -Cl, -Br or -I; the term "sulfhydryl" is art-recognized and refers to -SH; the term "hydroxyl" means -OH; and the term "sulfonyl"

is art-recognized and refers to $-SO_2^-$. "Halide" designates the corresponding anion of the halogens, and "pseudohalide" has the definition set forth on 560 of "Advanced Inorganic Chemistry" by Cotton and Wilkinson.

The terms "amine" and "amino" are art-recognized and refer to both unsubstituted and substituted amines, e.g., a moiety that may be represented by the general formulas:

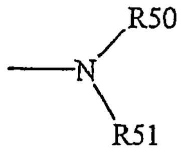 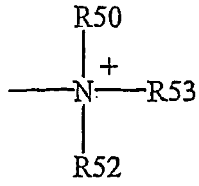

wherein R50, R51 and R52 each independently represent a hydrogen, an alkyl, an alkenyl, - $(CH_2)_m$-R61, or R50 and R51, taken together with the N atom to which they are attached complete a heterocycle having from 4 to 8 atoms in the ring structure; R61 represents an aryl, a cycloalkyl, a cycloalkenyl, a heterocycle or a polycycle; and m is zero or an integer in the range of 1 to 8. In certain embodiments, only one of R50 or R51 may be a carbonyl, e.g., R50, R51 and the nitrogen together do not form an imide. In other embodiments, R50 and R51 (and optionally R52) each independently represent a hydrogen, an alkyl, an alkenyl, or $-(CH_2)_m$-R61. Thus, the term "alkylamine" includes an amine group, as defined above, having a substituted or unsubstituted alkyl attached thereto, i.e., at least one of R50 and R51 is an alkyl group.

The term "acylamino" is art-recognized and refers to a moiety that may be represented by the general formula:

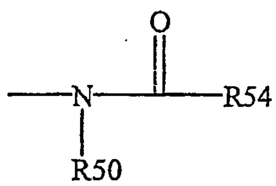

wherein R50 is as defined above, and R54 represents a hydrogen, an alkyl, an alkenyl or - $(CH_2)_m$-R61, where m and R61 are as defined above.

The term "amido" is art recognized as an amino-substituted carbonyl and includes a moiety that may be represented by the general formula:

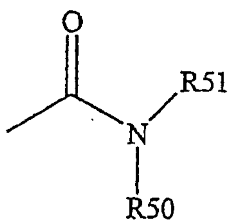

wherein R50 and R51 are as defined above. Certain embodiments of amides may not include imides which may be unstable.

The term "alkylthio" refers to an alkyl group, as defined above, having a sulfur radical attached thereto. In certain embodiments, the "alkylthio" moiety is represented by one of -S-alkyl, -S-alkenyl, -S-alkynyl, and $-S-(CH_2)_m$-R61, wherein m and R61 are defined above. Representative alkylthio groups include methylthio, ethyl thio, and the like.

The term "carbonyl" is art recognized and includes such moieties as may be represented by the general formulas:

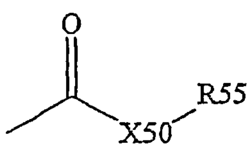 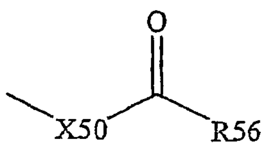

wherein X50 is a bond or represents an oxygen or a sulfur, and R55 and R56 represents a hydrogen, an alkyl, an alkenyl, $-(CH_2)_m$-R61 or a pharmaceutically acceptable salt, R56 represents a hydrogen, an alkyl, an alkenyl or $-(CH_2)_m$-R61,

where m and R61 are defined above. Where X50 is an oxygen and R55 or R56 is not hydrogen, the formula represents an "ester". Where X50 is an oxygen, and R55 is as defined above, the moiety is referred to herein as a carboxyl group, and particularly when R55 is a hydrogen, the formula represents a "carboxylic acid". Where X50 is an oxygen, and R56 is hydrogen, the formula represents a "formate". In general, where the oxygen atom of the above formula is replaced by sulfur, the formula represents a "thiolcarbonyl" group. Where X50 is a sulfur and R55 or R56 is not hydrogen, the formula represents a "thiolester." Where X50 is a sulfur and R55 is hydrogen, the formula represents a "thiolcarboxylic acid." Where X50 is a sulfur and R56 is hydrogen, the formula represents a "thiolformate." On the other hand, where X50 is a bond, and R55 is not hydrogen, the above formula represents a "ketone" group. Where X50 is a bond, and R55 is hydrogen, the above formula represents an "aldehyde" group.

The terms "alkoxyl" or "alkoxy" are art-recognized and refer to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as may be represented by one of -O-alkyl, -O-alkenyl, -O-alkynyl, -O--$(CH_2)_m$-R61, where m and R61 are described above.

The term "sulfonate" is art recognized and refers to a moiety that may be represented by the general formula:

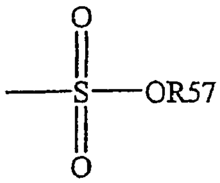

in which R57 is an electron pair, hydrogen, alkyl, cycloalkyl, or aryl.

The term "sulfate" is art recognized and includes a moiety that may be represented by the general formula:

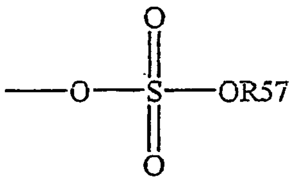

in which R57 is as defined above.

The term "sulfonamido" is art recognized and includes a moiety that may be represented by the general formula:

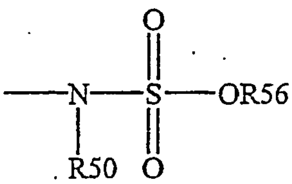

in which R50 and R56 are as defined above.

The term "sulfamoyl" is art-recognized and refers to a moiety that may be represented by the general formula:

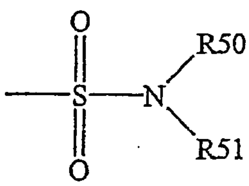

in which R50 and R51 are as defined above.

The term "sulfonyl" is art-recognized and refers to a moiety that may be represented by the general formula:

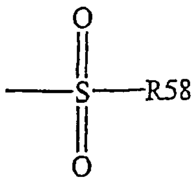

in which R58 is one of the following: hydrogen, alkyl, alkenyl, alkynyl, cycloalkyl, heterocyclyl, aryl or heteroaryl.

The term "sulfoxido" is art-recognized and refers to a moiety that may be represented by the general formula:

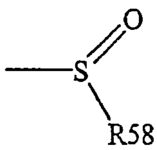

in which R58 is defined above.

The term "phosphoryl" is art-recognized and may in general be represented by the formula:

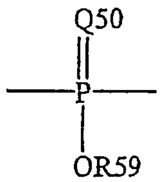

wherein Q 50 represents S or O, and R 59 represents hydrogen, a lower alkyl or an aryl. When used to substitute, e.g., an alkyl, the phosphoryl group of the phosphorylalkyl may be represented by the general formulas:

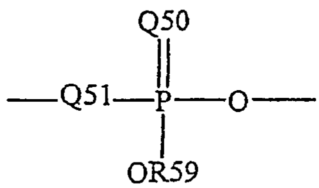 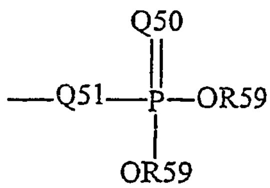

wherein Q50 and R59, each independently, are defined above, and Q51 represents O, S or N. When Q50 is S, the phosphoryl moiety is a "phosphorothioate".

The term "phosphoramidite" is art-recognized and may be represented in the general formulas:

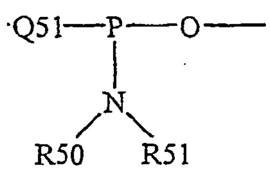 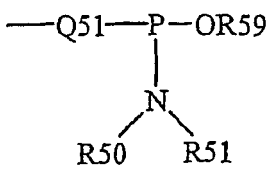

wherein Q51, R50, R51 and R59 are as defined above.

The term "phosphonamidite" is art-recognized and may be represented in the general formulas:

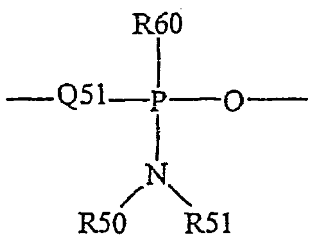 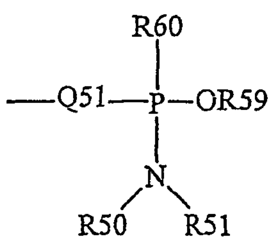

wherein Q51, R50, R51 and R59 are as defined above, and R60 represents a lower alkyl or an aryl.

Analogous substitutions may be made to alkenyl and alkynyl groups to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

The definition of each expression, e.g. alkyl, m, n, and the like, when it occurs more than once in any structure, is intended to be independent of its definition elsewhere in the same structure.

The term "selenoalkyl" is art-recognized and refers to an alkyl group having a substituted seleno group attached thereto. Exemplary "selenoethers" which may be substituted on the alkyl are selected from one of -Se-alkyl, -Se-alkenyl, -Se-alkynyl, and - Se-$(CH_2)_m$-R61, m and R61 being defined above.

The terms triflyl, tosyl, mesyl, and nonaflyl are art-recognized and refer to trifluoromethanesulfonyl, *p*-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, *p*-toluenesulfonate ester, m ethanesulfonate ester, and nonafluorobutanesulfonate ester functional groups and molecules that contain said groups, respectively.

The abbreviations Me, Et, Ph, Tf, Nf, Ts, and Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by organic chemists of ordinary skill in the art appears in the first issue of each volume of the Journal of Organic Chemistry; this list is typically presented in a table entitled Standard List of Abbreviations.

Certain compounds contained in compositions described herein may exist in particular geometric or stereoisomeric forms. In addition, compounds may also be optically active. Contemplated herein are all such compounds, including cis- and trans-isomers, Rand *S*-enantiomers, diastereomers, (D)-isomers, (L)-isomers, the racemic mixtures thereof, and other mixtures thereof. Additional asymmetric carbon atoms may be present in a substituent such as an alkyl, group. All such isomers, as well as mixtures thereof, are encompassed herein.

If, for instance, a particular enantiomer of a compound is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carbonyl, diastereomeric salts are formed with an appropriate optically-active acid or base, followed by resolution of the diastereomers thus formed by fractional crystallization or chromatographic means well known in the art, and subsequent recovery of the pure enantiomers.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. Heteroatoms such as nitrogen may have hydrogen substituents and/or any permissible substituents of organic compounds described herein which satisfy the valences of the heteroatoms. Compounds are not intended to be limited in any manner by the permissible substituents of organic compounds.

The chemical elements are identified in accordance with the Periodic Table of the Elements, C AS version, Handbook of Chemistry and Physics, 67th Ed., 1986-87, inside cover.

The term "protecting group" is art-recognized and refers to temporary substituents that protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed by Greene and Wuts in Protective Groups in Organic Synthesis (2nd ed., Wiley: New York, 1991).

The term "hydroxyl-protecting group" is art-recognized and refers to those groups intended to protect a hydroxyl group against undesirable reactions during synthetic procedures and includes, for example, benzyl or other suitable esters or ethers groups known in the art.

The term "carboxyl-protecting group" is art-recognized and refers to those groups intended to protect a carboxylic acid group, such as the C-terminus of an amino acid or peptide or an acidic or hydroxyl azepine ring substituent, against undesirable reactions during synthetic procedures and includes. Examples, for protecting groups for carboxyl groups involve, for example, benzyl ester, cyclohexyl ester, 4-nitrobenzyl ester, t-butyl ester, 4-pyridylmethyl ester, and the like.

The term "amino-blocking group" is art-recognized and refers to a group which will prevent an amino group from participating in a reaction carried out on some other functional group, but which can be removed from the amine when desired. Such groups are discussed by in Ch. 7 of Greene and Wuts, cited above, and by Barton, Protective Groups in Organic Chemistry c h. 2 (McOmie, ed., Plenum Press, New York, 1973). Examples of suitable groups include acyl protecting groups such as, to illustrate, formyl, dansyl, acetyl, benzoyl, trifluoroacetyl, succinyl, methoxysuccinyl,

benzyl and substituted benzyl such as 3,4-dimethoxybenzyl, o-nitrobenzyl, and triphenylmethyl; those of the formula -COOR where R includes such groups as methyl, ethyl, propyl, isopropyl, 2,2,2-trichloroethyl, 1-methyl-1-phenylethyl, isobutyl, t-butyl, t-amyl, vinyl, allyl, phenyl, benzyl, p-nitrobenzyl, o-nitrobenzyl, and 2,4-dichlorobenzyl; acyl groups and substituted acyl such as formyl, acetyl, chloroacetyl, dichloroacetyl, trichloroacetyl, trifluoroacetyl, benzoyl, and p-methoxybenzoyl; and other groups such as methanesulfonyl, p-toluenesulfonyl, p-bromobenzenesulfonyl, p-nitrophenylethyl, and p-toluenesulfonyl-aminocarbonyl. Preferred amino-blocking groups are benzyl ($-CH_2C_6H_5$), acyl [C(O)R1] or $SiR1_3$ where R1 is $C_1$-$C_4$ alkyl, halomethyl, or 2-halo-substituted-($C_2$-$C_4$ alkoxy), aromatic urethane protecting groups as, for example, carbonylbenzyloxy (Cbz); and aliphatic urethane protecting groups such as t-butyloxycarbonyl (Boc) or 9-fluorenylmethoxycarbonyl (FMOC).

The definition of each expression, e.g. lower alkyl, m, n, p and the like, when it occurs more than once in any structure, is intended to be independent of its definition elsewhere in the same structure.

The term "electron-withdrawing group" is art-recognized, and refers to the tendency of a substituent to attract valence electrons from neighboring atoms, i.e., the substituent is electronegative with respect to neighboring atoms. A quantification of the level of electron-withdrawing capability is given by the Hammett sigma ($\sigma$) constant. This well known constant is described in many references, for instance, March, Advanced Organic Chemistry 251-59 (McGraw Hill Book Company: New York, 1977). The Hammett constant values are generally negative for electron donating groups ($\sigma(P)$ = - 0.66 for $NH_2$) and positive for electron withdrawing groups ($\sigma(P)$ = 0.78 for a nitro group), $\sigma(P)$ indicating para substitution. Exemplary electron-withdrawing groups include nitro, acyl, formyl, sulfonyl, trifluoromethyl, cyano, chloride, and the like. Exemplary electron-donating groups include amino, methoxy, and the like.

The term "small molecule" is art-recognized and refers to a composition which has a molecular weight of less than about 2000 amu, or less than about 1000 amu, and even less than about 500 amu. Small molecules may be, for example, nucleic acids, peptides, polypeptides, peptide nucleic acids, peptidomimetics, carbohydrates, lipids or other organic (carbon containing) or inorganic molecules. Many pharmaceutical companies have extensive libraries of chemical and/or biological mixtures, often fungal, bacterial, or algal extracts, which can be screened with any of the assays described herein. The term "small organic molecule" refers to a small molecule that is often identified as being an organic or medicinal compound, and does not include molecules that are exclusively nucleic acids, peptides or polypeptides.

The term "modulation" is art-recognized and refers to up regulation (i.e., activation or stimulation), down regulation (i.e., inhibition or suppression) of a response, or the two in combination or apart.

The term "treating" is art-recognized and refers to curing as well as ameliorating at least one symptom of any condition or disease or preventing a condition or disease from worsening.

The term "prophylactic" or "therapeutic" treatment is art-recognized and refers to administration of a drug to a host. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

A "patient," "subject" or "host" to be treated by the subject method may mean either a human or non-human animal.

The term "mammal" is known in the art, and exemplary mammals include humans, primates, bovines, porcines, canines, felines, and rodents (e.g., mice and rats).

The term "bioavailable" when referring to a compound is art-recognized and refers to a form of a compound that allows for it, or a portion of the amount of compound administered, to be absorbed by, incorporated to, or otherwise physiologically available to a subject or patient to whom it is administered.

The term "pharmaceutically-acceptable salts" is art-recognized and refers to the relatively non-toxic, inorganic and organic acid addition salts of compounds, including, for example, those contained in compositions described herein.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such asp eanut oil, cottonseed oil, safflower oil, sesame oil, olive oil, c om oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The terms "systemic administration," "administered systemically," "peripheral administration" and "administered peripherally" are art-recognized and refer to the administration of a subject composition, therapeutic or other material other than directly into the central nervous system, such that it enters the patient's system and, thus, is subject to metabolism and other like processes.

The terms "parenteral administration" and "administered parenterally" are art-recognized and refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articulare, subcapsular, subarachnoid, intraspinal, and intrasternal injection and infusion.

Exemplary methods and compositions

Provided herein are diagnostic methods, e.g., methods for determining whether a subject has neuronal cell death or a neurodegenerative disorder. The method comprises determining the level or activity of the SIRT1 protein. The diagnostic method comprises (i) obtaining a biological sample of a subject and (ii) determining the level or activity of a sirtuin protein in the biological sample, wherein a higher level or activity of the sirtuin in the biological sample of the subject relative to a control indicates that the subject has or is likely to develop a neurodegenerative disease. The biological sample may be a cell sample, e.g., a blood sample, a spinal cord sample or a brain sample. Methods for determining the level of a sirtuin protein are known in the art and may involve using an antibody binding to the sirtuin protein. Methods for determining the activity of a sirtuin protein are also known in the art, and may involve determining its deacetylation efficiency. A control in the diagnostic assay may be a value corresponding to the level or activity of a sirtuin protein in an individual who does not have or is not likely to develop a neurodegenerative disease. A control may also be a value corresponding to the average of the level or activity of a sirtuin protein in two or more individuals who do not have or are not likely to develop a neurodegenerative disease. A control value may be an average of at least 5, 10, 50 or 100 individuals. A higher level or activity of a sirtuin in a biological sample of a subject relative to a control may include levels that are at least about 50%, 2, 3, 5, 10, 30, 50 or more fold higher in the subject than in the control.

"A method for diagnosing" includes any immunoassay, such as assays which utilize biotin and avidin or streptavidin, ELISAs, RIAs, Western blots, and immunoprecipitation. Diagnostic methods may use an antibody that specifically binds to a sirtuin. Other diagnostic assays comprise the use of nucleic acids, e.g., for determining the level of RNA, such as mRNA, or for determining the presence of a mutation in a sirtuin gene. The agent that is used in a diagnostic assay, e.g., an antibody or a nucleic acid, may be labeled and/or linked, covalently or not, to a solid surface.

A diagnostic method may comprise determining the level or activity of a sirtuin once or several times, e.g., several times within a determined period of time. For example, a diagnostic method may comprise obtaining a first biological sample of a subject, and obtaining a second biological sample several hours or days (e.g., 1, 2, 3, or 7 days) or weeks (e.g., 1, 2, 3 or 4 weeks) or months (e.g., 1, 2, 3, 6, 10 or more months) or years later. A change in the level of protein or activity of a sirtuin within the two samples may be indicative that a disease, e.g., a neurodegenerative disease, is evolving in the subject. An increase in the level of protein or activity of a sirtuin with time in a subject may indicate that the subject is developing a disease. A decrease in the level of protein or activity of a sirtuin with time in a subject may indicate that the disease or at least one or more symptoms thereof is being treated or prevented effectively.

A diagnosis of a disease may also comprise monitoring another characteristic of the disease, e.g., the presence or absence or level of a marker of the disease. For example, the diagnosis of Alzheimer's disease as described herein may be combined with the detection of β-amyloid plaques.

The practice of the present methods will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

**Examples**

**Example 1: Small molecule activators of SIRT1**

To identify compounds that modulate SIRT1 activity, we screened a number of small molecule libraries using a fluorescent deacetylation assay in 96-well plates[26]. The substrate used in the assay was a fluorogenic peptide based on the sequence encompassing the p53-K382 acetylation site, a known target of SIRT1 *in vivo*[20,21,27]. This substrate was preferred over a variety of other fluorogenic peptide substrates that were based on other known HDAC targets (Fig. 5). The small molecule libraries included analogues of nicotinamide, ε-acetyl lysine, $NAD^+$, nucleotides, nucleotide analogues and purinergic ligands. From the initial screen, several sirtuin inhibitors were found (Supplementary Table 7). However, the most striking outcome was the identification of two compounds, quercetin and piceatannol, that stimulated SIRT1 activity five and eight-fold, respectively (Table 1). Both quercetin and piceatannol have been previously identified as protein kinase inhibitors[28,29].

Comparison of the structures of the two activating compounds suggested a possible structure-activity relationship. Piceatannol comprises two phenyl groups trans to one another across a linking ethylene moiety. The trans-stilbene ring structures of piceatannol are superimposable on the flavonoid A and B rings of quercetin, with the ether oxygen and carbon-2 of the C ring aligning with the ethylene carbons in piceatannol (see structures, Table 1). Further, the 5, 7, 3' and 4' hydroxyl group positions in quercetin can be aligned, respectively, with the 3, 5, 3' and 4' hydroxyls of piceatannol.

Given the demonstrated longevity-enhancing effects of sirtuin activity in *S. cerevisiae*[7] and *C. elegans*[19], it was naturally of interest to further explore the structure-activity relationship among compounds that stimulate SIRT1. Both quercetin and piceatannol are polyphenols, members of a large and diverse group of plant secondary metabolites that includes flavones, stilbenes, flavanones, isoflavones, catechins (flavan-3-ols), chalcones, tannins and anthocyanidins[30,31]. Polyphenols noteworthy with respect to potential longevity-enhancing effects include resveratrol, a stilbene found in red wine and epigallocatechin gallate (EGCG) from green tea. Both have been suggested on the basis of epidemiological and mechanistic investigations to exert cancer chemopreventive and cardioprotective effects[30-32]. We therefore performed a secondary screen encompassing resveratrol, EGCG and additional representatives from a number of the polyphenol classes listed above. The screen emphasized flavones due to the great number of hydroxyl position variants available in this group[31]. The results of this screen are summarized in Supplementary Tables 1 - 6. In the tables, a "ratio to control rate" above 1 indicates that a compound with such a rate is an activator of the sirtuin tested and a number under 1 indicates that a compound is an inhibitor.

Additional potent SIRT1 activators were found among the stilbenes, chalcones and flavones (Table 1, Supplementary Tables 1 and 2). The six most active flavones had 3' and 4' hydroxyls (Supplementary Table 2), although it should be noted that the most active compound overall, resveratrol (3,5,4'-trihydroxystilbene), was more active than piceatannol, which differs only by its additional 3'-hydroxyl (Table 1). The importance of the 4'-hydroxyl to activity is underscored by the fact that each of the 12 most stimulatory flavones share this feature (Supplementary Tables 1 and 2).

Many, but not all of the most active compounds include hydroxyls in the two meta positions (e.g. 5,7-dihydroxylated flavones) of the ring (A ring), trans to that with the 4' or 3',4' pattern (B ring, see Table 1, Supplementary Tables 1 and 2). A potentially coplanar orientation of the trans phenyl rings may be important for activity since catechins and flavanones, which lack the 2,3 double-bond, have weak activity despite having equivalent hydroxylation patterns to various stimulatory flavones (compare Supplementary Tables 2 and 3 with 4 and 5). The absence of activity in the isoflavone genistein, although hydroxylated in an equivalent way to the stimulatory compounds apigenin and resveratrol (see Supplementary Tables 1, 2 and 4), is consistent with the idea that the trans positioning and spacing of the hydroxylated rings contributes strongly to activity.

The biological effects of polyphenols are frequently attributed to antioxidant, metal ion chelating and/or free-radical scavenging activity[30,32]. We considered the possibility that the apparent polyphenol stimulation of SIRT1 might simply represent the repair of oxidative and/or metal-ion induced damage incurred during preparation of the recombinant protein. Two features of our results argue against this being the case. First, a variety of free-radical protective compounds, including antioxidants, chelators and radical scavengers, failed to stimulate SIRT1 (see Supplementary Table 6.). Second, among various polyphenols of equivalent antioxidant capacity we observed diverse SIRT1 stimulating activity (e.g. compare resveratrol, quercetin and the epicatechins in Supplementary Tables 1, 2 and 5 and see [33]).

**Example 2: Resveratrol's effects on SIRT1 kinetics**

Detailed enzyme kinetic investigations were performed using the most potent activator, resveratrol. Dose-response experiments performed under the conditions of the polyphenol screening assays (25 μM $NAD^+$, 25 μM p53-382 acetylated peptide), showed that the activating effect doubled the rate at ~11 μM and was essentially saturated at 100 μM resveratrol (Fig. 1a). Initial enzyme rates, in the presence or absence of 100 μM resveratrol, were determined either as a function of acetyl-peptide concentration with high $NAD^+$ (3 mM $NAD^+$, Fig. 1b) or as a function of $NAD^+$ concentration

with high acetyl-peptide (1 mM p53-382 acetylated peptide, Fig. 1c). Although resveratrol had no significant effect on the two $V_{max}$ determinations (Figs. 1b, 1c), it had pronounced effects on the two apparent $K_m$s. Its effect on the acetylated peptide $K_m$ was particularly striking, amounting to a 35-fold decrease (Fig. 1b). Resveratrol also lowered the $K_m$ for $NAD^+$ over 5-fold (Fig. 1c). Since resveratrol acts only on $K_m$, it could be classified as an allosteric effector of 'K system' type[34]. This can imply that only the substrate binding affinity of the enzyme has been altered, rather than a rate-limiting catalytic step.

Our previous kinetic analysis of SIRT1 and Sir2[26] and our genetic analysis of Sir2's role in yeast lifespan extension[6,35] have implicated nicotinamide (a product of the sirtuin reaction) as a physiologically important inhibitor of sirtuin activity. Therefore the effects of resveratrol on nicotinamide inhibition were tested. In experiments similar to those of Figs. 1b and 1c, kinetic constants in the presence of 50 μM nicotinamide were determined either by varying the concentration of $NAD^+$ or that of the p53-382 acetylated peptide (Fig. 1d). Nicotinamide, in contrast to resveratrol, affects the SIRT1 $V_{max}$ (note 30% and 36% $V_{max}$ decreases in absence of resveratrol, Fig. 1d and see ref.[26]). In the presence of 50 μM nicotinamide, resveratrol appears to have complex, concentration-dependent effects on the kinetics of SIRT1 (Fig. 1d). Apparent $K_m$ for $NAD^+$ and acetylated substrate appear to actually be raised by 5 μM resveratrol when nicotinamide is present At 20 and 100 μM, in the presence of 50 μM nicotinamide, resveratrol lowers the $K_m$ for both $NAD^+$ and acetylated peptide, without reversing the nicotinamide-induced $V_{max}$ decrease. It has been proposed that sirtuins may bind nicotinamide at a second site, known as "the C pocket", distinct from the "B" site that interacts with the nicotinamide moiety of $NAD^+$[26]. In light of this potential complexity, further kinetic studies, supplemented by structural/crystallographic information, will likely be necessary to fully elucidate the interplay between the effects of nicotinamide and polyphenols.

**Example 3: Activating compounds extend yeast lifespan**

To investigate whether these compounds could stimulate sirtuins *in vivo,* we utilized *S. cerevisiae,* an organism in which the upstream regulators and downstream targets of Sir2 are relatively well understood. A resveratrol dose-response study of Sir2 deacetylation rates (Fig. 2a) indeed reveals that resveratrol stimulates Sir2 *in vitro,* with the optimum concentration of activator being 2- 5 μM. Levels of activation were somewhat lower than those for SIRT1, and unlike SIRT1, inhibition was seen at concentrations greater than ~100 μM.

Resveratrol and four other potent sirtuin activators, representatives of the stilbene, flavone, and chalcone families, were tested for their effect on yeast lifespan. Due to the potential impediment by the yeast cell wall or plasma membrane and suspected slow oxidation of the compound in the medium, we chose to use a concentration (10 μM) slightly higher than the optimal resveratrol concentration *in vitro.* As shown in Fig. 2b, quercetin and piceatannol had no significant effect on lifespan. In contrast, butein, fisetin and resveratrol increased average lifespan by 31, 55 and 70%, respectively, and all three significantly increased maximum lifespan (Fig. 2c). Concentrations of resveratrol higher than 10 μM provided no added lifespan benefit and there was no lasting effect of the compound on the lifespan of pre-treated young cells (Fig. 2d and data not shown).

For subsequent yeast genetic experiments we focused on resveratrol because it was the most potent SIRT1 activator and provided the greatest lifespan extension. Glucose restriction, a form of CR in yeast, resulted in no significant extension of the long-lived resveratrol-treated cells (Fig. 3a), indicating that resveratrol likely acts via the same pathway as CR. Consistent with this, resveratrol had no effect on the lifespan of a *sir2* null mutant (Fig. 3b). Given that resveratrol is reported to have fungicidal properties at high concentrations[36], and that mild stress can extend yeast lifespan by activating *PNC1*[6], it was plausible that resveratrol was extending lifespan by inducing *PNC1,* rather than acting on Sir2 directly. However, resveratrol extended the lifespan of a *pnc1* null mutant nearly as well as it did wild type cells (Fig. 3b). Together these data show that resveratrol acts downstream of *PNC1* and requires *SIR2* for its effect. Thus, the simplest explanation for our observations is that resveratrol increases lifespan by directly stimulating Sir2 activity.

A major cause of yeast aging is thought to stem from the inherent instability of the repetitive rDNA locus[2,5,37-39]. Homologous recombination between rDNA repeats can generate an extrachromosomal circular form of rDNA (ERC) that is replicated until it reaches toxic levels in old cells. Sir2 is thought to extend lifespan by suppressing recombination at the replication fork barrier of rDNA[40]. Consistent with the lifespan extension we observed for resveratrol, this compound reduced the frequency of rDNA recombination by ~60% (Fig. 3c), in a *SIR2*-dependent manner (Fig. 3d). In the presence of the Sir2 inhibitor nicotinamide, recombination was also decreased by resveratrol (Fig. 3c), in agreement with the kinetic data (see Fig. 1d). Interestingly, we found that resveratrol and other sirtuin activators had only minor effects on rDNA silencing (Fig. 3e and f). Work is underway to elucidate how these various compounds can differentially affect rDNA stability and silencing.

Another measure of lifespan in *S. cerevisiae* is the length of time cells can survive in a metabolically active but nutrient deprived state. Aging under these conditions (i.e. chronological aging) is primarily due to oxidative damage[41]. Resveratrol (10 μM or 100 μM) failed to extend chronological lifespan (not shown), indicating that the sirtuin-stimulatory effect of resveratrol may be more relevant *in vivo* than its antioxidant activity[30,31].

**Example 4: Effects of activators in human cells**

To test whether these compounds could stimulate human SIRT1 *in vivo,* we first employed a cellular deacetylase assay that we had developed. A schematic of the assay procedure is depicted in Fig. 4a. Cells are incubated with media containing the fluorogenic ε-acetyl-lysine substrate, 'Fluor de Lys' (FdL). This substrate, neutral when acetylated, becomes positively charged upon deacetylation and accumulates within cells (see Fig. 6a). Lysis of the cells and addition of the non-cell-permeable 'Developer' reagent releases a fluorophor specifically from those substrate molecules that have been deacetylated (Fig. 4a and see Methods). With HeLa cells growing adherently, 5-10% of the signal produced in this assay is insensitive to 1 μM trichostatin A (TSA), a potent inhibitor of class I and II HDACs but not sirtuins (class III)[42] (Figs. 6b and 6c).

A selection of SIRT1-stimulatory and non-stimulatory polyphenols were tested for their effects on this TSA-insensitive signal (Fig. 4b). Cellular deacetylation signals in the presence of each compound (y-axis, Fig. 4b) were plotted against their fold-stimulations of SIRT1 *in vitro* (x-axis, Fig. 4b, data from Supplementary Tables 1-3). For most of the compounds, the *in vitro* activity roughly corresponded to the cellular signal. Compounds with little or no *in vitro* activity clustered around the negative control (Group A, Fig. 4b). Another grouping, of strong *in vitro* activators is clearly distanced from the low activity cluster in both dimensions (Group B, Fig. 4b). A notable outlier was butein, a potent activator of SIRT1 *in vitro* which had no effect on the cellular signal. With allowances for possible variation among these compounds in properties unrelated to direct sirtuin stimulation, such as cell-permeability and rates of metabolism, these data are consistent with the idea that certain polyphenols can activate native sirtuins *in vivo.*

One known target of SIRT1 *in vivo* is lysine 382 of p53. Deacetylation of this residue by SIRT1 decreases the activity and half-life of p53[20,21,27]. To follow the acetylation status of K382 we generated a rabbit polyclonal antibody that recognizes the acetylated form of K382 (Ac-K382) on Western blots of whole cell lysates. As a control we showed that the signal was specifically detected in extracts from cells exposed to ionizing radiation (Fig. 4c), but not in extracts from cells lacking p53 or where arginine had been substituted for lysine 382 (data not shown). U2OS osteosarcoma cells were pre-treated for 4 hours with resveratrol (0.5 and 50 μM) and exposed to UV radiation. We consistently observed a marked decrease in the level of Ac-K382 in the presence of 0.5 μM resveratrol, compared to untreated cells (Fig. 4d). At higher concentrations of resveratrol (>50 μM) the effect was reversed (Fig. 4d and data not shown), consistent with previous reports of increased p53 activity at such concentrations[43]. The ability of low concentrations of resveratrol to promote deacetylation of p53 was diminished in cells expressing a dominant-negative *SIRT1* allele (H363Y) (Fig. 4e), demonstrating that SIRT1 is necessary for this effect. This biphasic dose-response of resveratrol could explain the dichotomy in the literature regarding the effects of resveratrol on cell survival[30,43,44].

Thus, we have discovered the first known class of small molecule sirtuin activators, all of which are plant polyphenols. These compounds can dramatically stimulate sirtuin activity *in vitro* and promote effects consistent with increased sirtuin activity *in vivo.* In human cells, resveratrol promotes SIRT1-mediated p53 deacetylation of K382. In yeast, the effect of resveratrol on lifespan is as great as any longevity-promoting genetic manipulation[6] and has been linked convincingly to the direct activation of Sir2. The correlation between lifespan and rDNA recombination, but not silencing, adds to the body of evidence that yeast aging is due to DNA instability[2,5,37-39] not gene dysregulation[45].

How can we explain the activation of the yeast and human sirtuins by so many plant metabolites? Sirtuins have been found in diverse eukaryotes, including fungi, protozoans, metazoans and plants[46,47], and likely evolved early in life's history[1]. Plants are known to produce a variety of polyphenols, including resveratrol, in response to stresses such as dehydration, nutrient deprivation, UV radiation and pathogens[48,49]. Therefore it is plausible that these compounds may be synthesized to regulate a sirtuin-mediated plant stress response. This would be consistent with the recently discovered relationship between environmental stress and Sir2 activity in yeast[6]. Perhaps these compounds have stimulatory activity on sirtuins from fungi and animals because they mimic an endogenous activator, as is the case for the opiates/endorphins, cannabinols/endocannabinoids and various polyphenols with estrogen-like activity[30,31]. Alternatively, animal and fungal sirtuins may have retained or developed an ability to respond to these plant metabolites because they are a useful indicator of a deteriorating environment and/or food supply.

**Example 5: Materials and Methods for Examples 1-4**

Compound libraries and deacetylation assays

$His_6$-tagged recombinant SIRT1 and GST-tagged recombinant Sir2 were prepared as previously described[26]. From 0.1 to 1 μg of SIRT1 and 1.5 μg of Sir2 were used per deacetylation assay (in 50 μl total reaction) as previously described[26]. SIRT1 assays and certain of those for Sir2 employed the p53-382 acetylated substrate ('Fluor de Lys-SIRT1', BIOMOL) rather than FdL.

Themed compound libraries (BIOMOL) were used for primary and secondary screening. Most polyphenol compounds were dissolved at 10 mM in dimethylsulfoxide (DMSO) on the day of the assay. For water soluble compounds

and negative controls, 1% v/v DMSO was added to the assay. *In vitro* fluorescence assay results were read in white 1/2-volume 96-well microplates (Corning Costar 3693) with a CytoFluor™II fluorescence plate reader (PerSeptive Biosystems, Ex. 360 nm, Em. 460 nm, gain = 85). HeLa cells were grown and the cellular deacetylation assays were performed and read, as above, but in full-volume 96-well microplates (Coming Costar 3595). Unless otherwise indicated all initial rate measurements were means of three or more replicates, obtained with single incubation times, at which point 5% or less of the substrate initially present had been deacetylated. Calculation of net fluorescence increases included subtraction of a blank value, which in the case of Sir2 was obtained by omitting the enzyme from the reaction and in the case of SIRT1 by adding an inhibitor (200 μM suramin or 1 mM nicotinamide) to the reaction prior to the acetylated substrate. A number of the polyphenols partially quenched the fluorescence produced in the assay and correction factors were obtained by determining the fluorescence increase due to a 3 μM spike of an FdL deacetylated standard (BIOMOL, catalog number KI -142). All error bars represent the standard error of the mean.

Media and Strains

All yeast strains were grown at 30°C in complete yeast extract/bactopeptone, 2.0% (w/v) glucose (YPD) medium except where stated otherwise. Calorie restriction was induced in 0.5% glucose. Synthetic complete (SC) medium consisted of 1.67% yeast nitrogen base, 2% glucose, 40 mg/litre each of auxotrophic markers. *SIR2* was integrated in extra copy and disrupted as described[5]. Other strains are described elsewhere[26]. For cellular deacetylation assays, HeLa S3 cells were used. U2OS osteosarcoma and human embryonic kidney (HEK 293) cells were cultured adherently in Dulbecco's Modified Eagle's Medium (DMEM) containing 10% fetal calf serum (FCS) with 1.0% glutamine and 1.0% penecillin/streptomycin. HEK 293 overexpressing dominant negative SIRT1 H363Y was a gift of R. Frye (U. Pittsburgh).

Lifespan determinations

Lifespan measurements were performed using PSY316AT *MAT*$\alpha$ as previously described[35]. All compounds for lifespan analyses were dissolved in 95% ethanol and plates were dried and used within 24 hours. Prior to lifespan analysis, cells were pre-incubated on their respective media for at least 15 hours. Following transfer to a new plate, cells were equilibrated on the medium for a minimum of 4 hours prior to micro-manipulating them. At least 30 cells were examined per experiment and each experiment was performed at least twice. Statistical significance of lifespan differences was determined using the Wilcoxon rank sum test. Differences are stated to be significant when the confidence is higher than 95%.

Silencing and recombination assays

Ribosomal DNA silencing assays using the *URA3* reporters were performed as previously described[26]. Ribosomal DNA recombination frequencies were determined by plating W303AR cells[37] on YPD medium with low adenine/histidine and counting the fraction of half-red sectored colonies using Bio-Rad Quantity One software as previously described[35]. At least 6000 cells were analyzed per experiment and all experiments were performed in triplicate. All strains were pre-grown for 15 hours with the relevant compound prior to plating.

Proteins and Western analyses

Recombinant Sir2-GST was expressed and purified from *E. coli* as previously described except that lysates were prepared using sonication[26]. Recombinant SIRT1 from E. *coli* was prepared as previously described[26]. Polyclonal antiserum against p53-AcK382 was generated using an acetylated peptide antigen as previously described[20], with the following modifications. Anti-Ac-K382 antibody was affinity purified using non-acetylated p53-K382 peptides and stored in PBS at -70°C and recognized an acetylated but not a non-acetylated p53 peptide. Western hybridizations using anti-acetylated K382 or anti-actin (Chemicon) antibody were performed at 1:1000 dilution of antibody. Hybridizations with polyclonal p53 antibody (Santa Cruz Biotech.) used 1:500 dilution of antibody. Whole cell extracts were prepared by lysing cells in buffer containing 150 mM NaCl, 1 mM $MgCl_2$, 10% glycerol, 1% NP40, 1 mM DTT and anti-protease cocktail (Roche).

References for Examples 1-4 and Background

1. Kenyon, C. Cell 105, 165-168 (2001).
2. Sinclair, D. A. Mech Ageing Dev 123, 857-67 (2002).

3. Hekimi, S. & Guarente Science 299, 1351-4 (2003).
4. Guarente, L. & Kenyon, C. Nature 408, 255-62. (2000).
5. Lin et al. Science 289, 2126-8. (2000).
6. Anderson et al. Nature 423, 181-5 (2003).
7. Kaeberlein et al. Genes Dev 13, 2570-80. (1999).
8. Landry et al. Proc Natl Acad Sci USA 97, 5807-11. (2000).
9. Imai et al. Nature 403, 795-800 (2000).
10. Smith et al. Proc Natl Acad Sci U SA 97, 6658-63. (2000).
11. Tanner et al. Proc Natl Acad Sci USA 97, 14178-82. (2000).
12. Tanny et al. Cell 99, 735-45. (1999).
13. Tanny et al. Proc Natl Acad Sci USA 98, 415-20. (2001).
14. Laurenson et al. Microbiol Rev 56, 543-60. (1992).
15. Smith et al. Genes Dev 11, 241-54. (1997).
16. Bryk, M. et al. Genes Dev 11,255-69. (1997).
17. Gottlieb et al. Cell 56, 771-6. (1989).
18. Aguilaniu et al. Science (2003).
19. Tissenbaum et al. Nature 410, 227-30. (2001).
20. Vaziri et al. Cell 107, 149-59. (2001).
21. Luo et al. Cell 107, 137-48. (2001).
22. Vergnes et al. Gene 296, 139-50 (2002).
23. Holzenberger et al. Nature 421, 182-7 (2003).
24. Shimokawa et al. Faseb J 17, 1108-9 (2003).
25. Tatar et al. Science 299, 1346-51 (2003).
26. Bitterman et al. JBiol Chem 277, 45099-107. (2002).
27. Langley et al. EMBO J21, 2383-2396 (2002).
28. Glossmann et al. Naunyn Schmiedebergs Arch Pharmacol 317, 100-2 (1981).
29. Oliver et al. JBiol Chem 269, 29697-703 (1994).
30. Ferguson et al. Mutat Res 475, 89-111 (2001).
31. Middleton et al. Pharmacol Rev 52, 673-751 (2000).
32. Jang et al. Science 275, 218-20 (1997).
33. Stojanovic et al. Arch Biochem Biophys 391, 79-89 (2001).
34. Monod et al. J. Mol. Biol. 12, 88-118 (1965).
35. Anderson et al. JBiol Chem 277, 18881-90. (2002).
36. Pont et al. J Phytopathol 130, 1-8 (1990).
37. Sinclair et al. Cell 91, 1033-42. (1997).
38. Defossez et al. Mol Cell 3, 447-55 (1999).
39. Park et al. Mol Cell Biol 19, 3848-56 (1999).
40. Benguria et al. Nucleic Acids Res 31, 893-8 (2003).
41. Longo et al. Science 299, 1342-6 (2003).
42. Denu et al. Trends Biochem Sci 28, 41-8 (2003).
43. Dong et al. Mutat Res 523-524, 145-50 (2003).
44.Nicolini et al. Neurosci Lett 302, 41-4 (2001).
45. Jazwinski, S. M. et al. Ann N Y Acad Sci 908, 21-30 (2000).
46. Pandey et al. Nucleic Acids Res 30, 5036-55 (2002).
47. Frye, R. A. Biochem Biophys Res Commun 273, 793-8. (2000).
48. Soleas et al. Clin Biochem 30, 91-113 (1997).
49. Coronado et al. Plant Physiol 108, 533-542 (1995).
50. Masoro, E. J.Exp Gerontol 35, 299-305. (2000).

**Example 6: <u>Localization of the activation domain of sirtuins to their N-terminus</u>**

Yeast Sir2 and human SIRT1 are very homologous and differ from human SIRT2 by the addition of an N-terminal domain that is absent in SIRT2. The effect of resveratrol was assayed on human recombinant SIRT2 as follows. Human recombinant SIRT2 was incubated at a concentration of 1.25μg/well with 25μM of Fluor de Lys-SIRT2 (BIOMOL cat. # KI-179) and 25μM NAD+ for 20 minutes at 37°C, as described above. The results, which are shown in Figure 7, indicate that, in contrast to SIRT1, increasing concentrations of resveratrol decrease SIRT2 activity. Thus, based on the difference in structure of SIRT1 and SIRT2, i.e., the absence of an N-terminal domain (see Fig. 8), it is believed that the N-terminal domain of SIRT1 and Sir2 is necessary for activation by the compounds described herein. In particular, it is

likely that the activator compounds described herein interact with the N-terminal portion of sirtuins. The N-terminal portion of SIRT1 that is necessary for the action of the compounds is from about amino acid 1 to about amino acid 176, and that of Sir2 is from about amino acid 1 to about amino acid 175.

**Example 7: Resveratrol extends the lifespan of *C. elegans***

50 *C. elegans* worms (strain N2) were grown in the presence or absence of 100 μM resveratrol for 17 days. On day 17, only 5 worms in the control group without resveratrol were alive, whereas 17 worms were alive in the group that was treated with resveratrol. Thus, the presence of resveratrol in the growth media of *C. elegans* extends their lifespan.

**Example 8: Identification of additional activators of sirtuins**

Using the screening assay described in Example 1, five more sirtuin activators have been identified. These are set forth in supplementary Table 8.

**Example 9: Identification of inhibitors of sirtuins**

Using the screening assay described in Example 1, more inhibitors were identified. These are set forth in the appended supplementary Table 8, and correspond to the compounds having a ratio to control rate of less than 1.

**Example 10: Identification of further activators and inhibitors of sirtuins**

Additional activators and inhibitors of sirtuins were identified, and are listed in Tables 9-13. In these Tables, "SE" stands for Standard error of the mean and N is the number of replicates used to calculate mean ratio to the control rate and standard error.

All SIRT1 rate measurements used in the calculation of "Ratio to Control Rate" were obtained with 25 μM $NAD^+$ and 25 μM p53-382 acetylated peptide substrate were performed as described above and in K.T. Hcwitz et al. Nature (2003) 425: 191. All ratio data were calculated from experiments in which the total deacetylation in the control reaction was 0.25-1.25 μM peptide or 1-5% of the initial concentration of acetylated peptide.

Stability determinations ($t_{1/2}$) derived from SIRT1 rate measurements performed in a similar way to those described above, except that 5 μM p53-382 acetylated peptide substrate was used rather than 25 μM. The fold-stimulation (ratio to control) obtained with a compound diluted from an aged stock solution was compared to an identical dilution from a stock solution freshly prepared from the solid compound. "$t_{1/2}$" is defined as the time required for the SIRT1 fold-stimulation of the compound from the aged solution to decay to one-half of that obtained from a freshly prepared solution. Ethanol stocks of resveratrol, BML-212 and BML-221 were prepared at 2.5 mM and the compounds were assayed at a final concentration of 50 μM. The water stock of resveratrol was 100 μM and the assay performed at 10 μM. Stocks were aged by storage at room temperature, in glass vials, under a nitrogen atmosphere.

The effect of some of these compounds on lifespan was determined in yeast and C. *elegans,* as described above. The results are set forth below in Table 19:

| Compound | % change in yeast replicative lifespan relative to untreated organisms (10 μM)[a] | % change in *C. elegans* lifespan relative to untreated organisms (100/500 μM)[b] |
|---|---|---|
| untreated | 100% | 100% |
| Resveratrol 3,5,4'-Trihydroxy-*trans*-stilbene | 170 - 180% | 110% |
| Pinosylvin 3,5-Dihydroxy-*trans*-stilbene | 114% | ND |
| BML-212 3,5-Dihydroxy-4'-fluoro-*trans*-stilbene | 98% | ND |
| BML-217 3,5-Dihydroxy-4'-chloro-*trans*-stilbene | 90% | ND |
| BML-221 3,4'-Dihydroxy-5-acetoxy-*trans*-stilbene | 165% | >100% (ongoing) |
| BML-233 | ND | 70% (10) |

(continued)

| Compound | % change in yeast replicative lifespan relative to untreated organisms (10 $\mu$M)[a] | % change in *C. elegans* lifespan relative to untreated organisms (100/500 $\mu$M)[b] |
|---|---|---|
| 3,5-Dihydroxy-4'-methoxy-*trans*-stilbene | | 50% (500) |
| a. Replicative lifespans performed using 2% (w/v) glucose standard yeast compete medium (YPD) under standard conditions.<br>b. Lifespan assays performed on N2 worms using *E. coli* as food under standard conditions.<br>ND. Not determined. | | |

The results indicate that resveratrol significantly extends lifespan in yeast and in *C. elegans.* Since BML-233 was shown to be a strong activator of sirtuins (see above), the results obtained in *C. elegans* may indicate that the compound is toxic to the cells.

Without wanting to be limited to particular structures, it appears that the following structure/activity relationships exist SIRT1 activation results from several of these new analogs confirmed the importance of planarity, or at least the potential for planarity, between and within the two rings of the active compounds. Reduction of the double bond of the ethylene function, between, the two rings essentially abolishes activity (compare Resveratrol, Table A and Dihydroresveratrol, Table E). Replacement of a phenyl moiety with a cyclohexyl group is nearly as detrimental to SIRT1 stimulating activity (compare Pinosylvin, Table 9 and BML-224, Table 12). Amide bonds are thought to have a partially double bond character. However, replacement of the ethylene function with a carboxamide abolished activity (compare Pinosylvin, Table 9, with BML-219, Table 13). It is possible that this effect could be due in part to the position that carbonyl oxygen must assume in the conformation that places the two rings *trans* to one another. If so, a compound in which the positions of the amide nitrogen and carbonyl are reversed might be expected to have greater activity.

In twelve of the analogs resveratrol's 4'-hydroxyl was replaced with various functionalities (see Tables 9 and 10, BML-221 in Table 11, BML-222 in Tables 12). Although none of the replacements tried led to substantial increases in SIRT1 stimulating activity, this parameter was, in general, remarkably tolerant of substitutions at this position. Small groups (H- in Pinosylvin, Cl- in BML-217, $-CH_3$ in BML-228) did the least to decrease activity. There is some evidence of a preference in the enzyme's stilbene binding/activation site for unbranched (ethyl in BML-225, azido in BML-232, $-SCH_3$ in BML-230) and hydrophobic functions (compare isopropyl in BML-231 to acetoxy in BML-221, acetamide in BML-222). Solution stability relative to resveratrol was strongly increased by one of the two 4'-substitutions (acetoxy, BML-221) tested for this so far.

Resveratrol is currently one of the most potent known activator of "SIRT1. The collection of analogs described above, particularly the group entailing substitutions at the 4' position, may be instrumental in informing the design of new SIRT1 ligands with improved pharmacological properties. One parameter that may be of interest in this regard is stability. One 4'-substituted analog, BML-221, displays a vast improvement in solution stability relative to resveratrol and although diminished in *in vitro* SIRT1 activating ability, retains much of resveratrol's biological activity (see lifespan data). The 4'-hydroxyl of resveratrol is thought to be of primary importance to resveratrol's free-radical scavenging reactivity (S. Stojanovic et al. Arch. Biochem. Biophys. 2001 391 79). Most of the 4'-substituted analogs have yet to be tested for solution stability, but if resveratrol's instability in solution is due to redox reactivity, many of the other analogs would be expected to also exhibit improved stability.

The results obtained with 4'-substituted analogs may indicate promising routes to explore while seeking to increase SIRT1 binding affinity. For example, the efficacy of the 4'-ethyl compound (BML-225) might indicate the presence of a narrow, hydrophobic binding pocket at the SIRT1 site corresponding to the 4' end of resveratrol. Several new series of 4'-substituted analogs are planned, the simplest comprising straight-chain aliphatic groups of various lengths.

**Example 11: Dose-response analysis of SIRT1 deacetylation by resveratrol and BML-230**

SIRT1 initial rates as a function of activator concentration were determined at 25 $\mu$M each of $NAD^+$ and p53-382 acetylated peptide, with 20 minutes incubations. Plots of the dose responses of SIRT1 to BML-230 and resveratrol show that the BML-230-stimulated activity exceeds that stimulated by resveratrol at all concentrations tested (Figure 9a). This could be due to a greater binding affinity of SIRT1 for BML-230, greater activity of the SIRT1/BML-230 complex or some combination of the two. A plot of the ratio of the rates of BML-230-stimulated enzyme to that of resveratrol-stimulated enzyme suggests that increased binding affinity does contribute to the improvement in activity of BML-230 (Figure 9b). A simple two state model of the binding and activation process assumes that the observed rate (v) is the sum of the fractional contributions of the unliganded and liganded enzymes, where $v_0$ is the unstimulated rate, $v_1$ is the rate of the

enzyme with bound ligand-1 (LI) and $K_{L1}$ is the dissociation constant of the enzyme/ligand-1 complex:

$$v = v_0(1-[L1]/(K_{L1} + [L1])) + v_1(-[L1]/(K_{L1} + [L1])$$

A similar equation can be prepared for ligand-2 and the ratio (R) of the two rates calculated, an equation which will include, given the conditions of Figure 9, the substitution [L]=[L1]=[L2]. It can be shown that if the two ligand dissociation constants were equal ($K_{L1}=K_{L2}=K_L$), this ratio would be:

$$R = (v_0K_L + v_1[L])/ (v_0K_L + v_2[L])$$

If $K_{L1} \neq K_{L2}$, this ratio would instead be:

$$R = (v_1[L]^2 + (v_0K_{L1} + v_1K_{L2} )[L] + v_0K_{L1}K_{L2})/ (v_2[L]^2 + (v_0K_{L2} + v_2K_{L1} )[L] + v_0K_{L1}K_{L2})$$

In the first case the plot of R vs. [L] would be a simple hyperbola that monotonically approaches $v_1/v_2$ as [L] increases. In the second case, as in Fig. 9b, the plot would pass through a maximum before approaching $v_1/v_2$ at higher [L] values. The data of Fig. 9b would imply that $v_1/v_2$ (rate for pure SIRT1BML-230 divided by that for pure SIRT1/resveratrol) is no more than ~1.4 (R at 500 μM) and that the SIRT1/BML-230 complex indeed has a lower dissociation constant than SLRT1/resveratrol ($K_{L1} < K_{L2}$).

One of the difficulties in the use of resveratrol as a pharmacologic agent is the relatively low serum concentrations of the aglycone form that can be achieved and maintained when it is administered orally (<<1 μM; see for example D.M Goldberg et al. Clin. Biochem. 2003 36 79). Increasing the SIRT1 binding affinity of synthetic derivatives will improve this aspect of the drug. As sest forth above, various replacements of the resveratrol 4'-hydroxyl e.g. the H- of pinosylvin or Cl- of BML-217, did not significantly diminish the SIRT1 activating effect. The results obtained with BML-230 indicate that it will be possible to actually increase SIRT1/activator binding affinity by modifications at that site. The 4'-thiomethyl of BML-230 therefore represents a new starting point in seeking further improvements in SIRT1 binding affinity by the synthesis of related derivatives (e.g. 4'-thioethyl etc.).

**Example 12: <u>Survival rates</u>**

Human 293 were grown to exponential phase under standard conditions and subjected to a dose of compound (50 micromolar) for 96 hours. The number of live cells each time point was counted using a Coulter counter.

Table 24: Survival statistics of 293 cells:

| Time (h) | Resveratrol | Thio-Methyl BML-230 | Ethyl BML-225 | Methyl BML-228 | Isopropyl BML-231 |
|---|---|---|---|---|---|
| 0 | 100% | 100% | 100% | 100% | 100% |
| 48 | 5% | 55% | 5% | 46% | 0% |
| 96 | 0% | 57% | 8% | 32% | 0% |

The results indicate that thiomethyl (BML-230) was the least toxic on 293 cells.

**Example 13: <u>Sirtuin activators mimic calorie restriction and delay aging in metazoans</u>**

Caloric restriction (CR) extends lifespan in numerous species. In the budding yeast *S. cerevisiae,* this effect requires Sir2[1], a member of the sirtuin family of $NAD^+$ -dependent deacetylases[2,3]. Sirtuin-activating compounds (STACs) can promote the survival of human cells and extend the replicative lifespan of yeast[4]. Here we show that resveratrol and other STACs activate sirtuins from *Caenorhabditis elegans* and *Drosophila melanogaster* and extend the lifespan of these animals up to 29% without reducing fecundity. Lifespan extension is dependent on functional Sir2 and is not observed when nutrients are restricted. Together these data indicate that STACs slow metazoan ageing by mechanisms related to CR.

Sir2-like proteins (sirtuins) are a family of $NAD^+$-dependent deacetylases conserved from *E.coli* to humans[5,9] (Fig. 10a) that play important roles in gene silencing, DNA repair, rDNA recombination and ageing in model

organisms[2,10-12]. When diet is restricted (calorie restriction, CR), lifespan is extended in diverse species, suggesting there is a conserved mechanism for nutrient regulation of ageing[13-17]. In budding yeast, extra copies this gene extend lifespan by 30% apparently by mimicking CR[1,18]. We recently described a group of compounds (STACs) that stimulate the catalytic activity of yeast and human sirtuins, and extend the replicative lifespan of yeast cells up to 60%[4].

To establish whether STACs could activate sirtuins from multicellular animals, we developed a cell-based deacetylation assay for *D. melanogaster* S2 cells. Several classes of polyphenolic STACs, including chalcones, flavones and stilbenes, increased the rate of deacetylation in an $NAD^+$-dependent manner (Fig. 10b). To determine whether this activity was due to direct stimulation of a Sir2 homolog, we purified recombinant SIR-2.1 of *C. elegans* and dSir2 of *D. melanogaster* and determined the effect of various STACs on enzymatic activity *in vitro* (Fig. 10c, d). In a dose-dependent manner, resveratrol stimulated deacetylation up to 2.5-fold for SIR-2.1 (Fig. 10e) and 2.4-fold for dSir2 (Fig. 10f). As previously observed with the yeast and human Sir2 enzymes, resveratrol lowered the $K_m$ of Slur-2.1 for the co-substrate $NAD^+$ (Fig. 10g).

Because resveratrol can significantly extend replicative lifespan in yeast[4], we asked whether STACs could also extend lifespan in the metazoans *C. elegans* and *D. melanogaster.* Wild-type worms were transferred to plates containing 0 or 100 μM of resveratrol shortly after reaching adulthood. Lifespan was reproducibly extended up to 15%, using either heat-killed or live *E. coli* as food supply (Fig. 11a, c respectively) and mortality was decreased across all adult ages (Fig. 14). To test whether the lifespan extension depends on functional SIR-2.1, we constructed a *sir-2.1* null mutant. The lifespan of this strain was not appreciably shorter than the wildtype N2 control and adults treated with resveratrol did not exhibit a significant lifespan extension relative to untreated worms (Fig. 11b, d). There was no decrease in fecundity associated with resveratrol treatment (Fig. 11e). To rule out the possibility that resveratrol was causing the animals to eat less, thereby inducing a CR effect indirectly, we measured feeding rates of both L4 larval and adult worms with or without resveratrol and found no differences (Fig. 11f).

We also tested whether STACs could extend lifespan in *D. melanogaster* using the standard laboratory wild type strain Canton-S. and normal fly culturing conditions (vials), and a *yw* marked wild type strain and demographic culturing conditions (cages) (Table 20). Across independent tests in males and females, lifespan was extended up to 23% with fisetin and up to 29% with resveratrol (Fig. 12a, c, e). Increased longevity was associated with reduced mortality prior to day 40 (Fig. 14). A restricted diet increased lifespan by 40% in females and by 14% in males (averaged across trials), and under these conditions neither resveratrol nor fisetin further increased longevity (Fig. 12b, d, f), suggesting that resveratrol extends lifespan through a mechanism related to CR.

Surprisingly, while diet manipulations that extend *D. melanogaster* longevity typically reduce fecundity[19,20], longevity-extending doses of resveratrol modestly increased egg production(10 μM resveratrol: 69.8 eggs/5days, s.e.= 2.2; control: 59.9 eggs/5days, s.e. = 2.2; $t$ = 3.17, P = 0.0017), particularly in the earliest days of adult life (Fig. 12g). The increase in egg production suggests that the lifespan extending effect of resveratrol in *D. melanogaster* was not due to CR induced by food aversion or lack of appetite. Consistent with this, no decrease in food uptake was seen with resveratrol-fed flies (Fig. 12h). Furthermore, resveratrol-fed flies maintained normal weight (Fig. 12i), except during days 3 through when resveratrol fed females were laying significantly more eggs than control fed females.

To determine whether resveratrol extends fly lifespan in a Sir2-dependent manner, we analyzed a *dSir2* allelic series with increasing amounts of dSir2. Adult offspring from crosses between independently derived alleles of *dSir2* were tested. Resveratrol failed to extend lifespan in flies completely lacking functional dSir2 ($dSir2^{4.5}/dSir2^{5.26}$) (Fig. 13a, b) or in flies in which dSir2 is severely decreased ($dSir2^{17}/dSir2^{KG00871}$) (Fig. 13c, d). Resveratrol increased longevity a small but statistically significant amount in flies homozygous for a hypomorphic *dSir2* allele ($dSir2^{KG0087}/dSir2^{KG0087}$) (Table 20, Trial 6) and increased lifespan up to 17% in flies with one copy of the hypomorphic allele and one copy of a wild-type *dSir2* (Canton-S/$dSir2^{KG008}$) (Table 20, Trial 7). These data demonstrate that the ability of resveratrol to extend fly lifespan requires functional Sir2.

We previously reported that STACs extend the lifespan of replicating yeast cells by mimicking CR[4]. In yeast, chronological and reproductive aging are inseparable in the measure of replicative lifespan. Here we show that STACs can extend lifespan in C. *elegans* and *D. melanogaster,* both of which are comprised of primarily non-dividing (post-mitotic) cells as adults, and whose somatic and reproductive aging are independent measures of senescence. In both species, resveratrol increases lifespan in a Sir2-dependent manner and, at least for the fly, this action appears to function through a pathway common to CR.

Our observation that resveratrol can increase longevity without an apparent cost of reproduction is counter to prevalent concepts of senescence evolution. However, STACs may still entail trade-offs under some environmental conditions[21,22] or in the context of selection acting upon the network of traits that determine fitness[23,24]. Plants synthesize STACs such as resveratrol in response to stress and nutrient limitations, possibly to activate their own sirtuin pathways[4]. These molecules may activate animal sirtuins because they serve as plant defense mechanisms against consumers or because they are ancestrally orthologous to endogenous activators within metazoans. Alternatively, animals may use plant stress molecules as a cue to prepare for a decline in their environment or food supply[4]. Understanding the adaptive significance, endogenous function, and evolutionary origin of sirtuin activators will lead to further insights into the underlying mechanisms of longevity regulation and aid in the development of interventions that provide the health benefits of CR.

**Example 14: <u>Materials and methods for Example 14</u>**

<u>Sirtuin purification</u>

$His_6$-tagged recombinant SIR-2.1 and dSir2 were purified from *E. coli* BL21(DE3) *plysS* cells harboring either pET28a-sir-2.1 or pRSETc-dSir2 plasmids. Cells were grown in LB medium, containing kanamycin (50 μg/mL) for pET28a-sir-2.1 or ampicillin (100 μg/ml) and chloramphenicol (25 μg/ml) for pRSETc-dSir2 at 30°C (dSir2) or 37°C (SIR-2.1) to an $OD_{600}$ of 0.6-0.8. After addition of IPTG (1 mM, flasks were shifted to 16°C for 20 h. Cell pellets were resuspended in cold PBS buffer containing 300 mM NaCl, 0.5 mM DTT, 0.5 mM PMSF and EDTA-free protease inhibitor tablets and lysed by sonication. $Ni^{2+}$-NTA beads were added to the clarified extract and after 1-3 hours they were loaded on a column, washed with buffer (50 mM Tris. Cl pH 7.4, 200 mM NaCl, 30 mM imidazole) then eluted with the same buffer containing 600 mM imidazole.

<u>Deacetylation assays</u>

From 0.1 to 1 μg of SIR-2.1 and 1 μg of dSir2 were used per deacetylation assay as previously described with modifications (SIR-2.1: 200 μM $NAD^+$, 10 μM Fluor de Lys, FdL; dSir2: 25 μM $NAD^+$, 10 μM FdL)[26]. STACs were dissolved at 10 mM in dimethylsulfoxide (DMSO) the day of the assay. *In vitro* fluorescence assay results were read in 96-well microplates (Coming Costar 3693) with a Wallac Victor Multilabel counter (Perkin Elmer, excitation at 360 nm, emission at 450 nm). *Drosophila* S2 cells were grown in Schneider media with fetal calf serum at 23-28°C, seeded at $9x10^4$ cells/well, grown overnight and then exposed to 1 μM TSA, 500 μM polyphenols, and 200 μM FdL for 2 hr. Deacetylation of FdL with lysate from whole cells was determined as described[4]. Unless otherwise indicated all initial rate measurements were means of three or more replicates obtained with single incubation times, at which point 5% or less of the substrate initially present was deacetylated.

<u>*C. elegans* media, strains, lifespan, and feeding assays</u>

Bristol N2 (*Caenorhabditis* Genetics Center) was used as the wild-type strain. The *sir-2.1* mutant strain was generated by backcrossing VC199 (*sir-2.1(ok434)*) to N2 four times. Cultures were grown on standard NGM media and maintained on *E. coli* strain OP50. For the lifespan assays, synchronized animals were transferred to treatment plates as young adults (2 d after hatching, day 0 of assay), and were transferred to fresh treatment plates every 2 days for the first 6 to 8 days of the assay. Treatment plates were standard NGM media with the reproductive suppressant FUdR (Sigma; 100mg/-L) containing resveratrol or solvent (DMSO, which does not affect lifespan) added either directly into the agar before pouring (for live OP50 trials) or diluted into PBS and added to the surface of a dry plate to the indicated final concentration (for dead OP50 trials). For some lifespan trials, heat-killed OP50 were used as a food source. OP50 cultures were heated to 65°C for 30 minutes, then pelleted and resuspended in 1/10 volume in S Basal supplemented with 10mM $MgSO_4$. In all assays, worms were monitored daily for mortality by gently probing with a platinum pick. Assays were performed at 24°C.

To assay worm feeding rates, worms at the indicated stages where placed on treatment plates (no FUdR) for 4-5 hours, then videoed for 1 minute using a Pixelink PL-662 camera. The frame rate was slowed and the pumping rate of the pharynx was counted. To assay fecundity, gravid hermaphrodites (5 per plate, raised from synchronized L1s on normal or treatment plates) were allowed to lay eggs on their respective media for 5 hours, and the total number of eggs was counted.

<u>*D. melanogaster* media, strains, feeding assay and lifespan assays</u>

Survival assays were conducted independently with adult *D. melanogaster* in two laboratories. In the first laboratory, all trials used an *yw* marked wild-type strain. Larvae were reared on standard cornmeal-sugar-yeast (CSY) agar diet (cornmeal 5%, sucrose 10.5%, SAF yeast 2%, and agar 0.7%). Newly eclosed adults were placed in 1L demography cages with approximately 75 males and 75 females. Three to four replicate 1L demography cages were used for each treatment group in each trial. Every two days, dead flies were removed and scored, and food vials were replenished. Food vials contained cornmeal-sugar-yeast diet with SAF yeast as either 2% or 3% by weight. Test compounds in 100 μl of EtOH (or blank EtOH in controls) were mixed into melted aliquots of the adult food media to make a final concentration of 0, 10 or 100 μM. Fresh stock solutions and adult media were prepared weekly. In the second laboratory, lifespan trials were conducted with the wild type strain Canton-S, *sir2* [4.5] and *dSir2* [5.26] (S. Smolik, University

of Oregon), *dSir2*[17] (S. Astrom, Stockholm University, Sweden), and *dSir2*[KG00871] (Drosophila Stock Center, Bloomington, IN). Larvae for all tests were reared on standard cornmeal-sugar-yeast diet. Newly eclosed adults were incubated in plastic shell vials containing 5 ml of 15% sugar-yeast diet (15% SY) or 5% sugar-yeast (5% SY) diet (15% SY: 15% yeast, 15% sucrose, 2% agar; 5% SY: 5% yeast, 5% sucrose, 2% agar as per Ref. [20]). In all trials, ~20 males with ~20 females were placed into each of 10 vials/treatment group. Every two days, flies were passed into new vials and dead flies were counted. Resveratrol in EtOH (or EtOH alone in controls) was added to the media during its preparation after it had cooled to 65°C and mixed vigorously. Final compound concentrations were 0, 10, 100 or 200 μM. Fresh stock solution and adult media was prepared weekly.

Feeding rate was measured in *yw* females with the crop-filling assay[27]. Females were held overnight with water and placed on 2% CSY diet containing food colour (FDA Blue 1) and 0, 10 or 100 μM resveratrol with EtOH. The presence of dye-marked food in the crop was scored in sets of 20 females across five 5-minute intervals. For body mass measurements, 10 vials with 20 males and 20 females each of wild type CS-5 flies were kept on 15% SY diet with EtOH or with resveratrol in EtOH (10μM). Males and females were weighed daily.

References for Examples 13 and 14:

1. Lin, S. J., Defossez, P. A. & Guarente, L. Requirement of NAD and SIR2 for lifespan extension by calorie restriction in Saccharomyces cerevisiae. Science 289, 2126-8. (2000).
2. Gasser, S. C. M. The molecular biology of the SIR proteins. Gene 279, 1-16 (2001).
3. Hekimi, S. & Guarente, L. Genetics and the specificity of the aging process. Science 299, 1351-4 (2003).
4. Howitz, K. T. et al. Small molecule activators of sirtuins extend Saccharomyces cerevisiae lifespan. Nature 425, 191-6 (2003).
5. Landry, J. et al. The silencing protein SIR2 and its homologs are NAD-dependent protein deacetylases. Proc Natl Acad Sci U S A 97, 5807-11. (2000).
6. Imai, S., Armstrong, C. M., Kaeberlein, M. & Guarente,, L. Transcriptional silencing and longevity protein Sir2 is an NAD- dependent histone deacetylase. Nature 403, 795-800 (2000).
7. Smith, J. S. et al. A phylogenetically conserved NAD+-dependent protein deacetylase activity in the Sir2 protein family. Proc Natl Acad Sci U S A 97, 6658-63. (2000).
8. Tanner, K. G., Landry, J., Sternglanz, R. & Denu, J. M. Silent information regulator 2 family of NAD- dependent histone/protein deacetylases generates a unique product, 1-O-acetyl-ADP-ribose. Proc Natl Acad Sci US A 97, 14178-82. (2000).
9. Tanny, J. C., Dowd, G. J., Huang, J., Hilz, H. & Moazed, D. An enzymatic activity in the yeast Sir2 protein that is essential for gene silencing. Cell 99, 735-45. (1999).
10. Guarente, L. Sir2 links chromatin silencing, metabolism, and aging. Genes Dev 14, 1021-6. (2000).
11. Tissenbaum, H. A. & Guarente, L. Increased dosage of a sir-2 gene extends lifespan in Caenorhabditis elegans. Nature 410,227-30. (2001).
12. Rogina, B., Helfand, S. L. & Frankel, S. Longevity regulation by Drosophila Rpd3 deacetylase and caloric restriction. Science 298, 1745. (2002).
13. Jiang, J. C., Jaruga, E., Repnevskaya, M. V. & Jazwinski, S. M. An intervention resembling caloric restriction prolongs life span and retards aging in yeast. Faseb J 14, 2135-7. (2000).
14. Kenyon, C. A conserved regulatory mechanism for aging. Cell 105, 165-168 (2001).
15. Masoro, E. J. Caloric restriction and aging: an update. Exp Gerontol 35, 299-305. (2000).
16. Koubova, J. & Guarente, L. How does calorie restriction work? Genes Dev 17, 313-21 (2003).
17. Sinclair, D. A. Paradigms and pitfalls of yeast longevity research. Mech Ageing Dev 123, 857-67 (2002).
18. Kaeberlein, M., McVey, M. & Guarente, L. The SIR2/3/4 complex and SIR2 alone promote longevity in Saccharomyces cerevisiae by two different mechanisms. Genes Dev 13, 2570-80. (1999).
19. Chippindale, A. K., Leroi, Armand M., Kim, Sung B., and Rose, Michael R. Phenotypic plasticity and selection in Drosophila life-history evolution. Journal of Evolutionary Biology 6, 171-193 (1993).
20. Chapman, T. & Partridge, L. Female fitness in Drosophila melanogaster: an interaction between the effect of nutrition and of encounter rate with males. Proc R Soc Lond B Biol Sci 263, 755-9 (1996).
21. Walker, D. W., McColl, G., Jenkins, N. L., Harris, J. & Lithgow, G. J. Evolution of lifespan in C. elegans. Nature 405,296-7 (2000).
22. Marden, J. H., Rogina, B., Montooth, K. L. & Helfand, S. L. Conditional tradeoffs between aging and organismal performance of Indy long-lived mutant flies. Proc Natl Acad Sci U S A 100, 3369-73 (2003).
23. Schmid-Hempel, P. On the evolutionary ecology of host-parasite interaction: addressing the question with regard to bumblebees and their parasites. Naturwissenschaften 88, 147-58 (2001).

24. Ebert, D. & Bull, J. J. Challenging the trade-off model for the evolution of virulence: is virulence management feasible? Trends Microbiol 11, 15-20 (2003).
25. Soleas, G. J., Diamandis, E. P. & Goldberg, D. M. Resveratrol: a molecule whose time has come? And gone? Clin Biochem 30, 91-113 (1997).
26. Bitterman, K. J., Anderson, R. M., Cohen, H. Y., Latorre-Esteves, M. & Sinclair, D. A. Inhibition of silencing and accelerated aging by nicotinamide, a putative negative regulator of yeast sir2 and human SIRT1. JBiol Chem 277, 45099-107. (2002).
27. Edgecomb, R. S., Harth, C. E. & Schneiderman, A. M. Regulation of feeding behavior in adult Drosophila melanogaster varies with feeding regime and nutritional state. J Exp Biol 197, 215-35 (1994).

**Example 15: Identification of additional activators and inhibitors or sirtuins**

The following high-throughput screening protocol was used to identify additional small molecule sirtuin activators and inhibitors from an ICCB library.

The following wells were designated for control reactions: a) with enzyme; DMSO blank, b) with enzyme; with resveratrol (50 μM) positive control. The reaction mixture contains (final): 0.5 units/reaction SIRT1 deacetylase (BIOMOL); 200 μM $NAD^+$; 5 μM Fluor de Lys-SIRT1 substrate (BIOMOL); buffer (25 mM Tris/Cl, pH 8.0, 137 mM NaCl, 2.7 mM KCl, 1 mM $MgCl_2$, and 1 mg/ml BSA). In addition, a reaction mixture containing no enzyme was made so that each well receiving compound has a corresponding "no enzyme control" well. Reactions were performed in black 384 well plates (NUNC) in a final volume of 25 μl/ well.

The reactions were started by combining enzyme and substrate in a reaction mixture immediately prior to aliquoting in plates (or substrate only for "no enzyme control" plates). Mixture were aliquoted to plates using Biotek μFill (Biotek Instruments). Control mixtures were manually added to designated wells. A library compound was added at a desired concentration by pin transfer to both "with enzyme" and "no enzyme" plates. Compounds were added in at least triplicate (with enzyme reaction in duplicate and no enzyme controls) at a final concentration of roughly 50 μM. The plates were incubated at 37° C for 30-60 minutes. Then 25 wμl of 1x Developer II (BIOMOL) plus 2 mM nicotinamide were added to all wells to stop the reactions. The reactions were left for at least 30 minutes at 37°C for the signal to develop. The plates were read in a microplate-reading fluorometer capable of excitation at a wavelength in the range of 350-380 nm and detection of emitted light in the range of 440-460 nm. A read time of 0.1 sec per well was used.

The following positive controls were used: resveratrol, resveratrol. 4"-methyl ether (3,5-dihydroxy-4'-methoxy-trans-stilbene, also referred to herein as BML-233, and set forth in Table 10), and pinosylvin, which activated SIRT1 2.2 fold, 2.1 fold and 3.28 fold, respectively. The activators are listed in Table 21 and the inhibitors are listed in Table 22.

**Example 16: SIRT1 deacetylase protects against age-dependent neurodegeneration A. Materials and Methods**

*Protein preparation and western blots*

The mice were sacrificed by intraperitoneal injection of chloral hydrate. Total protein extracts of mouse spinal cord, mouse forebrain, mouse hippocampus or human prefontal cortex were obtained by homogenization in SDS-urea β-mercaptoethanol (0.5% SDS, 8M urea in 7.4 phosphate buffer) or Triton X-100 (10 mM Tris-HCl [pH 7.5], 150 mM NaCl, 1 mM EDTA [pH 8.0], and 1% Triton). The protein concentration was estimated by the Bradford procedure (Bio-Rad Laboratories, Hercules, CA). Proteins were fractionated on 7.5% SDS-PAGE and blotted on a nitrocellulose or PVDF membrane for western blot analysis. Membranes were incubated with antibodies against SIRT1 (07-131, Upstate), α-Tubulin (B512, Sigma), actin (MAB 1501, Chemicon), FAK (C-20, Santa Cruz Biotechnology), Bax (N-20, Santa Cruz), GFP (B-2, Santa Cruz). The western blots were revealed by RENAISSANCE, a Western blot chemiluminescence kit from NEN Life Science (Boston, MA). Quantitations were corrected with levels of actin, α-Tubulin and FAK and performed with the Labscan program (Image Master, 2D software v 3.10, Amersham Pharmacia biotech).

***Culture, transfection and treatment of primary neurons***

Rat cortical/primary neurons were isolated, cultured and transfected with Lipofectamine 2000 according to Nguyen, M. D. et al. (Nat Cell Biol 6, 595-608 (2004)) in a ratio 3 (SIRT1 or SIRT1 H363Y or p53 RNAi): 1 (p25-GFP, WT SOD1 or SOD1G93A or GFP). Treatment of primary cortical neurons with ionomycin (1 μm), hydrogen peroxide (25 μm) or Resveratrol (50 nm to 500 nm) were performed according to Lee, M. S. et al. (Nature 405, 360-4 (2000)).

***Immunofluorescence of primary neurons and human prefontal cortex tissues***

Staining on cells were performed according to Nguyen, M. D. et al. (Nat Cell Biol 6, 595-608 (2004)) with

antibodies against Tubulin (Tuj1; α-Tubulin, B512, Sigma Aldrich), GFP (Molecular Probes), SOD1 (Biodesign), FLAG (M2, Sigma). Staining of human prefontal cortex tissues cord tissues were performed according to Cruz, J. C. et al. (Neuron 40, 471-83 (2003)) with antibodies against SIRT1 (07-131, Upstate) and APP/Aβ (4G8). ***Generation of SOD1$^{G37R}$ transgenic mice and p25 inducible transgenic mice***

Transgenic mice overexpressing SOD1$^{G37R}$ (line 29) (G37R) and p25-CK trangenic have been generated as described previously and have been maintained on a pure C57BL6 background (Cruz, J. C., et al., Neuron 40, 471-83 (2003); Nguyen, M. D. et al., Neuron 30, 135-47 (2001).

***Cannulation and injections***

Double cannulae (Plastic 1) were implanted seven days before the experiments under 1.2 % avertin anesthesia (0.4 ml/mouse), as described previously (Fischer, A., et al., J Neurosci 24, 1962-6 (2004). For resveratrol injection the cannulae were placed into both lateral brain ventricles, AP - 0.5 mm, lateral 1 mm, depth 2 mm. Resveratrol (5μg/μl) or vehicle were injected bilaterally 2-3 x/week (5μg Resveratrol/1μl/mouse) using a microinjector (CMA/Microdialysis) over a 60 s period, so that a volume of 0.5 μl was injected in each side. Resveratrol (25%DMSO/artificial cerebrospinal fluid) was prepared fresh immediately before each injection. For SILT1 lentivirus injection cannulae were placed in the dorsal hippocampus, AP-1.5 mm, lateral 1 mm, depth 2 mm. SIRT1-HA lentivirus (1.5 μl) was injected as described above into the left hippocampus whereas SIRT1-HA lentivirus (1.5 μl) was injected into the right hippocampus of 1 week induced CK-p25 mice. Number of GFP neurons were counted 1 to 2 mm caudal to the injection site. A ratio of neurons control side/neurons SIRT1 side was calculated to quantify variations in % of neurons between both sides.

***Fear Conditioning***

The fear conditiong apparatus (TSE Systems) consisted of two test boxes with defined light and background noise that were connected to a control unit and a PC computer. The experimental protocols were designed and performed using TSE fear conditioning software. Boxl contained a grid to apply the electric foot-shock and was cleaned with 70% ethanol before each training or test session. The second box had no grid and was cleaned with 1% acetic acid before each test This box was used to analyze tonedependent fear memories. Fear conditioning consisted of a single exposure to context (Box 1; 3 min) followed by a foot shock (2s, 0.7 mA, constant current). Context-dependent freezing was measured 24 h later every 10$^{th}$ s over 180 s by two observers in a blind manner and expressed as % of toal number of observations.

***Generation of RNAi***

P53 RNAi sequence were selected based on the criteria proposed by Sui et al. (Sui, G. et al., Proc Natl Acad Sci U S A 99, 5515-20 (2002)). Complementary hair pin sequences were commercially synthesized and cloned into pSilencer 2.0 under promoter U6 (Ambion). Sequence for p53 are base pairs: gga gtc ttc cag tgt gat gat (SEQ ID NO: 32). A random sequence without homology to any known mRNA was used for control RNAi. All RNAi constructs were tested in cell lines and primary neuronal cultures.

***Immunoprecipitation***

Immunoprecipitations were performed according to Nguyen, M. D. et al. (Nat Cell Biol 6, 595-608 (2004)) on 8 forebrains from p25 transgenic mice and wild type mice with a monoclonal antibody directed against p53 (Ab-3) (Calbiochem/Oncogene). Membranes were probed with a home made Ac-p53 Ab and a mouse monoclonal p53 antibody (pAb-240, Abeam).

B. Results

A progressive loss of neurons with age underlies a variety of debilitating neurological disorders including Alzheimer's disease (AD) and amyotrophic lateral sclerosis (ALS), yet few effective treatments are currently available. The SIR2 gene promotes longevity in a variety of organisms and may underlie the health benefits of caloric restriction, a diet that delays aging and neurodegeneration in mammals. We describe herein that a human homologue of *SIR2,* SIRT1, is upregulated in brain tissue from Alzheimer's patients, in mouse models for AD, ALS and in primary neurons challenged with neurotoxic insults. In cell-based models for AD/Tauopathies and ALS, SIRT1 and resveratrol, a SIRT1-activating molecule, both promote neuronal survival. In the p25 transgenic mouse, a model of AD/Tauopathies, resveratrol reduced neurodegeneration in the hippocampus and decreased acetylation of p53, a known SIRT1 substrate. Two downstream p53 effectors that mediate cell death, caspase 3 and Bax, were also attenuated. Furthermore, injection of SIRT1 lentivirus

in the hippocampus of p25 mice prevents massive neurodegeneraton. Thus, SIRT1 provides a unique molecular link between aging and human neurodegenerative disorders and the SIRT1 activation is a promising avenue for therapeutic intervention.

Although neurodegenerative disorders are relatively cell-type specific, many of the underlying pathogenic processes are similar, including protein misfolding, oxidative stress, cytoskeletal abnormalities, disruption of calcium homeostasis, and inflammation, all of which increase during aging (Bossy-Wetzel et al., Nat Med 10 Suppl, S2-9 (2004); Forman, M. S. et al., Nat Med 10, 1055-63 (2004); Selkoe, D. J., Nat Cell Biol 6, 1054-61 (2004)). The existence of related mechanisms underlying neurodegeneration raises the possibility of developing a class of therapeutic interventions that treat a variety of neurological disorders by activating the body's own defenses against age-related deterioration and cell death (Bossy-Wetzel et al., Nat Med 10 Suppl, S2-9 (2004); Foreman, M. S. et al., Nat Med 10, 1055-63 (2004); Selkoe, D. J., Nat Cell Biol 6, 1054-61 (2004)). Studies from yeast identified the evolutionary-conserved $NAD^+$-dependent deacetylase Sir2 as a critical regulator of the aging process (Anderson, R. M. et al., Science 302, 2124-6 (2003); Anderson, R. M. et al., Nature 423, 181-5 (2003); Cohen, H. Y. et al., Science 305, 390-2 (2004); Howitz, K T. et al., Nature 425, 191-6 (2003); Kaeberlein, M. et al., Genes Dev 13, 2570-80 (1999); Imai, S. et al., Nature 403, 795-800 (2000)). An additional copy of the *SIR2* gene extends life span in yeast and metazoans by a process seemingly analogous to caloric restriction (Anderson, R. M. et al., Science 302, 2124-6 (2003); Anderson, R. M. et al., Nature 423, 181-5 (2003); Lin, S. J. et al., Science 289, 2126-8 (2000)), a diet that delays diseases of aging in mammals including neurodegeneration (Cohen, H. Y. et al., Science 305, 390-2 (2004); Howitz, K. T. et al., Nature 425, 191-6 (2003); Brunet, A. et al., Science 303, 2011-5 (2004); Motta, M. C. et al., Cell 116, 551-63 (2004); Langley, E. et al., Embo J 21, 2383-96 (2002); Cohen, H. Y. et al., Mol Cell 13, 627-38 (2004); Luo, J. et al., Cell 107, 137-48 (2001); Vaziri, H. et al., Cell 107, 149-59 (2001)). Mammals possess seven Sir2 homologues (SIRT1-7) whose biological functions remain poorly defined. The SIRT1 gene is believed to provide cell protection during times of cell stress (Cohen, H. Y. et al., Science 305, 390-2 (2004); Brunet, A. et al., Science 303, 2011-5 (2004); Motta, M. C. et al., Cell 116, 551-63 (2004); Langley, E. et al., Embo J 21, 2383-96 (2002); Cohen, H. Y. et al., Mol Cell 13, 627-38 (2004)). Consistent with this, knockdown of the *SIRT1* gene in cultured mouse dorsal roots ganglion sensory neurons abrogates the protective effects of increased $NAD^+$ synthesis on axonal degeneration following acute axotomy (Araki, T., et al., Science 305, 1010-3 (2004)).

We hypothesized that SIRT1 levels may increase as a protective response to neurodegenerative disorders such as AD in human patients, and surveyed SIRT1 levels in normal non-demented post-mortem brain samples (n=9) and postmortem brains from patients with different stages of AD (n=11). For evaluation of human brains, we utilized Braak and Braak classification of progressive neuronal changes in AD (Braak, H. & Break, E., J Neural Transm Suppl 53, 127-40 (1998). Using neurofibrillary tangles and a neuropil thread characteristic distribution pattern, Braak and Braak classification recognizes the following six stages in disease progression: I-II, transentorhinal (clinically silent cases); III-IV, limbic (incipient AD); V-VI, neocortical (fully-developed AD) (Braak, H. & Braak, E., J Neural Transm Suppl 53, 127-40 (1998). As detected by immunoblotting, AD samples exhibited higher levels of SIRT1 protein when compared to controls ($2.55 \pm 0.23$ vs $1.36 \pm 0.27$; P (T<=t) two-tails: 0.004) (Figure 43A), although no consistent correlation was found between levels of SIRT1 and the Braak and Braak stages. The localization of SIRT1 in paraffin-embedded prefontal cortex tissues from three individuals (control #1 and AD #1 and 2) was then determined using indirect immunofluorescence. Consistent with immunoblot data, there was a dramatically higher level of SIRT1 in neurons of gray matter of prefontal cortex from AD samples while the white matter regions of all samples rarely exhibited SIRT1-positive cells (Figure 43B). The areas of intense SIRT1 staining were not confined to neurons adjacent to β-amyloid plaques, a hallmark of AD pathology (AD samples #2 and #3; Fig.1C). Collectively, these results indicate that SIRT1 is upregulated in regions of the brain that are degenerating.

To corroborate these *in vivo* results, we determined levels of SIRT1 in various mouse models for human age-dependent neurodegeneration. Mice expressing a toxic co-activator of cyclin-dependent kinase 5 (CDK5), p25, display massive degeneration of forebrain with features of AD/Tauopathies (Cruz, J. C. et al. Neuron 40, 471-83 (2003)), whereas transgenic mice expressing a mutant form of superoxide dismutase 1 (SOD1G37R), which has been linked to human ALS, exhibit severe motor neuron and axon degeneration in spinal cord (Wong, P. C. et al., Neuron 14, 1105-16 (1995); Gurney, M. E. et al., Science 264, 1772-5 (1994)).

In the forebrains of p25 transgenic mice (n=9) SIRT1 protein levels increased as early as two weeks after p25 induction and persisted throughout the progression of the pathology to 12 weeks (Figure 37A-B). Microarray analysis of brain tissue samples from these mice (n=3) showed that SIRT1 mRNA levels progressively increased as the disease progressed, but the levels of the other Sir2 family members, SIRT2-7, did not change (Figure 37C). In spinal cord of mutant SOD1G37R mice, SIRT1 was only slightly upregulated at 4 months (n=4), a stage with little degeneration but levels of SIRT1 were highly expressed when severe neurodegeneration was evident at 10 to 12 months (n=8) (Nguyen, M. D. et al., Neuron 30, 135-47 (2001)) (Figure 37D-E). Mice expressing a mutant form of Amyloid Precursor Protein (APP) linked to Familial AD (PDAPP-V717F, n=7; 2-12 months) (Games, D. et al., Nature 373, 523-7 (1995)) do not exhibit significant degeneration although they display, in an age-dependent manner, substantial β-amyloid plaques, a hallmark of AD (Games, D. et al., Nature 373, 523-7 (1995)) (Figure 38). These mice showed no significant increase in

SIRT1 in the forebrain (Figure 38). These results indicate that SIRT1 levels correlate with neurodegeneration accompanied by progressive and severe loss of neurons, but not with β-amyloid plaque pathology in the absence of severe degeneration.

Since both p25 and mutant SOD1 trigger disruption of calcium homeostasis and generate oxidative stress (Cruz, J. C. et al., Curr Opin Neurobiol 14, 390-4 (2004); Bruijn, L. I. et al., Annu Rev Neurosci 27, 723-49 (2004)), we tested whether SIRT1 is induced in neurons in response to ionomycin (1 μm), a calcium ionophore, or hydrogen peroxide (25 μm), a free-radical generator. These specific stresses have previously been shown to trigger the deterioration of neuronal morphology and the formation of p25 in cultured neurons (Lee, M. S. et al., Nature 405, 360-4 (2000); Kusakawa, G. et al., J Biol Chem 275, 17166-72 (2000); Nath, R. et al., Biochem Biophys Res Commun 274, 16-21 (2000)). Treatment of primary cortical neurons with either ionomycin or $H_2O_2$ rapidly induced SIRT1 protein expression, and the increased levels lasted up to 1 hour (Figure 37F). Thus, SIRT1 is not only induced in mouse models of neurodegeneration but also in primary cultured neurons under neurotoxic stresses.

To understand the physiological significance of SIRT1 activation in context of neurodegeneration, we first tested the effects of resveratrol, a polyphenolic SIRT1 activating compound (STAC) (Howitz, K. T. et al., Nature 425, 191-6 (2003)), on the viability of primary mouse neurons expressing p25 or mutant SOD1G93A (Patrick, G. N. et al., Nature 402, 615-22 (1999)). Doses of up to 500 nM resveratrol showed no evidence of toxicity to primary neurons transfected with GFP (Figure 39A). Transfection with p25-GFP resulted in a high degree of cell death (50% after 24 hours) (Lee, M. S. et al., Nature 405, 360-4 (2000); Patrick, G. N. et al., Nature 402, 615-22 (1999); Zhang, J., Krishnamurthy et al. J Neurochem 81, 307-13 (2002); Hamdane, M. et al., J Biol Chem 278, 34026-34 (2003)), based on a decreased integrity of neuronal processes (GFP and Tubulin staining), condensed chromatin and disrupted nuclear morphology as shown with DAPI staining (Figure 39B-C). Resveratrol treatment reduced by 45% the extent of cell death caused by p25 (Figure 39B-C) (P(T<=t) two tails: 0.01). Resveratrol also provided 45 % protection against SOD1G93A toxicity (P(T<=t) two tails: 0.01) (Figure 39D-E). These results are in line with a recent report showing that in cultured neurons derived from transgenic mice overexpressing a mutant (109Q) huntingtin, resveratrol suppressed the neurotoxic effects of the mutant protein (Parker, J. A. et al., Nat Genet 37, 349-50 (2005)).

To directly verify the protective role of SIRT1 in neurodegeneration, we transfected primary neurons with p25-GFP or SOD1G93A together with either SIRT1 or SIRT1 lacking catalytic activity (H363Y). Forty eight hours after transfection, approximately 80% of neurons with p25-GFP underwent degeneration (Figure 40B). Overexpression of SIRT1, but not H363Y, rescued the rate of cell death from 80% to 50% (82.7 +/- 7 % vs 53.0 +/-6.2 %; P (T<=t) two-tails: 0.001; 82.7 +/- 7 % vs 78.0 +/- 5.8 %; P (T<=t) two-tails: 0.27) (Figure 40A-B; Figure 41). The morphology of the p25-GFP/SIRT1-overexpressing neurons appeared normal and indistinguishable from control GFP-transfected neurons. This protective effect was not due to an effect of SIRT1 on the stability of p25-GFP because similar levels of p25-GFP were detected in the presence or absence of SIRT1 overexpression (Figure 40C).

We also sought to determine whether SIRT1 overexpression protected against mutant SOD1-induced neurotoxicty. Approximately 60% (62.3 +/- 8.9 %) of primary neurons transfected with mutant SOD1G93A but not wild-type SOD1 exhibited cytoskeletal disruption and SOD1 aggregates, two hallmarks of ALS-linked SOD1 toxicity (Figure 40D-E). The overexpression of SIRT1, but not H363Y, also protected against SOD1G93A toxicity with a 50% reduction of neurons displaying the degenerative phenotype (62.3 +/- 8.9 % vs 30.1 +/- 10.2 %; P (T<=t) two-tails: 0.001; 62.3 +/- 8.9 % vs 78.0 +/- 11.0 %; P (T<=t) two-tails: 0.33) (Figure 40D-E). Together, these results indicate that increased levels of SIRT1 in primary neurons confer potent protection against neurotoxicity induced by p25 or mutant SOD1. The observation that the H363Y mutant did not confer protection demonstrates that the deacetylase activity of SIRT1 is required for neuroprotection.

To test the neuroprotective effects of resveratrol *in vivo,* resveratrol (Resv) or vehicle (Veh) were introduced by ICV (see Fischer, A. et al., J Neurosci 24, 962-6 (2004)) in 2 week-induced p25 mice for 3 weeks at a dose of 5 μg/μl - 1μl injected every 2 to 3 days (Veh-treated animals, n=5; Resv-treated animals, n=9) (Figure 44A). After 5 weeks of p25 induction, cell death and neurodegeneration were evident in the hippocampus of the vehicle-treated animals, consistent with prior observations (Cruz, J. C. et al., Neuron 40, 471-83 (2003)). In contrast, administration of resveratrol reduced neurodegeneration in CA1 and CA3 regions of the hippocampus, as revealed by lower levels of two apoptotic markers, Bax and activated caspase 3, and a marker of astrogliosis, GFAP (Figure 44B-D, F; Figure 47). GFP immunostaining, which labels p25-GFP-expressing neurons, was more robust in the hippocampus of resveratrol-treated animals, suggesting the neurons were better able to tolerate and survive p25 expression (Figure 44D). At higher magnification, CA1 neurons from resveratrol-treated brains exhibited a better preservation of dendritic morphology (Figure 44E). We previously reported that by 5-6 weeks of induction, p25 mice have dramatically decreased associative learning capabilities as revealed by contextual fear conditioning paradigm (Fischer et al., Neuron in press). Together, these results show that resveratrol provides neuroprotection in an animal model of CNS degeneration that features massive neuronal loss and tau pathologies (Figure 44E-F).

To gain insights into the mechanism by which resveratrol confers neuroprotection *in vivo,* we hypothesized that p53 plays a key role in mediating neuroprotection for the following reasons. First, p25/Cdk5 is known to phosphorylate

p53 and upregulate its transcriptional activity (Zhang, J., et al., J Neurochem 81, 307-13 (2002)). Second, we found that p53 protein levels are significantly increased in p25 transgenic mice (Figure 45A). Third, this increase is accompanied by an increase in the acetylation status of lysine 382 of p53 (Figure 45B), a modification known to stabilize p53 and potentiate its apoptotic function of p53 (Langley, E. et al., Embo J 21, 2383-96 (2002); Luo, J. et al., Cell 107, 137-48 (2001); Vaziri, H. et al., Cell 107,149-59 (2001)). Fourth, lysine 382 of p53 (K382-p53) is a well-characterized SIRT1 target and activation of SIRT1 by resveratrol would be consistent with the effects we observed *in vivo.*

We first tested whether p53 contributes to p25-induced cell death by co-transfecting cortical neurons with p25-GFP together with either a control siRNA vector or p53 siRNA vector. Knockdown of p53 provided a 25% increase in cell survival (Figure 45C), which is similar in extent to what we observed for resveratrol-treated neurons (see Figure 39) and the acetylation status of K382-p53 was also reduced by resveratrol treatment (Figure 45D) (Langley, E. et al., Embo J 21, 2383-95 (2002); Vaziri, H. et al., Cell 107, 149-59 (2001)). Next, we asked whether resveratrol had a similar effect *in vivo.* As shown in Figure 45D, the acetylation status of K382-p53 was lower in resveratrol-treated hippocampal tissue of p25 transgenic mice, relative to vehicle. Overall p53 levels were also decreased by resveratrol-treatment, which is consistent with the deacetylated form being less stable (Luo, J. et al., Cell 107, 137-48 (2001)).

Further, to ascertain a role for SIRT 1 in neuroprotection *in vivo,* we introduced lentivirus carrying HA-tagged SIRT1 or control virus into the hippocampus of p25 mice (n=4) by stereotaxic injection as described in Methods. Briefly, 2 week induced p25 transgenic mice were subjected to a single injection of control or SIRT1 expressing virus in each side of the brain. Mice were sacrificed 3 weeks after the viral injections. GFP immunofluorescence staining showed that GFP positive p25 expressing neurons are more prominent in the CA1 regions receiving the SIRT1 virus compared to those receiving the control virus (Figure 46A-F), indicating that they can tolerate higher levels of p25, as seen in resveratrol treated animals (Figure 44). In general, the SIRT1 virus injected side exhibited 38% + / - 13% higher number of neurons than the control virus injected side, and the neurons were morphologically healthier. At higher magnification, it was clear that the surviving neurons expressed SIRT1 as detected by co-immunostaining with HA and GFP antibodies (Figure 46G-I). These results provide futher evidence that that resveratrol’s protective effects are due to SIRT1 activation and demonstrate a neuroprotective role of SIRT1 *in vivo.*

Together, these results show that it is possible to slow neurodegeneration with resveratrol, a SIRT1-activating molecule, and by expression of SIRT1. Also provided is evidence that the neuroprotective effect is due, at least in part, to deacetylation of K382-p53 (Figure 45). We do not rule out the possibility that other known substrates of SIRT1 are involved, such as Ku70, a protein that sequesters the apoptotic protein Bax from mitochondria (Brunet, A. et al., Science 303, 2011-5 (2004); Cohen, H. Y. et al., Mol Cell 13, 627-38 (2004)). Resveratrol may also stimulate the deacetylation of FOXO3/4 transcription factors, thereby enhancing gene expression of anti-oxidative molecules and upregulating DNA repair (Brunet, A. et al., Science 303, 2011-5 (2004); Nguyen, M. D. et al., Cell Death Differ 9, 1294-306 (2002); Smith, P. D. et al., Cell Cycle 3, 289-91 (2004)).

SIRT1 is thought to be a key regulator of an evolutionarily conserved pathway that allows organisms cope with and survival adversity. Consistent with this, yeast Sir2 and mammalian SIRT1 are upregulated by various biological stresses including caloric restriction, the diet that prevents numerous diseases of aging in mammals (Bordone, L. & Guarente, L., Nat Rev Mol Cell Biol 6, 298-305 (2005); Lombard, D. B. et al., Cell 120, 497-512 (2005); Lamming, D. W. et al., Mol Microbiol 53, 1003-9 (2004)). As described herein, we show for the first time the ability of SIRT1-activating molecules to prevent an age-dependent neurodegenerative diseases, and predict that such molecules will prove efficacious in a variety of other diseases associated with aging. Interestingly, the SIRT1 gene resides in a locus on chromosome 10 that is associated with familial AD (WIPO, international publication WO 2005/004815 A2). It is possible that mutations or polymorphisms in SIRT1 affect the susceptibility of individuals to neurodegeneration.

## Claims

1. A method for determining whether a subject has a neurodegenerative disease comprising:

   determining the level or activity of a sirtuin protein in a biological sample obtained from a subject, wherein higher level or activity of the sirtuin protein in the biological sample of the subject relative to a control level indicates that the subject has a neurodegenerative disease, wherein the sirtuin protein is SIRT1.

2. The method of claim 1, wherein the biological sample is a cell sample.

3. The method of claim 2, wherein the cell sample is a brain sample.

4. The method of claim 2, wherein the cell sample is a blood sample.

5. The method of claim 2, wherein the cell sample is a spinal cord sample.

6. The method of any one of claims 1-5, wherein the level of the sirtuin protein in the biological sample is determined using an antibody.

**7.** The method of any one of claims 1-5, wherein the activity of the sirtuin protein in the biological sample is measured by determining deacetylation activity of the sirtuin protein.

**8.** The method of any one of claims 1-7, wherein the control level corresponds to the level or activity of a sirtuin protein in a biological sample from an individual who does not have a neurodegenerative disease.

**9.** The method of any one of claims 1-7, wherein the control level corresponds to the average of the level or activity of a sirtuin protein in biological samples from 2 or more individuals who do not have a neurodegenerative disease.

**10.** The method of any one of claims 1-9, wherein the level or activity of the sirtuin protein is determined once.

**11.** The method of any one of claims 1-9, wherein the level or activity of the sirtuin protein is determined more than once.

**12.** The method of any one of claims 1-11, wherein the neurodegenerative disease is selected from the group consisting of: Alzheimer's disease, amyotrophic lateral sclerosis (ALS), a spongiform encephalopathy, dementia (including Lewy Body disease, mild cognitive impairment (MCI), Primary Senile Degenerative Dementia, Alzheimer Type Senile Dementia and Alzheimer Type Dementia), Parkinsonian disorders (including Parkinson's disease, Lewy Body disease and Parkinsonism-linked to chromosome 17 (FTDP-17)), Progressive Supranuclear Palsy (also known as Steele-Richardson-Olszewski Syndrome or Disease, Progressive Supranuclear Ophthalmoplegia), Pick's disease and corticobasal degeneration.

**13.** The method of claim 12, wherein the neurodegenerative disease is Alzheimer's disease.

**14.** The method of claim 12, wherein the neurodegenerative disease is amyotrophic lateral sclerosis (ALS).

**15.** The method of claim 11, wherein a change in the level of protein or activity of the sirtuin protein within the two samples indicates that the neurodegenerative disease is progressing in the subj ect.

## Patentansprüche

**1.** Verfahren zum Bestimmen, ob ein Individuum eine neurodegenerative Erkrankung aufweist, umfassend:

Bestimmen der Menge oder Aktivität eines Sirtuin-Proteins in einer von einem Individuum erhaltenen biologischen Probe, wobei eine höhere Menge oder Aktivität des Sirtuin-Proteins in der biologischen Probe des Individuums relativ zu einer Kontrollmenge anzeigt, dass das Individuum eine neurodegenerative Erkrankung aufweist, wobei das Sirtuin-Protein SIRT1 ist.

**2.** Verfahren gemäß Anspruch 1, wobei die biologische Probe eine Zellprobe ist.

**3.** Verfahren gemäß Anspruch 2, wobei die Zellprobe eine Gehirnprobe ist.

**4.** Verfahren gemäß Anspruch 2, wobei die Zellprobe eine Blutprobe ist.

**5.** Verfahren gemäß Anspruch 2, wobei die Zellprobe eine Rückenmarksprobe ist.

**6.** Verfahren gemäß einem der Ansprüche 1-5, wobei die Menge des Sirtuin-Proteins in der biologischen Probe unter Verwendung eines Antikörpers bestimmt wird.

**7.** Verfahren gemäß einem der Ansprüche 1-5, wobei die Aktivität des Sirtuin-Proteins in der biologischen Probe durch Bestimmen der Deacetylierungsaktivität des Sirtuin-Proteins gemessen wird.

**8.** Verfahren gemäß einem der Ansprüche 1-7, wobei die Kontrollmenge der Menge oder Aktivität eines Sirtuin-Proteins in einer biologischen Probe von einem Individuum entspricht, das keine neurodegenerative Erkrankung aufweist.

**9.** Verfahren gemäß einem der Ansprüche 1-7, wobei die Kontrollmenge dem Durchschnittswert der Menge oder Aktivität eines Sirtuin-Proteins in biologischen Proben von 2 oder mehr Individuen entspricht, die keine neurodegenerative Erkrankung aufweisen.

**10.** Verfahren gemäß einem der Ansprüche 1-9, wobei die Menge oder Aktivität des Sirtuin-Proteins einmal bestimmt wird.

**11.** Verfahren gemäß einem der Ansprüche 1-9, wobei die Menge oder Aktivität des Sirtuin-Proteins mehr als einmal bestimmt wird.

**12.** Verfahren gemäß einem der Ansprüche 1-11, wobei die neurodegenerative Erkrankung ausgewählt ist aus der Gruppe, bestehend aus: Alzheimer-Krankheit, amyotropher lateraler Sklerose (ALS), einer spongiformen Enzephalopathie, Demenz (einschließlich Lewy-Körperchen-Krankheit, milder kognitiver Beeinträchtigung (MCI), primär seniler degenerativer Demenz, seniler Demenz vom Alzheimer-Typ und Demenz vom Alzheimer-Typ), Parkinson-Störungen (einschließlich Parkinson-Krankheit, Lewy-Körperchen-Krankheit und Parkinsonismus, gekoppelt an Chromosom 17 (FTDP-17)), progressiver supranukleärer Blicklähmung (auch bekannt als Steele-Richardson-Olszewski-Syndrom oder -Erkrankung, progressiver supranukleärer Ophthalmoplegie), Pick-Krankheit und kortikobasaler Degeneration.

**13.** Verfahren gemäß Anspruch 12, wobei die neurodegenerative Erkrankung Alzheimer-Krankheit ist.

**14.** Verfahren gemäß Anspruch 12, wobei die neurodegenerative Erkrankung amyotrophe laterale Sklerose (ALS) ist.

**15.** Verfahren gemäß Anspruch 11, wobei eine Veränderung in der Menge an Protein oder Aktivität des Sirtuin-Proteins innerhalb der beiden Proben anzeigt, dass die neurodegenerative Erkrankung in dem Individuum fortschreitet.

## Revendications

**1.** Méthode pour déterminer si un sujet a une maladie neurodégénérative, comprenant:

déterminer le taux ou l'activité d'une protéine sirtuine dans un échantillon biologique obtenu à partir d'un sujet, un taux supérieur ou une activité supérieure de la protéine sirtuine dans l'échantillon biologique du sujet par rapport à un taux témoin indiquant que le sujet a une maladie neurodégénérative, la protéine sirtuine étant SIRT1.

**2.** Méthode selon la revendication 1, dans laquelle l'échantillon biologique est un échantillon cellulaire.

**3.** Méthode selon la revendication 2, dans laquelle l'échantillon cellulaire est un échantillon de cerveau.

**4.** Méthode selon la revendication 2, dans laquelle l'échantillon cellulaire est un échantillon sanguin.

**5.** Méthode selon la revendication 2, dans laquelle l'échantillon cellulaire est un échantillon de moelle épinière.

**6.** Méthode selon l'une quelconque des revendications 1 à 5, dans laquelle le taux de la protéine sirtuine dans l'échantillon biologique est déterminé à l'aide d'un anticorps.

**7.** Méthode selon l'une quelconque des revendications 1 à 5, dans laquelle l'activité de la protéine sirtuine dans l'échantillon biologique est mesurée par détermination de l'activité désacétylation de la protéine sirtuine.

**8.** Méthode selon l'une quelconque des revendications 1 à 7, dans laquelle le taux témoin correspond au taux ou à l'activité d'une protéine sirtuine dans un échantillon biologique provenant d'un individu qui n'a pas une maladie neurodégénérative.

**9.** Méthode selon l'une quelconque des revendications 1 à 7, dans laquelle le taux témoin correspond à la moyenne du taux ou de l'activité d'une protéine sirtuine dans des échantillons biologiques provenant d'au moins 2 individus qui n'ont pas une maladie neurodégénérative.

**10.** Méthode selon l'une quelconque des revendications 1 à 9, dans laquelle le taux ou l'activité de la protéine sirtuine est déterminé une fois.

**11.** Méthode selon l'une quelconque des revendications 1 à 9, dans laquelle le taux ou l'activité de la protéine sirtuine est déterminé plus d'une fois.

**12.** Méthode selon l'une quelconque des revendications 1 à 11, dans laquelle la maladie neurodégénérative est choisie dans le groupe consistant en : la maladie d'Alzheimer, la sclérose latérale amyotrophique (SLA), une encéphalopathie spongiforme, la démence (y compris la maladie à corps de Lewy, le trouble cognitif léger (MCI), la démence sénile dégénérative primaire, la démence sénile de type Alzheimer et la démence de type Alzheimer), les troubles parkinsoniens (y compris la maladie de Parkinson, la maladie à corps de Lewy et le parkinsonisme lié au chromosome 17 (FTDP-17)), la paralysie supranucléaire progressive (également connue comme syndrome ou maladie de Steele-Richardson-Olszewski, ophtalmoplégie supranucléaire progressive), la maladie de Pick et la dégénérescence cortico-basale.

**13.** Méthode selon la revendication 12, dans laquelle la maladie neurodégénérative est la maladie d'Alzheimer.

**14.** Méthode selon la revendication 12, dans laquelle la maladie neurodégénérative est la sclérose latérale amyotrophique (SLA).

**15.** Méthode selon la revendication 11, dans laquelle un changement du taux de protéine ou de l'activité de la protéine sirtuine dans les deux échantillons indique que la maladie neurodégénérative progresse chez le sujet.

Fig. 1

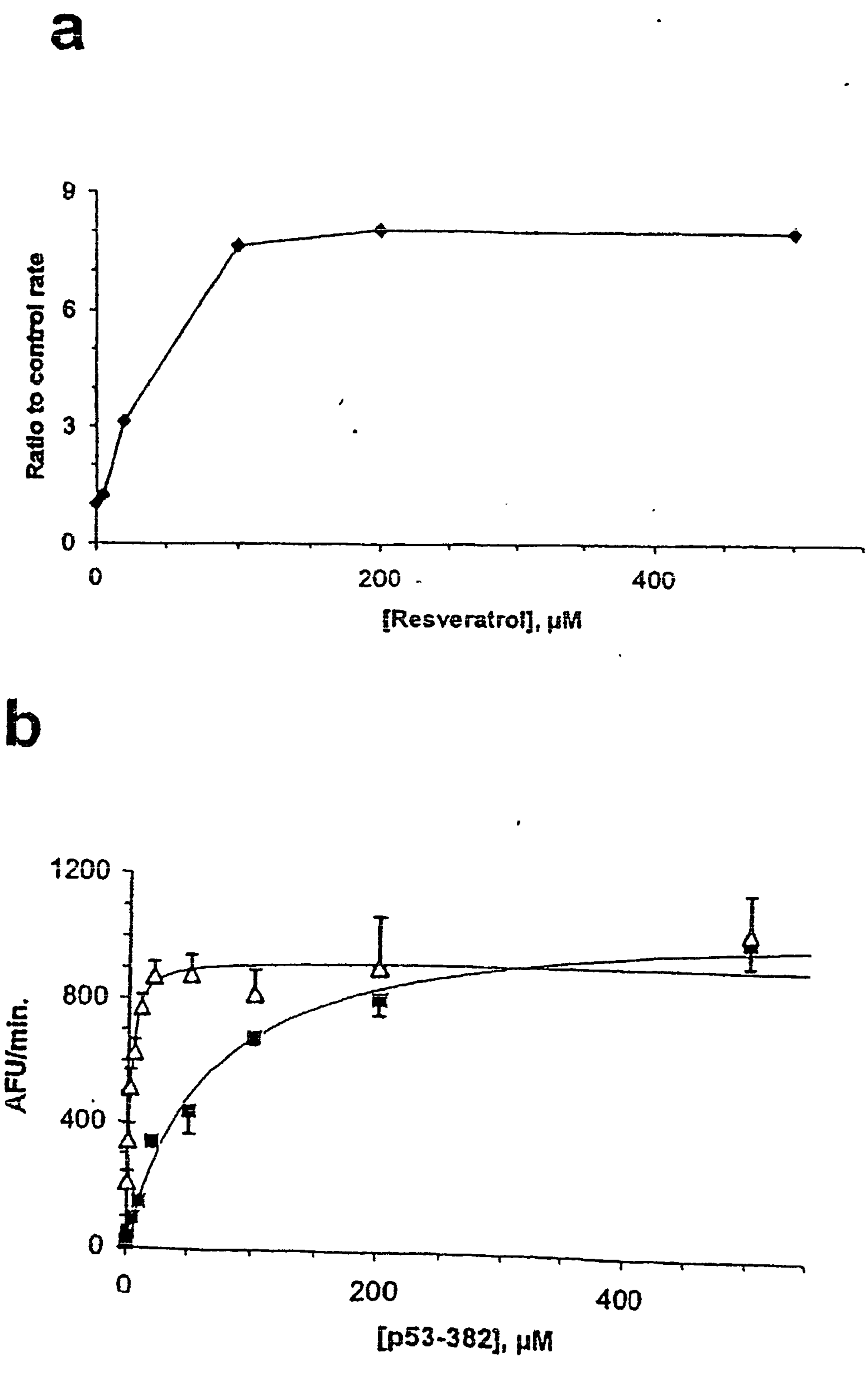
a
9
6
3
0
Ratio to control rate
0
200
400
[Resveratrol], µM
b
1200
800
400
0
AFU/min.
0
200
400
[p53-382], µM

Fig. 1

c

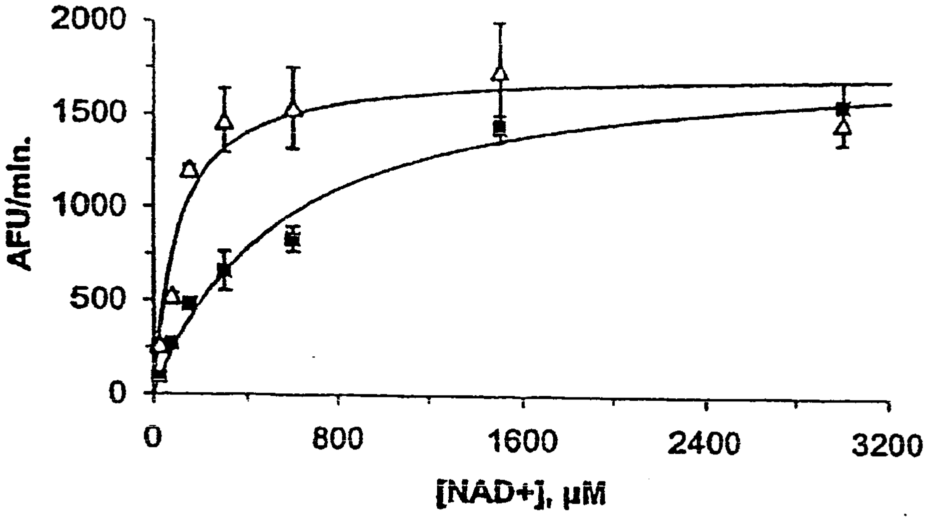

AFU/min.
2000
1500
1000
500
0
0
800
1600
2400
3200
[NAD+], μM


d

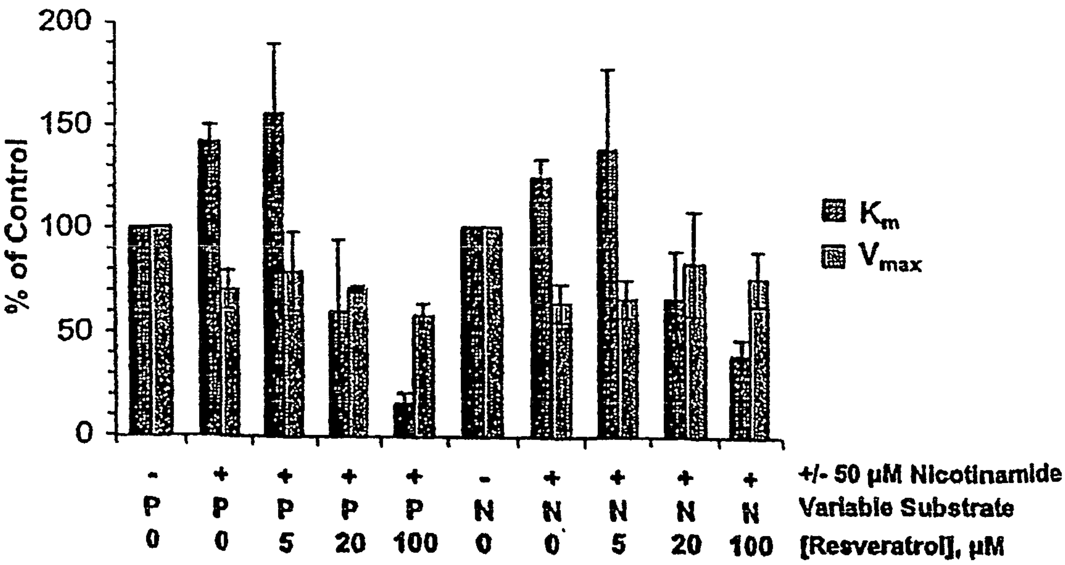

% of Control
200
150
100
50
0
Km
Vmax
- + + + + - + + + +
+/- 50 μM Nicotinamide
P P P P P N N N N N
Variable Substrate
0 0 5 20 100 0 0 5 20 100
[Resveratrol], μM

# Figure 2

a

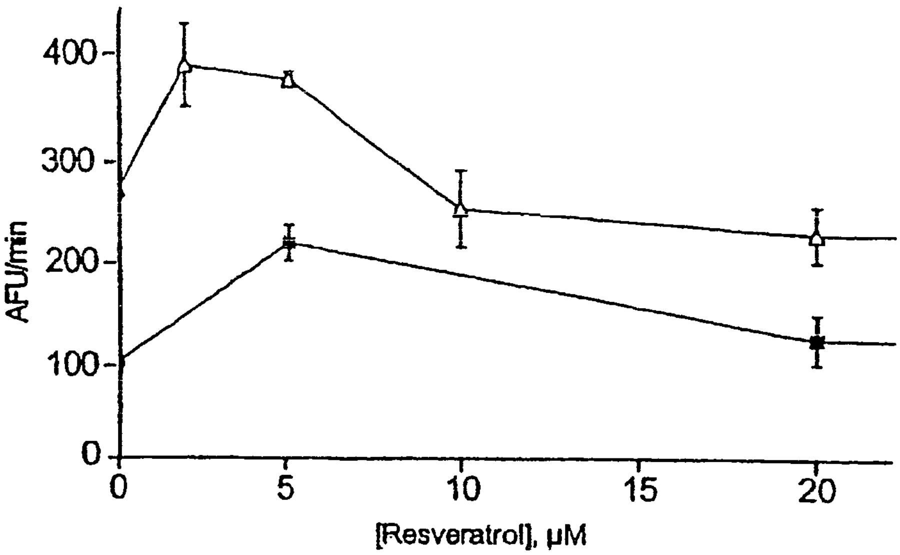

400
300
200
100
0
AFU/min
0
5
10
15
20
[Resveratrol], µM


b

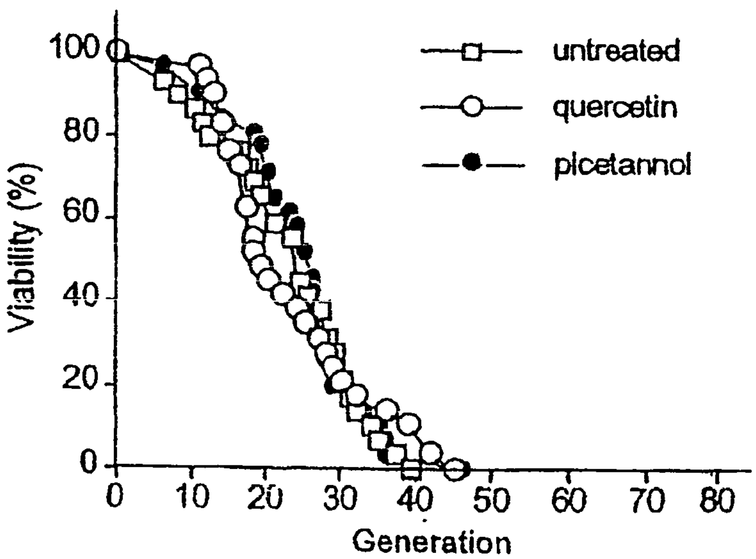

100
80
60
40
20
0
Viability (%)
0
10
20
30
40
50
60
70
80
Generation
untreated
quercetin
picetannol

# Figure 2

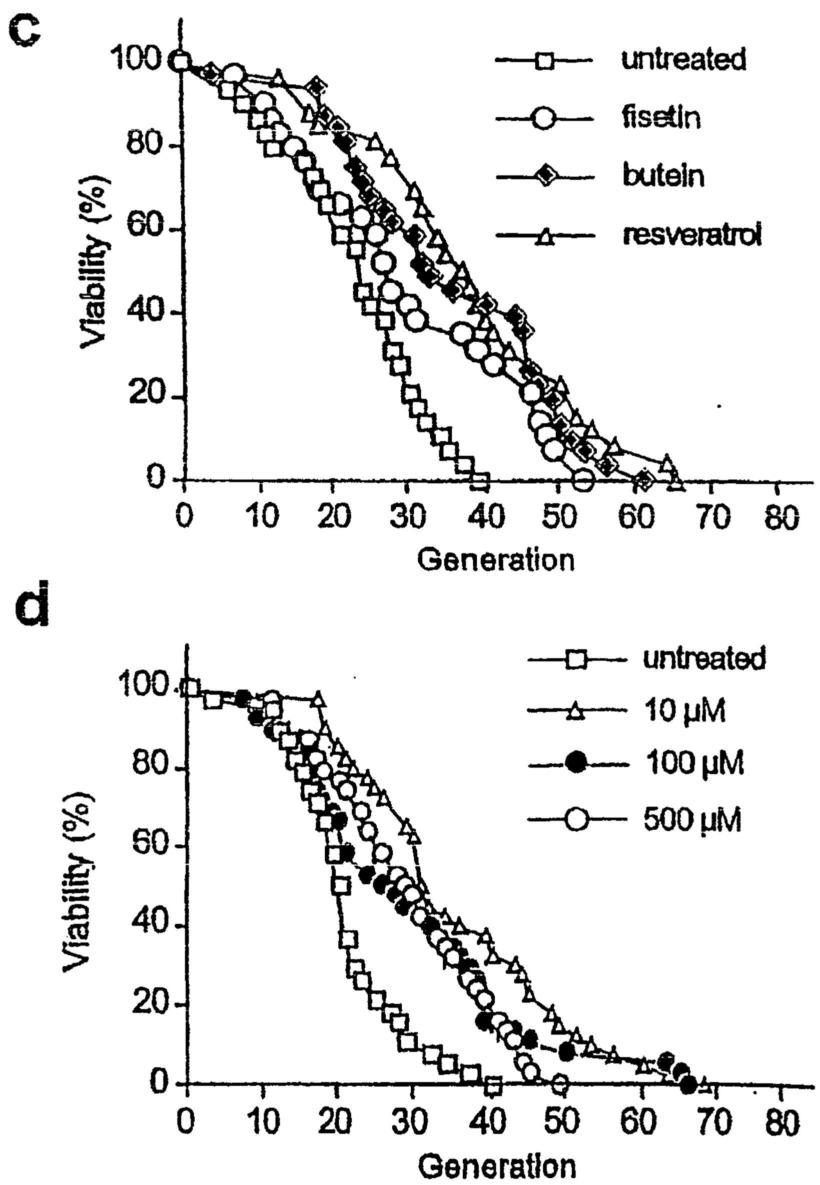
c
100
80
60
40
20
0
Viability (%)
0
10
20
30
40
50
60
70
80
Generation
untreated
fisetin
butein
resveratrol
d
100
80
60
40
20
0
Viability (%)
0
10
20
30
40
50
60
70
80
Generation
untreated
10 μM
100 μM
500 μM

Figure 3

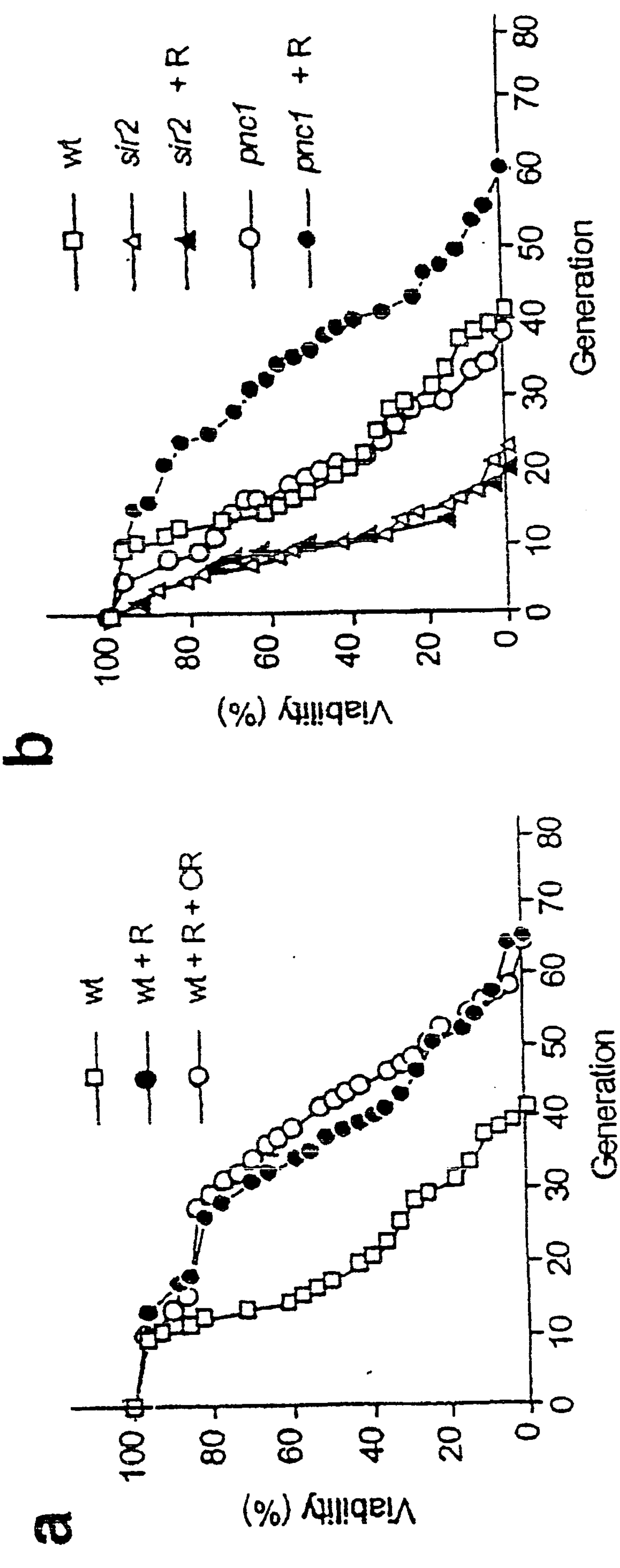
a
wt
wt + R
wt + R + CR
Viability (%)
Generation
b
wt
sir2
sir2 + R
pnc1
pnc1 + R
Viability (%)
Generation

Figure 3

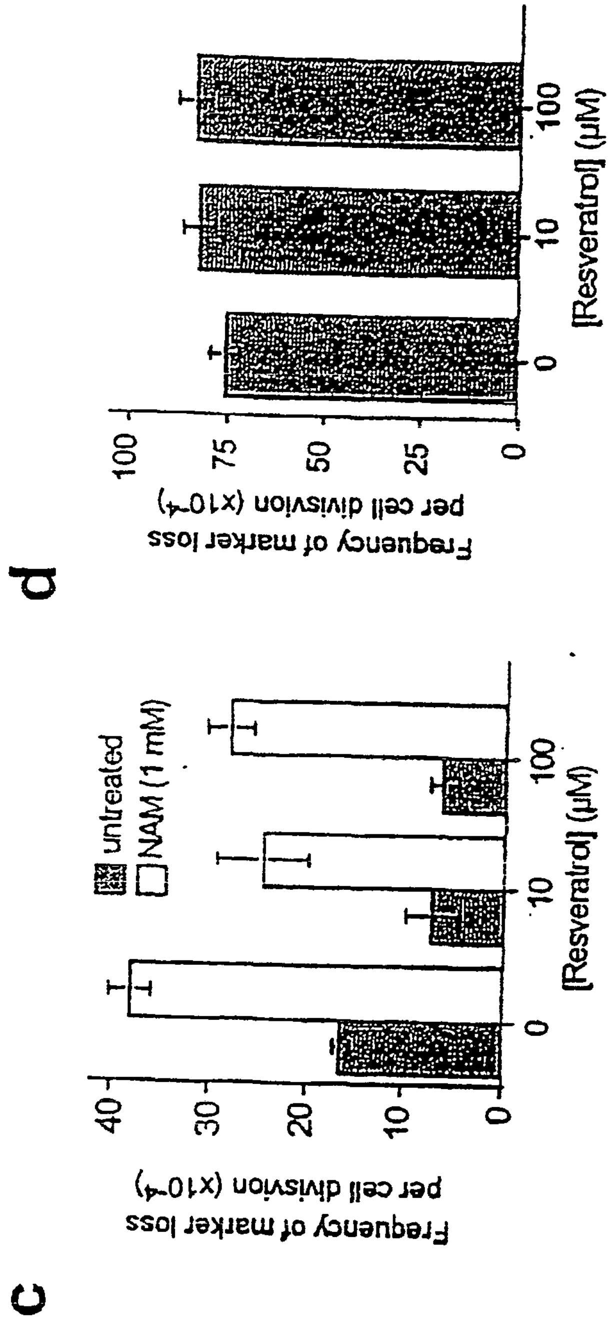
c
Frequency of marker loss per cell division (x10^-4)
40
30
20
10
0
untreated
NAM (1 mM)
0
10
100
[Resveratrol] (μM)
d
Frequency of marker loss per cell division (x10^-4)
100
75
50
25
0
0
10
100
[Resveratrol] (μM)

Figure 3

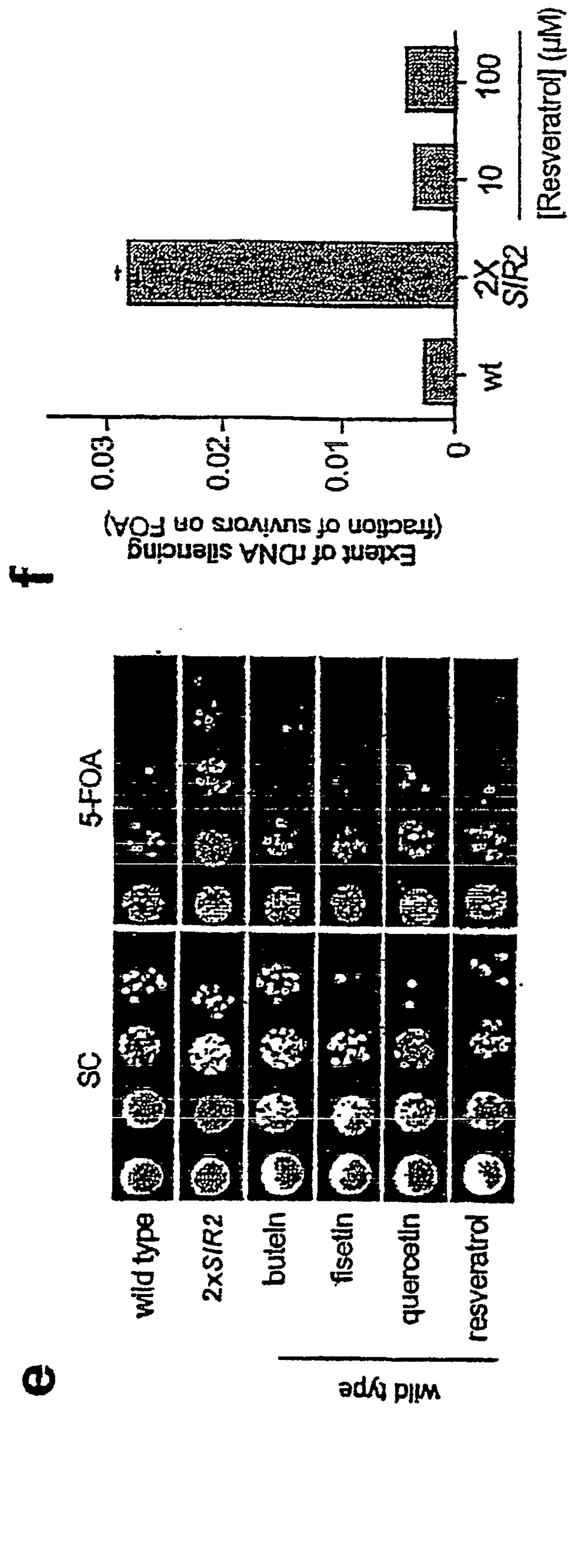
e
wild type
wild type
2xSIR2
butein
fisetin
quercetin
resveratrol
SC
5-FOA
f
Extent of rDNA silencing
(fraction of suvivors on FOA)
0.03
0.02
0.01
0
wt
2X SIR2
10
100
[Resveratrol] (μM)

# Figure 4

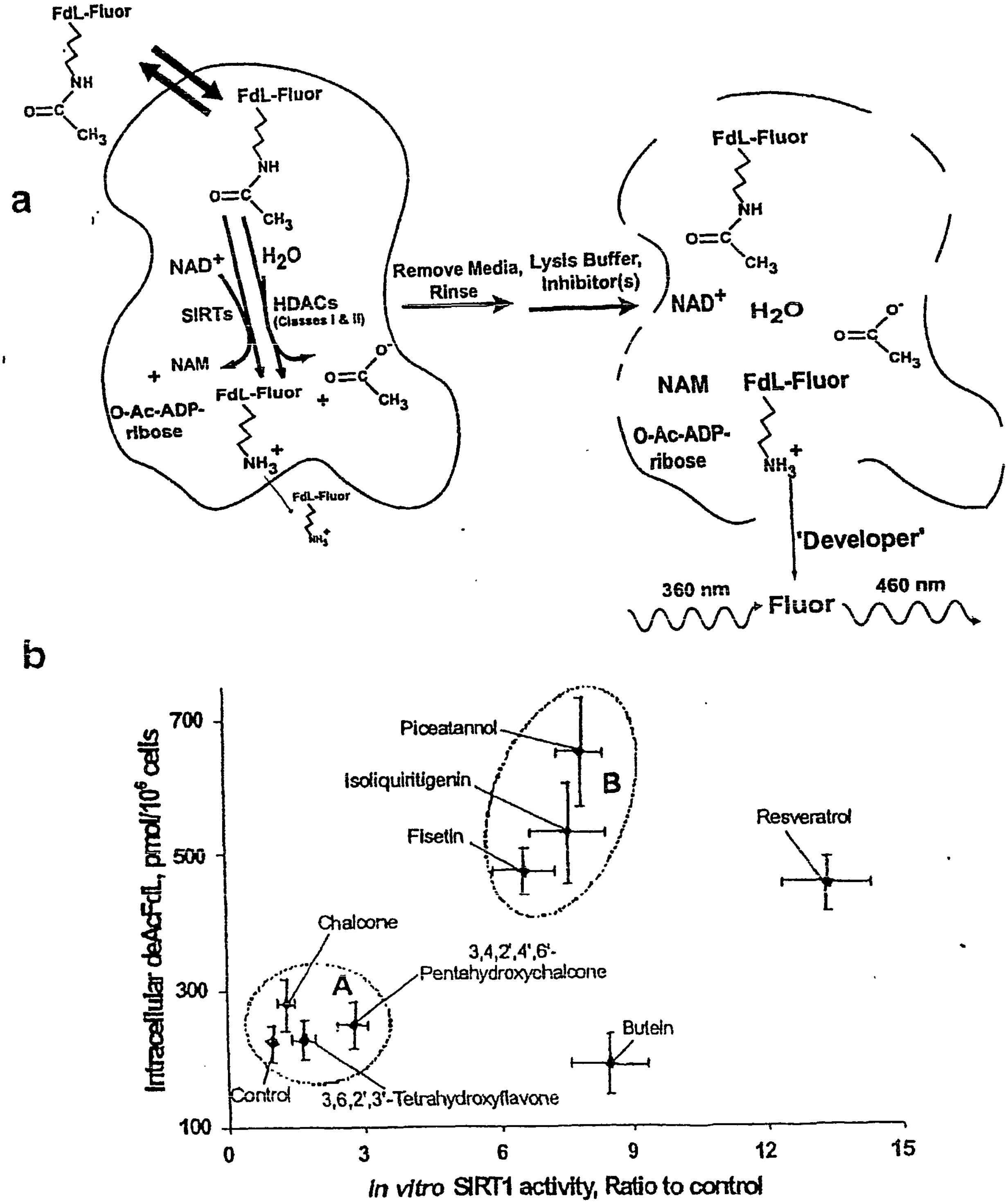
a
FdL-Fluor
NAD+
H2O
SIRTs
HDACs
(Classes I & II)
NAM
O-Ac-ADP-ribose
Remove Media, Rinse
Lysis Buffer, Inhibitor(s)
'Developer'
360 nm
Fluor
460 nm
b
Intracellular deAcFdL, pmol/10^6 cells
in vitro SIRT1 activity, Ratio to control
100
300
500
700
0
3
6
9
12
15
Piceatannol
Isoliquiritigenin
Fisetin
B
Resveratrol
Chalcone
3,4,2',4',6'-Pentahydroxychalcone
A
Butein
Control
3,6,2',3'-Tetrahydroxyflavone

Figure 4

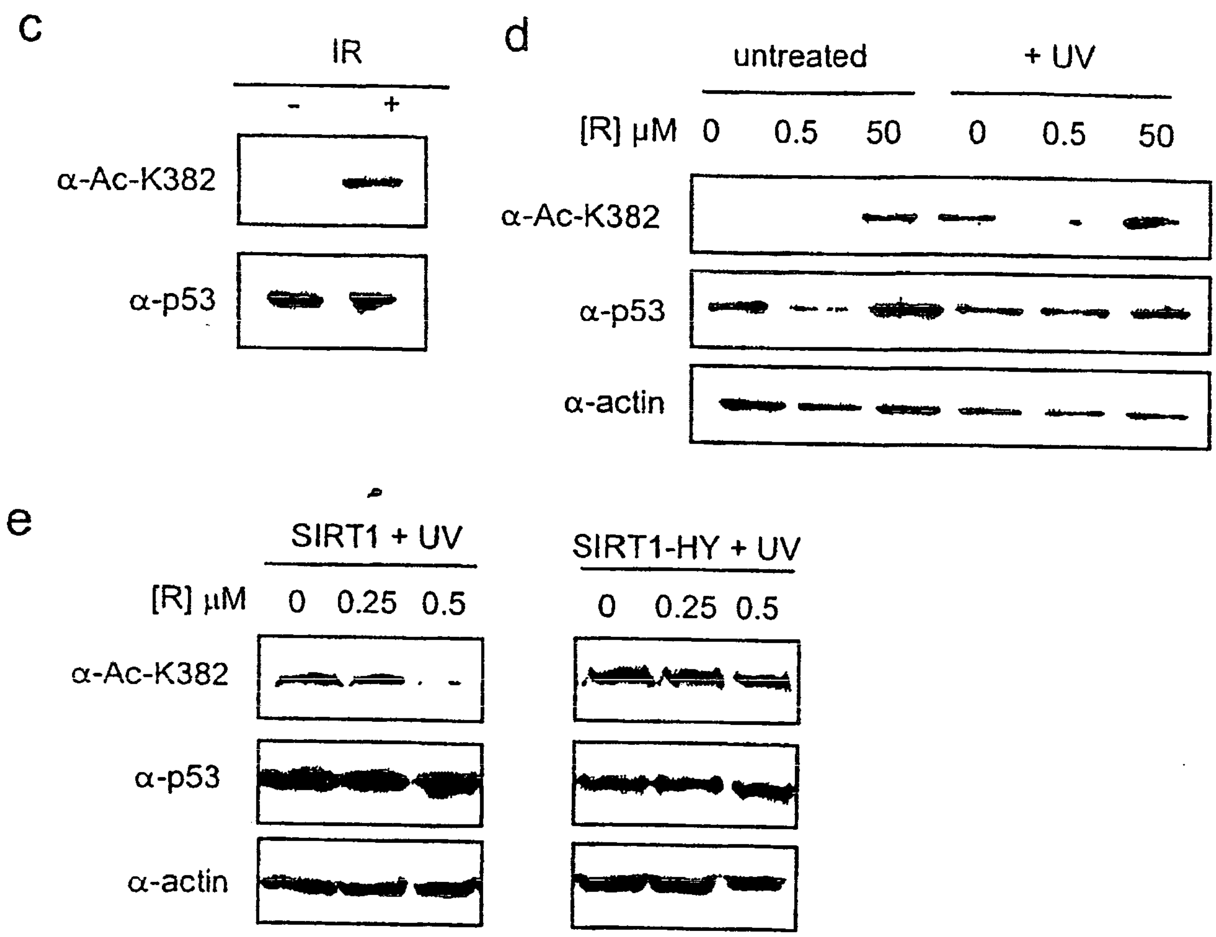
c
IR
-
+
α-Ac-K382
α-p53
d
untreated
+ UV
[R] μM
0
0.5
50
0
0.5
50
α-Ac-K382
α-p53
α-actin
e
SIRT1 + UV
SIRT1-HY + UV
[R] μM
0
0.25
0.5
0
0.25
0.5
α-Ac-K382
α-p53
α-actin

Figure 5

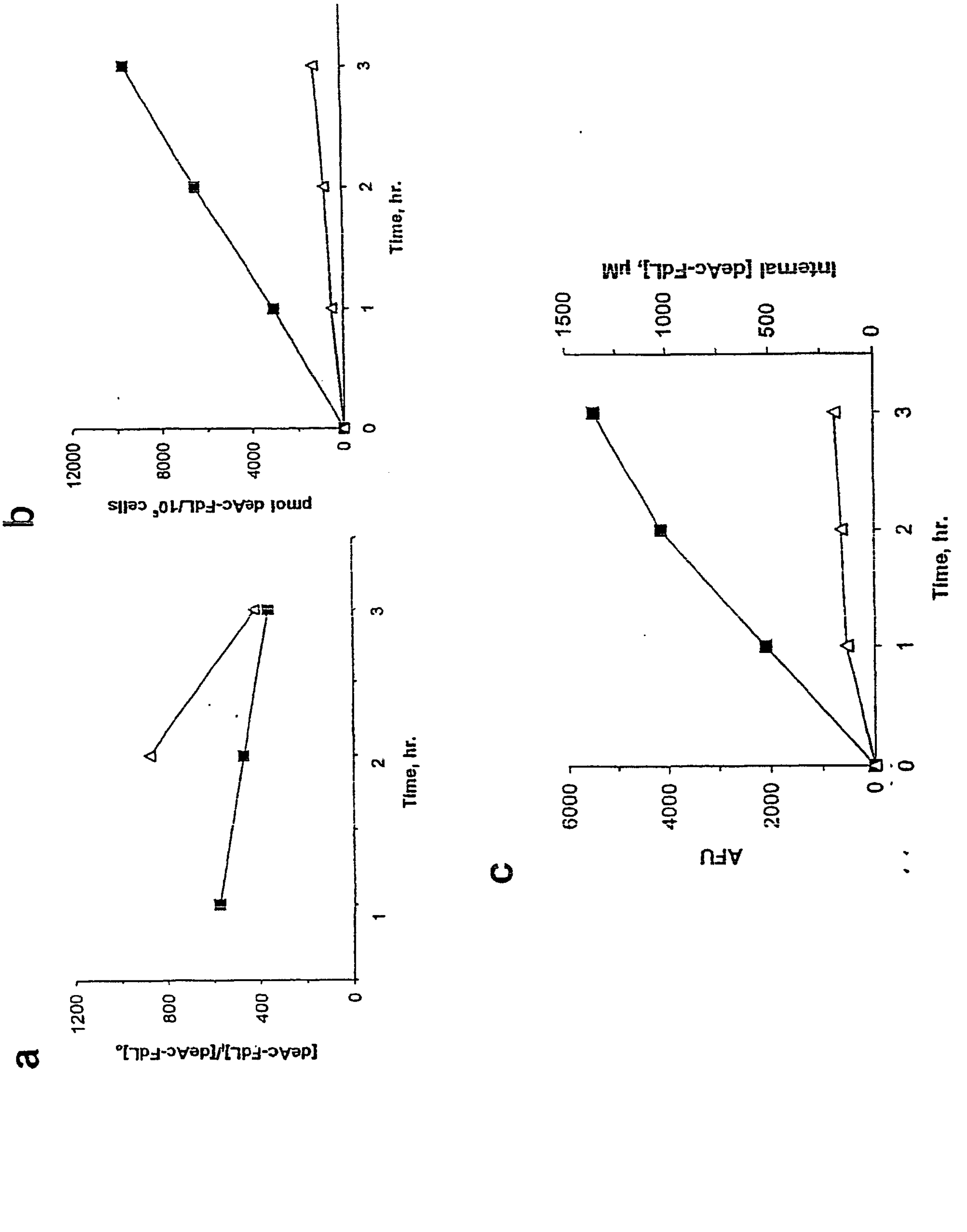
a
[deAc-FdL]$_i$/[deAc-FdL]$_o$
Time, hr.
b
pmol deAc-FdL/10$^6$ cells
Time, hr.
c
AFU
Internal [deAc-FdL], µM
Time, hr.

Figure 6

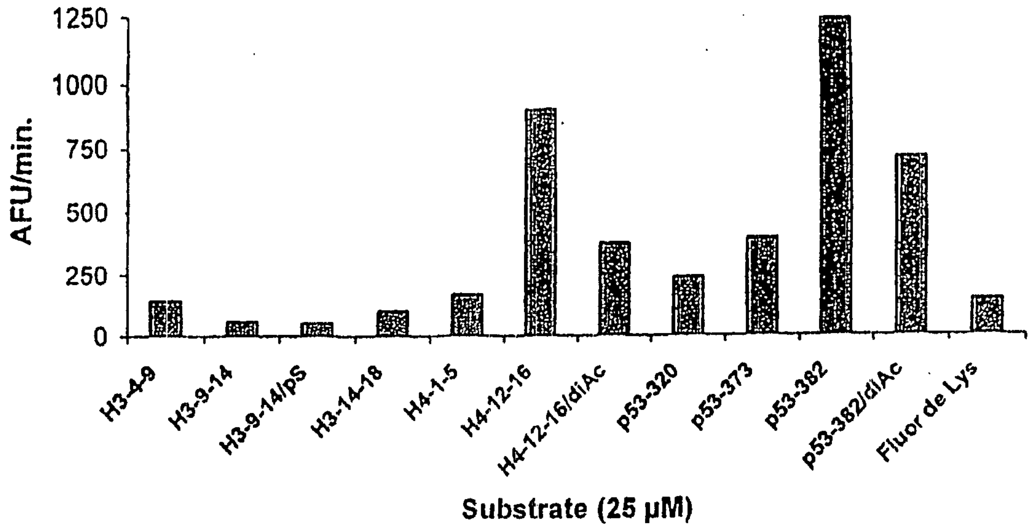


| Substrate Name<br>Sequence Source-Residue #(s), (manufacturer's substrate name, (BIOMOL, Plymouth Meeting, PA) | Sequence |
|---|---|
| H3-4-9 | K(Ac)QTARK(Ac) |
| H3-9-14 | K(Ac)STGGK(Ac) |
| H3-9-14/pS | K(Ac)-S(PO3)-TGGK(Ac) |
| H3-14-18 | K(Ac)APRK(Ac) |
| H4-1-5 | SGRGK(Ac) |
| H4-12-16 (Fluor de Lys-H4-AcK16) | KGGAK(Ac) |
| H4-12-16/diAc | K(Ac)GGAK(Ac) |
| p53-320 (Fluor de Lys-SIRT2) | QPKK(Ac) |
| p53-373 | K(Ac)SKK(Ac) |
| p53-382 (Fluor de Lys-SIRT1) | RHKK(Ac) |
| p53-382/di-Ac (Fluor de Lys-HDAC8) | RHK(Ac)K(Ac) |
| ε-acetyl lysine (Fluor de Lys, FdL) | K(Ac) |

# Figure 7

| | AFU/min | SE . | AFU/20 mi | SD |
|---|---|---|---|---|
| 0 | 96.35835 | 7.819439 | 1927.167 | 270.8733 |
| 2 | 105.3334 | 5.886086 | 2106.667 | 203.9 |
| 5 | 98.15 | 13.83784 | 1963 | 472.4288 |
| 20 | 98.575 | 4.85032 | 1971.5 | 168.02 |
| 100 | 60.85835 | 9.009262 | 1217.167 | 312.09 |
| 200 | 32.43335 | 1.127565 | 648.687 | 39.06 |
| 500 | 5.33335 | 9.047858 | 108.667 | 313.42 |

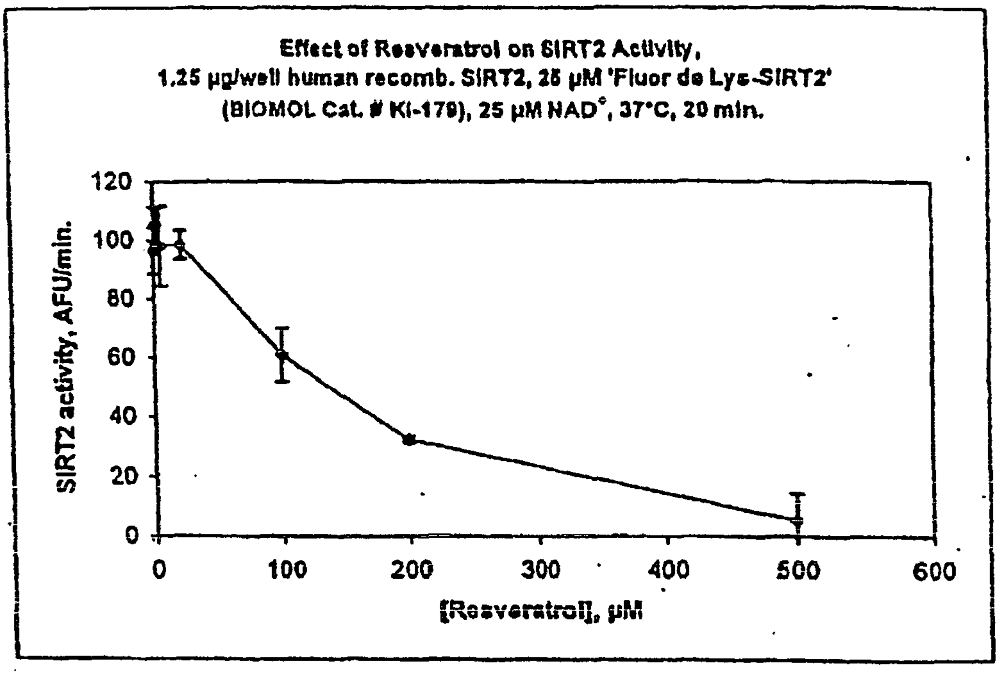

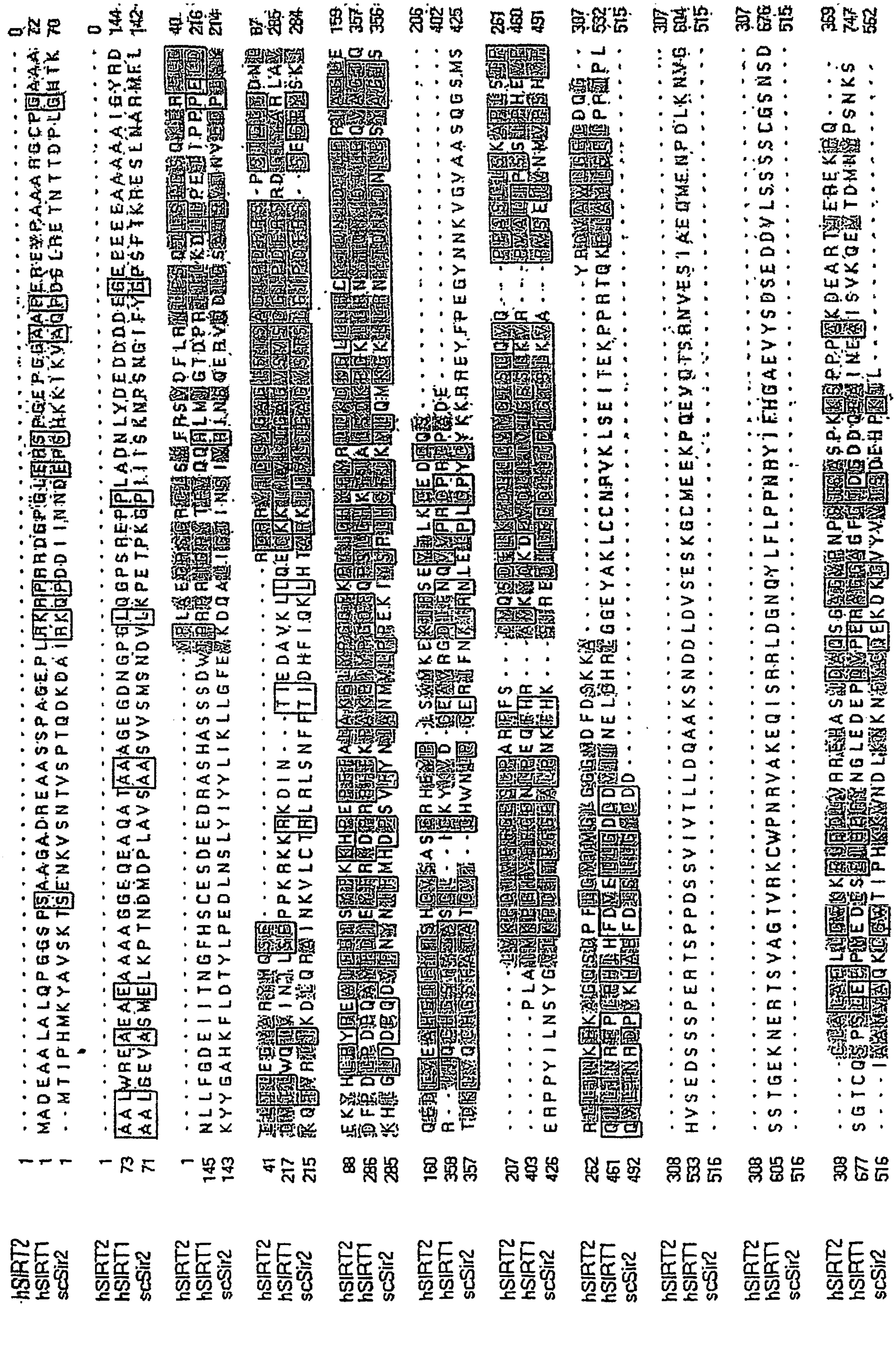

Figure 8

# Figure 9

a

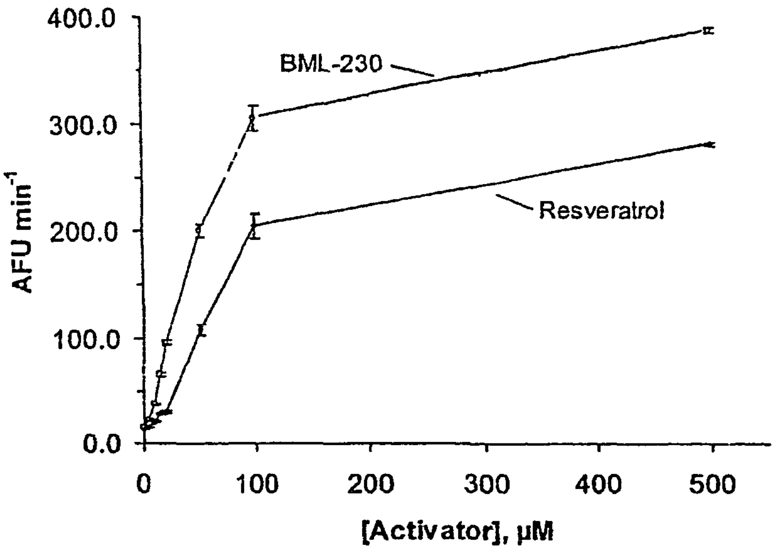


b

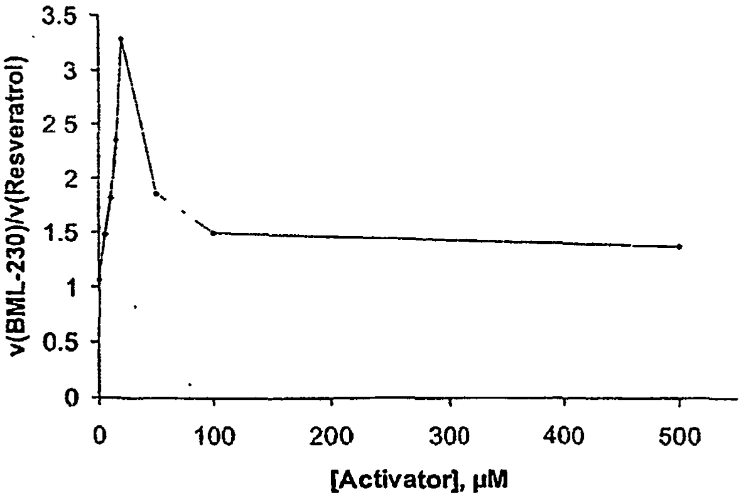


**Figure 9.** Resveratrol and BML-230 dose responses of SIRT1 catalytic rate. **a,** SIRT1 initial rates as a function of activator concentration were determined at 25 μM each of $NAD^+$ and p53-382 acetylated peptide, with 20 min. incubations. Points represent the mean of three determinations and error bars are standard errors of the mean. **b,** Ratio of BML-230-activated to resveratrol-activated SIRT1 rates as a function of activator concentration. Ratios calculated from data of **a**.

Figure 10

a

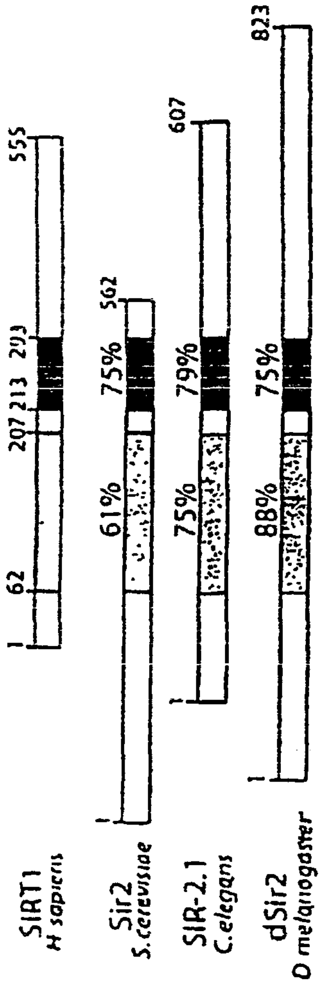
SIRT1
H sapiens
Sir2
S. cerevisiae
SIR-2.1
C. elegans
dSir2
D melanogaster
1
62
207
213
233
555
562
607
823
61%
75%
79%
88%

# Figure 10

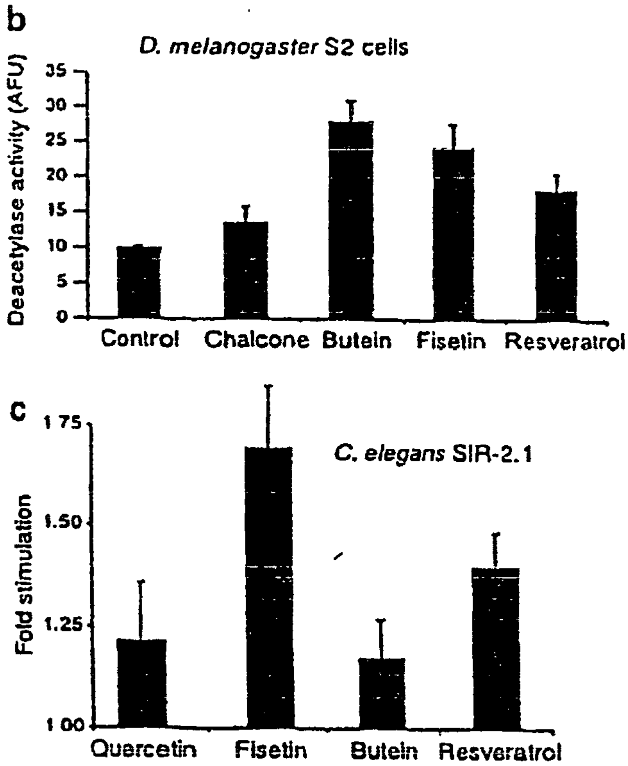
b
D. melanogaster S2 cells
Deacetylase activity (AFU)
35
30
25
20
15
10
5
0
Control
Chalcone
Butein
Fisetin
Resveratrol
c
1.75
1.50
1.25
1.00
Fold stimulation
C. elegans SIR-2.1
Quercetin
Fisetin
Butein
Resveratrol

Figure 10

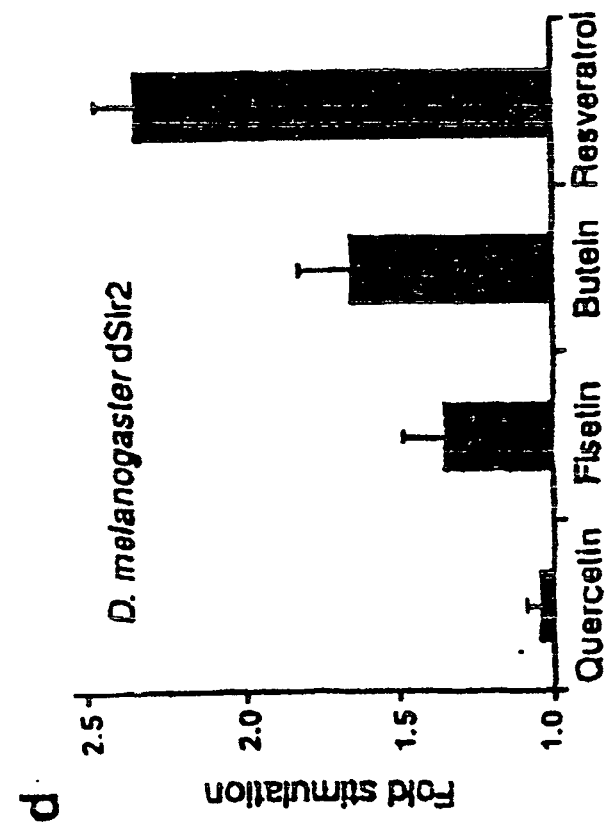
d
D. melanogaster dSir2
Fold stimulation
2.5
2.0
1.5
1.0
Quercetin
Fisetin
Butein
Resveratrol

Figure 10

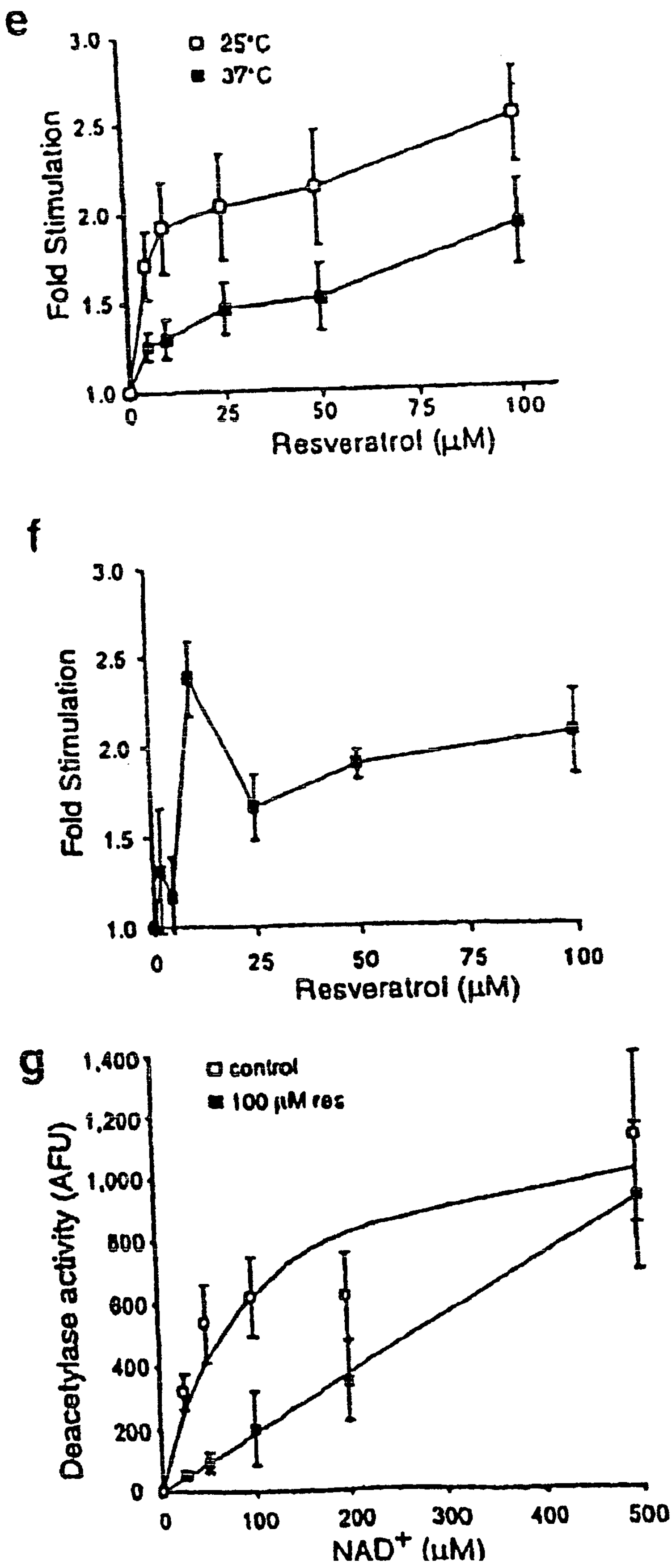
e
25°C
37°C
Fold Stimulation
3.0
2.5
2.0
1.5
1.0
0
25
50
75
100
Resveratrol (μM)
f
Fold Stimulation
3.0
2.5
2.0
1.5
1.0
0
25
50
75
100
Resveratrol (μM)
g
control
100 μM res
Deacetylase activity (AFU)
1,400
1,200
1,000
800
600
400
200
0
0
100
200
300
400
500
NAD+ (μM)

Figure 11

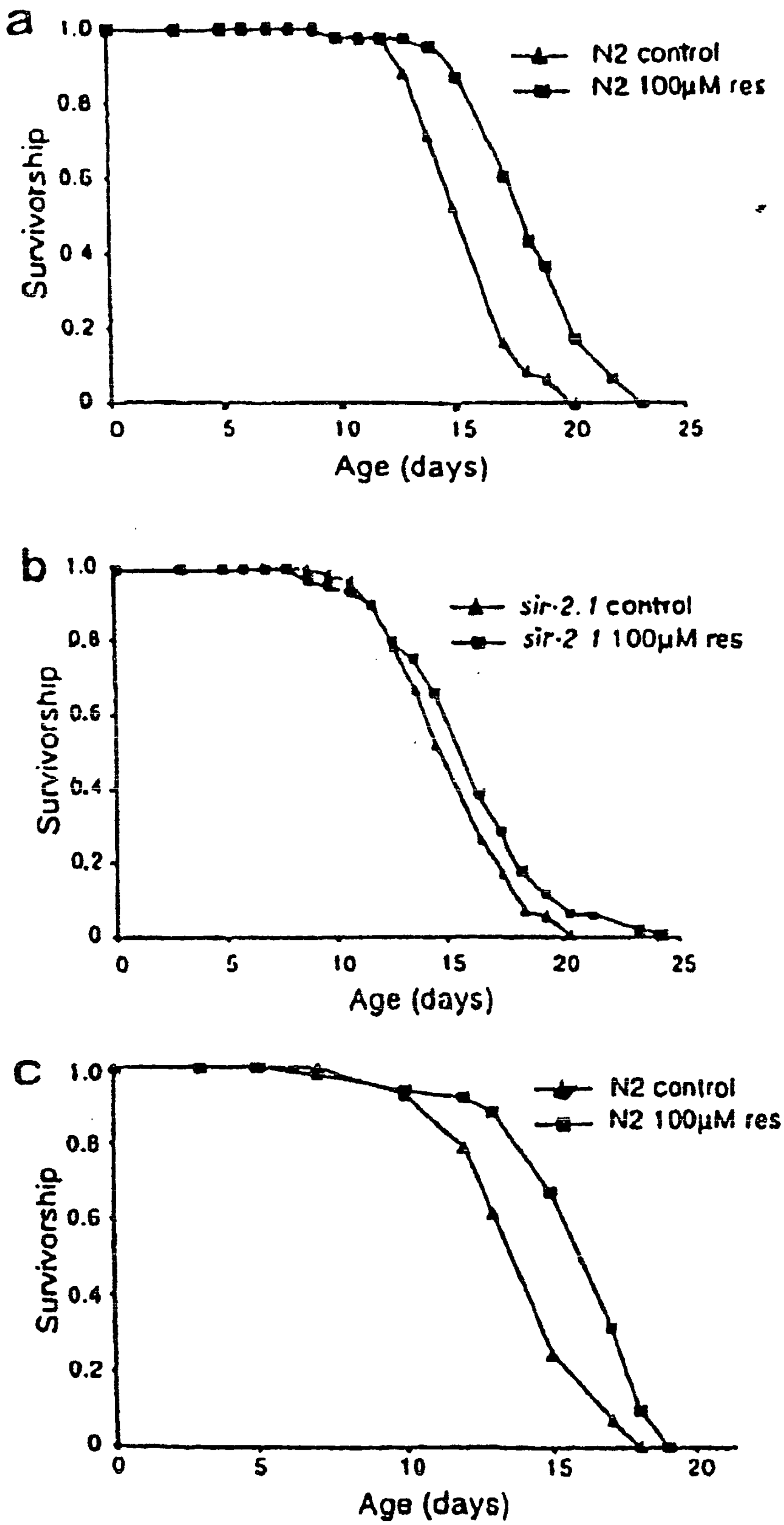
a
N2 control
N2 100µM res
Survivorship
Age (days)
b
sir-2.1 control
sir-2.1 100µM res
Survivorship
Age (days)
c
N2 control
N2 100µM res
Survivorship
Age (days)

Figure 11

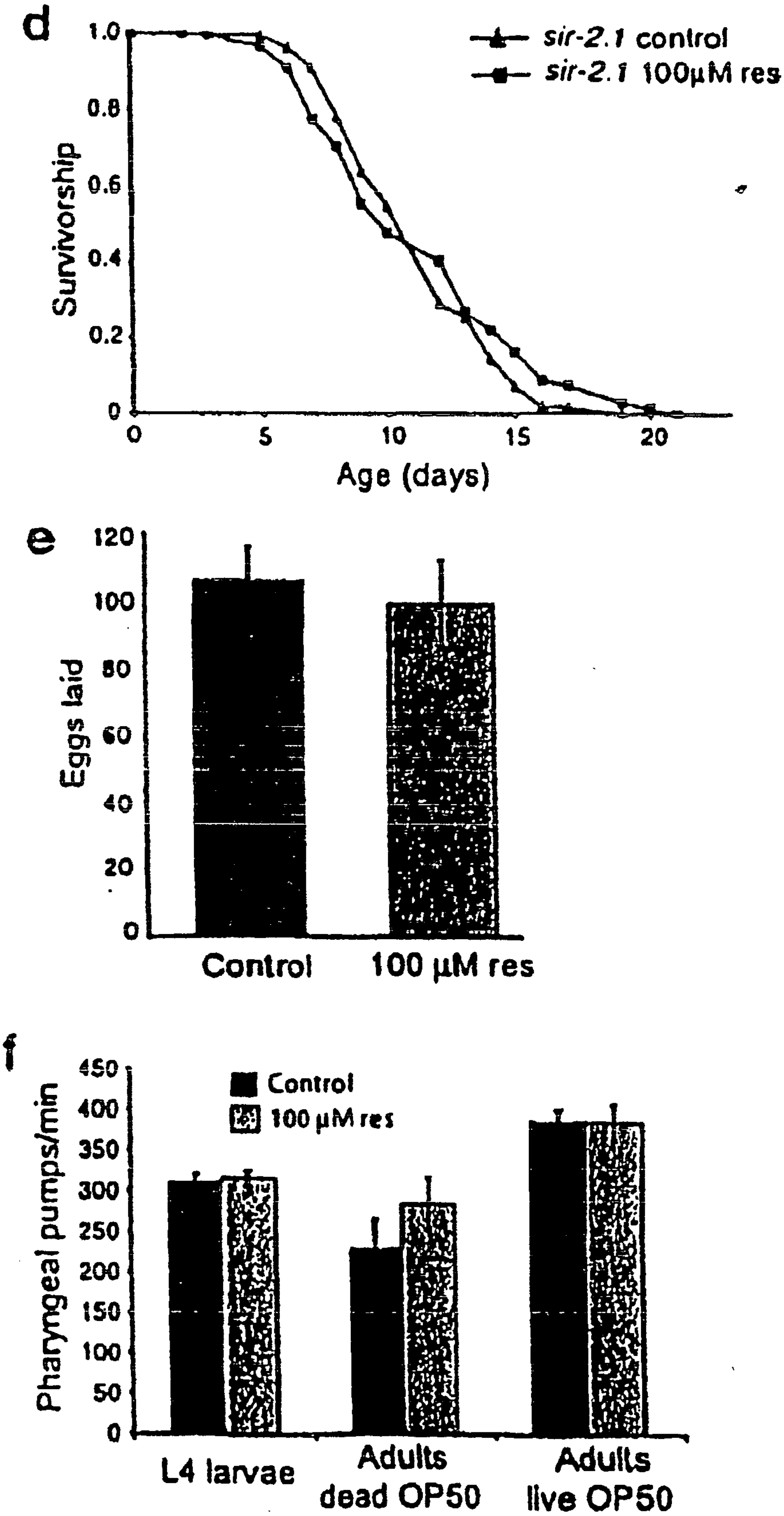
d
sir-2.1 control
sir-2.1 100µM res
Survivorship
1.0
0.8
0.6
0.4
0.2
0
0
5
10
15
20
Age (days)
e
Eggs laid
120
100
80
60
40
20
0
Control
100 µM res
f
Pharyngeal pumps/min
450
400
350
300
250
200
150
100
50
0
Control
100 µM res
L4 larvae
Adults dead OP50
Adults live OP50

Figure 12

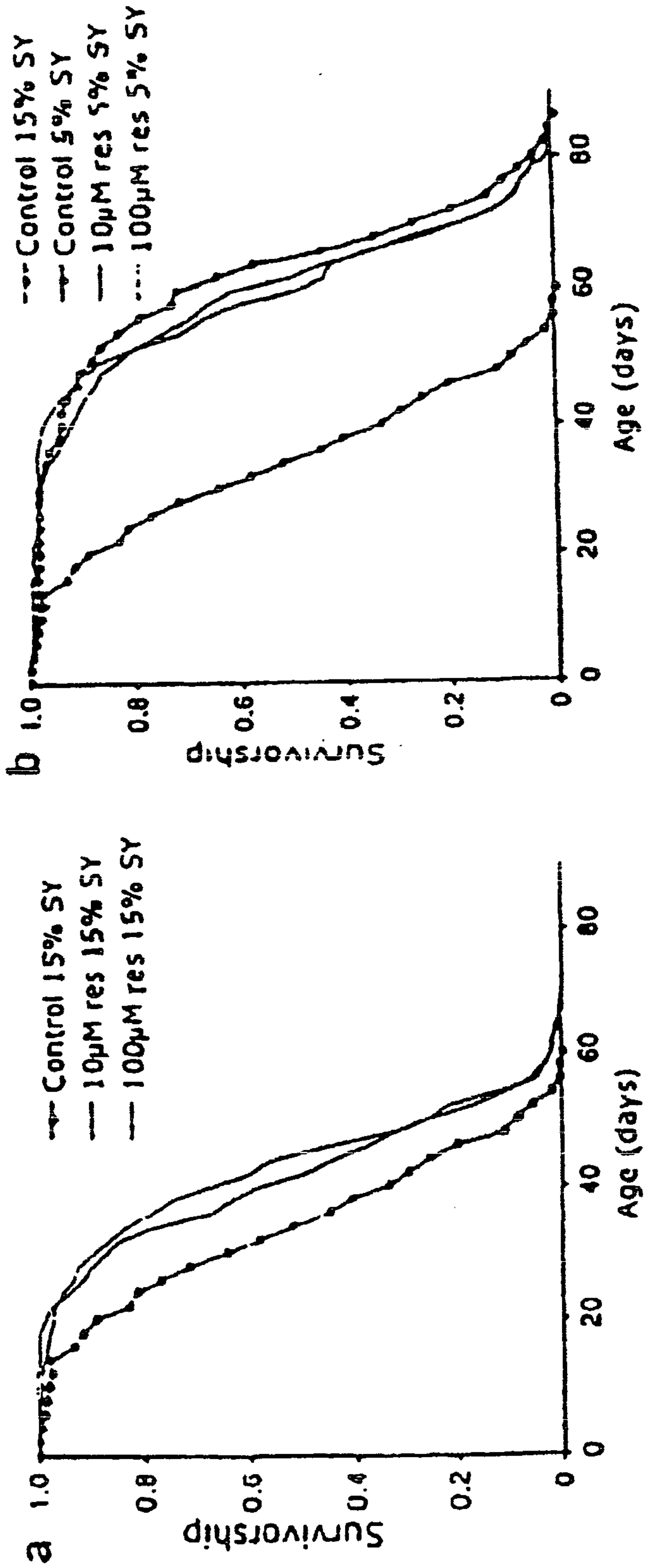
a
b
Survivorship
Age (days)
Control 15% SY
10μM res 15% SY
100μM res 15% SY
Control 15% SY
Control 5% SY
10μM res 5% SY
100μM res 5% SY

Figure 12

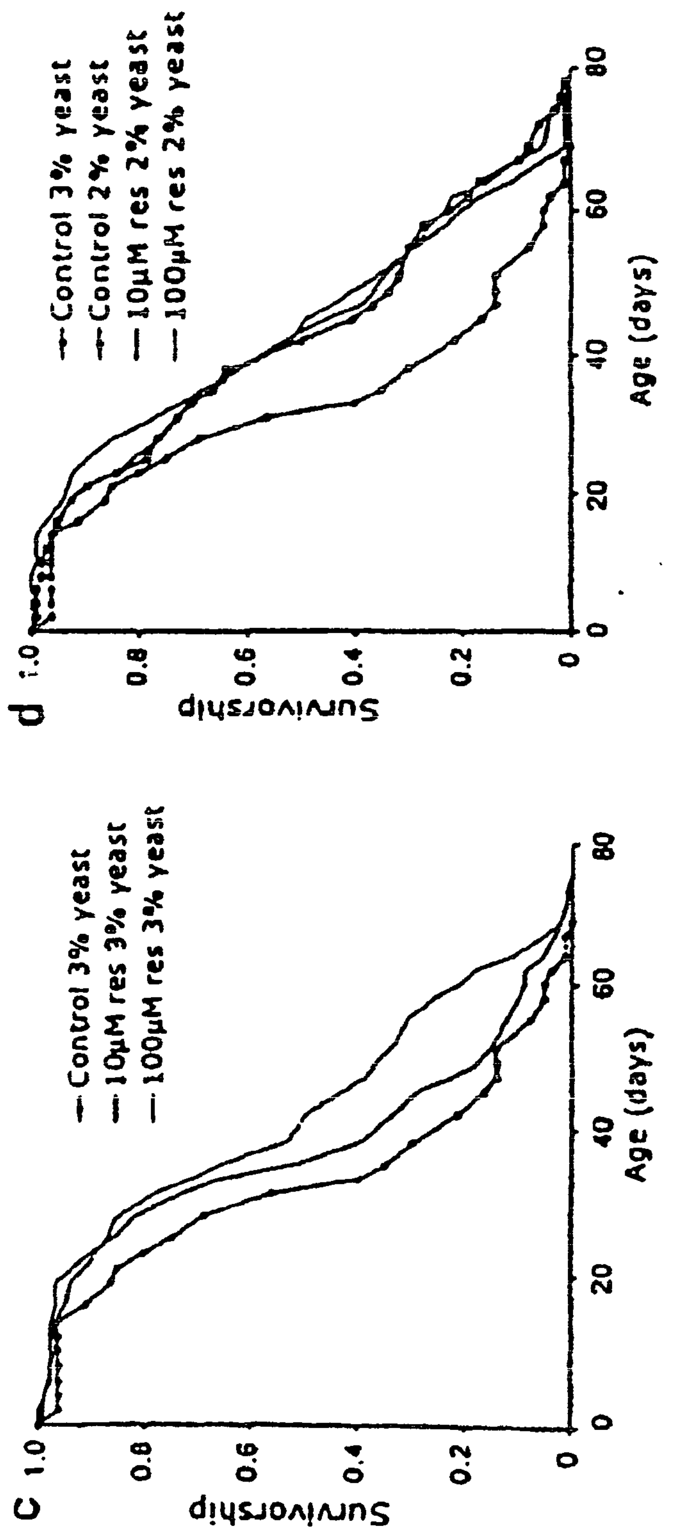
c
Survivorship
1.0
0.8
0.6
0.4
0.2
0
0
20
40
60
80
Age (days)
Control 3% yeast
10μM res 3% yeast
100μM res 3% yeast
d
Survivorship
1.0
0.8
0.6
0.4
0.2
0
0
20
40
60
80
Age (days)
Control 3% yeast
Control 2% yeast
10μM res 2% yeast
100μM res 2% yeast

Figure 12

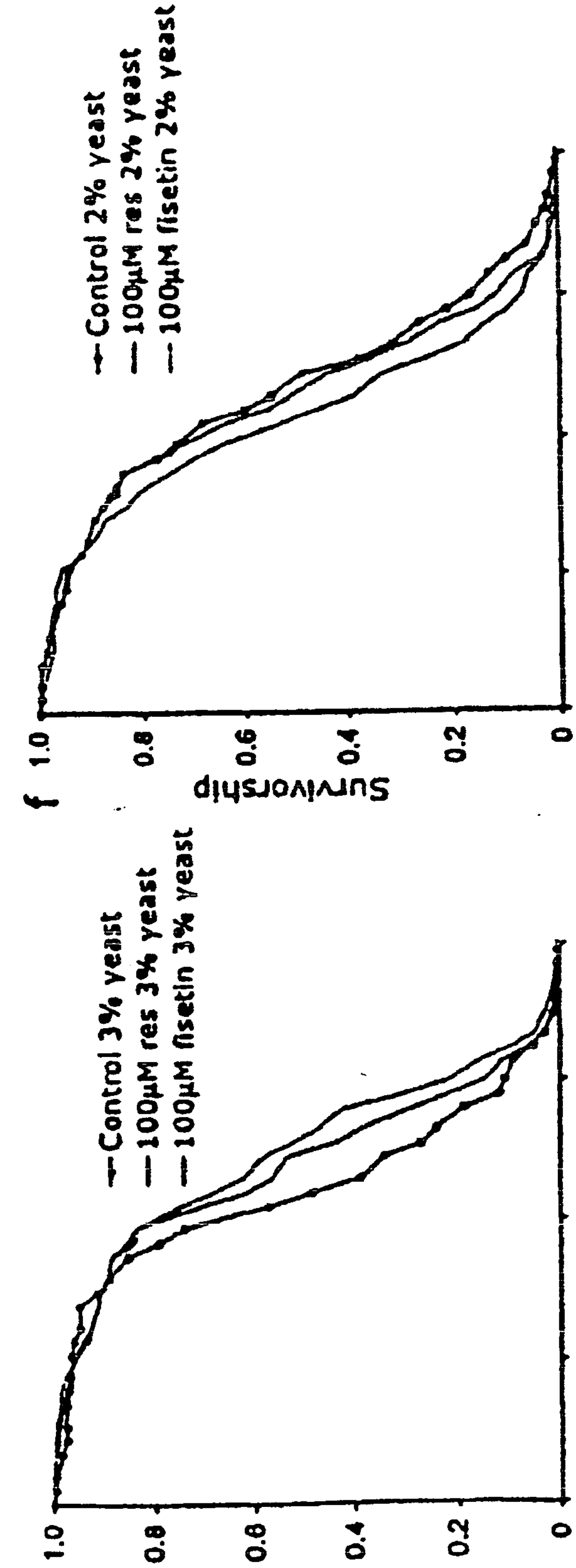
e
Survivorship
Age (days)
Control 3% yeast
100μM res 3% yeast
100μM fisetin 3% yeast
f
Survivorship
Age (days)
Control 2% yeast
100μM res 2% yeast
100μM fisetin 2% yeast

Figure 12

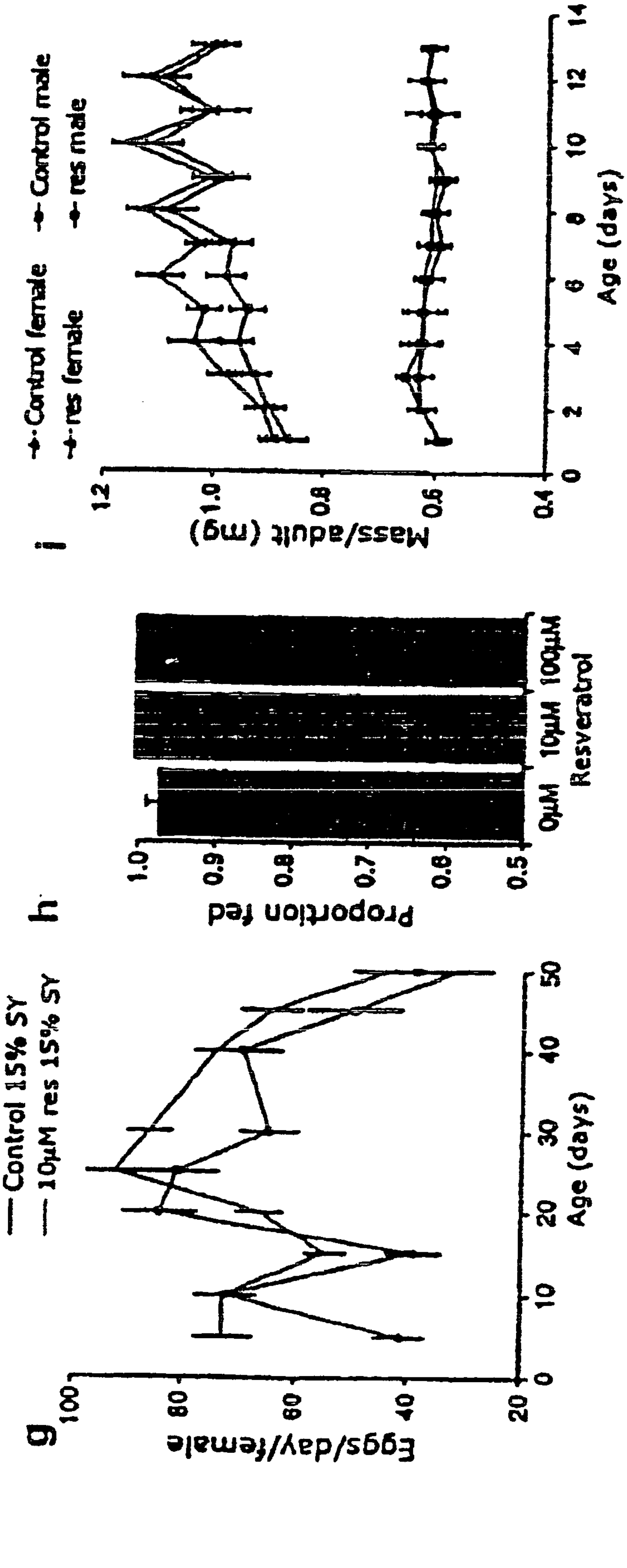
g
Eggs/day/female
Age (days)
Control 15% SY
10µM res 15% SY
h
Proportion fed
0µM 10µM 100µM
Resveratrol
i
Mass/adult (mg)
Age (days)
Control female
res female
Control male
res male

Figure 13

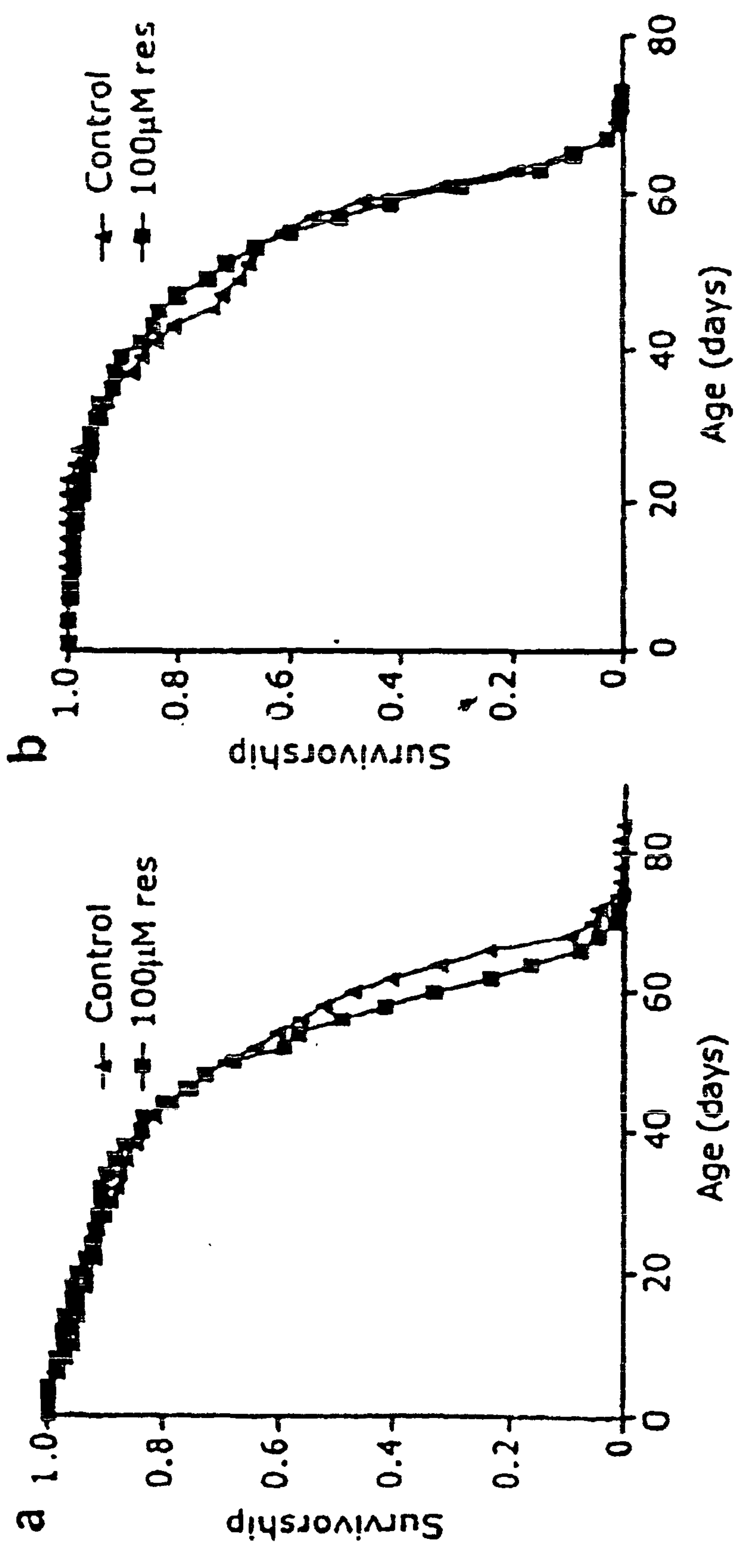
a
b
Survivorship
1.0
0.8
0.6
0.4
0.2
0
Age (days)
0
20
40
60
80
Control
100μM res

Figure 13

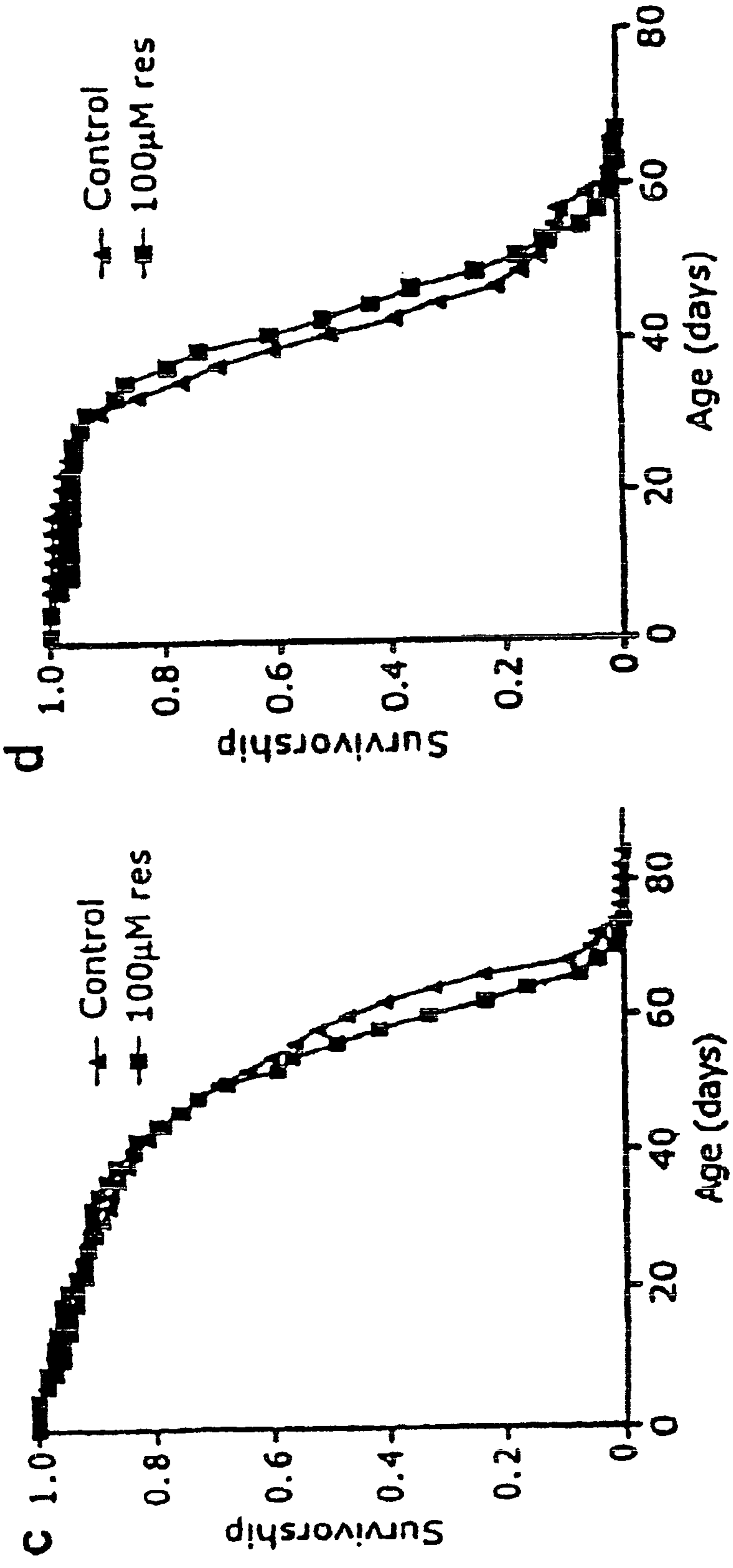
c
d
Survivorship
1.0
0.8
0.6
0.4
0.2
0
20
40
60
80
Age (days)
Control
100µM res

Figure 14

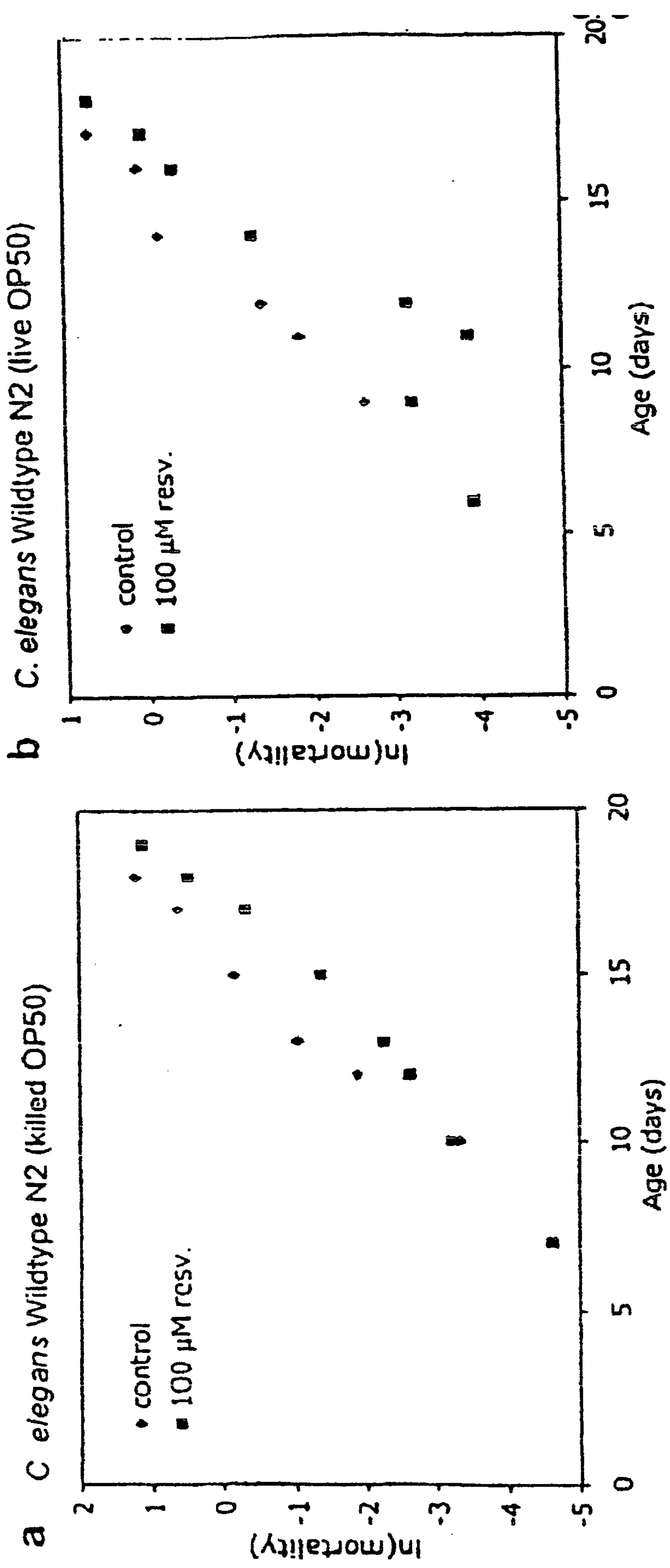
a
C elegans Wildtype N2 (killed OP50)
control
100 µM resv.
ln(mortality)
Age (days)
b
C. elegans Wildtype N2 (live OP50)
control
100 µM resv.
ln(mortality)
Age (days)

Figure 14

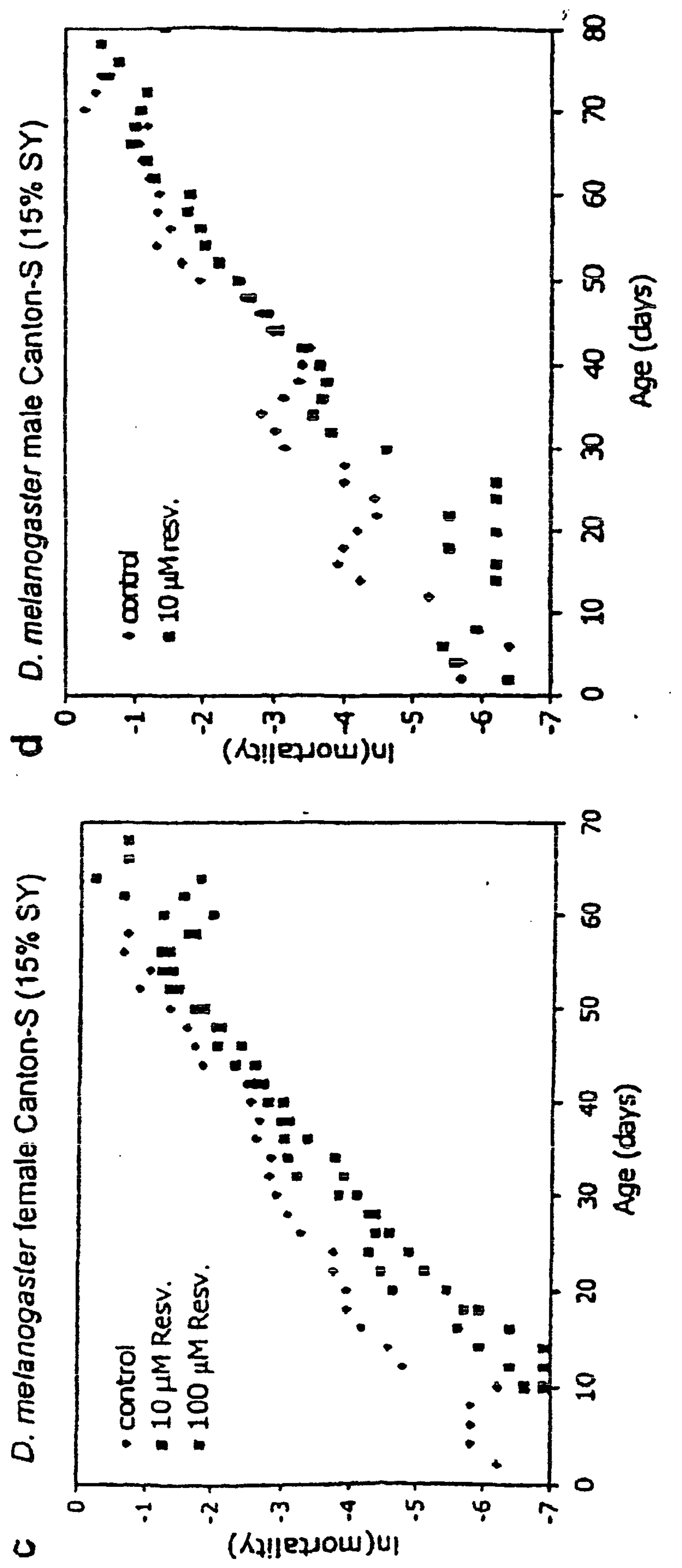
c
D. melanogaster female Canton-S (15% SY)
control
10 µM Resv.
100 µM Resv.
ln(mortality)
Age (days)
d
D. melanogaster male Canton-S (15% SY)
control
10 µM resv.
ln(mortality)
Age (days)

**Figure 15**
**Table 1. Stimulation of SIRT1 Catalytic Rate by Plant Polyphenols (100 µM).**

| Compound | Ratio to Control Rate Mean ± SE | Structure |
|---|---|---|
| Resveratrol (3,5,4'-Trihydroxy-*trans*-stilbene) | 13.4 ± 1.0 | 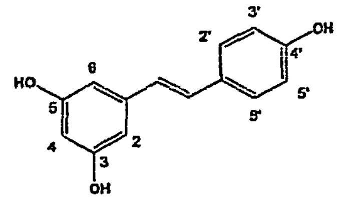 |
| Butein (3,4,2',4'-Tetrahydroxychalcone) | 8.53 ± 0.89 | 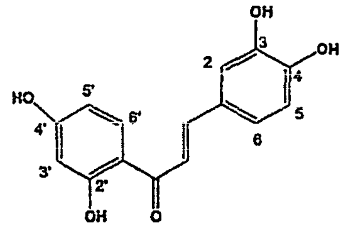 |
| Piceatannol (3,5,3',4'-Tetrahydroxy-*trans*-stilbene) | 7.90 ± 0.50 | 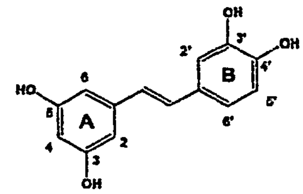 |
| Isoliquiritigenin (4,2',4'-Trihydroxychalcone) | 7.57 ± 0.84 | 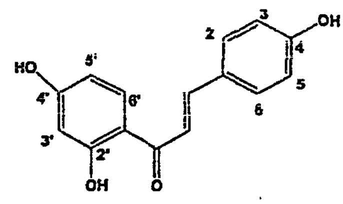 |
| Fisetin (3,7,3',4'-Tetrahydroxyflavone) | 6.58 ± 0.69 | 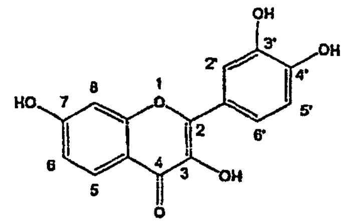 |
| Quercetin (3,5,7,3',4'-Pentahydroxyflavone) | 4.59 ± 0.47 | 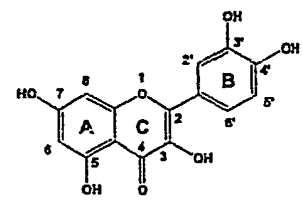 |

Abbreviation: SE, Standard error of the mean. Rate measurements with 25 µM $NAD^+$ and 25 µM p53-382 acetylated peptide substrate were performed as described in Methods. All ratio data were calculated from experiments in which the total deacetylation in the control reaction was 0.25-1.25 µM peptide or 1-5% of the initial concentration of acetylated peptide.

**Figure 16**
**Supplementary Table 1. Effects of Stilbenes and Chalcones (100 µM) on SIRT1 Rate.**

| Compound | Ratio to Control Rate Mean ± SE | Replicates | Structure Skeleton |
|---|---|---|---|
| Resveratrol (3,5,4'-Trihydroxy-*trans*-stilbene) | 13.4 ± 1.0 | 10 | |
| Piceatannol (3,5,3',4'-Tetrahydroxy-*trans*-stilbene) | 7.90 ± 0.50 | 7 | 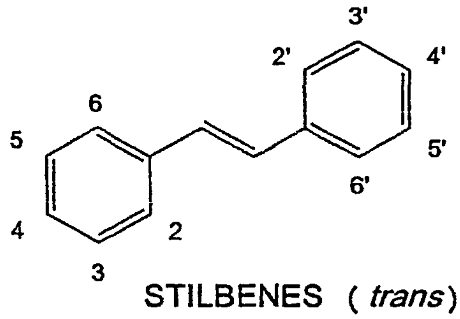 |
| Deoxyrhapontin (3,5-Dihydroxy-4'-methoxystilbene 3-O-β-D-glucoside) | 1.94 ± 0.21 | 6 | STILBENES ( *trans*) |
| *trans*-Stilbene | 1.48 ± 0.15 | 6 | |
| Rhapontin 3,3',5-Trihydroxy-4'-methoxystilbene 3-O-β-D-glucoside | 1.40 ± 0.37- | 6 | |
| *cis*-Stilbene | 1.14 ± 0.29 | 6 | |
| Butein (3,4,2',4'-Tetrahydroxychalcone) | 8.53 ± 0.89 | 6 | |
| Isoliquiritigen (4,2',4'-Trihydroxychalcone) | 7.57 ± 0.84 | 6 | 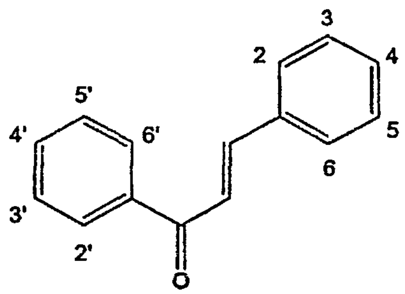 |
| 3,4,2',4',6'-Pentahydroxychalcone | 2.80 ± 0.32 | 6 | CHALCONES |
| Chalcone | 1.34 ± 0.17 | 6 | |

Abbreviation: SE, Standard error of the mean. Rate measurements with 25 µM $NAD^+$ and 25 µM p53-382 acetylated peptide substrate were performed as described in Methods. All ratio data were calculated from experiments in which the total deacetylation in the control reaction was 0.25-1.25 µM peptide or 1-5% of the initial concentration of acetylated peptide.

**Figure 17**
**Supplementary Table 2. Effects of Flavones (100 μM) on SIRT1 Rate (Part I).**

| Compound | Ratio to Control Rate Mean ± SE | Replicates | Structure Skeleton |
|---|---|---|---|
| Fisetin (3,7,3',4'-Tetrahydroxyflavone) | 6.58 ± 0.69 | 9 | |
| 5,7,3',4',5'-Pentahydroxyflavone | 6.05 ± 0.98 | 6 | |
| Luteolin (5,7,3',4'-Tetrahydroxyflavone) | 5.66 ± 0.80 | 6 | |
| 3,6,3',4'-Tetrahydroxyflavone | 5.45 ± 0.57 | 12 | |
| Quercetin (3,5,7,3',4'-Pentahydroxyflavone) | 4.59 ± 0.47 | 16 | 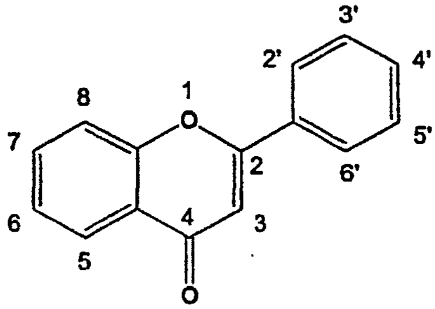<br>FLAVONES |
| 7,3',4',5'-Tetrahydroxyflavone | 3.62 ± 0.56 | 6 | |
| Kaempferol (3,5,7,4'-Tetrahydroxyflavone) | 3.55 ± 0.56 | 6 | |
| 6-Hydroxyapigenin (5,6,7,4'-Tetrahydroxyflavone; Scutellarein) | 3.06 ± 0.29 | 6 | |
| Apigenin (5,7,4'-Trihydroxyflavone) | 2.77 ± 0.40 | 6 | |
| 3,6,2',4'-Tetrahydroxyflavone | 2.10 ± 0.22 | 6 | |
| 7,4'-Dihydroxyflavone | 1.91 ± 0.17 | 6 | |

Abbreviation: SE, Standard error of the mean. Rate measurements with 25 μM $NAD^+$ and 25 μM p53-382 acetylated peptide substrate were performed as described in Methods. All ratio data were calculated from experiments in which the total deacetylation in the control reaction was 0.25-1.25 μM peptide or 1-5% of the initial concentration of acetylated peptide.

**Figure 18**
**Supplementary Table 3. Effects of Flavones (100 µM) on SIRT1 Rate (Part II).**

| Compound | Ratio to Control Rate Mean ± SE | Replicates | Structure Skeleton |
|---|---|---|---|
| 7,8,3',4'-Tetrahydroxyflavone | 1.91 ± 0.39 | 6 | |
| 3,6,2',3'-Tetrahydroxyflavone | 1.74 ± 0.27 | 6 | |
| 4'-Hydroxyflavone | 1.73 ± 0.12 | 6 | |
| 5,4'-Dihydroxyflavone | 1.56 ± 0.15 | 6 | |
| 5,7-Dihydroxyflavone | 1.51 ± 0.18 | 6 | 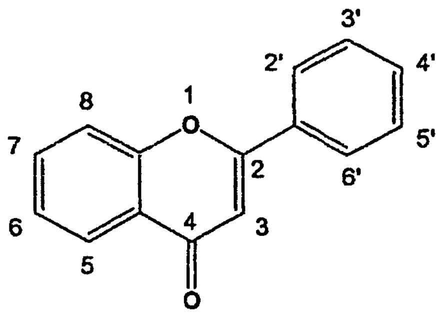 |
| Morin (3,5,7,2',4'-Pentahydroxyflavone) | 1.461 ± 0.071 | 6 | |
| Flavone | 1.41 ± 0.23 | 6 | FLAVONES |
| 5-Hydroxyflavone | 1.22 ± 0.19 | 6 | |
| Myricetin (Cannabiscetin; 3,5,7,3',4',5'-Hexahydroxyflavone) | 0.898 ± 0.070 | 12 | |
| 3,7,3',4',5'-Pentahydroxyflavone | 0.826 ± 0.074 | 12 | |
| Gossypetin (3,5,7,8,3',4'-Hexahydroxyflavone) | 0.723 ± 0.062 | 6 | |

Abbreviation: SE, Standard error of the mean. Rate measurements with 25 µM $NAD^+$ and 25 µM p53-382 acetylated peptide substrate were performed as described in Methods. All ratio data were calculated from experiments in which the total deacetylation in the control reaction was 0.25-1.25 µM peptide or 1-5% of the initial concentration of acetylated peptide.

Figure 19

**Supplementary Table 4. Effects of Isoflavones, Flavanones and Anthocyanidins (100 μM) on SIRT1 Rate**

| Compound | Ratio to Control Rate Mean ± SE | Replicates | Structure Skeleton |
|---|---|---|---|
| Daidzein (7,4'-Dihydroxyisoflavone) | 2.28 ± 0.74 | 2 | 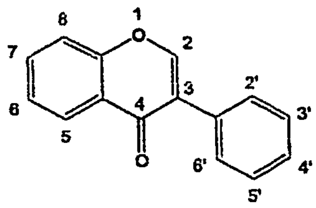 |
| Genistein (5,7,4'-Trihydroxyisoflavone) | 1.109 ± 0.026 | 2 | ISOFLAVONES |
| Naringenin (5,7,4'-Trihydroxyflavanone) | 2.10 ± 0.23 | 6 | 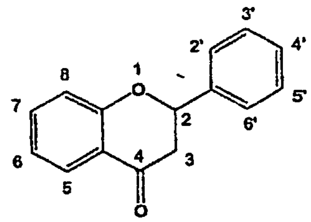 |
| 3,5,7,3',4'-Pentahydroxyflavanone | 1.97 ± 0.22 | 5 | |
| Flavanone | 1.92 ± 0.24 | 6 | FLAVANONES |
| Pelargonidin chloride (3,5,7.4'-Tetrahydroxyflavylium chloride) | 1.586 ± 0.037 | 2 | |
| Cyanidin chloride (3,5,7,3',4'-Pentahydroxyflavylium chloride) | 0.451 ± 0.015 | 2 | 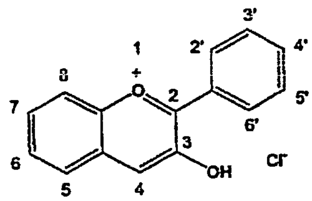 |
| Delphinidin chloride (3,5,7,3',4',5'-Hexahydroxyflavylium chloride) | 0.4473 ± 0.0071 | 2 | ANTHOCYANIDINS ( Flavylium Chloride Salts) |

Abbreviation: SE, Standard error of the mean. Rate measurements with 25 μM $NAD^+$ and 25 μM p53-382 acetylated peptide substrate were performed as described in Methods. All ratio data were calculated from experiments in which the total deacetylation in the control reaction was 0.25-1.25 μM peptide or 1-5% of the initial concentration of acetylated peptide.

**Figure 20**
**Supplementary Table 5. Effects of Catechins (Flavan-3-ols) (100 μM) on SIRT1 Rate.**

| Compound | Ratio to Control Rate Mean ± SE | Replicates | Structure Skeleton/Structure |
|---|---|---|---|
| (-)-Epicatechin (Hydroxy Sites: 3,5,7,3',4') | 1.53 ± 0.31 | 4 | |
| (-)-Catechin (Hydroxy Sites: 3,5,7,3',4') | 1.41 ± 0.21 | 4 | 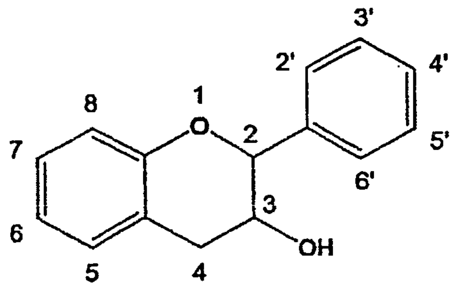 |
| (-)-Gallocatechin (Hydroxy Sites: 3,5,7,3',4',5') | 1.35 ± 0.25 | 4 | CATECHINS ( Flavan-3-ols) |
| (+)-Catechin (Hydroxy Sites: 3,5,7,3',4') | 1.31 ± 0.19 | 4 | |
| (+)-Epicatechin (Hydroxy Sites: 3,5,7,3',4') | 1.26 ± 0.20 | 4 | |
| (-)-Epigallocatechin (Hydroxy Sites: 3,5,7,3',4',5') | 0.41 ± 0.11 | 4 | |
| (-)-Epigallocatechin Gallate (Hydroxy Sites: 3*,5,7,3',4',5'; *Position of gallate ester) | 0.32 ± 0.12 | 4 | 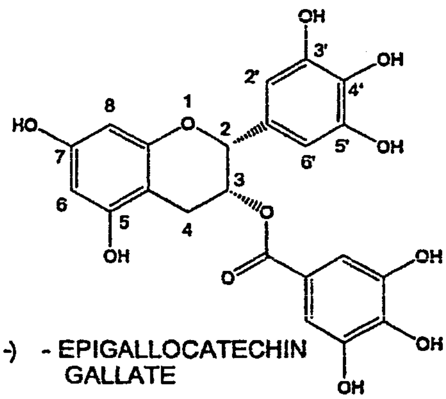 ( -) - EPIGALLOCATECHIN GALLATE |

Abbreviation: SE, Standard error of the mean. Rate measurements with 25 μM $NAD^+$ and 25 μM p53-382 acetylated peptide substrate were performed as described in Methods. All ratio data were calculated from experiments in which the total deacetylation in the control reaction was 0.25-1.25 μM peptide or 1-5% of the initial concentration of acetylated peptide.

# Figure 21

**Supplementary Table 6. Effects of Free Radical Protective Compounds (100 μM) on SIRT1 Rate.**

| Compound | Ratio to Control Rate Mean ± SE | Replicates | Protective Mechanism |
|---|---|---|---|
| Hinokitiol (b-Thujaplicin; 2-hydroxy-4-isopropyl-2,4,6-cycloheptatrien-1-one) | 2.48 ± 0.15 | 2 | Iron Chelator |
| L-(+)-Ergothioneine ((S)-a-Carboxy-2,3-dihydro-N,N,N-trimethyl-2-thioxo-1H-imidazole-4-ethanaminium inner salt) | 2.06 ± 0.48 | 2 | Antioxidant, Peroxynitrite Scavenger |
| Caffeic Acid Phenyl Ester | 1.80 ± 0.16 | 2 | Iron Chelator |
| MCI-186 (3-Methyl-1-phenyl-2-pyrazolin-5-one) | 1.2513 ± 0.0080 | 2 | Radical Scavenger and Antioxidant |
| HBED (N,N'-Di-(2-hydroxybenzyl)ethylenediamine-N,N'-diacetic acid•HCl•H2O) | 1.150 ± 0.090 | 2 | Iron Chelator |
| Ambroxol (trans-4-(2-Amino-3,5-dibromobenzylamino) cyclohexane•HCl) | 1.075 ± 0.0026 | 2 | Radical Scavenger |
| U-83836E ((-)-2-((4-(2,6-di-1-Pyrrolidinyl-4-pyrimidinyl)-1-piperazinyl)methyl)-3,4-dihydro-2,5,7,8-tetramethyl-2H-1-benzopyran-6-ol•2HCl) | 1.030 ± 0.055 | 2 | "Lazaroid" aminosteroid, Peroxidation inhibitor |
| Trolox (6-Hydroxy-2,5,7,8-tetramethylchroman-2-carboxylic acid) | 0.995 ± 0.019 | 2 | Antioxidant |

Abbreviation: SE, Standard error of the mean. Rate measurements with 25 μM $NAD^+$ and 25 μM p53-382 acetylated peptide substrate were performed as described in Methods. All ratio data were calculated from experiments in which the total deacetylation in the control reaction was 0.25-1.25 μM peptide or 1-5% of the initial concentration of acetylated peptide.

**Figure 22**
**Supplementary Table 7. Effects of Miscellaneous Compounds (100 µM) on SIRT1 Catalytic Rate.**

| Compound | Ratio to Control Rate Mean ± SE | Replicates | Structure & Activities |
|---|---|---|---|
| Dipyridamole (2,6-bis(Diethanol amino)-4,8-dipiperidino-pyrimido[5,4-d]pyrimidine | 3.54 ± 0.20 | 2 | 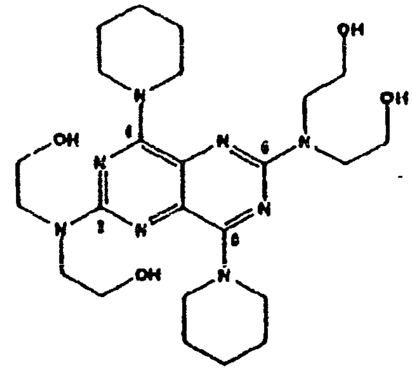 Inhibitor of Adenosine Transport, Phosphodiesterase, 5-Lipoxygenase |
| Nicotinamide | 0.428 ± 0.019 | 42 | 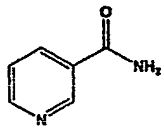 Sirtuin Reaction Product/Inhibitor |
| NF279 | 0.0035 ± 0.0011 | 3 | 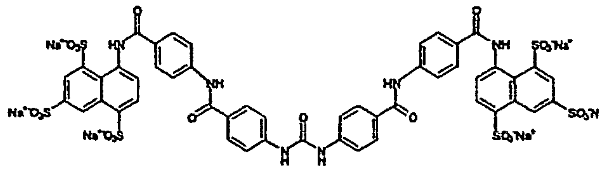 Purinergic Receptor Antagonist |
| NF023 | -0.0016 ± 0.0015 | 3 | 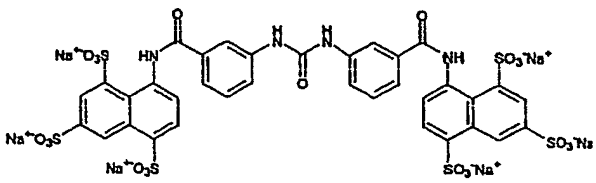 G-protein Antagonist |

**Figure 22**
**Supplementary Table 7 (cont.)**

| | | | |
|---|---|---|---|
| Suramin | -0.0002 ± 0.0010 | 3 | 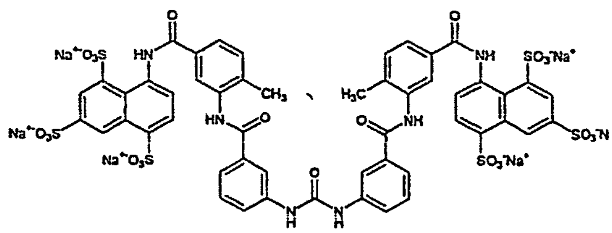<br>G-protein Antagonist, Reverse Transcriptase Inhibitor |

Abbreviation: SE, Standard error of the mean. Rate measurements with 25 μM $NAD^+$ and 25 μM p53-382 acetylated peptide substrate were performed as described in Methods. All ratio data were calculated from experiments in which the total deacetylation in the control reaction was 0.25-1.25 μM peptide or 1-5% of the initial concentration of acetylated peptide.

**Figure 23**
**Supplementary Table 8. Effects of Various Modulators on SIRT1 Rate.**

| Compound, (Concentration) | Ratio to Control Rate Mean ± SE | Replicates | Structure |
|---|---|---|---|
| ZM 336372, (100 µM) | 3.5 ± 1.1 | 3 | 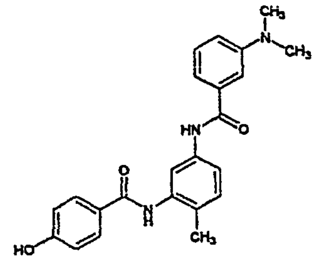 |
| Camptothecin, (10 µM) | 2.92 ± 0.41 | 3 | 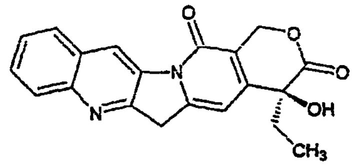 |
| Coumestrol, (10 µM) | 2.30 ± 0.31 | 2 | 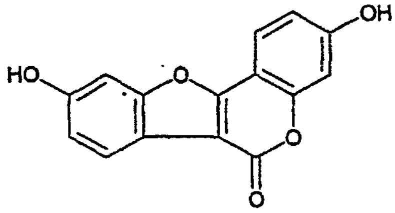 |
| NDGA, (100 µM) | 1.738 ± 0.088 | 3 | 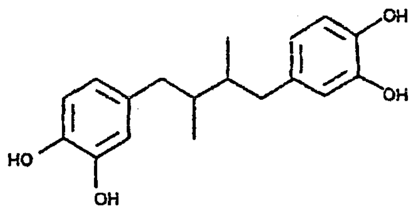 |
| Esculetin, (10 µM) | 1.737 ± 0.082 | 3 | 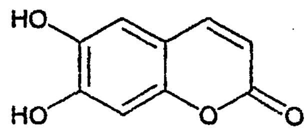 |
| Sphingosine | 0.069 ± 0.028 | 3 | 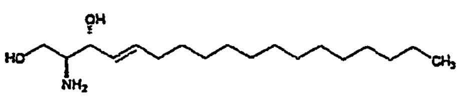 |

Abbreviation: SE, Standard error of the mean. Rate measurements with 25 µM $NAD^+$ and 25 µM p53-382 acetylated peptide substrate were performed as described in Methods. All ratio data were calculated from experiments in which the total deacetylation in the control reaction was 0.25-1.25 µM peptide or 1-5% of the initial concentration of acetylated peptide.

**Figure 24. Table 9. SIRT1 Rate Effects of New Resveratrol Analogs (100 μM).**

| Compound | Ratio to Control Rate Mean ± SE | N | Structure | Stability in Solution $t_{1/2}$, hrs. |
|---|---|---|---|---|
| BML-230 (3,5-Dihydroxy-4'-thiomethyl-*trans*-stilbene) | 11.8 ± 1.9 | 12 | 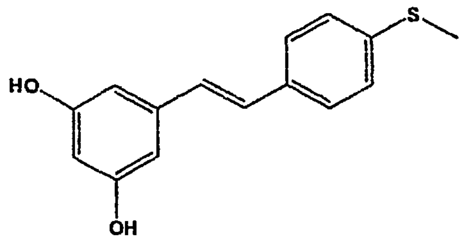 | |
| Resveratrol (3,5,4'-Trihydroxy-*trans*-stilbene) | 10.7 ± 0.4 | 49 | 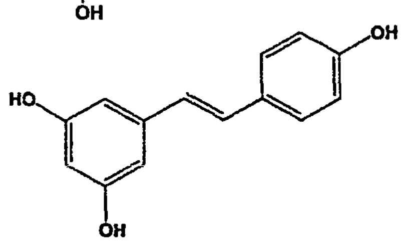 | 59 (ethanol), 20 (water) |
| BML-217 (3,5-Dihydroxy-4'-chloro-*trans*-stilbene) | 10.6 ± 0.4 | 3 | 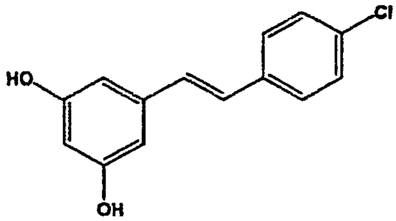 | |
| Pinosylvin (3,5-Dihydroxy-*trans*-stilbene) | 9.95 ± 0.45 | 3 | 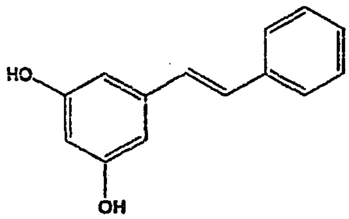 | |
| BML-225 (3,5-Dihydroxy-4'-ethyl-*trans*-stilbene) | 9.373 ± 0.014 | 3 | 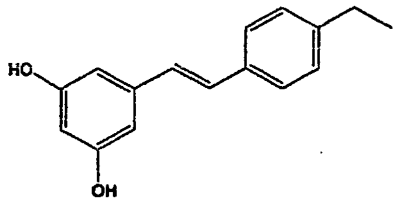 | |
| BML-212 (3,5-Dihydroxy-4'-fluoro-*trans*-stilbene) | 8.20 ± 0.69 | 3 | 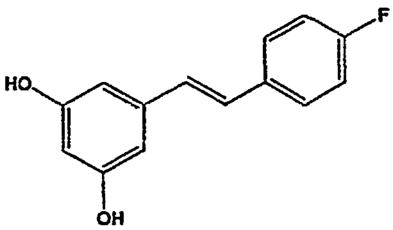 | 66 (ethanol) |

Figure 25

Table 10. SIRT1 Rate Effects of New Resveratrol Analogs (100 μM).

| Compound | Ratio to Control Rate Mean ± SE | N | Structure | Stability in Solution $t_{1/2}$, hrs. |
|---|---|---|---|---|
| BML-228 (3,5-Dihydroxy-4'-methyl-*trans*-stilbene) | 7.72 ± 0.12 | 3 | 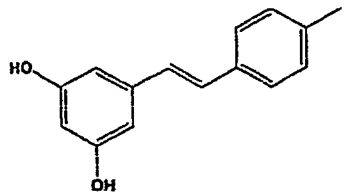 | |
| BML-232 (3,5-Dihydroxy-4'-azido-*trans*-stilbene) | 7.24 ± 0.12 | 3 | 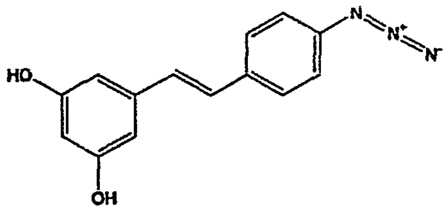 | |
| BML-229 (3,5-Dihydroxy-4'-nitro-*trans*-stilbene) | 6.78 ± 0.22 | 3 | 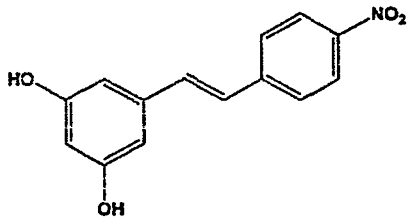 | |
| BML-231 (3,5-Dihydroxy-4'-isopropyl-*trans*-stilbene) | 6.01 ± 0.15 | 3 | 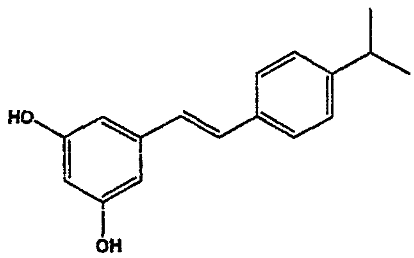 | |
| BML-233 3,5-Dihydroxy-4'-methoxy-*trans*-stilbene | 5.48 ± 0.33 | 6 | 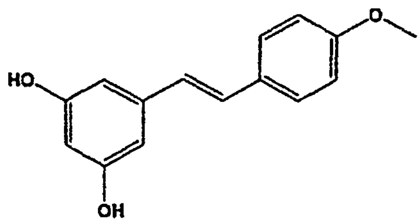 | |

# Figure 26

Table 11. SIRT1 Rate Effects of New Resveratrol Analogs (100 μM).

| Compound | Ratio to Control Rate Mean ± SE | N | Structure | Stability in Solution $t_{1/2}$, hrs. |
|---|---|---|---|---|
| Rhapontin aglycone (3,5,3'Trihydroxy-4'-methoxy-*trans*-stilbene) | 4.060 ± 0.069 | 3 | 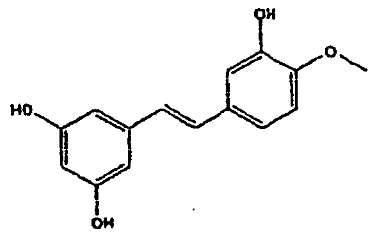 | |
| BML-227 (3,4'-Dihydroxy-5-acetoxy-*trans*-stilbene) | 3.340 ± 0.093 | 3 | 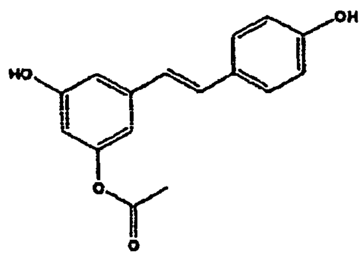 | |
| BML-221 (3,5-Dihydroxy-4'-acetoxy-*trans*-stilbene) | 3.05 ± 0.54 | 6 | 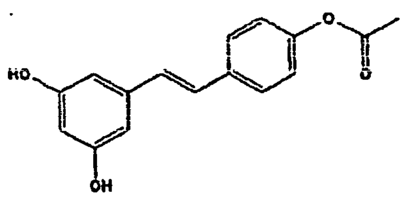 | 504 (ethanol) |
| BML-218 (E)-1-(3,5-Dihydroxyphenyl)-2-(2-napthyl) ethene | 3.05 ± 0.37 | 6 | 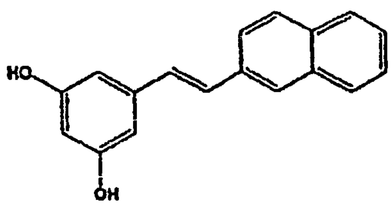 | |
| BML-216 3-Hydroxystilbene | 2.357 ± 0.074 | 3 | 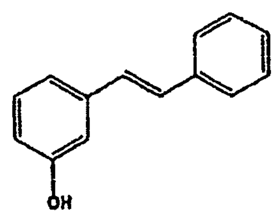 | |

# Figure 27

Table 12. SIRT1 Rate Effects of New Resveratrol Analogs (100 µM).

| Compound | Ratio to Control Rate Mean ± SE | N | Structure | Stability in Solution $t_{1/2}$, hrs. |
|---|---|---|---|---|
| BML-226 (3,5-Dimethoxymethoxy-4'-thiomethyl-*trans*-stilbene) | 2.316 ± 0.087 | 3 | 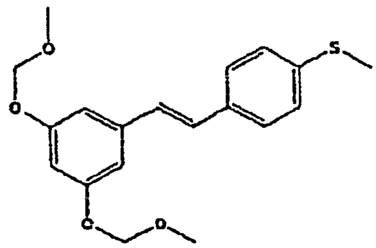 | |
| BML-222 (3,5-Dihydroxy-4'-acetamide-*trans*-stilbene) | 1.88 ± 0.11 | 3 | 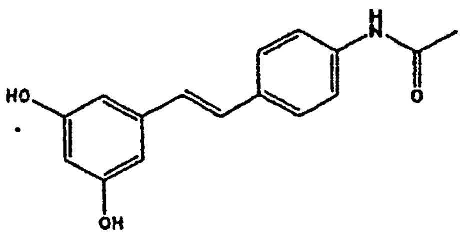 | |
| BML-215 3,4-Dihydroxy-*trans*-stilbene | 1.64 ± 0.10 | 6 | 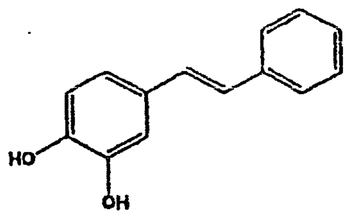 | |
| BML-224 (E)-1-(3,5-Dihydroxyphenyl)-2-(cyclohexyl) ethene | 1.287 ± 0.042 | 3 | 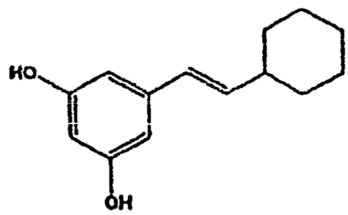 | |
| 3,4-Dimethoxy-*trans*-stilbene | 1.127 ± 0.019 | 3 | 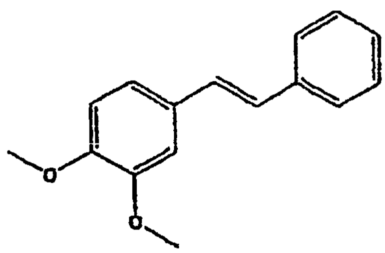 | |

# Figure 28

Table 13. SIRT1 Rate Effects of New Resveratrol Analogs (100 μM).

| Compound | Ratio to Control Rate Mean ± SE | N | Structure | Stability in Solution $t_{1/2}$, hrs. |
|---|---|---|---|---|
| Dihydroresveratrol (1-(3,5-Dihydroxyphenyl)-2-(4-hydroxyphenyl) ethane) | 1.08 ± 0.14 | 4 | 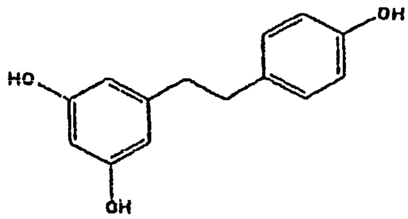 | |
| 4-Hydroxy-*trans*-stilbene | 0.943 ± 0.039 | 3 | 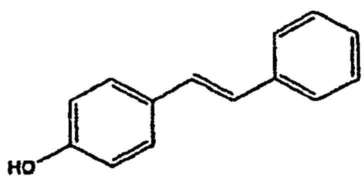 | |
| BML-219 *N*-phenyl-(3,5-dihydroxy)benzamide | 0.902 ± 0.014 | 3 | 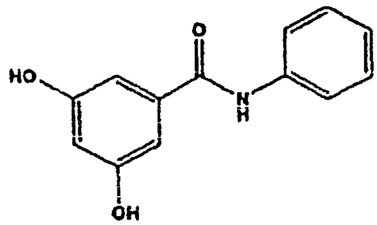 | |
| 3,5-Dihydroxy-4'-nitro-*trans*-stilbene | 0.870 ± 0.019 | 3 | 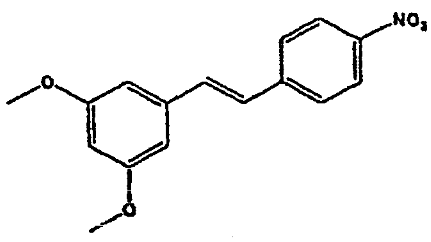 | |
| 4-Methoxy-*trans*-stilbene | 0.840 ± 0.089 | 3 | 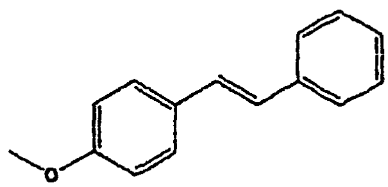 | |

**Figure 29**
**Table 14. Resveratrol Analog Synthetic Intermediates**

| Compound | Benzylphosphonate | Aldehyde | Structure |
|---|---|---|---|
| BML-217 (3,5-Dihydroxy-4'-chloro-*trans*-stilbene) | Diethyl 3-5-dimethoxybenzyl phosphonate | 4-Chlorobenzaldehyde | 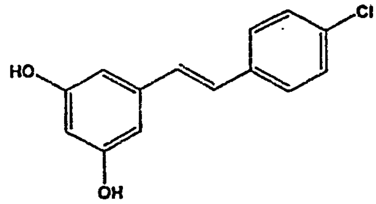 |
| Resveratrol (3,5,4'-Trihydroxy-*trans*-stilbene) | N/A | N/A | 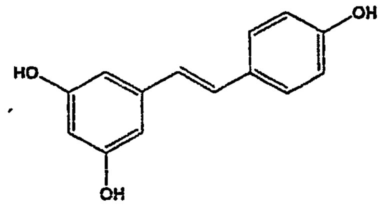 |
| Pinosylvin (3,5-Dihydroxy-*trans*-stilbene) | Diethyl benzyl phosphonate | 3,5-Dimethoxy benzaldehyde | 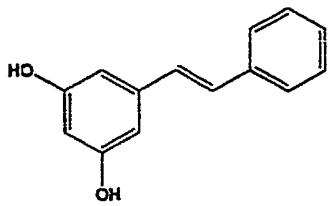 |
| BML-225 (3,5-Dihydroxy-4'-ethyl-*trans*-stilbene) | Diethyl 3-5-dimethoxybenzyl phosphonate | 4-Ethylbenzaldehyde | 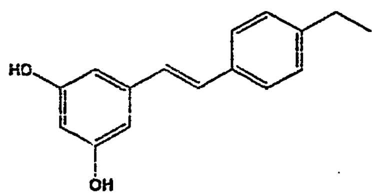 |
| BML-212 (3,5-Dihydroxy-4'-fluoro-*trans*-stilbene) | Diethyl 4-fluoro benzylphosphonate | 3,5-Dimethoxy benzaldehyde | 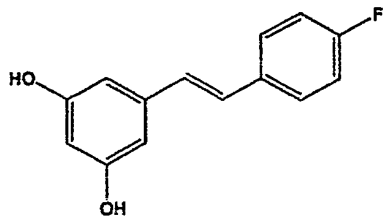 |
| BML-228 (3,5-Dihydroxy-4'-methyl-*trans*-stilbene) | Diethyl 3-5-dimethoxybenzyl phosphonate | 4-Methylbenzaldehyde | 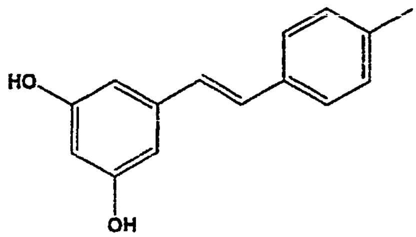 |

**Figure 30**
**Table 15. Resveratrol Analog Synthetic Intermediates**

| Compound | Benzylphosphonate | Aldehyde | Structure |
|---|---|---|---|
| BML-232 (3,5-Dihydroxy-4'-azido-*trans*-stilbene) | Diethyl 4-azido benzylphosphonate | 3,5-Dimethoxymethoxy benzaldehyde | 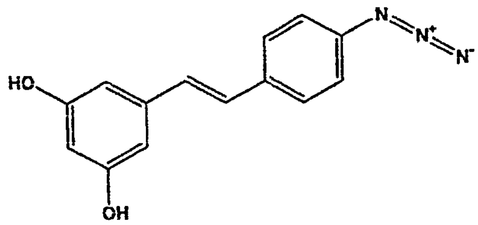 |
| BML-230 (3,5-Dihydroxy-4'-thiomethyl-*trans*-stilbene) | Diethyl 4-methylthio benzylphosphonate | 3,5-Dimethoxymethoxy benzaldehyde | 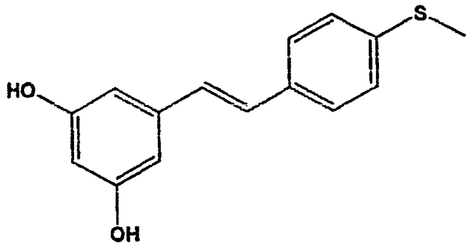 |
| BML-229 (3,5-Dihydroxy-4'-nitro-*trans*-stilbene) | Diethyl 3-5-dimethoxybenzyl phosphonate | 4-Nitrobenzaldehyde | 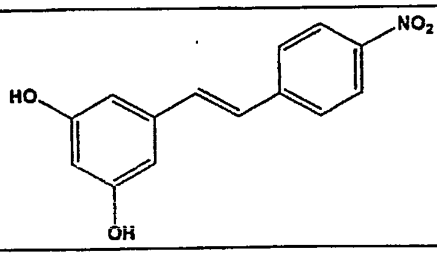 |
| BML-231 (3,5-Dihydroxy-4'-isopropyl-*trans*-stilbene) | Diethyl 3-5-dimethoxybenzyl phosphonate | 4-Isopropyl benzaldehyde | 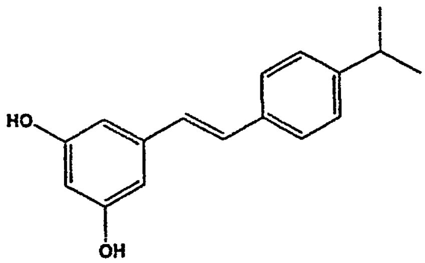 |
| 3,5-Dihydroxy-4'-methoxy-*trans*-stilbene | N/A | N/A | 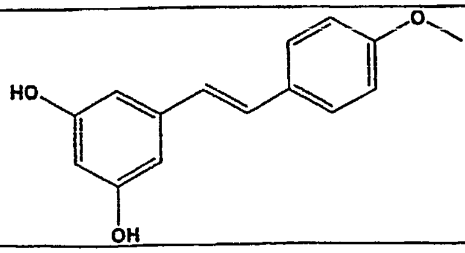 |

**Figure 31**
**Table 16. Resveratrol Analog Synthetic Intermediates**

| Compound | Benzylphosphonate | Aldehyde | Structure |
|---|---|---|---|
| Rhapontin aglycone (3,5,3'Trihydroxy-4'-methoxy-*trans*-stilbene) | N/A | N/A | 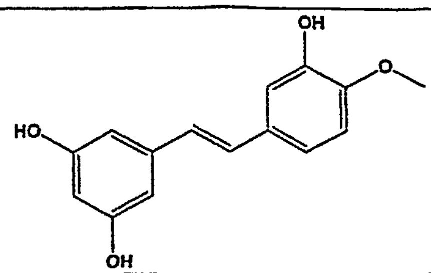 |
| BML-227 (3,4'-Dihydroxy-5-acetoxy-*trans*-stilbene) | N/A | N/A | 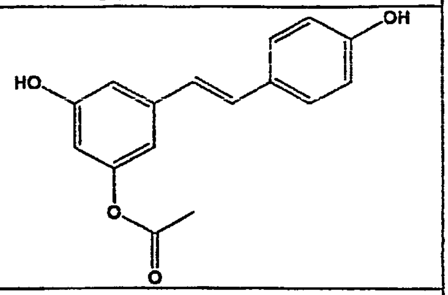 |
| BML-221 (3,5-Dihydroxy-4'-acetoxy-*trans*-stilbene) | N/A | N/A | 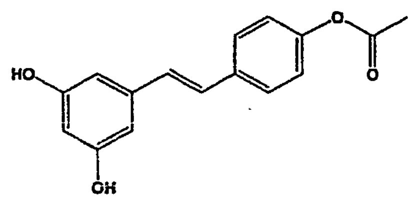 |
| BML-218 (E)-1-(3,5-Dihydroxyphenyl)-2-(2-napthyl) ethene | Diethyl 3-5-dimethoxybenzyl phosphonate | 2-Naphthaldehyde | 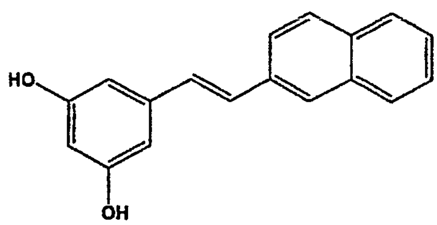 |
| BML-216 3-Hydroxystilbene | Benzylphosphonate | 3-Methoxy benzaldehyde | 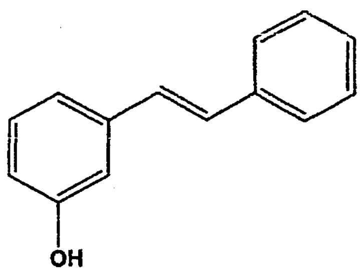 |

**Figure 32**
**Table 17. Resveratrol Analog Synthetic Intermediates**

| Compound | Benzylphosphonate | Aldehyde | Structure |
|---|---|---|---|
| BML-226 (3,5-Dimethoxymethoxy-4'-thiomethyl-*trans*-stilbene) | Diethyl 4-methylthio benzylphosphonate | 3,5dimethoxymethoxy benzaldehyde | 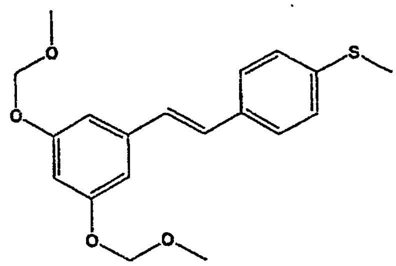 |
| BML-222 (3,5-Dihydroxy-4'-acetamide-*trans*-stilbene) | Diethyl 4-acetamido benzylphosphonate | 3,5-dimethoxymethoxy benzaldehyde | 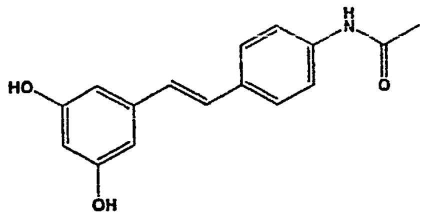 |
| BML-215 3,4-Dihydroxy-*trans*-stilbene | Benzylphosphonate | 3,4-Dimethoxy benzaldehyde | 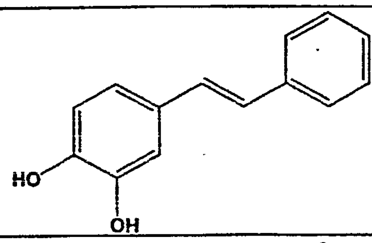 |
| BML-224 (E)-1-(3,5-Dihydroxyphenyl)-2-(cyclohexyl) ethene | 3,5-Dimethoxy benzylphosphonate | Cyclohexane carboxaldehyde | 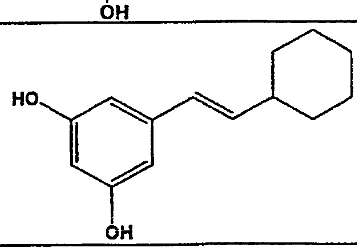 |
| 3,4-Dimethoxy-*trans*-stilbene | Benzylphosphonate | 3,4-Dimethoxy benzaldehyde | 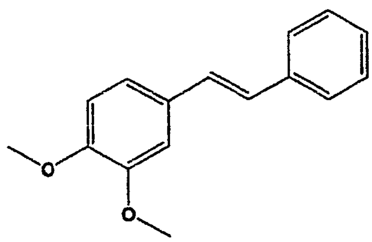 |

**Figure 33**
**Table 18. Resveratrol Analog Synthetic Intermediates**

| Compound | Benzylphosphonate | Aldehyde | Structure |
|---|---|---|---|
| Dihydroresveratrol (1-(3,5-Dihydroxyphenyl)-2-(4-hydroxyphenyl) ethane) | N/A | N/A | 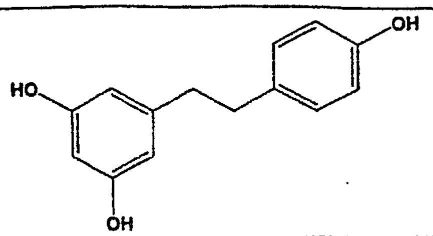 |
| BML-214 4-Hydroxy-*trans*-stilbene | Benzylphosphonate | 4-Methoxy benzaldehyde | 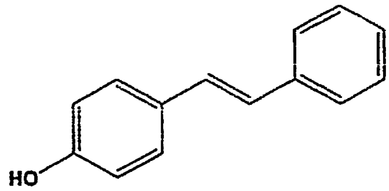 |
| BML-219 *N*-phenyl-(3,5-dihydroxy)benzamide | N/A | N/A | 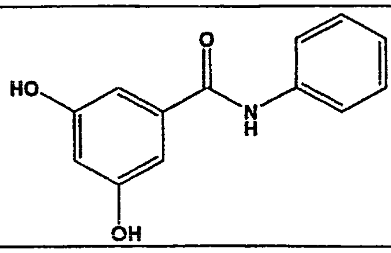 |
| 3,5-Dihydroxy-4'-nitro-*trans*-stilbene | 3,5-Dimethoxy benzylphosphonate | 4-Nitrobenzaldehdye | 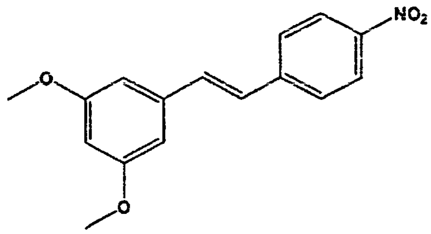 |
| 4-Methoxy-*trans*-stilbene | Benzylphosphonate | 4-Methoxy benzaldehyde | 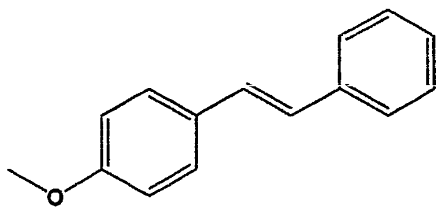 |

# Figure 34

## Table 20

Female

| Trial | Genotype | Diet | Treatment | N(0) | Median lifespan days | Median lifespan % change† | Mean lifespan days | Mean lifespan s.e. | Log-rank Test $\chi 2$ | Log-rank Test prob |
|---|---|---|---|---|---|---|---|---|---|---|
| 1 | Canton-S | 15% SY | control | 189 | 35 | | 34.5 | 0.83 | | |
| | | | 10μM Resv | 203 | 45 | **28.6** | 43.2 | 0.73 | 53.8 | <0.0001 |
| | | | 100μMResv | 189 | 41 | **17.1** | 41.8 | 0.75 | 34.2 | <0.0001 |
| | | | 200uM Resv | 189 | 36 | 2.9 | 36.6 | 0.65 | 0.14 | 0.71 |
| | | 5% SY | control | 198 | 66 | | 63.6 | 0.93 | | |
| | | | 10uM Resv | 203 | 63 | *-4.5* | 60 | 0.9 | 11.2 | 0.0008 |
| | | | 100uM Resv | 194 | 60 | *-9.1* | 60.8 | 0.87 | 8.7 | 0.0032 |
| | | | 200uM Resv | 202 | 66 | 0.0 | 63.9 | 0.99 | 0.99 | 0.32 |
| 2 | yw | 3% CSY | control | 80 | 29 | | 30.5 | 1.2 | | |
| | | | 10uM Resv | 93 | 32 | 10.3 | 34.6 | 1.1 | 5.5 | 0.019 |
| | | | 100uM Resv | 100 | 36 | **24.1** | 38 | 1.3 | 19.7 | <0.0001 |
| | | 2% CSY | control | 106 | 36 | | 37.9 | 1.3 | | |
| | | | 10μM Resv | 103 | 36 | 0.0 | 38.6 | 1.1 | 0.65 | 0.42 |
| | | | 100uM Resv | 127 | 37 | 2.8 | 38.4 | 1.2 | 0.003 | 0.95 |
| 3 | yw | 3% CSY | control | 237 | 43 | | 45.3 | 0.8 | | |
| | | | 10μM Resv | 223 | 47 | 9 3 | 46 5 | 0.78 | 0.16 | 0.69 |
| | | | 100uM Resv | 274 | 51 | **18.6** | 50.7 | 0.81 | 28.7 | <0.0001 |
| | | | 10μM Fisetin | 305 | 43 | 0.0 | 42 9 | 0.81 | 1.85 | 0.17 |
| | | | 100uM Fisetin | 288 | 53 | **23.3** | 48.6 | 0.76 | 10.3 | 0.0013 |
| | | 2% CSY | control | 311 | 47 | | 47.4 | 0.82 | | |
| | | | 10uM Resv | 456 | 53 | 12.8 | 49.7 | 0.66 | 2.45 | 0.118 |
| | | | 100uM Resv | 300 | 43 | *-8.5* | 43.2 | 0.74 | 21.5 | <0.0001 |
| | | | 10μM Fisetin | 307 | 45 | -4.3 | 47 | 0.82 | 0.11 | 0.737 |
| | | | 100μM Fisetin | 300 | 46 | -2.1 | 45.9 | 0.8 | 3.98 | 0.046 |
| 4 | SIR2 loss of function *dSir2* [4.5]/*dSir2* [5.26] | 15% SY | control | 175 | 58 | | 53.5 | 1.2 | | |
| | | | 100uM Resv | 196 | 54 | *-6.9* | 51.5 | 1 | 16.9 | <0.0001 |
| 5 | SIR2 hypomorphism *dSir2* [17]/KG00871 | 15% SY | control | 185 | 55 | | 51.7 | 0.84 | | |
| | | | 100μM Resv | 183 | 54 | -1.8 | 51.7 | 0.86 | 0.29 | 0.59 |
| 6 | SIR2 hypomorphism KG00871/KG00871 | 15% SY | control | 184 | 50 | | 47 | 1.1 | | |
| | | | 10μM Resv | 184 | 52 | **4.0** | 49.1 | 1.2 | 10.9 | 0.0009 |
| | | | 100μM Resv | 173 | 52 | **4.0** | 50.2 | 1 | 6.98 | 0.0083 |
| | | | 200μM Resv | 141 | 48 | -4.0 | 43.3 | 1.6 | 7.23 | 0.027 |
| 7 | SIR2 hypomorphism KG00871/Canton-S | 15% SY | control | 194 | 62 | | 59.2 | 1.3 | | |
| | | | 10μM Resv | 199 | 72 | **16.1** | 67.7 | 1.1 | 26.1 | <0.0001 |
| | | | 100uM Resv | 195 | 63 | 1.6 | 59.3 | 1.5 | 1.62 | 0.202 |
| | | | 200uM Resv | 186 | 73 | **17.7** | 67 | 1.2 | 22.1 | <0.0001 |

† percent change is relative to control

**Bold: increase in lifespan** at significance criterion: p < 0.01

*Italics: decrease in lifespan* at signficance criterion: p < 0.01

SY: sugar-yeast diet CSY: cornmeal-sugar-yeast diet

## Figure 34

### Table 20 (cont.)

MALE

| Trial | Genotype | Diet | Treatment | N(0) | Median lifespan days | Median lifespan % change† | Mean lifespan days | Mean lifespan s.e. | Log-rank Test χ 2 | Log-rank Test prob |
|---|---|---|---|---|---|---|---|---|---|---|
| 1 | Canton-S | 15% SY | control | 189 | 52 | | 47.4 | 0.51 | | |
| | | | 10μM Resv | 182 | 56 | **7.7** | 53.9 | 0.11 | 17.8 | <0.0001 |
| | | | 100μMResv | 188 | 53 | 1.9 | 50.8 | 0.9 | 0.8 | 0.383 |
| | | | 200μM Resv | 198 | 49 | -5.8 | 47.9 | 0.95 | 0.0 | 0.838 |
| | | 5% SY | control | 180 | 67 | | 67.9 | 0.93 | | |
| | | | 10μM Resv | 180 | 66 | *-1.5* | 63.8 | 1.1 | 7.0 | 0.0081 |
| | | | 100μM Resv | 179 | 70 | 4.5 | 70.2 | 0.95 | 3.2 | 0.07 |
| | | | 200μM Resv | 174 | 70 | 4.5 | 69.1 | 1.2 | 5.4 | 0.02 |
| 2 | yw | 3% CSY | control | 113 | 38 | | 40.1 | 1.1 | | |
| | | | 10μM Resv | 98 | 40 | 5.3 | 44 | 1.2 | 3.8 | 0.053 |
| | | | 100μM Resv | 118 | 49 | **28.9** | 47.5 | 1.2 | 16.4 | <0.0001 |
| | | 2% CSY | control | 118 | 41 | | 42.7 | 1.2 | | |
| | | | 10μM Resv | 102 | 43 | 4.9 | 43.6 | 1.3 | 1.3 | 0.26 |
| | | | 100μM Resv | 100 | 51 | **24.4** | 48.4 | 1.6 | 21.7 | <0.0001 |
| 3 | yw | 3% CSY | control | 210 | 55 | | 53.5 | 1.2 | | |
| | | | 10μM Resv | 218 | 65 | **18.2** | 57.9 | 1.3 | 14 0 | 0.0002 |
| | | | 100μM Resv | 308 | 64 | **16.4** | 62 | 0.97 | 38.7 | <0.0001 |
| | | | 10μM Fisetin | 284 | 50 | -9.1 | 52.2 | 1 | 0.0 | 0.958 |
| | | | 100uM Fisetin | 285 | 67 | **21.8** | 60.4 | 0 94 | 17.2 | <0.0001 |
| | | 2% CSY | control | 281 | 58 | | 57.6 | 0.98 | | |
| | | | 10μM Resv | 284 | 55 | -5.2 | 55.2 | 1 | 1.6 | 0.21 |
| | | | 100μM Resv | 290 | 48 | *-17.2* | 50.1 | 0.88 | 42.8 | <0.0001 |
| | | | 10μM Fisetin | 274 | 54 | *-6.9* | 54.1 | 0.99 | 7.8 | 0.0052 |
| | | | 100μM Fisetin | 290 | 52 | *-10.3* | 51.6 | 1 | 17.1 | <0.0001 |
| 4 | SIR2 loss of function *dSir2* [4.5]/*dSir2* [5.26] | 15% SY | control | 168 | 64 | | 61.7 | 1.1 | | |
| | | | 100μM Resv | 166 | 61 | *-4.7* | 57.3 | 0.94 | 24.5 | <0.0001 |
| 5 | SIR2 hypomorphism *dSir2* [17]/KG00871 | 15% SY | control | 168 | 38 | | 39.4 | 0.72 | | |
| | | | 100μM Resv | 177 | 40 | 5.3 | 40.6 | 0.78 | 1.6 | 0.21 |
| 6 | SIR2 hypomorphism KG00871/KG00871 | 15% SY | control | 167 | 53 | | 50.1 | 1.2 | | |
| | | | 10μM Resv | 152 | 59 | **11.3** | 55.9 | 1.1 | 8.4 | 0.0037 |
| | | | 100μM Resv | 163 | 59 | **11.3** | 56.4 | 1 | 10.8 | 0.001 |
| | | | 200μM Resv | 139 | 54 | 1.9 | 50.8 | 1.5 | 2.4 | 0.125 |
| 7 | SIR2 hypomorphism KG00871/Canton-S | 15% SY | control | 172 | 68 | | 67.2 | 0.85 | | |
| | | | 10μM Resv | 185 | 74 | **8.8** | 69 | 1.2 | 7.9 | 0.005 |
| | | | 100μM Resv | 171 | 69 | 1.5 | 64.8 | 1.3 | 0.4 | 0.507 |
| | | | 200uM Resv | 176 | 73 | **7.4** | 71.1 | 0.94 | 14.3 | 0.0002 |

† percent change is relative to control

**Bold: increase in lifespan** at significance criterion: $p < 0.01$

*Italics: decrease in lifespan* at signficance criterion: $p < 0.01$

SY: sugar-yeast diet    CSY: cornmeal-sugar-yeast diet

Figure 35A

**Table 21.** Sirtuin activators.

| Compound | Fold Activation | Structure | Included in formula number |
|---|---|---|---|
| 2-[1-(2-hydroxyphenyl) ethylidene] hydrazine-1-carbothioamide | 1.1 | 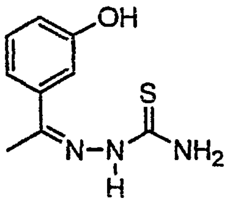 | 32 |
| prop-2-ynyl 3-(2,6-dichlorophenyl)-5-methylisoxazole-4-carboxylate | 1.1 | 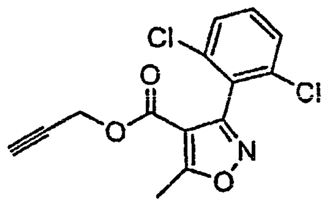 | 33 |
| 4-{3-[(3,5-dichloro-2-hydroxybenzylidene)amino]propyl}-4,5-dihydro-1H-pyrazol-5-one | 1.2 | 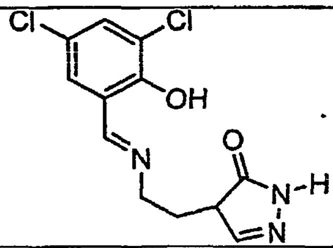 | 34 |
| 6-(phenylthio)-2-[2-(2-pyridyl)ethyl]-2,3-dihydro-1H-benzo[de]isoquinoline-1,3-dione | 1.15 | 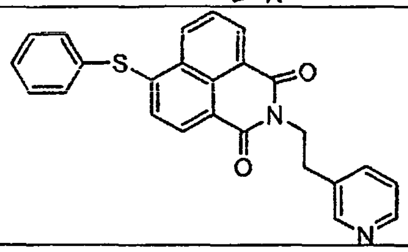 | 35 |
| 5-[(4-chloroanilino)methylene]-3-(4-chlorophenyl)-1lambda~6~,3-thiazolane-1,1,4-trione | 1.15 | 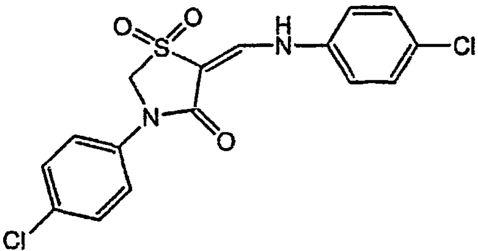 | 36 |
| 2-(4-chlorophenyl)-7-methylimidazo[1,2-a]pyridine-3-carbaldehyde O-(3-fluorobenzyl)oxime | 1.1 | 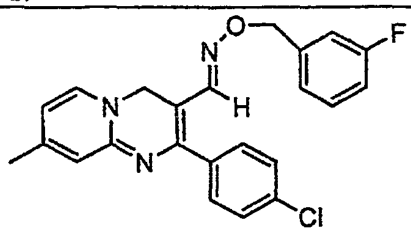 | 37 |

Figure 35B

| | | | |
|---|---|---|---|
| 2-(4-tert-butylphenoxy)-N-(3-methoxyphenyl)acetamide | 1.12 | 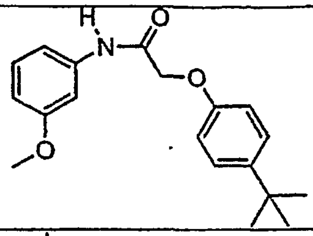 | 38 |
| 3,4,5-trimethoxy-N-(4-methyl-1,3-benzothiazol-2-yl)benzamide | 1.12 | 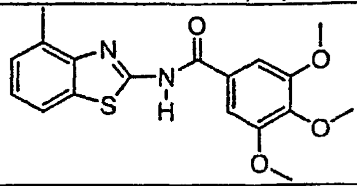 | 39 |
| 3-(1,3-benzodioxol-5-yl)-N-(pentafluorophenyl)acrylamide | 1.09 | 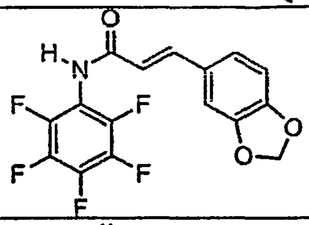 | 40 |
| 'ethyl [(4-cyano-1-morpholin-4-yl-5,6,7,8-tetrahydroisoquinolin-3-yl)thio]acetate | 1.11 | 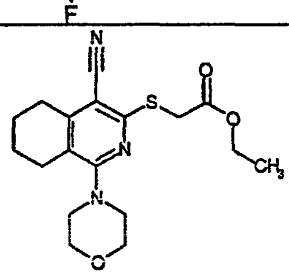 | 41 |
| 'ethyl 2-({[5-(4-methylphenyl)-7-(trifluoromethyl)-4,5,6,7-tetrahydropyrazolo[1,5-a]pyrimidin-3-yl]carbonyl}amino)-4,5,6,7-tetrahydro-1-benzothiophene-3-carboxylate | 1.1 | 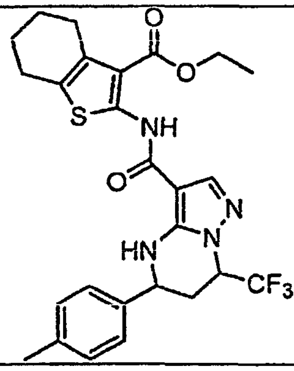 | 42 |
| '6-amino-3-(4-bromophenyl)-4-(3-hydroxy-4-methoxyphenyl)-1,4-dihydropyrano[2,3-c]pyrazole-5-carbonitrile | 1.1 | 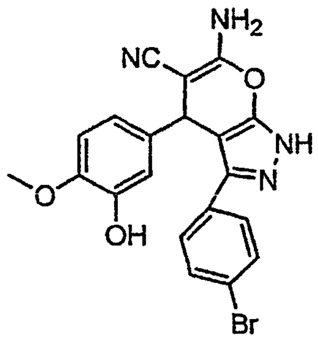 | 43 |

Figure 35C

| | | | |
|---|---|---|---|
| 'dimethyl 5-{[({4-oxo-5-[3-(trifluoromethyl)phenyl]-4,5-dihydro-1H-pyrazolo[3,4-d]pyrimidin-6-yl}thio)acetyl]amino}isophthalate | 1.08 | 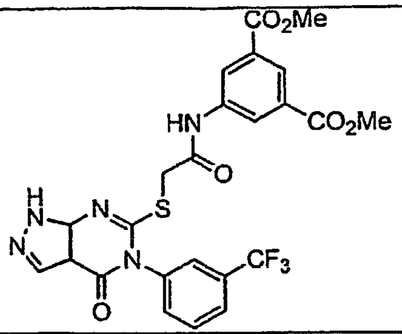 | 44 |
| 'N-{2-[4-(aminosulfonyl)phenyl]ethyl}-2-{[4-oxo-3-(tetrahydrofuran-2-ylmethyl)-3,4-dihydroquinazolin-2-yl]thio}acetamide | 1.05 | 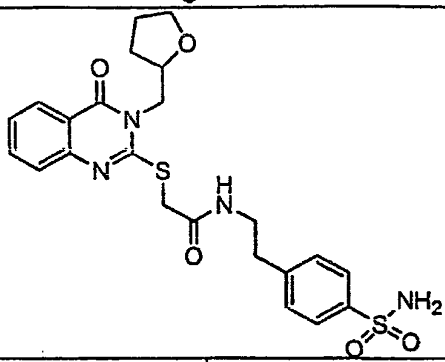 | 45 |
| 'N-{3-chloro-4-[(4-chloro-1-naphthyl)oxy]phenyl}-2-hydroxy-3,5-diiodobenzamide | 1.24 | 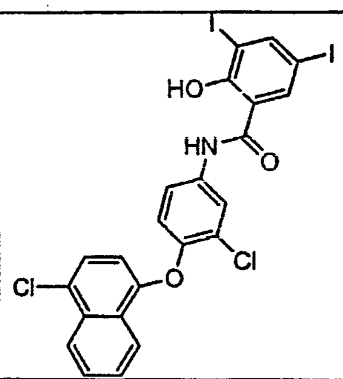 | 46 |
| | 1.2 | 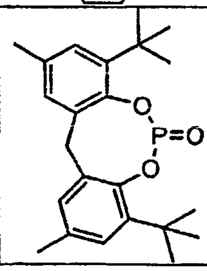 | 47 |

**Figure 35D**

| | | | |
|---|---|---|---|
| 'tetramethyl 5',5',9'-trimethyl-6'-(trifluoroacetyl)-5',6'-dihydrospiro[1,3-dithiole-2,1'-thiopyrano[2,3-c]quinoline]-2',3',4,5-tetracarboxylate | 1.14 | 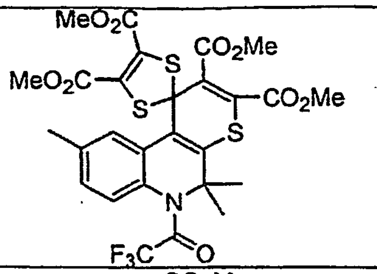 | 48 |
| 'dimethyl 2-[2,2,6-trimethyl-1-(3-methylbutanoyl)-3-thioxo-2,3-dihydroquinolin-4(1H)-ylidene]-1,3-dithiole-4,5-dicarboxylate | 1.17 | 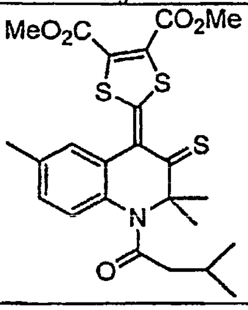 | 49 |
| 'ethyl 4-[5-[(cyanomethyl)thio]-2-thioxo[1,3]thiazolo[4',5':4,5]pyrimido[1,6-a]benzimidazol-3(2H)-yl]benzoate | 1.47 | 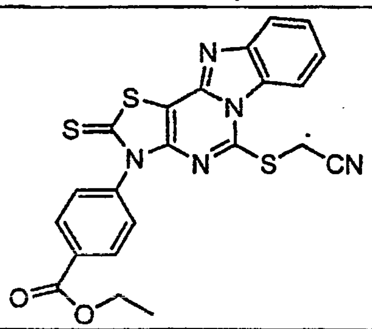 | 50 |
| '6-chloro-2,3-diphenyl-7-(trifluoromethyl)quinoxaline | 1.12 | 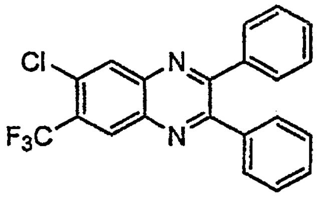 | 51 |
| '6-fluoro-2,3-bis(4-methylphenyl)quinoxaline | 1.27 | 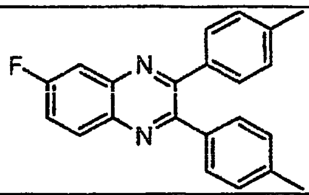 | 51 |
| | 1.1 | 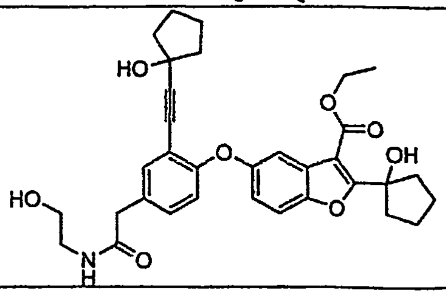 | 52 |

Figure 35E

| | | | |
|---|---|---|---|
| | 1.28 | 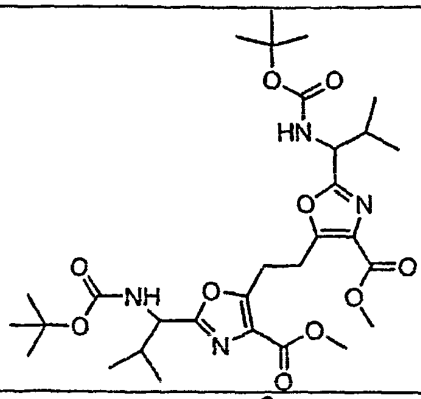 | 53 |
| Pyridine, 2-(p-chlorostyryl)-4-[[4-(diethylamino)-1-methylbutyl]amino]-, (E)- | 1.06 | 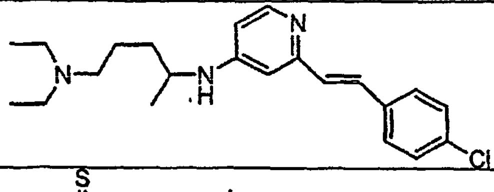 | 54 |
| Gloxazone | 1.16 | 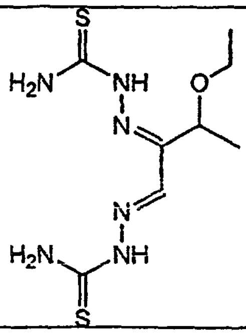 | 55 |
| | 1.25 | 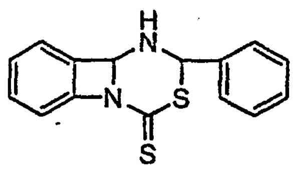 | 56 |
| | 1.1 | 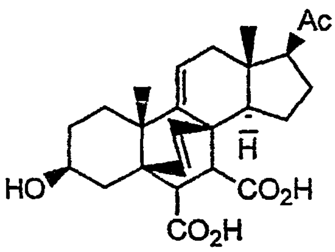 | 57 |

**Figure 35F**

| | | | |
|---|---|---|---|
| Ouabaine | 1.07 | 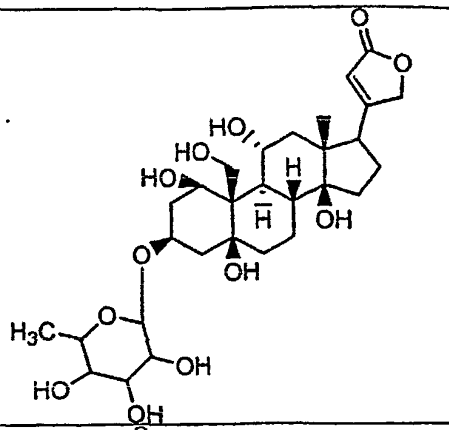 | 58 |
| | 1.16 | 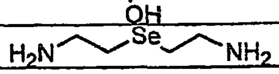 | 59 |
| | 1.06 | 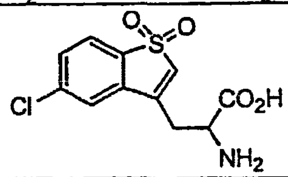 | 60 |
| Pinosylvin | 3.28 | 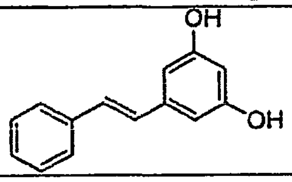 | 61 |
| Resveratrol 4"-Methyl Ether | 2.1 | 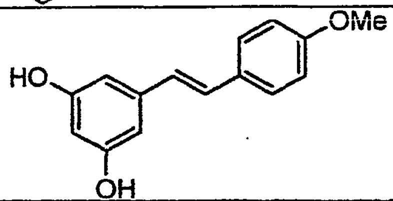 | 1 |
| Resveratrol | 2.2 | 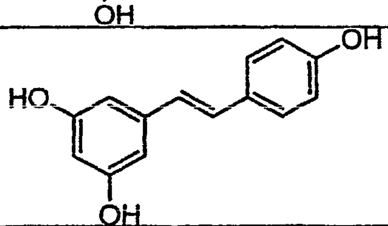 | 1 |
| Aloin | 1.2 | 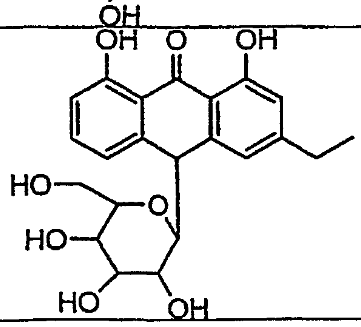 | 62 |

Figure 35G

| | | | |
|---|---|---|---|
| Piromidic Acid | 1.47 | 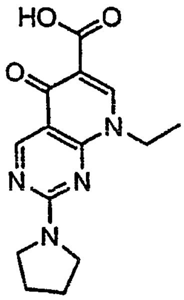 | 63 |
| Meclocycline Sulfosalicylate | 1.12 | 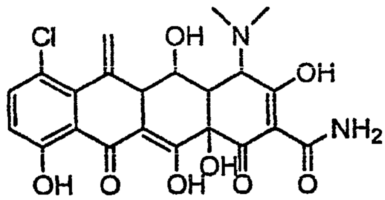 | 64 |
| Methacycline Hydrochloride | 1.14 | 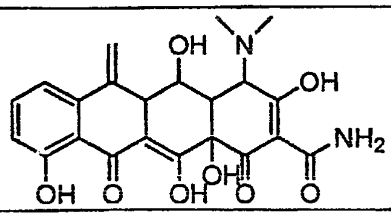 | 64 |
| Ofloxacin | 1.5 | 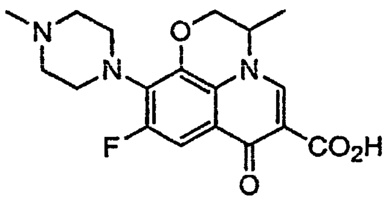 | 65 |

**Figure 36**

**Table 22.** Sirtuin inhibitors

| Compound | Fold Activation | Structure | Included in formula number |
|---|---|---|---|
| Chlortetracycline | <1 | 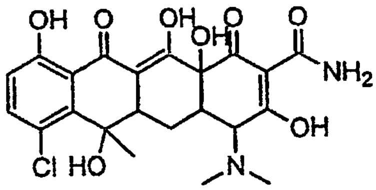 | 66 |
| | 0.27 | 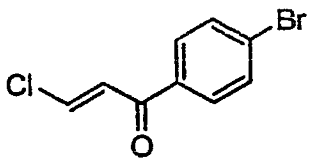 | 67 |
| Methotrexane | 0.53 | 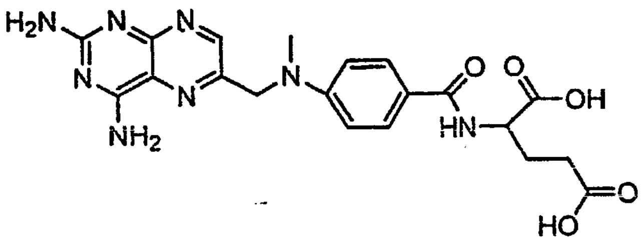 | 68 |

Figure 37

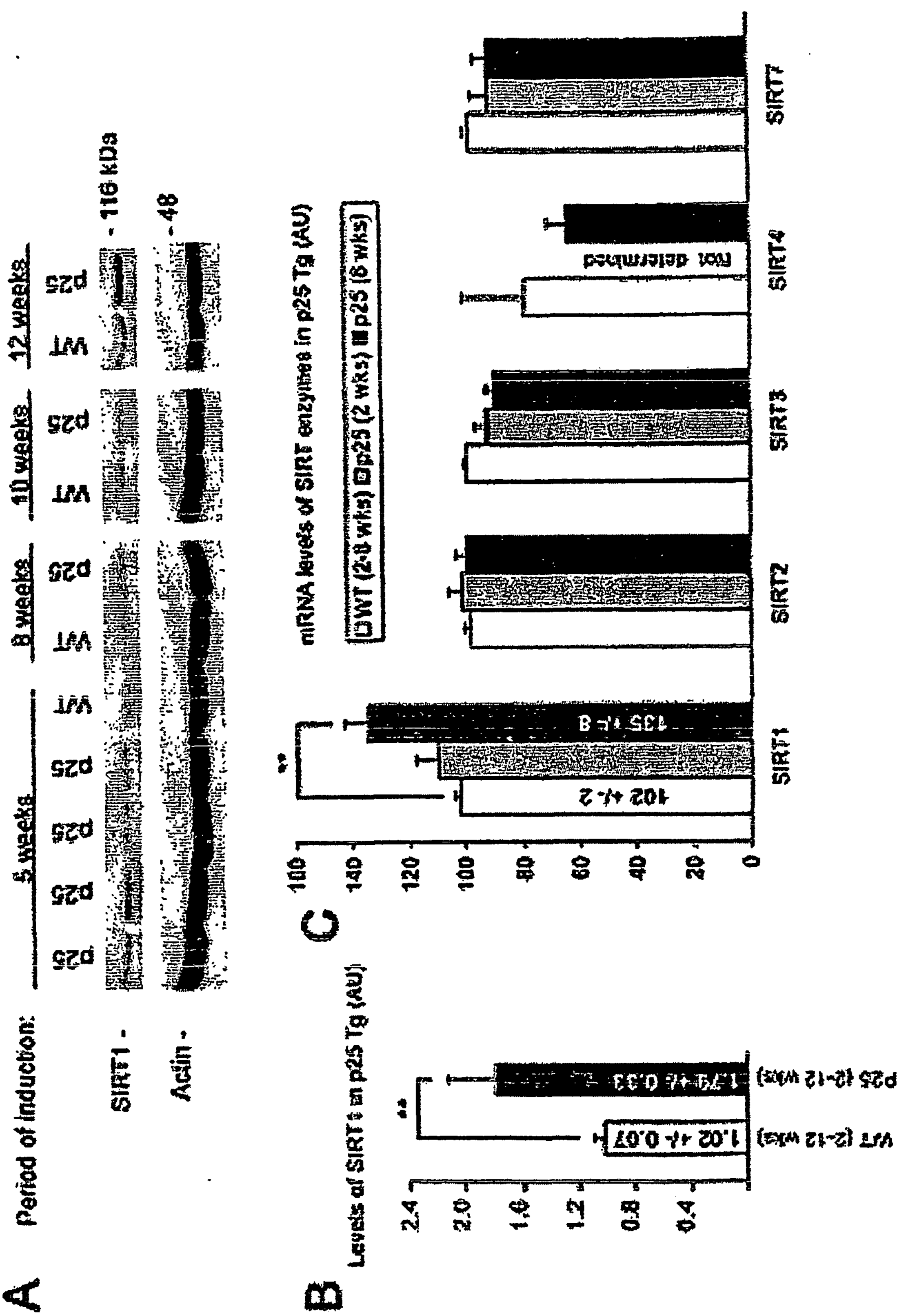
A
Period of induction:
5 weeks
8 weeks
10 weeks
12 weeks
SIRT1 -
Actin -
- 116 kDa
- 48
B
Levels of SIRT1 in p25 Tg (AU)
1.02 +/- 0.07
1.74 +/- 0.33
WT (2-12 wks)
p25 (2-12 wks)
C
mRNA levels of SIRT enzymes in p25 Tg (AU)
WT (2-8 wks)
p25 (2 wks)
p25 (8 wks)
102 +/- 2
135 +/- 8
not determined
SIRT1
SIRT2
SIRT3
SIRT4
SIRT7

Figure 37

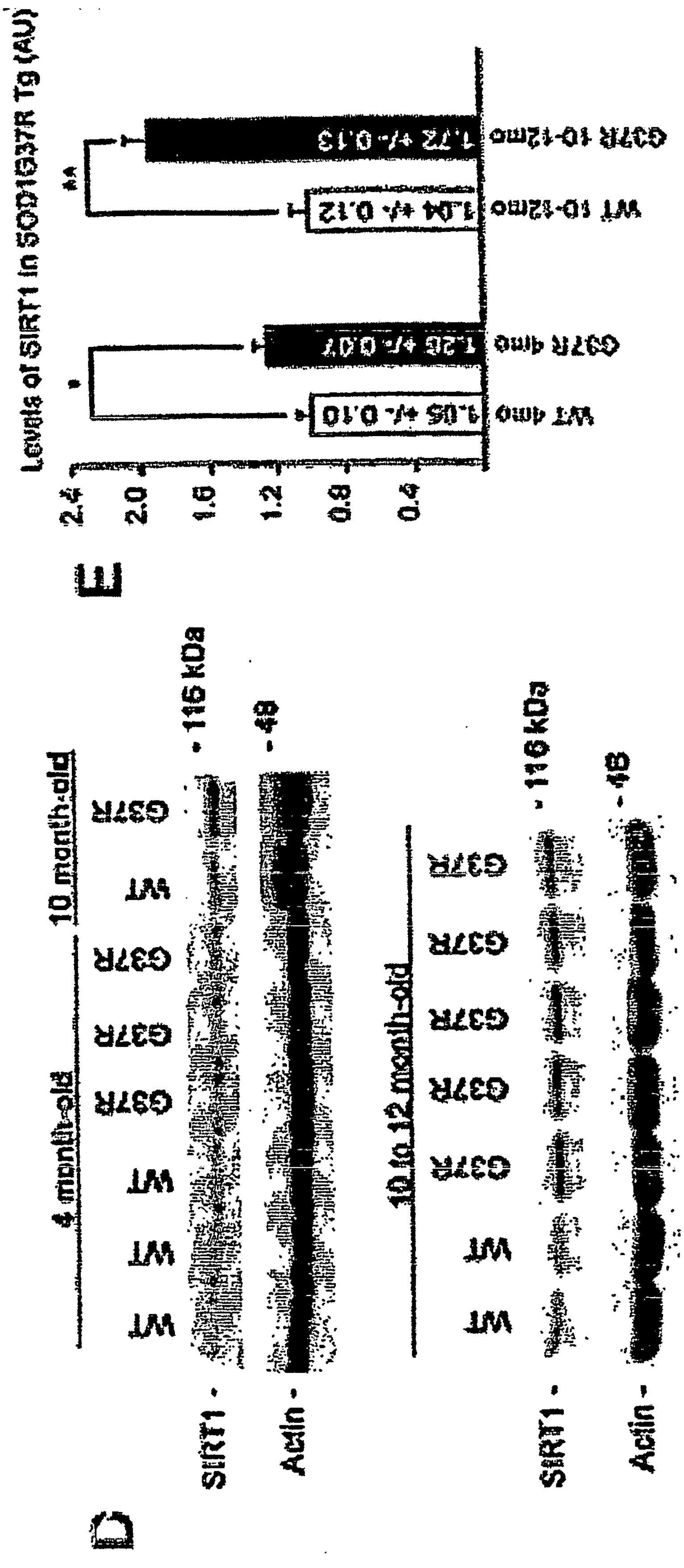
D
4 month-old
10 month-old
WT
WT
WT
G37R
G37R
G37R
WT
G37R
SIRT1 -
- 116 kDa
Actin -
- 48
10 to 12 month-old
WT
WT
G37R
G37R
G37R
G37R
G37R
SIRT1 -
- 116 kDa
Actin -
- 48
E
Levels of SIRT1 in SOD1G37R Tg (AU)
2.4
2.0
1.6
1.2
0.8
0.4
WT 4mo
G37R 4mo
WT 10-12mo
G37R 10-12mo
1.05 +/- 0.10
1.26 +/- 0.07
1.04 +/- 0.12
1.72 +/- 0.13
*
**

Figure 37

F

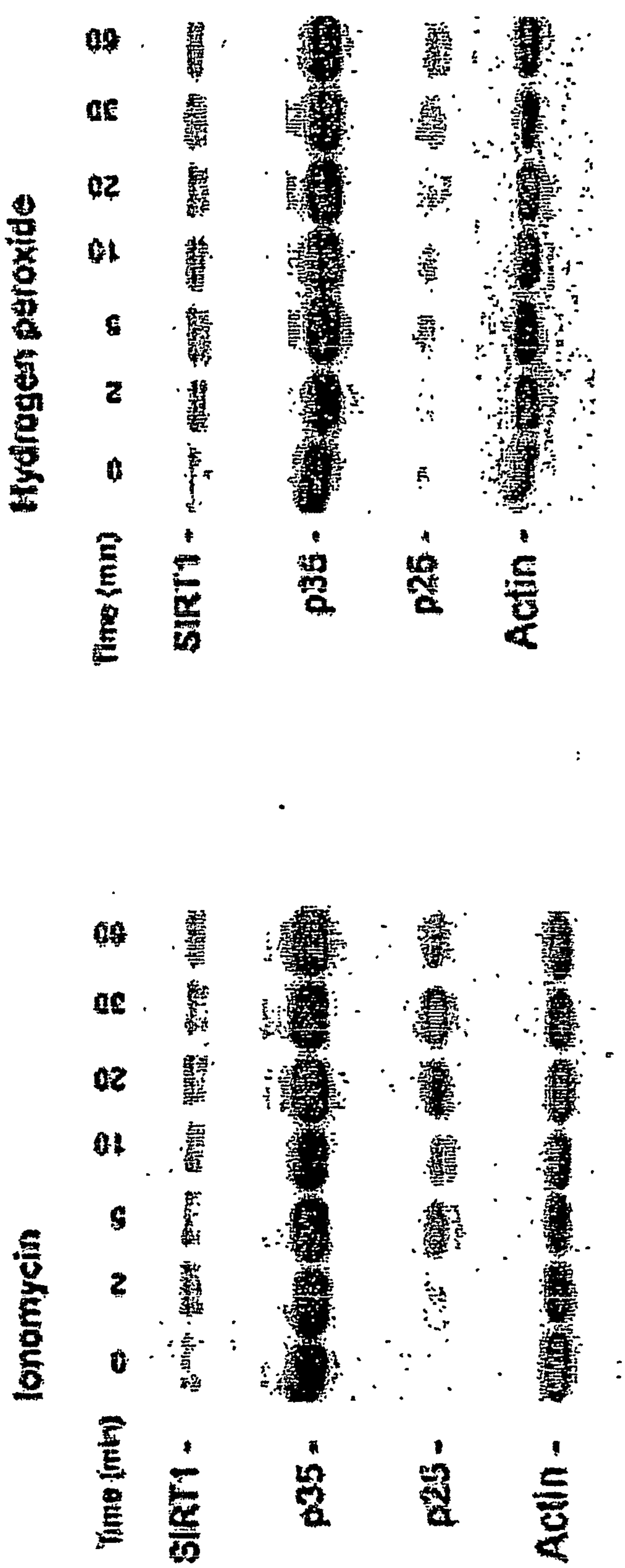
Ionomycin
Time (min)
0
2
5
10
20
30
60
SIRT1 -
p35 -
p25 -
Actin -
Hydrogen peroxide
Time (min)
0
2
5
10
20
30
60
SIRT1 -
p35 -
p25 -
Actin -

Figure 38

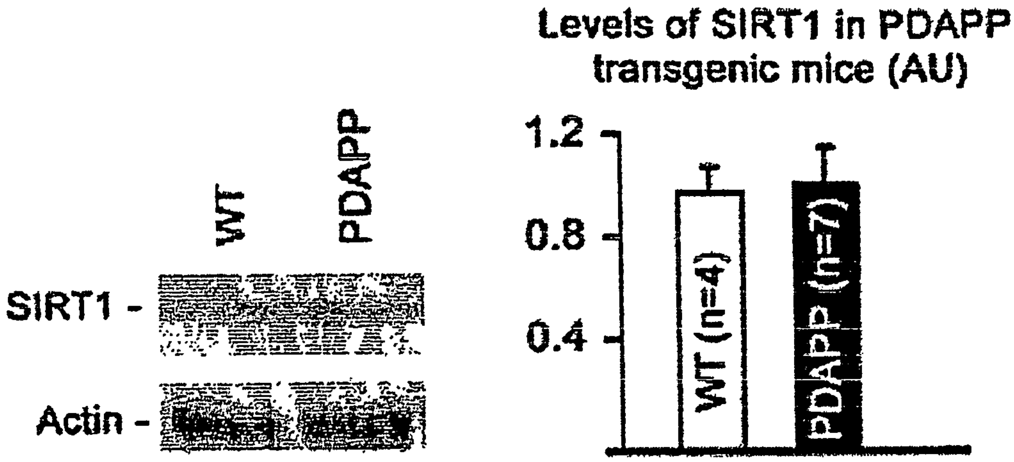
WT
PDAPP
SIRT1 -
Actin -
Levels of SIRT1 in PDAPP transgenic mice (AU)
1.2
0.8
0.4
WT (n=4)
PDAPP (n=7)

Figure 39

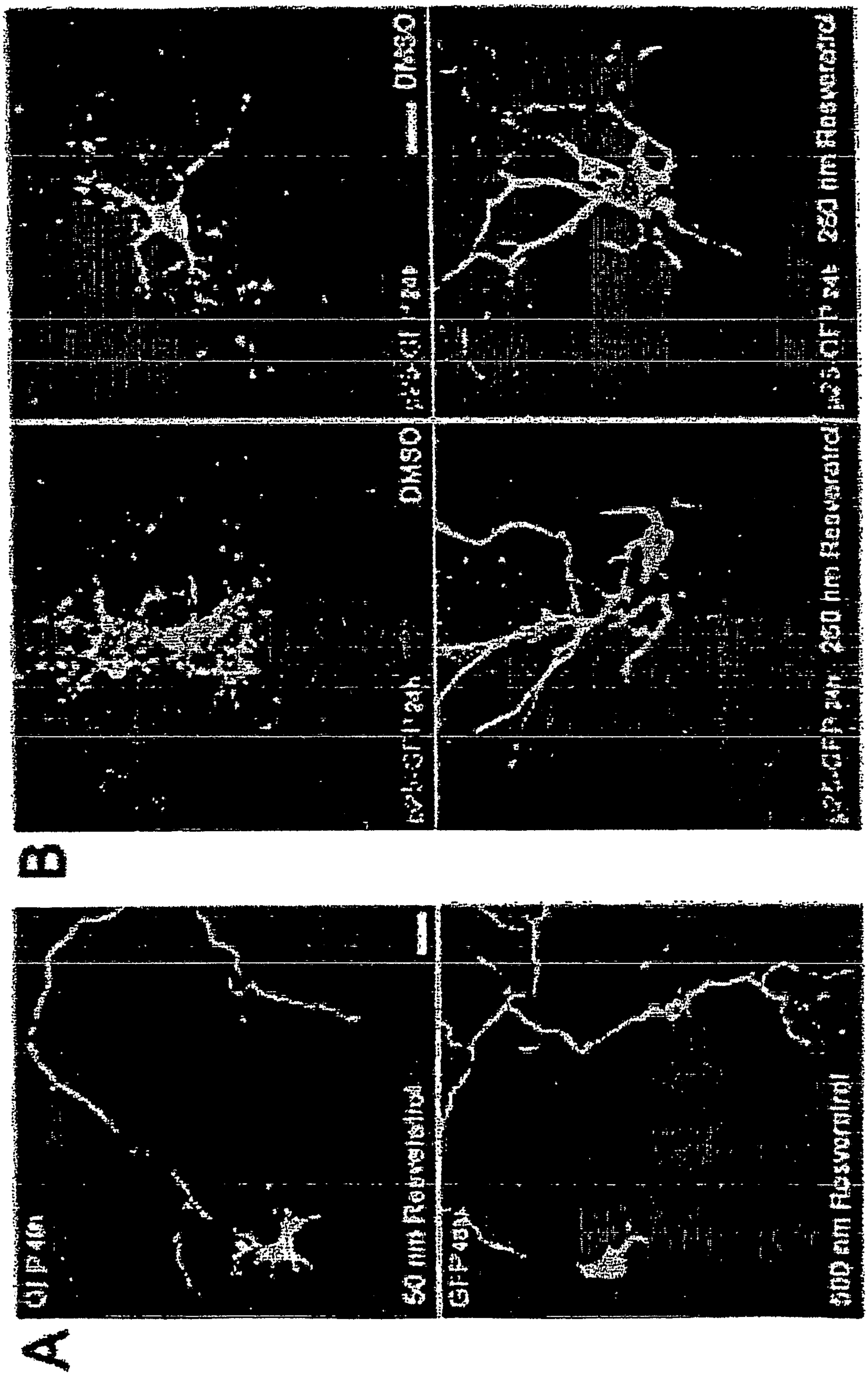
A
B
DMSO
DMSO
50 nm Resveratrol
500 nm Resveratrol
250 nm Resveratrol
250 nm Resveratrol

Figure 39

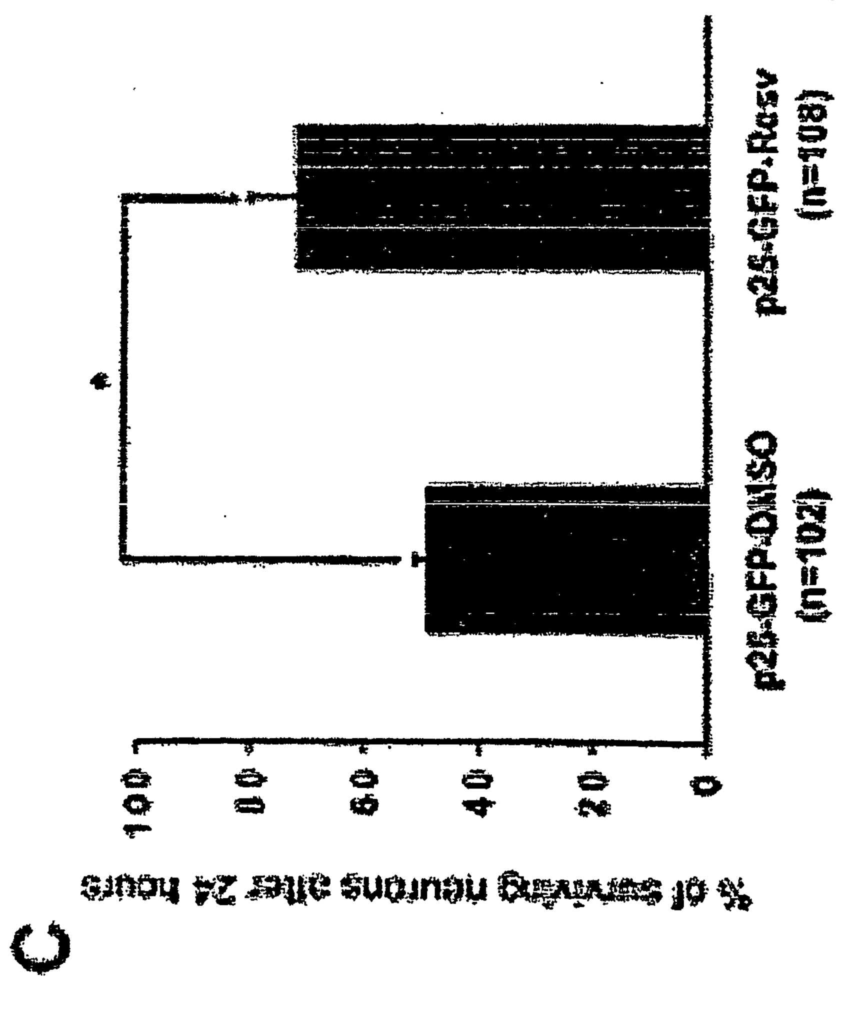
C
% of surviving neurons after 24 hours
0
20
40
60
80
100
*
p25-GFP-DMSO
(n=102)
p25-GFP-Rasv
(n=108)

Figure 39

D

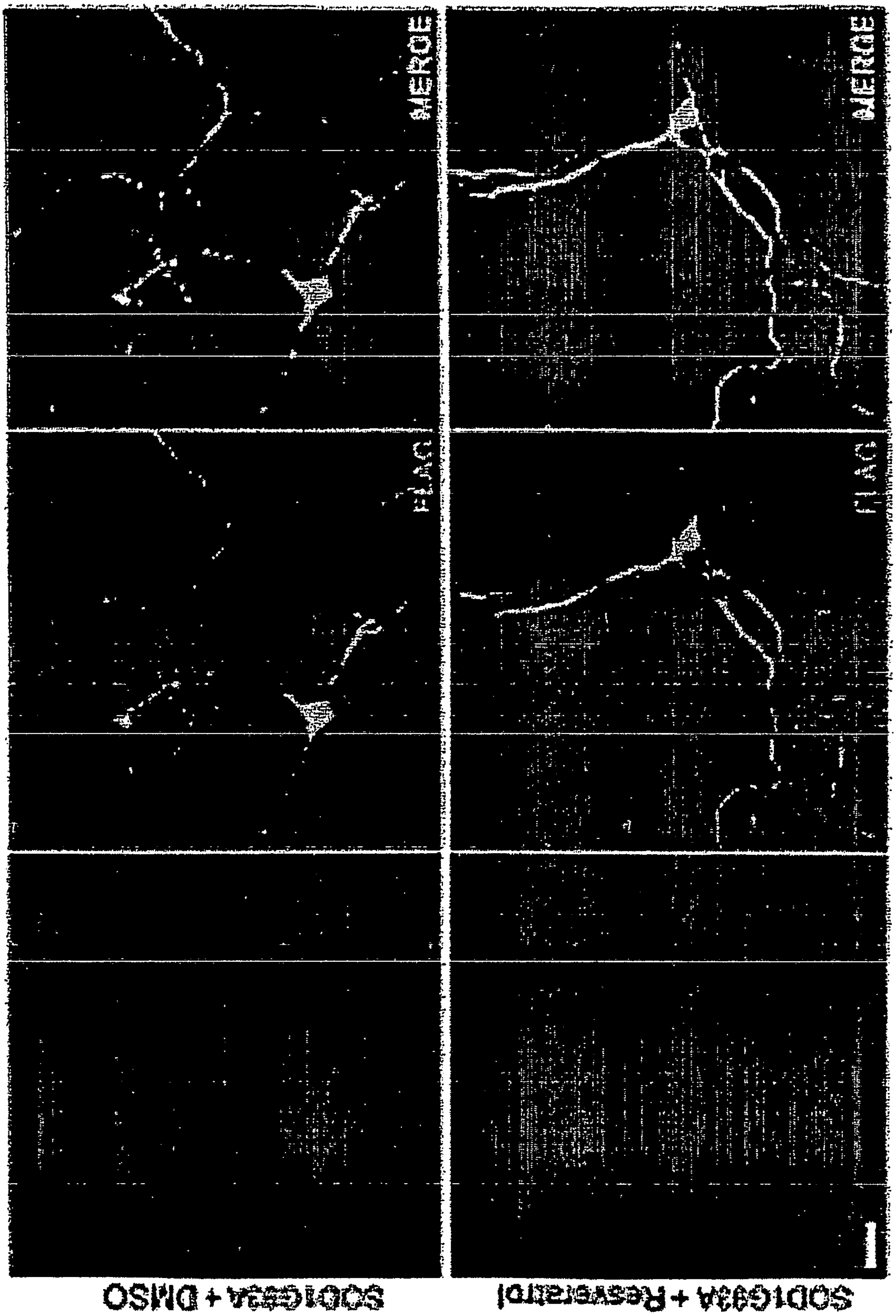

Figure 39

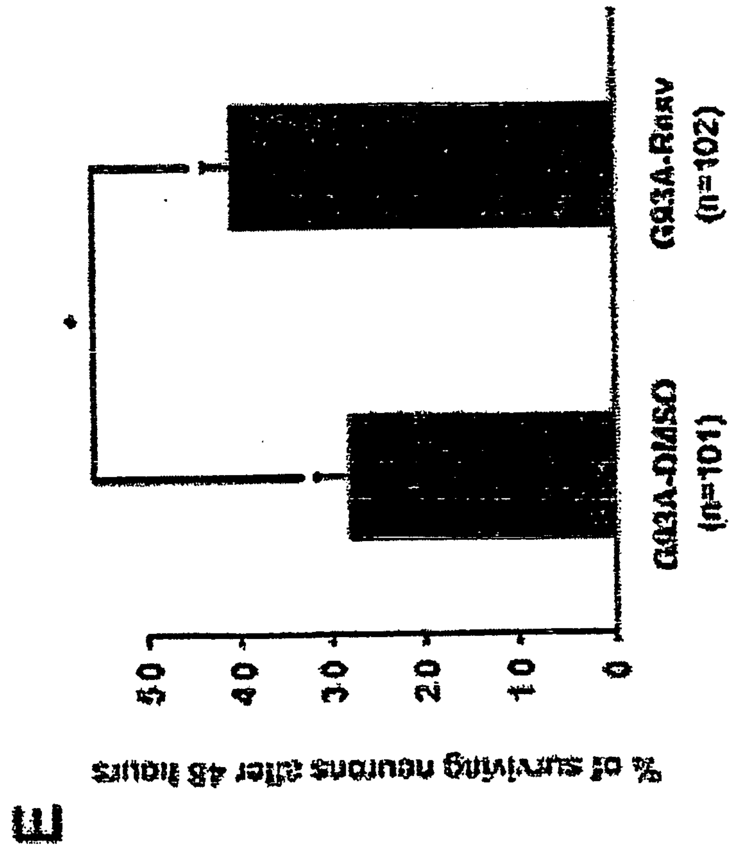
E
% of surviving neurons after 48 hours
0
10
20
30
40
50
*
G93A-DMSO
(n=101)
G93A-Rasv
(n=102)

Figure 40

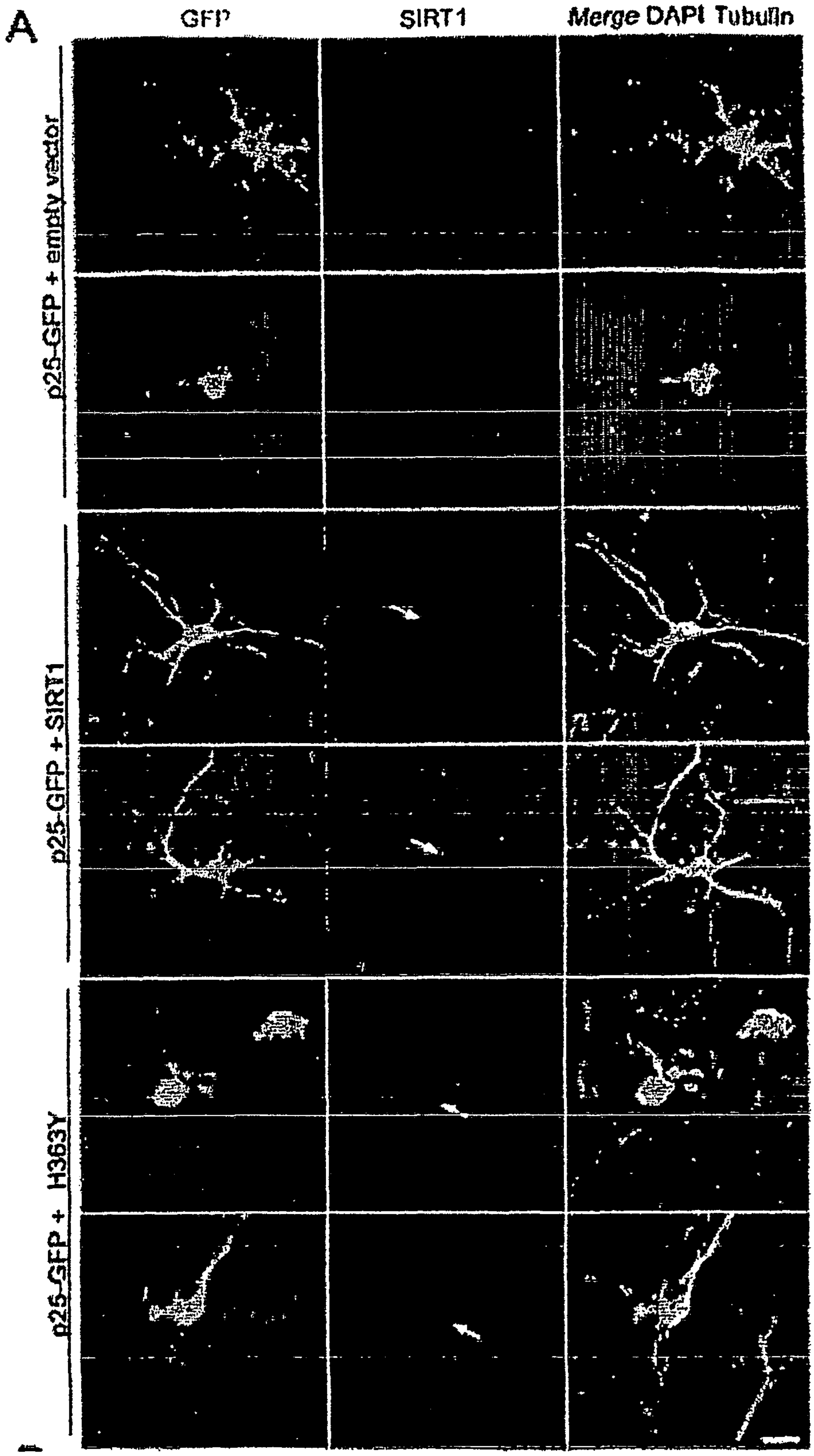
A
GFP
SIRT1
Merge DAPI Tubulin
p25-GFP + empty vector
p25-GFP + SIRT1
p25-GFP + H363Y

Figure 40

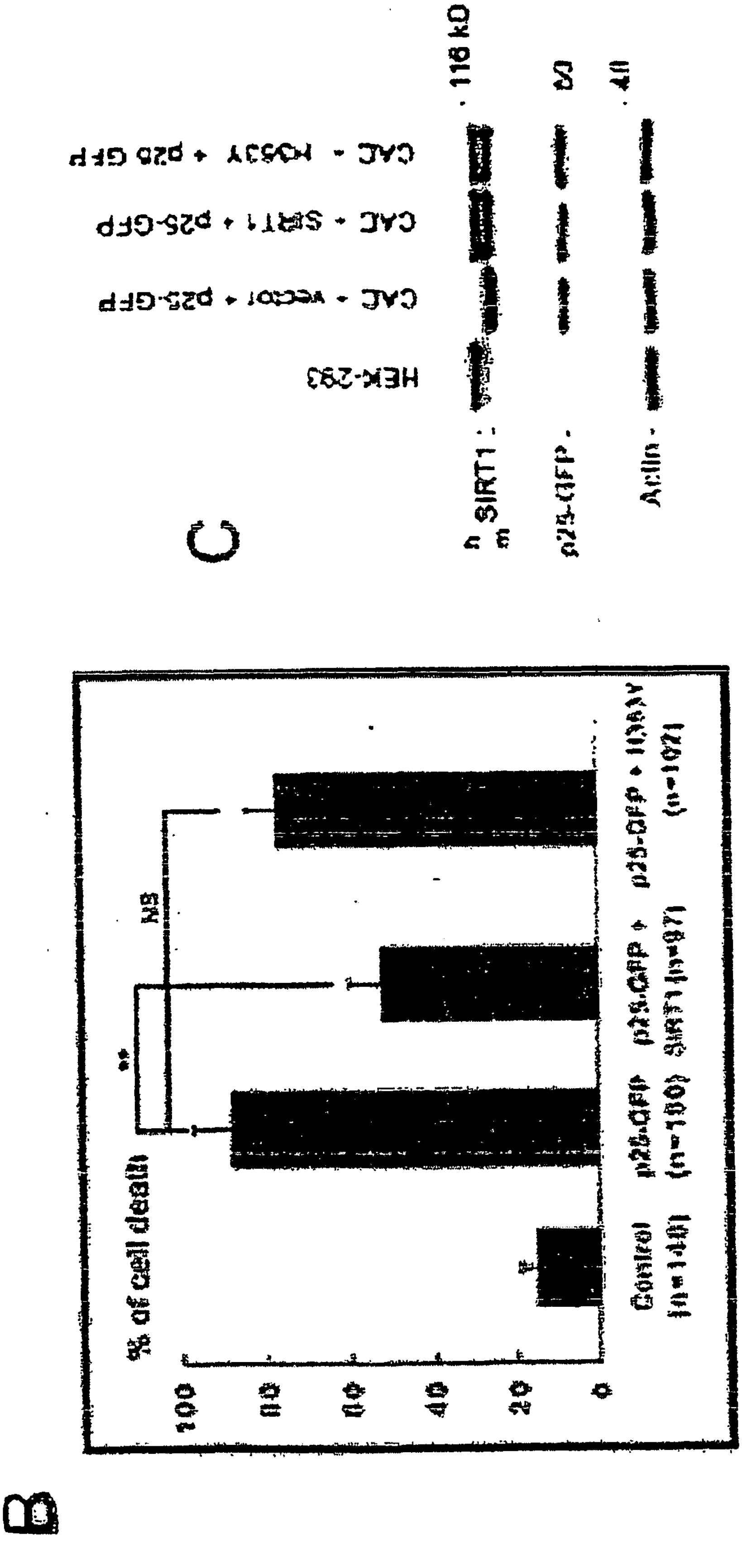
B
% of cell death
100
80
60
40
20
0
Control
p25-GFP
NS
**
C
HEK-293
CAC + vector + p25-GFP
CAC + SIRT1 + p25-GFP
CAC + H363Y + p25 GFP
SIRT1
p25-GFP
Actin
116 kD

D

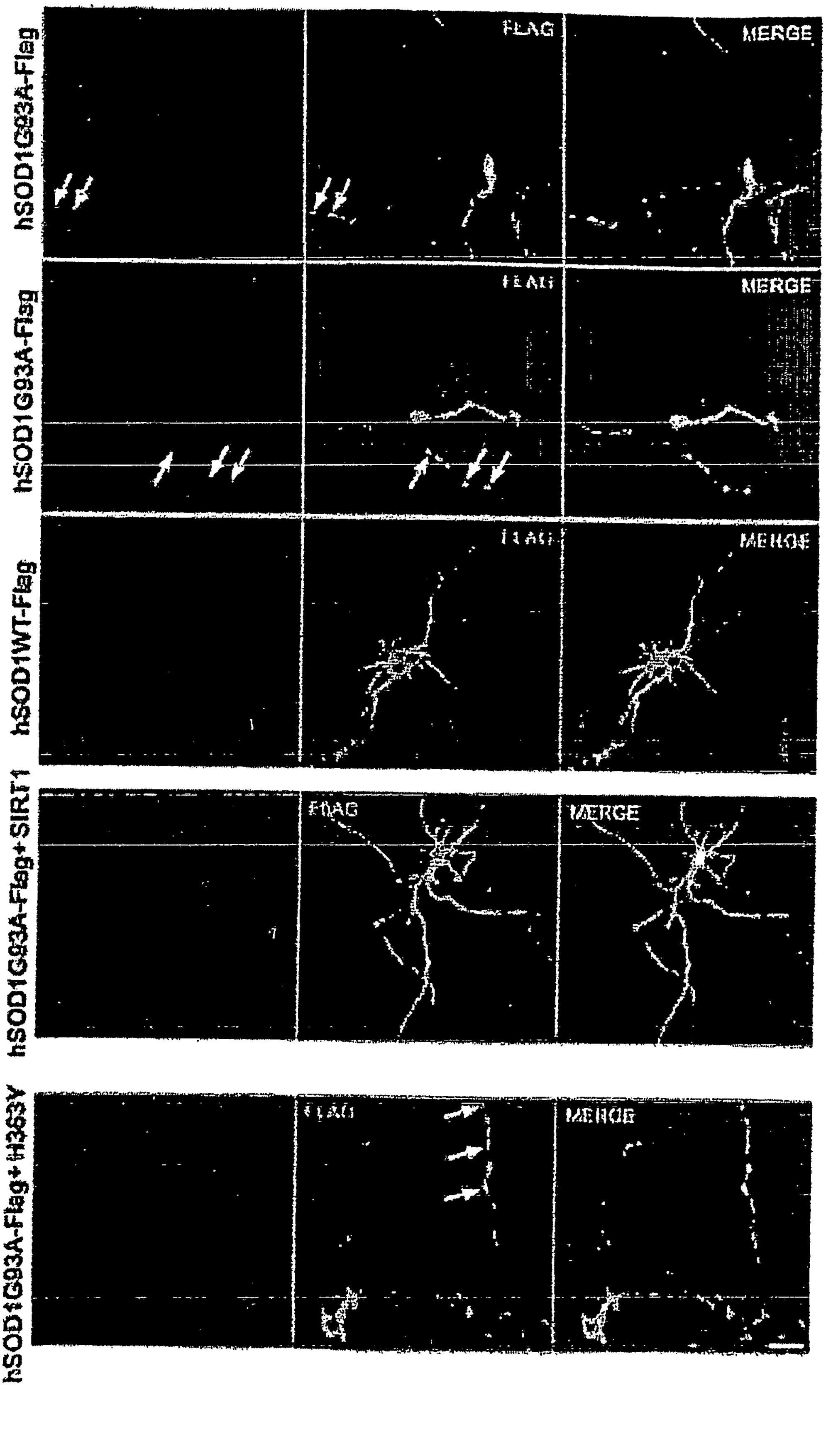
hSOD1G93A-Flag
FLAG
MERGE
hSOD1G93A-Flag
FLAG
MERGE
hSOD1WT-Flag
FLAG
MERGE
hSOD1G93A-Flag+SIRT1
FLAG
MERGE
hSOD1G93A-Flag+H363Y
FLAG
MERGE

Figure 40E

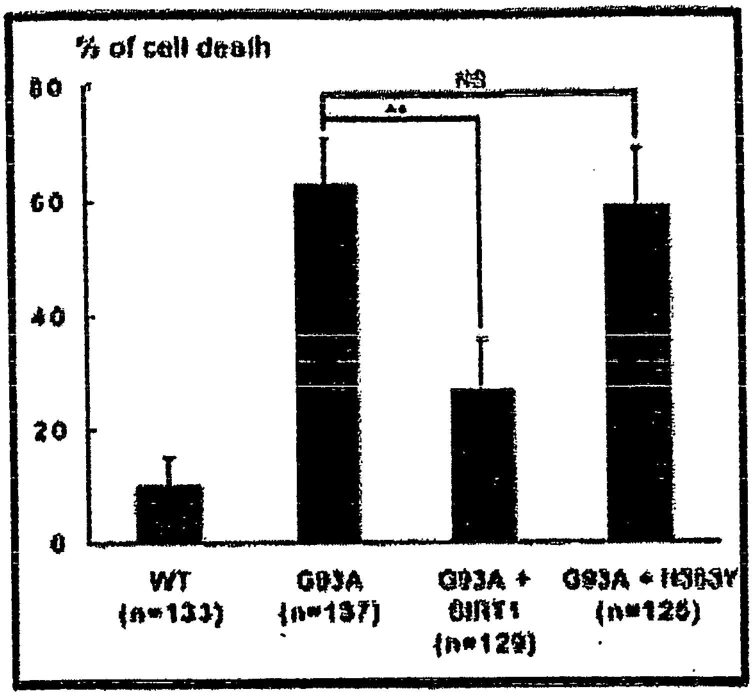
% of cell death
80
60
40
20
0
NS
**
WT
(n=131)
G93A
(n=137)
G93A +
SIRT1
(n=129)
G93A + H363Y
(n=125)

Figure 41

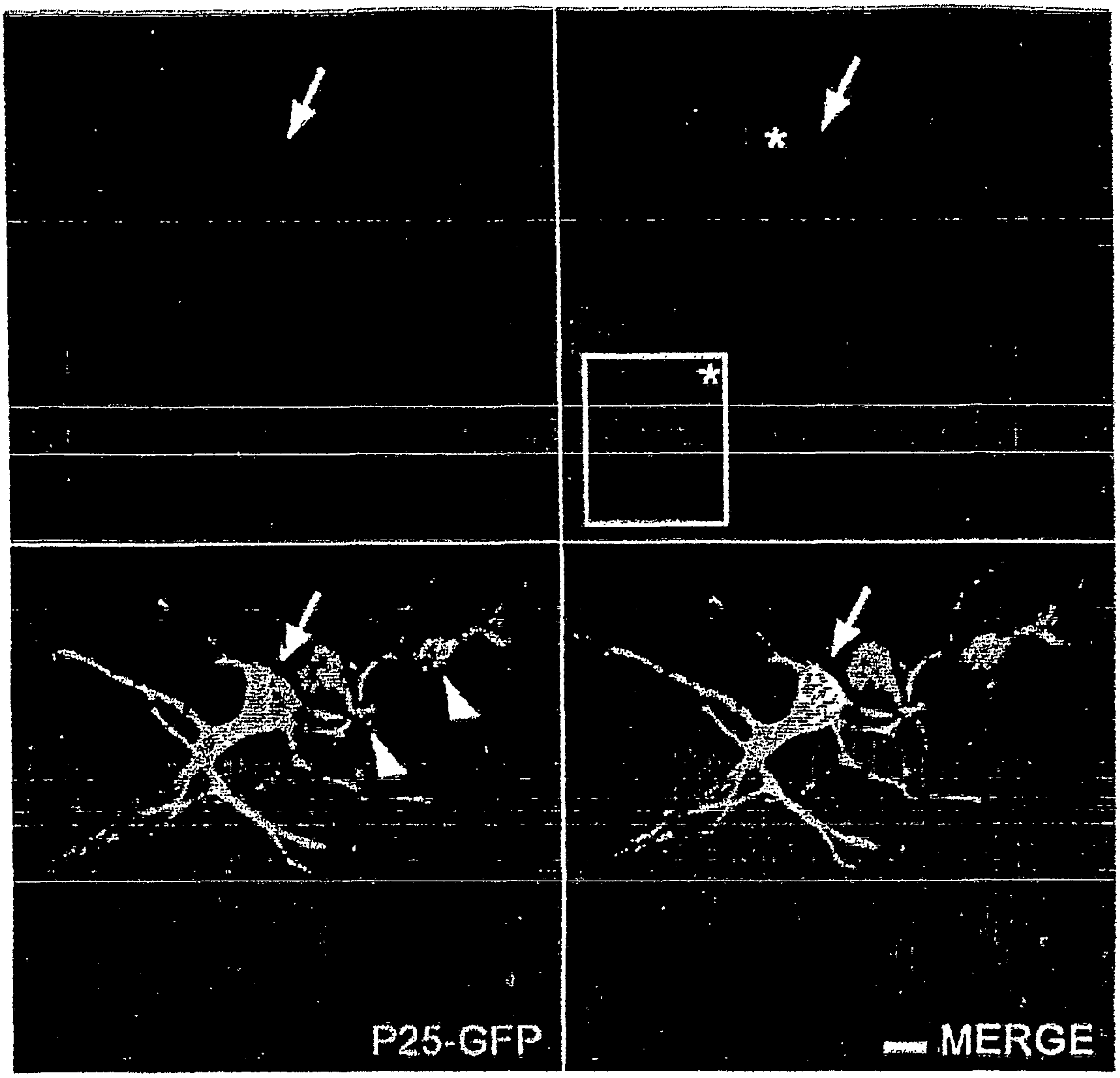

Figure 42

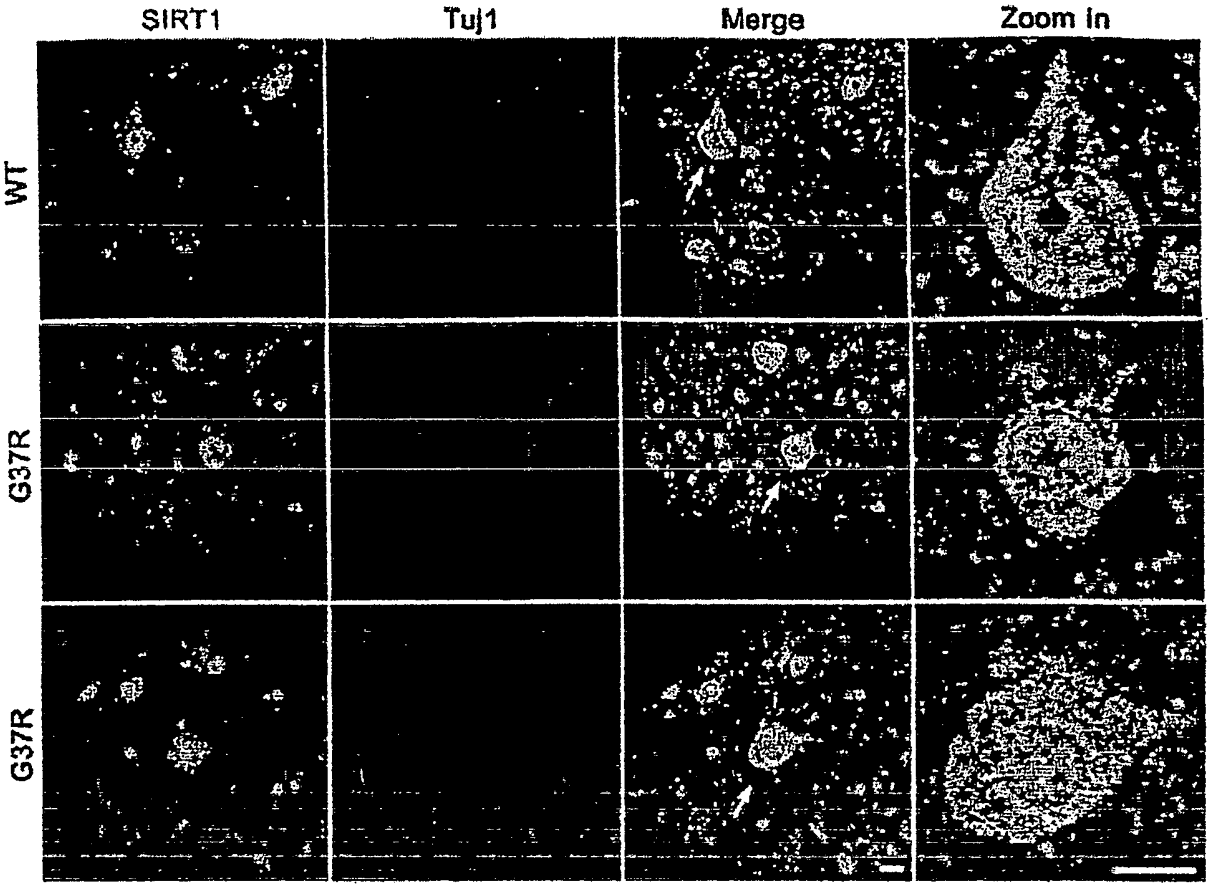

Figure 43

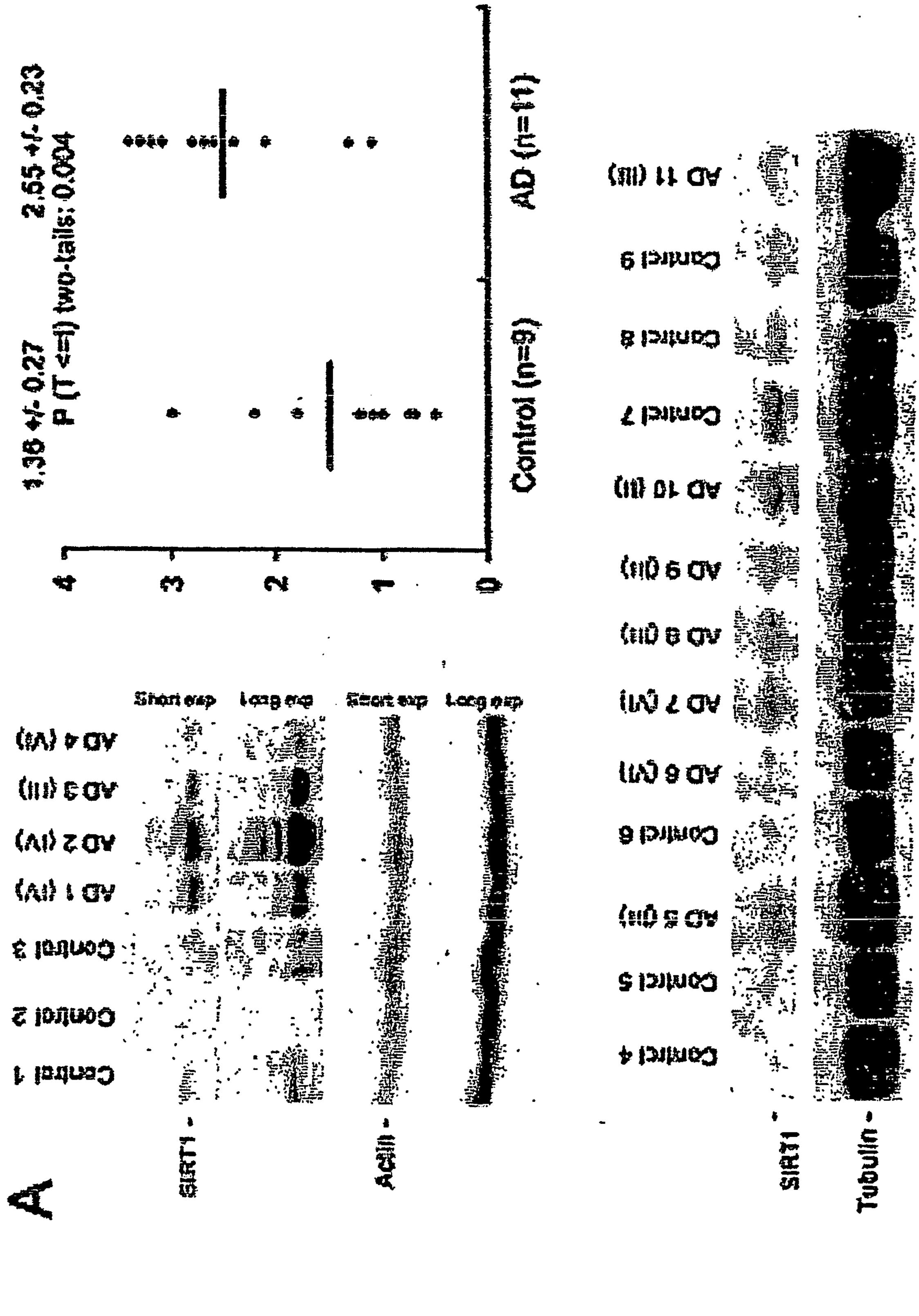
A
SIRT1
AcH3
Short exp
Long exp
Short exp
Long exp
Tubulin
1.38 +/- 0.27
2.55 +/- 0.23
P (T<=t) two-tails: 0.004
Control (n=9)
AD (n=11)
0
1
2
3
4

Figure 43

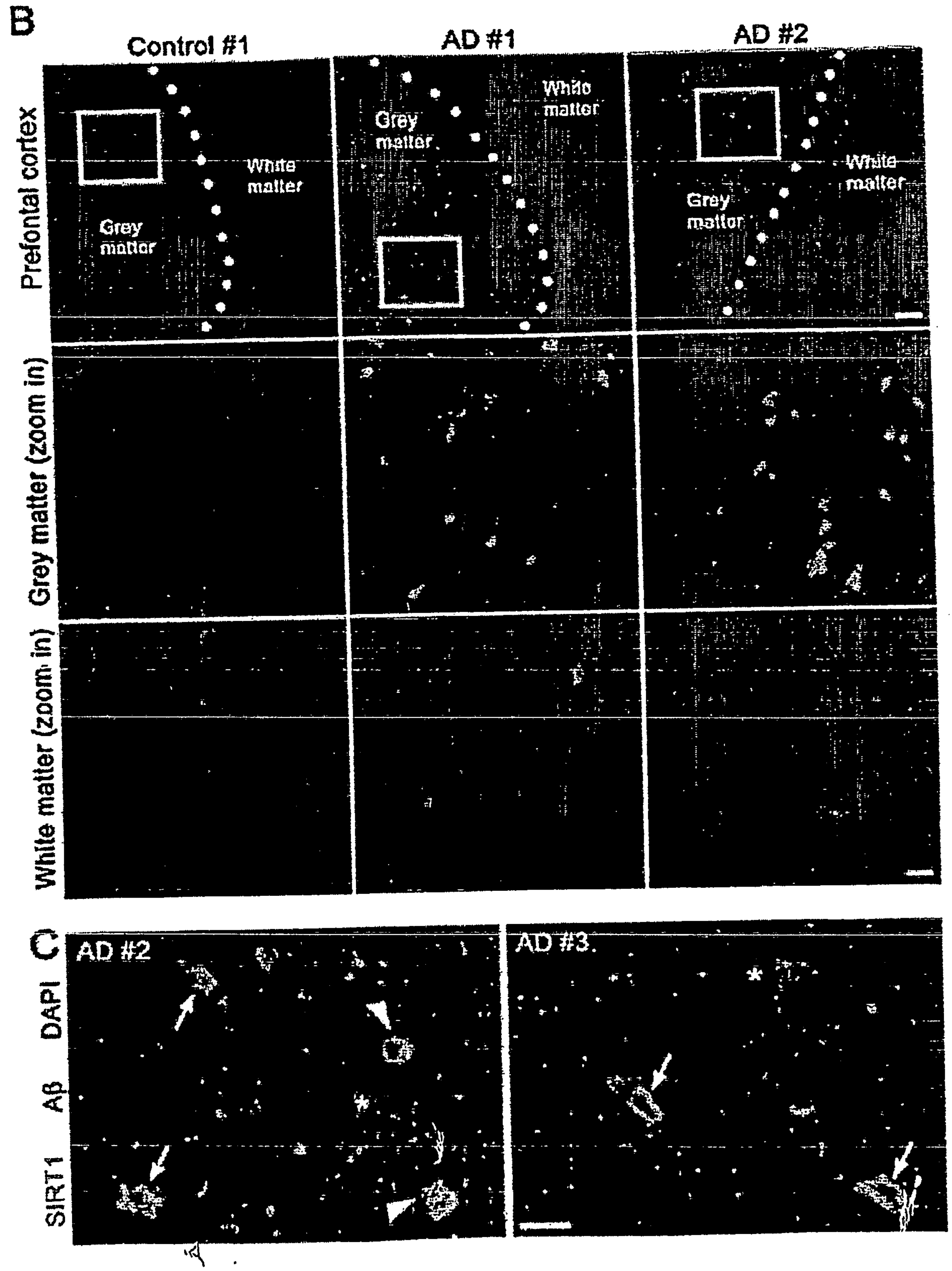
B
Control #1
AD #1
AD #2
Prefontal cortex
Grey matter (zoom in)
White matter (zoom in)
Grey matter
White matter
C
AD #2
AD #3
DAPI
Aβ
SIRT1

Figure 44

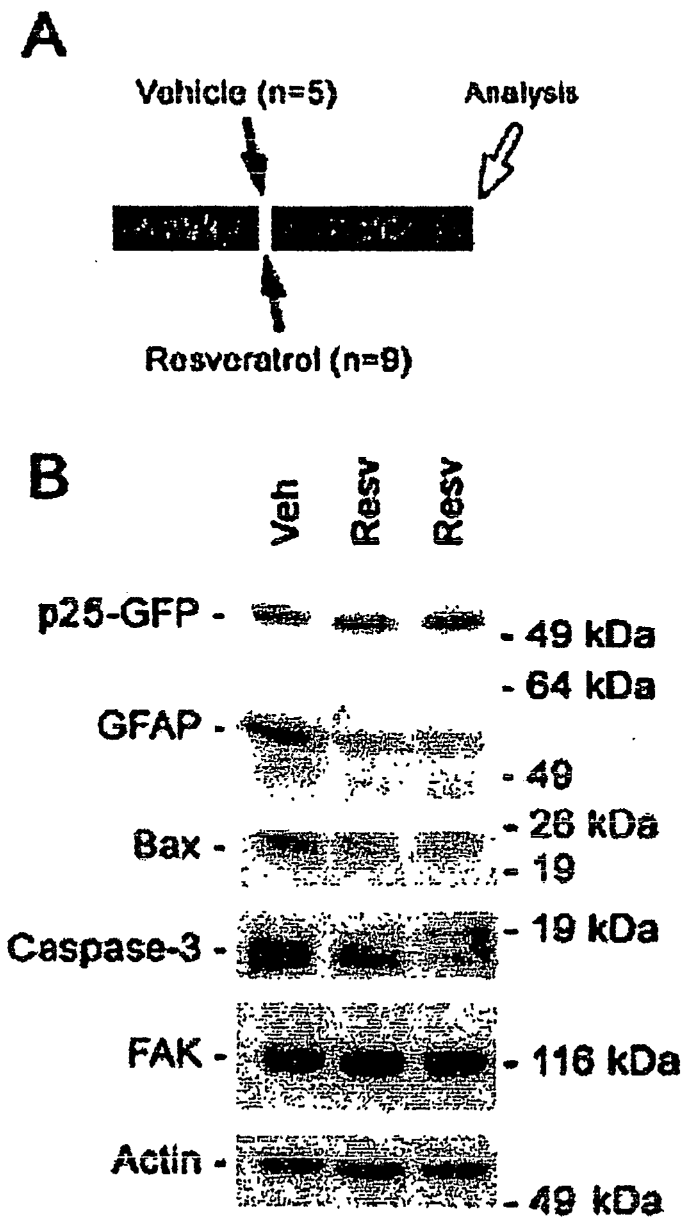
A
Vehicle (n=5)
Analysis
Resveratrol (n=8)
B
Veh
Resv
Resv
p25-GFP -
- 49 kDa
- 64 kDa
GFAP -
- 49
- 26 kDa
Bax -
- 19
Caspase-3 -
- 19 kDa
FAK -
- 116 kDa
Actin -
- 49 kDa

Figure 44

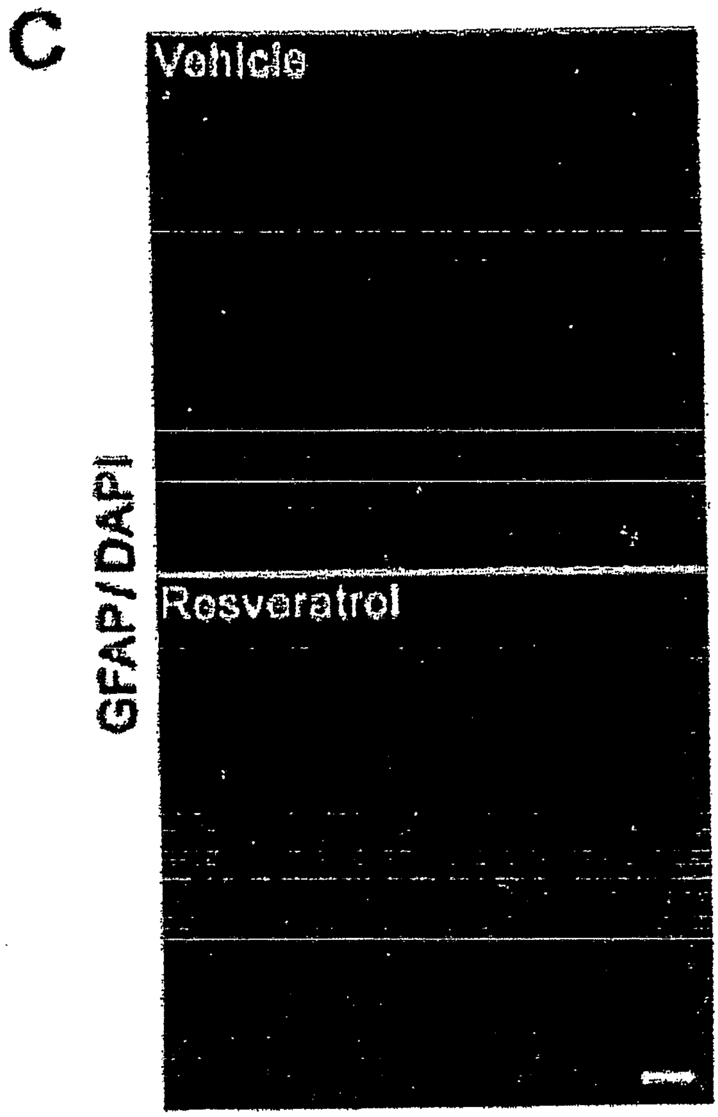

Figure 44

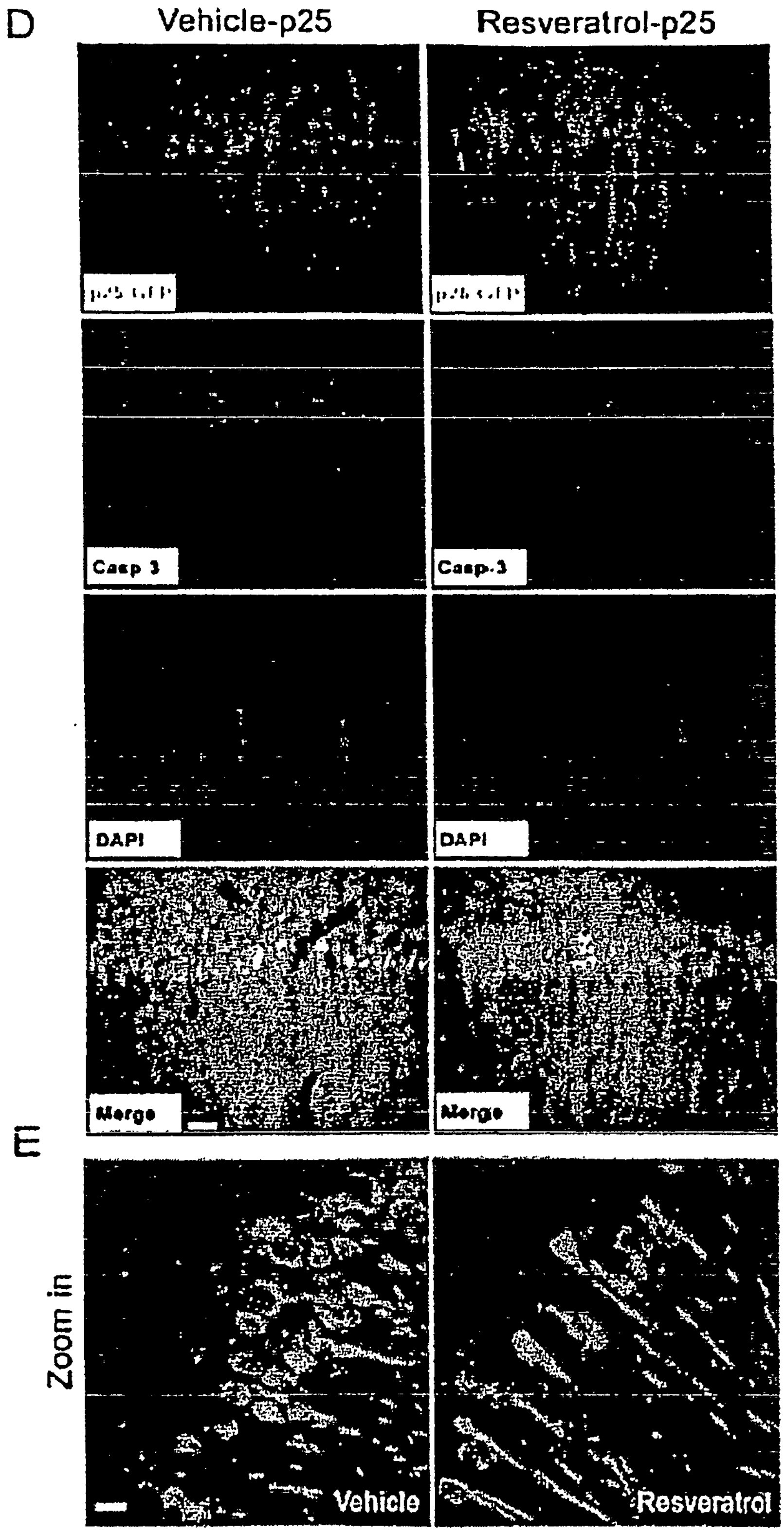
D
Vehicle-p25
Resveratrol-p25
Casp 3
Casp-3
DAPI
DAPI
Merge
Merge
E
Zoom in
Vehicle
Resveratrol

Figure 44

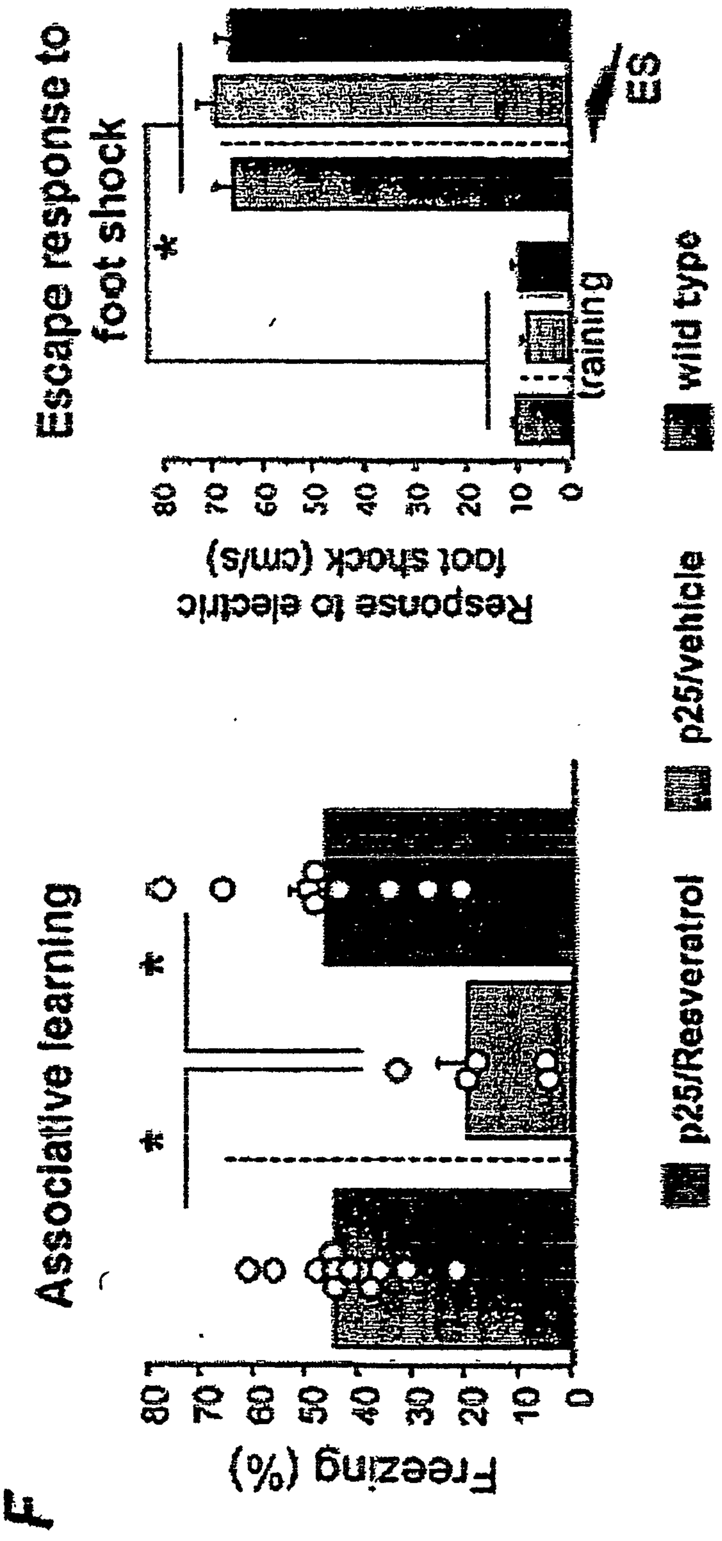
F
Associative learning
Freezing (%)
0
10
20
30
40
50
60
70
80
*
*
Escape response to foot shock
Response to electric foot shock (cm/s)
training
ES
p25/Resveratrol
p25/vehicle
wild type

Figure 45

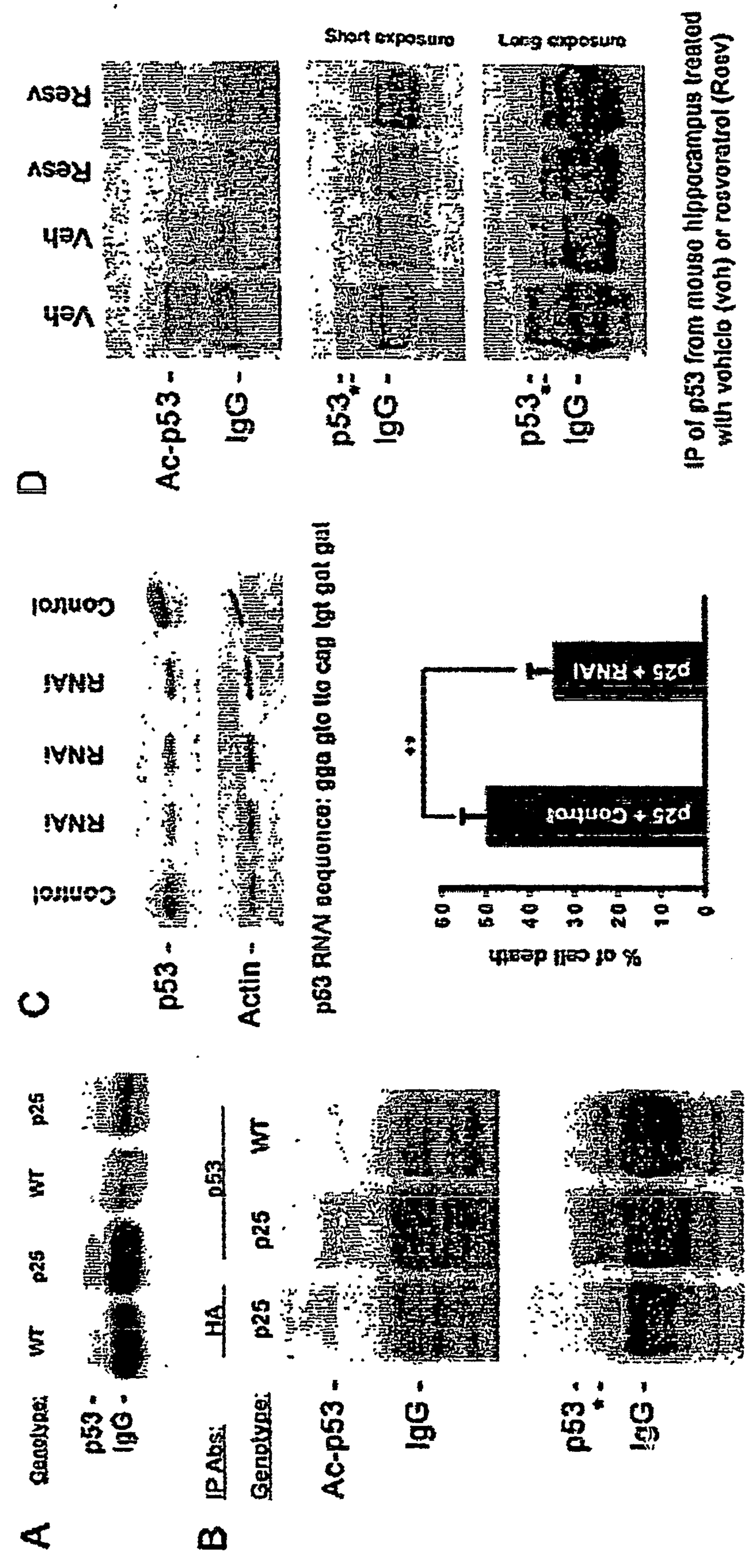
A
Genotype:
WT
p25
WT
p25
p53 -
IgG -
B
IP Abs:
HA
p53
Genotype:
p25
p25
WT
Ac-p53 -
IgG -
p53 -
IgG -
C
Control
RNAi
RNAi
RNAi
Control
p53 -
Actin -
p53 RNAi sequence: gga gtc ttc cag tgt gat gat
% of cell death
60
50
40
30
20
10
0
p25 + Control
p25 + RNAi
D
Veh
Veh
Resv
Resv
Ac-p53 -
IgG -
p53 -
IgG -
Short exposure
p53 -
IgG -
Long exposure
IP of p53 from mouse hippocampus treated
with vehicle (veh) or resveratrol (Resv)

Figure 46

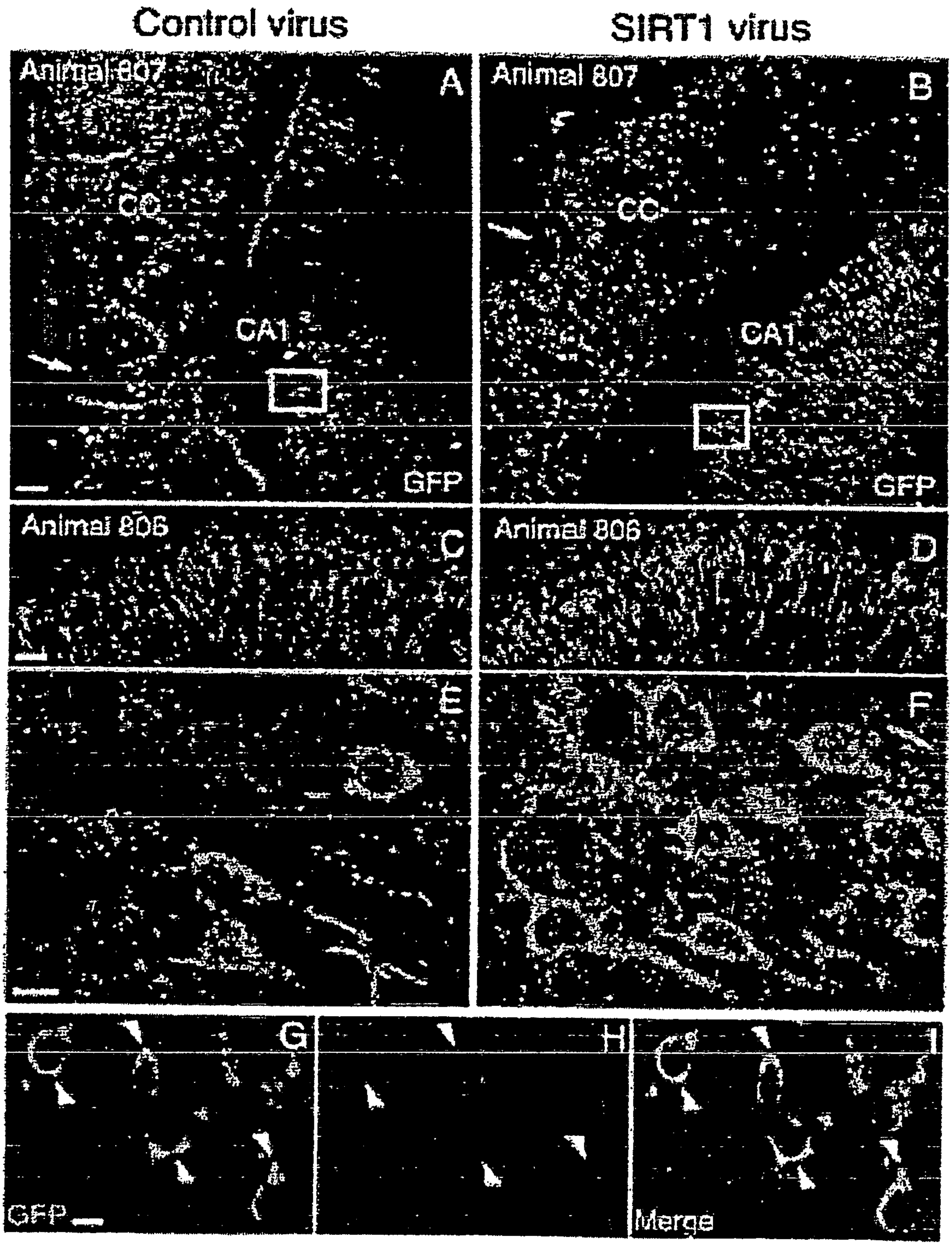
Control virus
SIRT1 virus
Animal 807
A
CC
CA1
GFP
Animal 807
B
CC
CA1
GFP
Animal 806
C
Animal 806
D
E
F
G
GFP
H
I
Merge

Figure 47

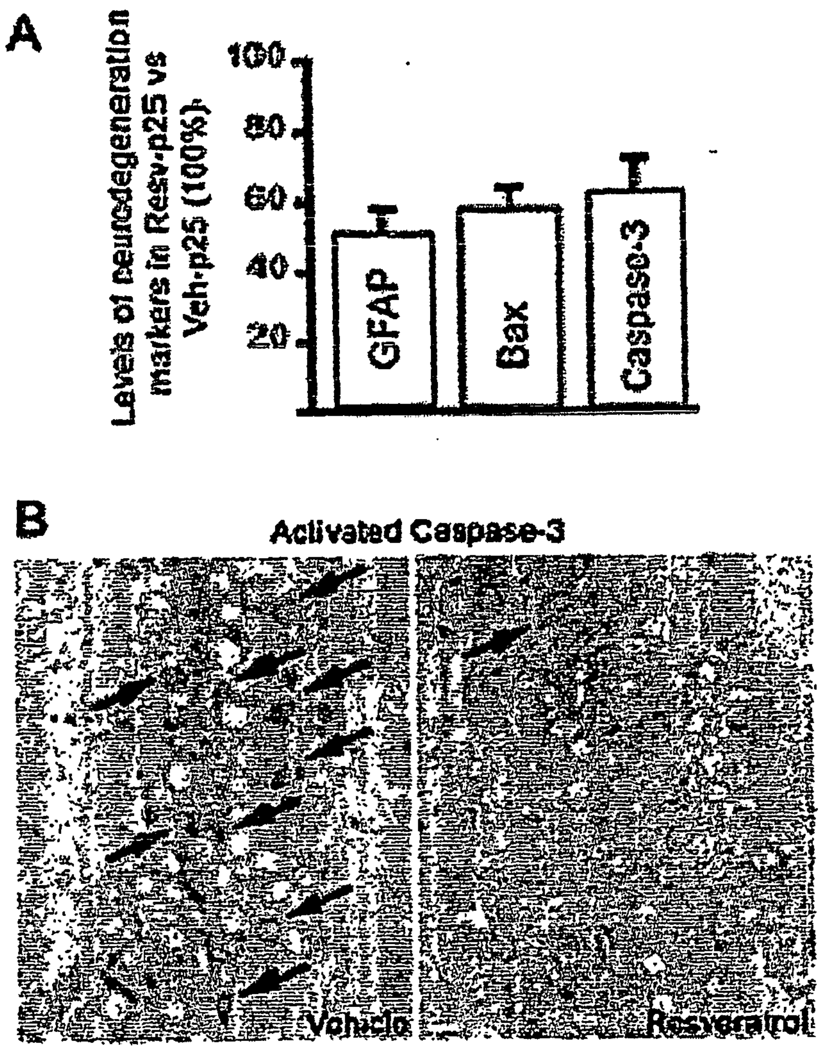
A
Levels of neurodegeneration markers in Resv-p25 vs Veh-p25 (100%)
100
80
60
40
20
GFAP
Bax
Caspase-3
B
Activated Caspase-3
Vehicle
Resveratrol

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A, Mullis
- WO 2005004815 A2
- US 2006008290 W
- US 10884022 B
- US 10884879 B
- US 60532158 B
- US 60483949 B


### Non-patent literature cited in the description


- Computer Methods for Macromolecular Sequence Analysis. Methods in Enzymology. Academic Press, Inc, 1996, vol. 266
- *Meth. Mol. Biol.,* 1997, vol. 70, 173-187
- Handbook of Chemistry and Physics. 1986
- **GREENE ; WUTS.** Protective Groups in Organic Synthesis. Wiley, 1991
- **BARTON.** Protective Groups in Organic Chemistry. Plenum Press, 1973
- Advanced Organic Chemistry. McGraw Hill Book Company, 1977, 251-59
- Molecular Cloning A Laboratory Manual. Cold Spring Harbor Laboratory Press, 1989
- Oligonucleotide Synthesis
- Nucleic Acid Hybridization
- Transcription And Translation
- **R. I. FRESHNEY.** Culture Of Animal Cells. Alan R. Liss, Inc, 1987
- Immobilized Cells And Enzymes. IRL Press, 1986
- **B. PERBAL.** *A Practical Guide To Molecular Cloning,* 1984
- Methods In Enzymology. Academic Press, Inc,
- Gene Transfer Vectors For Mammalian Cells. Cold Spring Harbor Laboratory, 1987
- Methods In Enzymology. vol. 154-155
- Immunochemical Methods In Cell And Molecular Biology. Academic Press, 1987
- Handbook Of Experimental Immunology. 1986, vol. I-IV
- Manipulating the Mouse Embryo. Cold Spring Harbor Laboratory Press, 1986
- **KENYON, C.** *Cell,* 2001, vol. 105, 165-168
- **SINCLAIR, D. A.** *Mech Ageing Dev,* 2002, vol. 123, 857-67
- **HEKIMI, S. ; GUARENTE.** *Science,* 2003, vol. 299, 1351-4
- **GUARENTE, L. ; KENYON, C.** *Nature,* 2000, vol. 408, 255-62
- **LIN et al.** *Science,* 2000, vol. 289, 2126-8
- **ANDERSON et al.** *Nature,* 2003, vol. 423, 181-5
- **KAEBERLEIN et al.** *Genes Dev,* 1999, vol. 13, 2570-80
- **LANDRY et al.** *Proc Natl Acad Sci USA,* 2000, vol. 97, 5807-11
- **IMAI et al.** *Nature,* 2000, vol. 403, 795-800
- **SMITH et al.** *Proc Natl Acad Sci U SA,* 2000, vol. 97, 6658-63
- **TANNER et al.** *Proc Natl Acad Sci USA,* 2000, vol. 97, 14178-82
- **TANNY et al.** *Cell,* 1999, vol. 99, 735-45
- **TANNY et al.** *Proc Natl Acad Sci USA,* 2001, vol. 98, 415-20
- **LAURENSON et al.** *Microbiol Rev,* 1992, vol. 56, 543-60
- **SMITH et al.** *Genes Dev,* 1997, vol. 11, 241-54
- **BRYK, M. et al.** *Genes Dev,* 1997, vol. 11, 255-69
- **GOTTLIEB et al.** *Cell,* 1989, vol. 56, 771-6
- **AGUILANIU et al.** *Science,* 2003
- **TISSENBAUM et al.** *Nature,* 2001, vol. 410, 227-30
- **VAZIRI et al.** *Cell,* 2001, vol. 107, 149-59
- **LUO et al.** *Cell,* 2001, vol. 107, 137-48
- **VERGNES et al.** *Gene,* 2002, vol. 296, 139-50
- **HOLZENBERGER et al.** *Nature,* 2003, vol. 421, 182-7
- **SHIMOKAWA et al.** *Faseb J,* 2003, vol. 17, 1108-9
- **TATAR et al.** *Science,* 2003, vol. 299, 1346-51
- **BITTERMAN et al.** *JBiol Chem,* 2002, vol. 277, 45099-107
- **LANGLEY et al.** *EMBO J,* 2002, vol. 21, 2383-2396
- **GLOSSMANN et al.** *Naunyn Schmiedebergs Arch Pharmacol,* 1981, vol. 317, 100-2
- **OLIVER et al.** *JBiol Chem,* 1994, vol. 269, 29697-703

- **FERGUSON et al.** *Mutat Res,* 2001, vol. 475, 89-111
- **MIDDLETON et al.** *Pharmacol Rev,* 2000, vol. 52, 673-751
- **JANG et al.** *Science,* 1997, vol. 275, 218-20
- **STOJANOVIC et al.** *Arch Biochem Biophys,* 2001, vol. 391, 79-89
- **MONOD et al.** *J. Mol. Biol.,* 1965, vol. 12, 88-118
- **ANDERSON et al.** *JBiol Chem,* 2002, vol. 277, 18881-90
- **PONT et al.** *J Phytopathol,* 1990, vol. 130, 1-8
- **SINCLAIR et al.** *Cell,* 1997, vol. 91, 1033-42
- **DEFOSSEZ et al.** *Mol Cell,* 1999, vol. 3, 447-55
- **PARK et al.** *Mol Cell Biol,* 1999, vol. 19, 3848-56
- **BENGURIA et al.** *Nucleic Acids Res,* 2003, vol. 31, 893-8
- **LONGO et al.** *Science,* 2003, vol. 299, 1342-6
- **DENU et al.** *Trends Biochem Sci,* 2003, vol. 28, 41-8
- **DONG et al.** *Mutat Res,* 2003, 523-524, 145-50
- **NICOLINI et al.** *Neurosci Lett,* 2001, vol. 302, 41-4
- **JAZWINSKI, S. M. et al.** *Ann N Y Acad Sci,* 2000, vol. 908, 21-30
- **PANDEY et al.** *Nucleic Acids Res,* 2002, vol. 30, 5036-55
- **FRYE, R. A.** *Biochem Biophys Res Commun,* 2000, vol. 273, 793-8
- **SOLEAS et al.** *Clin Biochem,* 1997, vol. 30, 91-113
- **CORONADO et al.** *Plant Physiol,* 1995, vol. 108, 533-542
- **MASORO, E. J.** *Exp Gerontol,* 2000, vol. 35, 299-305
- **K.T. HCWITZ et al.** *Nature,* 2003, vol. 425, 191
- **S. STOJANOVIC et al.** *Arch. Biochem. Biophys.,* 2001, vol. 391, 79
- **D.M GOLDBERG et al.** *Clin. Biochem.,* 2003, vol. 36, 79
- **LIN, S. J. ; DEFOSSEZ, P. A. ; GUARENTE, L.** Requirement of NAD and SIR2 for lifespan extension by calorie restriction. *Saccharomyces cerevisiae. Science,* 2000, vol. 289, 2126-8
- **GASSER, S. C. M.** The molecular biology of the SIR proteins. *Gene,* 2001, vol. 279, 1-16
- **HEKIMI, S. ; GUARENTE, L.** Genetics and the specificity of the aging process. *Science,* 2003, vol. 299, 1351-4
- **HOWITZ, K. T. et al.** Small molecule activators of sirtuins extend Saccharomyces cerevisiae lifespan. *Nature,* 2003, vol. 425, 191-6
- **LANDRY, J. et al.** The silencing protein SIR2 and its homologs are NAD-dependent protein deacetylases. *Proc Natl Acad Sci U S A,* 2000, vol. 97, 5807-11
- **IMAI, S. ; ARMSTRONG, C. M. ; KAEBERLEIN, M. ; GUARENTE,, L.** Transcriptional silencing and longevity protein Sir2 is an NAD- dependent histone deacetylase. *Nature,* 2000, vol. 403, 795-800
- **SMITH, J. S. et al.** A phylogenetically conserved NAD+-dependent protein deacetylase activity in the Sir2 protein family. *Proc Natl Acad Sci U S A,* 2000, vol. 97, 6658-63
- **TANNER, K. G. ; LANDRY, J. ; STERNGLANZ, R. ; DENU, J. M.** Silent information regulator 2 family of NAD- dependent histone/protein deacetylases generates a unique product, 1-O-acetyl-ADP-ribose. *Proc Natl Acad Sci US A,* 2000, vol. 97, 14178-82
- **TANNY, J. C. ; DOWD, G. J. ; HUANG, J. ; HILZ, H. ; MOAZED, D.** An enzymatic activity in the yeast Sir2 protein that is essential for gene silencing. *Cell,* 1999, vol. 99, 735-45
- **GUARENTE, L.** Sir2 links chromatin silencing, metabolism, and aging. *Genes Dev,* 2000, vol. 14, 1021-6
- **TISSENBAUM, H. A. ; GUARENTE, L.** Increased dosage of a sir-2 gene extends lifespan in Caenorhabditis elegans. *Nature,* 2001, vol. 410, 227-30
- **ROGINA, B. ; HELFAND, S. L. ; FRANKEL, S.** Longevity regulation by Drosophila Rpd3 deacetylase and caloric restriction. *Science,* 2002, vol. 298, 1745
- **JIANG, J. C. ; JARUGA, E. ; REPNEVSKAYA, M. V. ; JAZWINSKI, S. M.** An intervention resembling caloric restriction prolongs life span and retards aging in yeast. *Faseb J,* 2000, vol. 14, 2135-7
- **KENYON, C. A.** conserved regulatory mechanism for aging. *Cell,* 2001, vol. 105, 165-168
- **MASORO, E. J.** Caloric restriction and aging: an update. *Exp Gerontol,* 2000, vol. 35, 299-305
- **KOUBOVA, J. ; GUARENTE, L.** How does calorie restriction work?. *Genes Dev,* 2003, vol. 17, 313-21
- **SINCLAIR, D. A.** Paradigms and pitfalls of yeast longevity research. *Mech Ageing Dev,* 2002, vol. 123, 857-67
- **KAEBERLEIN, M. ; MCVEY, M. ; GUARENTE, L.** The SIR2/3/4 complex and SIR2 alone promote longevity in Saccharomyces cerevisiae by two different mechanisms. *Genes Dev,* 1999, vol. 13, 2570-80
- **CHIPPINDALE, A. K. ; LEROI, ARMAND M. ; KIM, SUNG B. ; ROSE, MICHAEL R.** Phenotypic plasticity and selection in Drosophila life-history evolution. *Journal of Evolutionary Biology,* 1993, vol. 6, 171-193

- **CHAPMAN, T. ; PARTRIDGE, L.** Female fitness in Drosophila melanogaster: an interaction between the effect of nutrition and of encounter rate with males. *Proc R Soc Lond B Biol Sci,* 1996, vol. 263, 755-9
- **WALKER, D. W. ; MCCOLL, G. ; JENKINS, N. L. ; HARRIS, J. ; LITHGOW, G. J.** Evolution of lifespan in C. elegans. *Nature,* 2000, vol. 405, 296-7
- **MARDEN, J. H. ; ROGINA, B. ; MONTOOTH, K. L. ; HELFAND, S. L.** Conditional tradeoffs between aging and organismal performance of Indy long-lived mutant flies. *Proc Natl Acad Sci U S A,* 2003, vol. 100, 3369-73
- **SCHMID-HEMPEL, P.** On the evolutionary ecology of host-parasite interaction: addressing the question with regard to bumblebees and their parasites. *Naturwissenschaften,* 2001, vol. 88, 147-58
- **EBERT, D. ; BULL, J. J.** Challenging the trade-off model for the evolution of virulence: is virulence management feasible?. *Trends Microbiol,* 2003, vol. 11, 15-20
- **SOLEAS, G. J. ; DIAMANDIS, E. P. ; GOLDBERG, D. M.** Resveratrol: a molecule whose time has come? And gone?. *Clin Biochem,* 1997, vol. 30, 91-113
- **BITTERMAN, K. J. ; ANDERSON, R. M. ; COHEN, H. Y. ; LATORRE-ESTEVES, M. ; SINCLAIR, D. A.** Inhibition of silencing and accelerated aging by nicotinamide, a putative negative regulator of yeast sir2 and human SIRT1. *JBiol Chem,* 2002, vol. 277, 45099-107
- **EDGECOMB, R. S. ; HARTH, C. E. ; SCHNEIDERMAN, A. M.** Regulation of feeding behavior in adult Drosophila melanogaster varies with feeding regime and nutritional state. *J Exp Biol,* 1994, vol. 197, 215-35
- **NGUYEN, M. D. et al.** *Nat Cell Biol,* 2004, vol. 6, 595-608
- **LEE, M. S. et al.** *Nature,* 2000, vol. 405, 360-4
- **CRUZ, J. C. et al.** *Neuron,* 2003, vol. 40, 471-83
- **NGUYEN, M. D. et al.** *Neuron,* 2001, vol. 30, 135-47
- **FISCHER, A. et al.** *J Neurosci,* 2004, vol. 24, 1962-6
- **SUI, G. et al.** *Proc Natl Acad Sci U S A,* 2002, vol. 99, 5515-20
- **BOSSY-WETZEL et al.** *Nat Med,* 2004, S2-9
- **FORMAN, M. S. et al.** *Nat Med,* 2004, vol. 10, 1055-63
- **SELKOE, D. J.** *Nat Cell Biol,* 2004, vol. 6, 1054-61
- **FOREMAN, M. S. et al.** *Nat Med,* 2004, vol. 10, 1055-63
- **ANDERSON, R. M. et al.** *Science,* 2003, vol. 302, 2124-6
- **ANDERSON, R. M. et al.** *Nature,* 2003, vol. 423, 181-5
- **COHEN, H. Y. et al.** *Science,* 2004, vol. 305, 390-2
- **HOWITZ, K T. et al.** *Nature,* 2003, vol. 425, 191-6
- **KAEBERLEIN, M. et al.** *Genes Dev,* 1999, vol. 13, 2570-80
- **IMAI, S. et al.** *Nature,* 2000, vol. 403, 795-800
- **LIN, S. J. et al.** *Science,* 2000, vol. 289, 2126-8
- **HOWITZ, K. T. et al.** *Nature,* 2003, vol. 425, 191-6
- **BRUNET, A. et al.** *Science,* 2004, vol. 303, 2011-5
- **MOTTA, M. C. et al.** *Cell,* 2004, vol. 116, 551-63
- **LANGLEY, E. et al.** *Embo J,* 2002, vol. 21, 2383-96
- **COHEN, H. Y. et al.** *Mol Cell,* 2004, vol. 13, 627-38
- **LUO, J. et al.** *Cell,* 2001, vol. 107, 137-48
- **VAZIRI, H. et al.** *Cell,* 2001, vol. 107, 149-59
- **ARAKI, T. et al.** *Science,* 2004, vol. 305, 1010-3
- **BRAAK, H. ; BREAK, E.** *J Neural Transm Suppl,* 1998, vol. 53, 127-40
- **BRAAK, H. ; BRAAK, E.** *J Neural Transm Suppl,* 1998, vol. 53, 127-40
- **WONG, P. C. et al.** *Neuron,* 1995, vol. 14, 1105-16
- **GURNEY, M. E. et al.** *Science,* 1994, vol. 264, 1772-5
- **GAMES, D. et al.** *Nature,* 1995, vol. 373, 523-7
- **CRUZ, J. C. et al.** *Curr Opin Neurobiol,* 2004, vol. 14, 390-4
- **BRUIJN, L. I. et al.** *Annu Rev Neurosci,* 2004, vol. 27, 723-49
- **KUSAKAWA, G. et al.** *J Biol Chem,* 2000, vol. 275, 17166-72
- **NATH, R. et al.** *Biochem Biophys Res Commun,* 2000, vol. 274, 16-21
- **PATRICK, G. N. et al.** *Nature,* 1999, vol. 402, 615-22
- **ZHANG, J. ; KRISHNAMURTHY et al.** *J Neurochem,* 2002, vol. 81, 307-13
- **HAMDANE, M. et al.** *J Biol Chem,* 2003, vol. 278, 34026-34
- **PARKER, J. A. et al.** *Nat Genet,* 2005, vol. 37, 349-50
- **FISCHER, A. et al.** *J Neurosci,* 2004, vol. 24, 962-6
- **FISCHER et al.** *Neuron*
- **ZHANG, J. et al.** *J Neurochem,* 2002, vol. 81, 307-13

- **LANGLEY, E. et al.** *Embo J,* 2002, vol. 21, 2383-95
- **NGUYEN, M. D. et al.** *Cell Death Differ,* 2002, vol. 9, 1294-306
- **SMITH, P. D. et al.** *Cell Cycle,* 2004, vol. 3, 289-91
- **BORDONE, L. ; GUARENTE, L.** *Nat Rev Mol Cell Biol,* 2005, vol. 6, 298-305
- **LOMBARD, D. B. et al.** *Cell,* 2005, vol. 120, 497-512
- **LAMMING, D. W. et al.** *Mol Microbiol,* 2004, vol. 53, 1003-9

SEQUENCE LISTING

<110> PRESIDENT AND FELLOWS OF HARVARD COLLEGE

<120> SIRTUIN RELATED THERAPEUTICS AND DIAGNOSTICS FOR NEURODEGENERATIVE DISEASES

<130> HMV-089.27

<140> PCT/US2006/008290
<141> 2006-03-07

<150> 10/884,022
<151> 2004-07-01

<150> 10/884,879
<151> 2004-07-01

<150> 60/532,158
<151> 2003-12-23

<150> 60/483,949
<151> 2003-07-01

<160> 31

<170> PatentIn Ver. 3.3

<210> 1
<211> 4107
<212> DNA
<213> Homo sapiens

<220>
<221> CDS
<222> (54)..(2294)

<400> 1

```
gtcgagcggg agcagaggag gcgagggagg agggccagag aggcagttgg aag atg    56
                                                           Met
                                                             1

gcg gac gag gcg gcc ctc gcc ctt cag ccc ggc ggc tcc ccc tcg gcg   104
Ala Asp Glu Ala Ala Leu Ala Leu Gln Pro Gly Gly Ser Pro Ser Ala
              5                  10                  15

gcg ggg gcc gac agg gag gcc gcg tcg tcc ccc gcc ggg gag ccg ctc   152
Ala Gly Ala Asp Arg Glu Ala Ala Ser Ser Pro Ala Gly Glu Pro Leu
         20                  25                  30

cgc aag agg ccg cgg aga gat ggt ccc ggc ctc gag cgg agc ccg ggc   200
Arg Lys Arg Pro Arg Arg Asp Gly Pro Gly Leu Glu Arg Ser Pro Gly
     35                  40                  45

gag ccc ggt ggg gcg gcc cca gag cgt gag gtg ccg gcg gcg gcc agg   248
Glu Pro Gly Gly Ala Ala Pro Glu Arg Glu Val Pro Ala Ala Ala Arg
 50                  55                  60                  65
```

```
ggc tgc ccg ggt gcg gcg gcg gcg gcg ctg tgg cgg gag gcg gag gca    296
Gly Cys Pro Gly Ala Ala Ala Ala Ala Leu Trp Arg Glu Ala Glu Ala
                70                  75                  80

gag gcg gcg gcg gca ggc ggg gag caa gag gcc cag gcg act gcg gcg    344
Glu Ala Ala Ala Ala Gly Gly Glu Gln Glu Ala Gln Ala Thr Ala Ala
            85                  90                  95

gct ggg gaa gga gac aat ggg ccg ggc ctg cag ggc cca tct cgg gag    392
Ala Gly Glu Gly Asp Asn Gly Pro Gly Leu Gln Gly Pro Ser Arg Glu
        100                 105                 110

cca ccg ctg gcc gac aac ttg tac gac gaa gac gac gac gac gag ggc    440
Pro Pro Leu Ala Asp Asn Leu Tyr Asp Glu Asp Asp Asp Asp Glu Gly
    115                 120                 125

gag gag gag gaa gag gcg gcg gcg gcg gcg att ggg tac cga gat aac    488
Glu Glu Glu Glu Glu Ala Ala Ala Ala Ala Ile Gly Tyr Arg Asp Asn
130                 135                 140                 145

ctt ctg ttc ggt gat gaa att atc act aat ggt ttt cat tcc tgt gaa    536
Leu Leu Phe Gly Asp Glu Ile Ile Thr Asn Gly Phe His Ser Cys Glu
                150                 155                 160

agt gat gag gag gat aga gcc tca cat gca agc tct agt gac tgg act    584
Ser Asp Glu Glu Asp Arg Ala Ser His Ala Ser Ser Ser Asp Trp Thr
            165                 170                 175

cca agg cca cgg ata ggt cca tat act ttt gtt cag caa cat ctt atg    632
Pro Arg Pro Arg Ile Gly Pro Tyr Thr Phe Val Gln Gln His Leu Met
        180                 185                 190

att ggc aca gat cct cga aca att ctt aaa gat tta ttg ccg gaa aca    680
Ile Gly Thr Asp Pro Arg Thr Ile Leu Lys Asp Leu Leu Pro Glu Thr
    195                 200                 205

ata cct cca cct gag ttg gat gat atg aca ctg tgg cag att gtt att    728
Ile Pro Pro Pro Glu Leu Asp Asp Met Thr Leu Trp Gln Ile Val Ile
210                 215                 220                 225

aat atc ctt tca gaa cca cca aaa agg aaa aaa aga aaa gat att aat    776
Asn Ile Leu Ser Glu Pro Pro Lys Arg Lys Lys Arg Lys Asp Ile Asn
                230                 235                 240

aca att gaa gat gct gtg aaa tta ctg caa gag tgc aaa aaa att ata    824
Thr Ile Glu Asp Ala Val Lys Leu Leu Gln Glu Cys Lys Lys Ile Ile
            245                 250                 255

gtt cta act gga gct ggg gtg tct gtt tca tgt gga ata cct gac ttc    872
Val Leu Thr Gly Ala Gly Val Ser Val Ser Cys Gly Ile Pro Asp Phe
        260                 265                 270

agg tca agg gat ggt att tat gct cgc ctt gct gta gac ttc cca gat    920
Arg Ser Arg Asp Gly Ile Tyr Ala Arg Leu Ala Val Asp Phe Pro Asp
    275                 280                 285

ctt cca gat cct caa gcg atg ttt gat att gaa tat ttc aga aaa gat    968
Leu Pro Asp Pro Gln Ala Met Phe Asp Ile Glu Tyr Phe Arg Lys Asp
290                 295                 300                 305
```

```
cca aga cca ttc ttc aag ttt gca aag gaa ata tat cct gga caa ttc     1016
Pro Arg Pro Phe Phe Lys Phe Ala Lys Glu Ile Tyr Pro Gly Gln Phe
                310                 315                 320

cag cca tct ctc tgt cac aaa ttc ata gcc ttg tca gat aag gaa gga     1064
Gln Pro Ser Leu Cys His Lys Phe Ile Ala Leu Ser Asp Lys Glu Gly
            325                 330                 335

aaa cta ctt cgc aac tat acc cag aac ata gac acg ctg gaa cag gtt     1112
Lys Leu Leu Arg Asn Tyr Thr Gln Asn Ile Asp Thr Leu Glu Gln Val
        340                 345                 350

gcg gga atc caa agg ata att cag tgt cat ggt tcc ttt gca aca gca     1160
Ala Gly Ile Gln Arg Ile Ile Gln Cys His Gly Ser Phe Ala Thr Ala
    355                 360                 365

tct tgc ctg att tgt aaa tac aaa gtt gac tgt gaa gct gta cga gga     1208
Ser Cys Leu Ile Cys Lys Tyr Lys Val Asp Cys Glu Ala Val Arg Gly
370                 375                 380                 385

gat att ttt aat cag gta gtt cct cga tgt cct agg tgc cca gct gat     1256
Asp Ile Phe Asn Gln Val Val Pro Arg Cys Pro Arg Cys Pro Ala Asp
                390                 395                 400

gaa ccg ctt gct atc atg aaa cca gag att gtg ttt ttt ggt gaa aat     1304
Glu Pro Leu Ala Ile Met Lys Pro Glu Ile Val Phe Phe Gly Glu Asn
            405                 410                 415

tta cca gaa cag ttt cat aga gcc atg aag tat gac aaa gat gaa gtt     1352
Leu Pro Glu Gln Phe His Arg Ala Met Lys Tyr Asp Lys Asp Glu Val
        420                 425                 430

gac ctc ctc att gtt att ggg tct tcc ctc aaa gta aga cca gta gca     1400
Asp Leu Leu Ile Val Ile Gly Ser Ser Leu Lys Val Arg Pro Val Ala
    435                 440                 445

cta att cca agt tcc ata ccc cat gaa gtg cct cag ata tta att aat     1448
Leu Ile Pro Ser Ser Ile Pro His Glu Val Pro Gln Ile Leu Ile Asn
450                 455                 460                 465

aga gaa cct ttg cct cat ctg cat ttt gat gta gag ctt ctt gga gac     1496
Arg Glu Pro Leu Pro His Leu His Phe Asp Val Glu Leu Leu Gly Asp
                470                 475                 480

tgt gat gtc ata att aat gaa ttg tgt cat agg tta ggt ggt gaa tat     1544
Cys Asp Val Ile Ile Asn Glu Leu Cys His Arg Leu Gly Gly Glu Tyr
            485                 490                 495

gcc aaa ctt tgc tgt aac cct gta aag ctt tca gaa att act gaa aaa     1592
Ala Lys Leu Cys Cys Asn Pro Val Lys Leu Ser Glu Ile Thr Glu Lys
        500                 505                 510

cct cca cga aca caa aaa gaa ttg gct tat ttg tca gag ttg cca ccc     1640
Pro Pro Arg Thr Gln Lys Glu Leu Ala Tyr Leu Ser Glu Leu Pro Pro
    515                 520                 525
```

```
aca cct ctt cat gtt tca gaa gac tca agt tca cca gaa aga act tca    1688
Thr Pro Leu His Val Ser Glu Asp Ser Ser Ser Pro Glu Arg Thr Ser
530                 535                 540                 545

cca cca gat tct tca gtg att gtc aca ctt tta gac caa gca gct aag    1736
Pro Pro Asp Ser Ser Val Ile Val Thr Leu Leu Asp Gln Ala Ala Lys
                550                 555                 560

agt aat gat gat tta gat gtg tct gaa tca aaa ggt tgt atg gaa gaa    1784
Ser Asn Asp Asp Leu Asp Val Ser Glu Ser Lys Gly Cys Met Glu Glu
            565                 570                 575

aaa cca cag gaa gta caa act tct agg aat gtt gaa agt att gct gaa    1832
Lys Pro Gln Glu Val Gln Thr Ser Arg Asn Val Glu Ser Ile Ala Glu
        580                 585                 590

cag atg gaa aat ccg gat ttg aag aat gtt ggt tct agt act ggg gag    1880
Gln Met Glu Asn Pro Asp Leu Lys Asn Val Gly Ser Ser Thr Gly Glu
    595                 600                 605

aaa aat gaa aga act tca gtg gct gga aca gtg aga aaa tgc tgg cct    1928
Lys Asn Glu Arg Thr Ser Val Ala Gly Thr Val Arg Lys Cys Trp Pro
610                 615                 620                 625

aat aga gtg gca aag gag cag att agt agg cgg ctt gat ggt aat cag    1976
Asn Arg Val Ala Lys Glu Gln Ile Ser Arg Arg Leu Asp Gly Asn Gln
                630                 635                 640

tat ctg ttt ttg cca cca aat cgt tac att ttc cat ggc gct gag gta    2024
Tyr Leu Phe Leu Pro Pro Asn Arg Tyr Ile Phe His Gly Ala Glu Val
            645                 650                 655

tat tca gac tct gaa gat gac gtc tta tcc tct agt tct tgt ggc agt    2072
Tyr Ser Asp Ser Glu Asp Asp Val Leu Ser Ser Ser Ser Cys Gly Ser
        660                 665                 670

aac agt gat agt ggg aca tgc cag agt cca agt tta gaa gaa ccc atg    2120
Asn Ser Asp Ser Gly Thr Cys Gln Ser Pro Ser Leu Glu Glu Pro Met
    675                 680                 685

gag gat gaa agt gaa att gaa gaa ttc tac aat ggc tta gaa gat gag    2168
Glu Asp Glu Ser Glu Ile Glu Glu Phe Tyr Asn Gly Leu Glu Asp Glu
690                 695                 700                 705

cct gat gtt cca gag aga gct gga gga gct gga ttt ggg act gat gga    2216
Pro Asp Val Pro Glu Arg Ala Gly Gly Ala Gly Phe Gly Thr Asp Gly
                710                 715                 720

gat gat caa gag gca att aat gaa gct ata tct gtg aaa cag gaa gta    2264
Asp Asp Gln Glu Ala Ile Asn Glu Ala Ile Ser Val Lys Gln Glu Val
            725                 730                 735

aca gac atg aac tat cca tca aac aaa tca tagtgtaata attgtgcagg      2314
Thr Asp Met Asn Tyr Pro Ser Asn Lys Ser
        740                 745

tacaggaatt gttccaccag cattaggaac tttagcatgt caaaatgaat gtttacttgt 2374

gaactcgata gagcaaggaa accagaaagg tgtaatattt ataggttggt aaaatagatt 2434
```

gtttttcatg gataattttt aacttcatta tttctgtact tgtacaaact caacactaac 2494

tttttttttt ttaaaaaaaa aaaggtacta agtatcttca atcagctgtt ggtcaagact 2554

aactttcttt taaaggttca tttgtatgat aaattcatat gtgtatatat aatttttttt 2614

gttttgtcta gtgagtttca acatttttaa agttttcaaa aagccatcgg aatgttaaat 2674

taatgtaaag ggacagctaa tctagaccaa agaatggtat tttcactttt ctttgtaaca 2734

ttgaatggtt tgaagtactc aaaatctgtt acgctaaact tttgattctt taacacaatt 2794

atttttaaac actggcattt tccaaaactg tggcagctaa ctttttaaaa tctcaaatga 2854

catgcagtgt gagtagaagg aagtcaacaa tatgtgggga gagcactcgg ttgtctttac 2914

ttttaaaagt aatacttggt gctaagaatt tcaggattat tgtatttacg ttcaaatgaa 2974

gatggctttt gtacttcctg tggacatgta gtaatgtcta tattggctca taaaactaac 3034

ctgaaaaaca aataaatgct ttggaaatgt ttcagttgct ttagaaacat tagtgcctgc 3094

ctggatcccc ttagttttga aatatttgcc attgttgttt aaatacctat cactgtggta 3154

gagcttgcat tgatcttttc cacaagtatt aaactgccaa aatgtgaata tgcaaagcct 3214

ttctgaatct ataataatgg tacttctact ggggagagtg taatattttg gactgctgtt 3274

ttccattaat gaggagagca acaggcccct gattatacag ttccaaagta ataagatgtt 3334

aattgtaatt cagccagaaa gtacatgtct cccattggga ggatttggtg ttaaatacca 3394

aactgctagc cctagtatta tggagatgaa catgatgatg taacttgtaa tagcagaata 3454

gttaatgaat gaaactagtt cttataattt atctttattt aaaagcttag cctgccttaa 3514

aactagagat caactttctc agctgcaaaa gcttctagtc tttcaagaag ttcatacttt 3574

atgaaattgc acagtaagca tttatttttc agaccatttt tgaacatcac tcctaaatta 3634

ataaagtatt cctctgttgc tttagtattt attacaataa aaagggtttg aaatatagct 3694

gttctttatg cataaaacac ccagctagga ccattactgc cagagaaaaa aatcgtattg 3754

aatggccatt tccctactta taagatgtct caatctgaat ttatttggct acactaaaga 3814

atgcagtata tttagttttc catttgcatg atgtttgtgt gctatagatg atattttaaa 3874

ttgaaaagtt tgttttaaat tatttttaca gtgaagactg ttttcagctc tttttatatt 3934

gtacatagtc ttttatgtaa tttactggca tatgttttgt agactgttta atgactggat 3994

atcttccttc aacttttgaa atacaaaacc agtgtttttt acttgtacac tgttttaaag 4054

tctattaaaa ttgtcatttg actttttttct gttaaaaaaa aaaaaaaaaa aaa 4107

<210> 2
<211> 747
<212> PRT
<213> Homo sapiens

<400> 2

Met Ala Asp Glu Ala Ala Leu Ala Leu Gln Pro Gly Gly Ser Pro Ser
1               5                   10                  15

Ala Ala Gly Ala Asp Arg Glu Ala Ala Ser Ser Pro Ala Gly Glu Pro
            20                  25                  30

Leu Arg Lys Arg Pro Arg Arg Asp Gly Pro Gly Leu Glu Arg Ser Pro
        35                  40                  45

Gly Glu Pro Gly Gly Ala Ala Pro Glu Arg Glu Val Pro Ala Ala Ala
    50                  55                  60

Arg Gly Cys Pro Gly Ala Ala Ala Ala Ala Leu Trp Arg Glu Ala Glu
65                  70                  75                  80

Ala Glu Ala Ala Ala Ala Gly Gly Glu Gln Glu Ala Gln Ala Thr Ala
                85                  90                  95

Ala Ala Gly Glu Gly Asp Asn Gly Pro Gly Leu Gln Gly Pro Ser Arg
            100                 105                 110

Glu Pro Pro Leu Ala Asp Asn Leu Tyr Asp Glu Asp Asp Asp Asp Glu
        115                 120                 125

Gly Glu Glu Glu Glu Glu Ala Ala Ala Ala Ala Ile Gly Tyr Arg Asp
    130                 135                 140

Asn Leu Leu Phe Gly Asp Glu Ile Ile Thr Asn Gly Phe His Ser Cys
145                 150                 155                 160

Glu Ser Asp Glu Glu Asp Arg Ala Ser His Ala Ser Ser Ser Asp Trp
                165                 170                 175

Thr Pro Arg Pro Arg Ile Gly Pro Tyr Thr Phe Val Gln Gln His Leu
            180                 185                 190

Met Ile Gly Thr Asp Pro Arg Thr Ile Leu Lys Asp Leu Leu Pro Glu
        195                 200                 205

Thr Ile Pro Pro Pro Glu Leu Asp Asp Met Thr Leu Trp Gln Ile Val
    210                 215                 220

Ile Asn Ile Leu Ser Glu Pro Pro Lys Arg Lys Lys Arg Lys Asp Ile
225                 230                 235                 240

Asn Thr Ile Glu Asp Ala Val Lys Leu Leu Gln Glu Cys Lys Lys Ile
                245                 250                 255

Ile Val Leu Thr Gly Ala Gly Val Ser Val Ser Cys Gly Ile Pro Asp
            260                 265                 270

Phe Arg Ser Arg Asp Gly Ile Tyr Ala Arg Leu Ala Val Asp Phe Pro
        275                 280                 285

```
Asp Leu Pro Asp Pro Gln Ala Met Phe Asp Ile Glu Tyr Phe Arg Lys
    290                 295                 300

Asp Pro Arg Pro Phe Phe Lys Phe Ala Lys Glu Ile Tyr Pro Gly Gln
305                 310                 315                 320

Phe Gln Pro Ser Leu Cys His Lys Phe Ile Ala Leu Ser Asp Lys Glu
                325                 330                 335

Gly Lys Leu Leu Arg Asn Tyr Thr Gln Asn Ile Asp Thr Leu Glu Gln
            340                 345                 350

Val Ala Gly Ile Gln Arg Ile Ile Gln Cys His Gly Ser Phe Ala Thr
        355                 360                 365

Ala Ser Cys Leu Ile Cys Lys Tyr Lys Val Asp Cys Glu Ala Val Arg
    370                 375                 380

Gly Asp Ile Phe Asn Gln Val Val Pro Arg Cys Pro Arg Cys Pro Ala
385                 390                 395                 400

Asp Glu Pro Leu Ala Ile Met Lys Pro Glu Ile Val Phe Phe Gly Glu
                405                 410                 415

Asn Leu Pro Glu Gln Phe His Arg Ala Met Lys Tyr Asp Lys Asp Glu
            420                 425                 430

Val Asp Leu Leu Ile Val Ile Gly Ser Ser Leu Lys Val Arg Pro Val
        435                 440                 445

Ala Leu Ile Pro Ser Ser Ile Pro His Glu Val Pro Gln Ile Leu Ile
    450                 455                 460

Asn Arg Glu Pro Leu Pro His Leu His Phe Asp Val Glu Leu Leu Gly
465                 470                 475                 480

Asp Cys Asp Val Ile Ile Asn Glu Leu Cys His Arg Leu Gly Gly Glu
                485                 490                 495

Tyr Ala Lys Leu Cys Cys Asn Pro Val Lys Leu Ser Glu Ile Thr Glu
            500                 505                 510

Lys Pro Pro Arg Thr Gln Lys Glu Leu Ala Tyr Leu Ser Glu Leu Pro
        515                 520                 525

Pro Thr Pro Leu His Val Ser Glu Asp Ser Ser Ser Pro Glu Arg Thr
    530                 535                 540

Ser Pro Pro Asp Ser Ser Val Ile Val Thr Leu Leu Asp Gln Ala Ala
545                 550                 555                 560

Lys Ser Asn Asp Asp Leu Asp Val Ser Glu Ser Lys Gly Cys Met Glu
                565                 570                 575

Glu Lys Pro Gln Glu Val Gln Thr Ser Arg Asn Val Glu Ser Ile Ala
            580                 585                 590
```

```
Glu Gln Met Glu Asn Pro Asp Leu Lys Asn Val Gly Ser Ser Thr Gly
        595                 600                 605

Glu Lys Asn Glu Arg Thr Ser Val Ala Gly Thr Val Arg Lys Cys Trp
    610                 615                 620

Pro Asn Arg Val Ala Lys Glu Gln Ile Ser Arg Arg Leu Asp Gly Asn
625                 630                 635                 640

Gln Tyr Leu Phe Leu Pro Pro Asn Arg Tyr Ile Phe His Gly Ala Glu
                645                 650                 655

Val Tyr Ser Asp Ser Glu Asp Asp Val Leu Ser Ser Ser Ser Cys Gly
            660                 665                 670

Ser Asn Ser Asp Ser Gly Thr Cys Gln Ser Pro Ser Leu Glu Glu Pro
        675                 680                 685

Met Glu Asp Glu Ser Glu Ile Glu Glu Phe Tyr Asn Gly Leu Glu Asp
    690                 695                 700

Glu Pro Asp Val Pro Glu Arg Ala Gly Gly Ala Gly Phe Gly Thr Asp
705                 710                 715                 720

Gly Asp Asp Gln Glu Ala Ile Asn Glu Ala Ile Ser Val Lys Gln Glu
                725                 730                 735

Val Thr Asp Met Asn Tyr Pro Ser Asn Lys Ser
            740                 745
```

<210> 3
<211> 1963
<212> DNA
<213> Homo sapiens

<220>
<221> CDS
<222> (201)..(1367)

<400> 3

```
gtgttgtacg aaagcgcgtc tgcggccgca atgtctgctg agagttgtag ttctgtgccc 60

tatcacggcc actcccattt ctggtgccgt cacgggacag agcagtcggt gacaggacag 120

agcagtcggt gacgggacac agtggttggt gacgggacag agcggtcggt gacagcctca 180

agggcttcag caccgcgccc atg gca gag cca gac ccc tct cac cct ctg gag 233
                      Met Ala Glu Pro Asp Pro Ser His Pro Leu Glu
                      1               5                   10

acc cag gca ggg aag gtg cag gag gct cag gac tca gat tca gac tct   281
Thr Gln Ala Gly Lys Val Gln Glu Ala Gln Asp Ser Asp Ser Asp Ser
            15                  20                  25

gag gga gga gcc gct ggt gga gaa gca gac atg gac ttc ctg cgg aac   329
Glu Gly Gly Ala Ala Gly Gly Glu Ala Asp Met Asp Phe Leu Arg Asn
        30                  35                  40
```

```
tta ttc tcc cag acg ctc agc ctg ggc agc cag aag gag cgt ctg ctg     377
Leu Phe Ser Gln Thr Leu Ser Leu Gly Ser Gln Lys Glu Arg Leu Leu
     45                  50                  55

gac gag ctg acc ttg gaa ggg gtg gcc cgg tac atg cag agc gaa cgc     425
Asp Glu Leu Thr Leu Glu Gly Val Ala Arg Tyr Met Gln Ser Glu Arg
 60                  65                  70                  75

tgt cgc aga gtc atc tgt ttg gtg gga gct gga atc tcc aca tcc gca     473
Cys Arg Arg Val Ile Cys Leu Val Gly Ala Gly Ile Ser Thr Ser Ala
                 80                  85                  90

ggc atc ccc gac ttt cgc tct cca tcc acc ggc ctc tat gac aac cta     521
Gly Ile Pro Asp Phe Arg Ser Pro Ser Thr Gly Leu Tyr Asp Asn Leu
             95                 100                 105

gag aag tac cat ctt ccc tac cca gag gcc atc ttt gag atc agc tat     569
Glu Lys Tyr His Leu Pro Tyr Pro Glu Ala Ile Phe Glu Ile Ser Tyr
        110                 115                 120

ttc aag aaa cat ccg gaa ccc ttc ttc gcc ctc gcc aag gaa ctc tat     617
Phe Lys Lys His Pro Glu Pro Phe Phe Ala Leu Ala Lys Glu Leu Tyr
    125                 130                 135

cct ggg cag ttc aag cca acc atc tgt cac tac ttc atg cgc ctg ctg     665
Pro Gly Gln Phe Lys Pro Thr Ile Cys His Tyr Phe Met Arg Leu Leu
140                 145                 150                 155

aag gac aag ggg cta ctc ctg cgc tgc tac acg cag aac ata gat acc     713
Lys Asp Lys Gly Leu Leu Leu Arg Cys Tyr Thr Gln Asn Ile Asp Thr
                160                 165                 170

ctg gag cga ata gcc ggg ctg gaa cag gag gac ttg gtg gag gcg cac     761
Leu Glu Arg Ile Ala Gly Leu Glu Gln Glu Asp Leu Val Glu Ala His
            175                 180                 185

ggc acc ttc tac aca tca cac tgc gtc agc gcc agc tgc cgg cac gaa     809
Gly Thr Phe Tyr Thr Ser His Cys Val Ser Ala Ser Cys Arg His Glu
        190                 195                 200

tac ccg cta agc tgg atg aaa gag aag atc ttc tct gag gtg acg ccc     857
Tyr Pro Leu Ser Trp Met Lys Glu Lys Ile Phe Ser Glu Val Thr Pro
    205                 210                 215

aag tgt gaa gac tgt cag agc ctg gtg aag cct gat atc gtc ttt ttt     905
Lys Cys Glu Asp Cys Gln Ser Leu Val Lys Pro Asp Ile Val Phe Phe
220                 225                 230                 235

ggt gag agc ctc cca gcg cgt ttc ttc tcc tgt atg cag tca gac ttc     953
Gly Glu Ser Leu Pro Ala Arg Phe Phe Ser Cys Met Gln Ser Asp Phe
                240                 245                 250

ctg aag gtg gac ctc ctc ctg gtc atg ggt acc tcc ttg cag gtg cag    1001
Leu Lys Val Asp Leu Leu Leu Val Met Gly Thr Ser Leu Gln Val Gln
            255                 260                 265

ccc ttt gcc tcc ctc atc agc aag gca ccc ctc tcc acc cct cgc ctg    1049
Pro Phe Ala Ser Leu Ile Ser Lys Ala Pro Leu Ser Thr Pro Arg Leu
        270                 275                 280
```

```
ctc atc aac aag gag aaa gct ggc cag tcg gac cct ttc ctg ggg atg   1097
Leu Ile Asn Lys Glu Lys Ala Gly Gln Ser Asp Pro Phe Leu Gly Met
    285                 290                 295

att atg ggc ctc gga gga ggc atg gac ttt gac tcc aag aag gcc tac   1145
Ile Met Gly Leu Gly Gly Gly Met Asp Phe Asp Ser Lys Lys Ala Tyr
300                 305                 310                 315

agg gac gtg gcc tgg ctg ggt gaa tgc gac cag ggc tgc ctg gcc ctt   1193
Arg Asp Val Ala Trp Leu Gly Glu Cys Asp Gln Gly Cys Leu Ala Leu
                320                 325                 330

gct gag ctc ctt gga tgg aag aag gag ctg gag gac ctt gtc cgg agg   1241
Ala Glu Leu Leu Gly Trp Lys Lys Glu Leu Glu Asp Leu Val Arg Arg
            335                 340                 345

gag cac gcc agc ata gat gcc cag tcg ggg gcg ggg gtc ccc aac ccc   1289
Glu His Ala Ser Ile Asp Ala Gln Ser Gly Ala Gly Val Pro Asn Pro
        350                 355                 360

agc act tca gct tcc ccc aag aag tcc ccg cca cct gcc aag gac gag   1337
Ser Thr Ser Ala Ser Pro Lys Lys Ser Pro Pro Pro Ala Lys Asp Glu
    365                 370                 375

gcc agg aca aca gag agg gag aaa ccc cag tgacagctgc atctcccagg     1387
Ala Arg Thr Thr Glu Arg Glu Lys Pro Gln
380                 385

cgggatgccg agctcctcag ggacagctga gccccaaccg ggcctggccc cctcttaacc 1447

agcagttctt gtctggggag ctcagaacat cccccaatct cttacagctc cctccccaaa 1507

actggggtcc cagcaaccct ggcccccaac cccagcaaat ctctaacacc tcctagaggc 1567

caaggcttaa acaggcatct ctaccagccc cactgtctct aaccactcct gggctaagga 1627

gtaacctccc tcatctctaa ctgcccccac ggggccaggg ctaccccaga acttttaact 1687

cttccaggac agggagcttc gggcccccac tctgtctcct gcccccgggg gcctgtggct 1747

aagtaaacca tacctaacct accccagtgt gggtgtgggc ctctgaatat aacccacacc 1807

cagcgtaggg ggagtctgag ccgggagggc tcccgagtct ctgccttcag ctcccaaagt 1867

gggtggtggg ccccccttcac gtgggaccca cttcccatgc tggatgggca gaagacattg 1927

cttattggag acaaattaaa aacaaaaaca actaac                           1963
```

<210> 4
<211> 389
<212> PRT
<213> Homo sapiens

<400> 4

```
Met Ala Glu Pro Asp Pro Ser His Pro Leu Glu Thr Gln Ala Gly Lys
1               5                   10                  15
```

Val Gln Glu Ala Gln Asp Ser Asp Ser Asp Ser Glu Gly Gly Ala Ala
20 25 30

Gly Gly Glu Ala Asp Met Asp Phe Leu Arg Asn Leu Phe Ser Gln Thr
35 40 45

Leu Ser Leu Gly Ser Gln Lys Glu Arg Leu Leu Asp Glu Leu Thr Leu
50 55 60

Glu Gly Val Ala Arg Tyr Met Gln Ser Glu Arg Cys Arg Arg Val Ile
65 70 75 80

Cys Leu Val Gly Ala Gly Ile Ser Thr Ser Ala Gly Ile Pro Asp Phe
85 90 95

Arg Ser Pro Ser Thr Gly Leu Tyr Asp Asn Leu Glu Lys Tyr His Leu
100 105 110

Pro Tyr Pro Glu Ala Ile Phe Glu Ile Ser Tyr Phe Lys Lys His Pro
115 120 125

Glu Pro Phe Phe Ala Leu Ala Lys Glu Leu Tyr Pro Gly Gln Phe Lys
130 135 140

Pro Thr Ile Cys His Tyr Phe Met Arg Leu Leu Lys Asp Lys Gly Leu
145 150 155 160

Leu Leu Arg Cys Tyr Thr Gln Asn Ile Asp Thr Leu Glu Arg Ile Ala
165 170 175

Gly Leu Glu Gln Glu Asp Leu Val Glu Ala His Gly Thr Phe Tyr Thr
180 185 190

Ser His Cys Val Ser Ala Ser Cys Arg His Glu Tyr Pro Leu Ser Trp
195 200 205

Met Lys Glu Lys Ile Phe Ser Glu Val Thr Pro Lys Cys Glu Asp Cys
210 215 220

Gln Ser Leu Val Lys Pro Asp Ile Val Phe Phe Gly Glu Ser Leu Pro
225 230 235 240

Ala Arg Phe Phe Ser Cys Met Gln Ser Asp Phe Leu Lys Val Asp Leu
245 250 255

Leu Leu Val Met Gly Thr Ser Leu Gln Val Gln Pro Phe Ala Ser Leu
260 265 270

Ile Ser Lys Ala Pro Leu Ser Thr Pro Arg Leu Leu Ile Asn Lys Glu
275 280 285

Lys Ala Gly Gln Ser Asp Pro Phe Leu Gly Met Ile Met Gly Leu Gly
290 295 300

Gly Gly Met Asp Phe Asp Ser Lys Lys Ala Tyr Arg Asp Val Ala Trp
305 310 315 320

Leu Gly Glu Cys Asp Gln Gly Cys Leu Ala Leu Ala Glu Leu Leu Gly
325 330 335

```
Trp Lys Lys Glu Leu Glu Asp Leu Val Arg Arg Glu His Ala Ser Ile
            340                 345                 350

Asp Ala Gln Ser Gly Ala Gly Val Pro Asn Pro Ser Thr Ser Ala Ser
        355                 360                 365

Pro Lys Lys Ser Pro Pro Pro Ala Lys Asp Glu Ala Arg Thr Thr Glu
    370                 375                 380

Arg Glu Lys Pro Gln
385
```

<210> 5
<211> 1931
<212> DNA
<213> Homo sapiens

<220>
<221> CDS
<222> (257)..(1312)

<400> 5

```
cgaaagcgcg tctgcggccg caatgtctgc tgagagttgt agttctgtgc cctatcacgg 60

ccactcccat ttctggtgcc gtcacgggac agagcagtcg gtgacaggac agagcagtcg 120

gtgacgggac acagtggttg gtgacgggac agagcggtcg gtgacagcct caagggcttc 180

agcaccgcgc ccatggcaga gccagaccga ctcagattca gactctgagg gaggagccgc 240

tggtggagaa gcagac atg gac ttc ctg cgg aac tta ttc tcc cag acg ctc 292
                  Met Asp Phe Leu Arg Asn Leu Phe Ser Gln Thr Leu
                  1               5                   10

agc ctg ggc agc cag aag gag cgt ctg ctg gac gag ctg acc ttg gaa   340
Ser Leu Gly Ser Gln Lys Glu Arg Leu Leu Asp Glu Leu Thr Leu Glu
        15                  20                  25

ggg gtg gcc cgg tac atg cag agc gaa cgc tgt cgc aga gtc atc tgt   388
Gly Val Ala Arg Tyr Met Gln Ser Glu Arg Cys Arg Arg Val Ile Cys
    30                  35                  40

ttg gtg gga gct gga atc tcc aca tcc gca ggc atc ccc gac ttt cgc   436
Leu Val Gly Ala Gly Ile Ser Thr Ser Ala Gly Ile Pro Asp Phe Arg
45                  50                  55                  60

tct cca tcc acc ggc ctc tat gac aac cta gag aag tac cat ctt ccc   484
Ser Pro Ser Thr Gly Leu Tyr Asp Asn Leu Glu Lys Tyr His Leu Pro
                65                  70                  75

tac cca gag gcc atc ttt gag atc agc tat ttc aag aaa cat ccg gaa   532
Tyr Pro Glu Ala Ile Phe Glu Ile Ser Tyr Phe Lys Lys His Pro Glu
            80                  85                  90

ccc ttc ttc gcc ctc gcc aag gaa ctc tat cct ggg cag ttc aag cca   580
Pro Phe Phe Ala Leu Ala Lys Glu Leu Tyr Pro Gly Gln Phe Lys Pro
        95                  100                 105
```

```
acc atc tgt cac tac ttc atg cgc ctg ctg aag gac aag ggg cta ctc    628
Thr Ile Cys His Tyr Phe Met Arg Leu Leu Lys Asp Lys Gly Leu Leu
    110                 115                 120

ctg cgc tgc tac acg cag aac ata gat acc ctg gag cga ata gcc ggg    676
Leu Arg Cys Tyr Thr Gln Asn Ile Asp Thr Leu Glu Arg Ile Ala Gly
125                 130                 135                 140

ctg gaa cag gag gac ttg gtg gag gcg cac ggc acc ttc tac aca tca    724
Leu Glu Gln Glu Asp Leu Val Glu Ala His Gly Thr Phe Tyr Thr Ser
                145                 150                 155

cac tgc gtc agc gcc agc tgc cgg cac gaa tac ccg cta agc tgg atg    772
His Cys Val Ser Ala Ser Cys Arg His Glu Tyr Pro Leu Ser Trp Met
            160                 165                 170

aaa gag aag atc ttc tct gag gtg acg ccc aag tgt gaa gac tgt cag    820
Lys Glu Lys Ile Phe Ser Glu Val Thr Pro Lys Cys Glu Asp Cys Gln
        175                 180                 185

agc ctg gtg aag cct gat atc gtc ttt ttt ggt gag agc ctc cca gcg    868
Ser Leu Val Lys Pro Asp Ile Val Phe Phe Gly Glu Ser Leu Pro Ala
    190                 195                 200

cgt ttc ttc tcc tgt atg cag tca gac ttc ctg aag gtg gac ctc ctc    916
Arg Phe Phe Ser Cys Met Gln Ser Asp Phe Leu Lys Val Asp Leu Leu
205                 210                 215                 220

ctg gtc atg ggt acc tcc ttg cag gtg cag ccc ttt gcc tcc ctc atc    964
Leu Val Met Gly Thr Ser Leu Gln Val Gln Pro Phe Ala Ser Leu Ile
                225                 230                 235

agc aag gca ccc ctc tcc acc cct cgc ctg ctc atc aac aag gag aaa    1012

Ser Lys Ala Pro Leu Ser Thr Pro Arg Leu Leu Ile Asn Lys Glu Lys
            240                 245                 250

gct ggc cag tcg gac cct ttc ctg ggg atg att atg ggc ctc gga gga    1060
Ala Gly Gln Ser Asp Pro Phe Leu Gly Met Ile Met Gly Leu Gly Gly
        255                 260                 265

ggc atg gac ttt gac tcc aag aag gcc tac agg gac gtg gcc tgg ctg    1108
Gly Met Asp Phe Asp Ser Lys Lys Ala Tyr Arg Asp Val Ala Trp Leu
    270                 275                 280

ggt gaa tgc gac cag ggc tgc ctg gcc ctt gct gag ctc ctt gga tgg    1156
Gly Glu Cys Asp Gln Gly Cys Leu Ala Leu Ala Glu Leu Leu Gly Trp
285                 290                 295                 300

aag aag gag ctg gag gac ctt gtc cgg agg gag cac gcc agc ata gat    1204
Lys Lys Glu Leu Glu Asp Leu Val Arg Arg Glu His Ala Ser Ile Asp
                305                 310                 315

gcc cag tcg ggg gcg ggg gtc ccc aac ccc agc act tca gct tcc ccc    1252
Ala Gln Ser Gly Ala Gly Val Pro Asn Pro Ser Thr Ser Ala Ser Pro
            320                 325                 330
```

```
aag aag tcc ccg cca cct gcc aag gac gag gcc agg aca aca gag agg   1300
Lys Lys Ser Pro Pro Pro Ala Lys Asp Glu Ala Arg Thr Thr Glu Arg
        335                 340                 345

gag aaa ccc cag tgacagctgc atctcccagg cgggatgccg agctcctcag       1352
Glu Lys Pro Gln
    350

ggacagctga gccccaaccg ggcctggccc cctcttaacc agcagttctt gtctggggag 1412

ctcagaacat cccccaatct cttacagctc cctccccaaa actggggtcc cagcaaccct 1472

ggcccccaac cccagcaaat ctctaacacc tcctagaggc caaggcttaa acaggcatct 1532

ctaccagccc cactgtctct aaccactcct gggctaagga gtaacctccc tcatctctaa 1592

ctgcccccac ggggccaggg ctaccccaga acttttaact cttccaggac agggagcttc 1652

gggcccccac tctgtctcct gcccccgggg gcctgtggct aagtaaacca tacctaacct 1712

accccagtgt gggtgtgggc ctctgaatat aacccacacc cagcgtaggg ggagtctgag 1772

ccgggagggc tcccgagtct ctgccttcag ctcccaaagt gggtggtggg ccccccttcac 1832

gtgggaccca cttcccatgc tggatgggca gaagacattg cttattggag acaaattaaa 1892

aacaaaaaca actaacaaaa aaaaaaaaaa aaaaaaaaa                        1931
```

<210> 6
<211> 352
<212> PRT
<213> Homo sapiens

<400> 6

```
Met Asp Phe Leu Arg Asn Leu Phe Ser Gln Thr Leu Ser Leu Gly Ser
1               5                   10                  15

Gln Lys Glu Arg Leu Leu Asp Glu Leu Thr Leu Glu Gly Val Ala Arg
            20                  25                  30

Tyr Met Gln Ser Glu Arg Cys Arg Arg Val Ile Cys Leu Val Gly Ala
        35                  40                  45

Gly Ile Ser Thr Ser Ala Gly Ile Pro Asp Phe Arg Ser Pro Ser Thr
    50                  55                  60

Gly Leu Tyr Asp Asn Leu Glu Lys Tyr His Leu Pro Tyr Pro Glu Ala
65                  70                  75                  80

Ile Phe Glu Ile Ser Tyr Phe Lys Lys His Pro Glu Pro Phe Phe Ala
                85                  90                  95

Leu Ala Lys Glu Leu Tyr Pro Gly Gln Phe Lys Pro Thr Ile Cys His
            100                 105                 110

Tyr Phe Met Arg Leu Leu Lys Asp Lys Gly Leu Leu Leu Arg Cys Tyr
        115                 120                 125
```

```
Thr Gln Asn Ile Asp Thr Leu Glu Arg Ile Ala Gly Leu Glu Gln Glu
    130                 135                 140

Asp Leu Val Glu Ala His Gly Thr Phe Tyr Thr Ser His Cys Val Ser
145                 150                 155                 160

Ala Ser Cys Arg His Glu Tyr Pro Leu Ser Trp Met Lys Glu Lys Ile
                165                 170                 175

Phe Ser Glu Val Thr Pro Lys Cys Glu Asp Cys Gln Ser Leu Val Lys
            180                 185                 190

Pro Asp Ile Val Phe Phe Gly Glu Ser Leu Pro Ala Arg Phe Phe Ser
        195                 200                 205

Cys Met Gln Ser Asp Phe Leu Lys Val Asp Leu Leu Leu Val Met Gly
    210                 215                 220

Thr Ser Leu Gln Val Gln Pro Phe Ala Ser Leu Ile Ser Lys Ala Pro
225                 230                 235                 240

Leu Ser Thr Pro Arg Leu Leu Ile Asn Lys Glu Lys Ala Gly Gln Ser
                245                 250                 255

Asp Pro Phe Leu Gly Met Ile Met Gly Leu Gly Gly Gly Met Asp Phe
            260                 265                 270

Asp Ser Lys Lys Ala Tyr Arg Asp Val Ala Trp Leu Gly Glu Cys Asp
        275                 280                 285

Gln Gly Cys Leu Ala Leu Ala Glu Leu Leu Gly Trp Lys Lys Glu Leu
    290                 295                 300

Glu Asp Leu Val Arg Arg Glu His Ala Ser Ile Asp Ala Gln Ser Gly
305                 310                 315                 320

Ala Gly Val Pro Asn Pro Ser Thr Ser Ala Ser Pro Lys Lys Ser Pro
                325                 330                 335

Pro Pro Ala Lys Asp Glu Ala Arg Thr Thr Glu Arg Glu Lys Pro Gln
            340                 345                 350
```

<210> 7
<211> 1927
<212> DNA
<213> Homo sapiens

<220>
<221> CDS
<222> (103)..(1299)

<400> 7

```
ggcgccgggg gcgggggtgg gaggcggagg cggggccggg gcgccgcggg cggggcgccg 60

ggggcggggc gagtccggag gactcctcgg actgcgcgga ac atg gcg ttc tgg    114
                                               Met Ala Phe Trp
                                               1
```

```
ggt tgg cgc gcc gcg gca gcc ctc cgg ctg tgg ggc cgg gta gtt gaa    162
Gly Trp Arg Ala Ala Ala Ala Leu Arg Leu Trp Gly Arg Val Val Glu
  5                   10                  15                  20

cgg gtc gag gcc ggg gga ggc gtg ggg ccg ttt cag gcc tgc ggc tgt    210
Arg Val Glu Ala Gly Gly Gly Val Gly Pro Phe Gln Ala Cys Gly Cys
                25                  30                  35

cgg ctg gtg ctt ggc ggc agg gac gat gtg agt gcg ggg ctg aga ggc    258
Arg Leu Val Leu Gly Gly Arg Asp Asp Val Ser Ala Gly Leu Arg Gly
            40                  45                  50

agc cat ggg gcc cgc ggt gag ccc ttg gac ccg gcg cgc ccc ttg cag    306
Ser His Gly Ala Arg Gly Glu Pro Leu Asp Pro Ala Arg Pro Leu Gln
        55                  60                  65

agg cct ccc aga ccc gag gtg ccc agg gca ttc cgg agg cag ccg agg    354
Arg Pro Pro Arg Pro Glu Val Pro Arg Ala Phe Arg Arg Gln Pro Arg
    70                  75                  80

gca gca gct ccc agt ttc ttc ttt tcg agt att aaa ggt gga aga agg    402
Ala Ala Ala Pro Ser Phe Phe Phe Ser Ser Ile Lys Gly Gly Arg Arg
 85                  90                  95                 100

tcc ata tct ttt tct gtg ggt gct tca agt gtt gtt gga agt gga ggc    450
Ser Ile Ser Phe Ser Val Gly Ala Ser Ser Val Val Gly Ser Gly Gly
                105                 110                 115

agc agt gac aag ggg aag ctt tcc ctg cag gat gta gct gag ctg att    498
Ser Ser Asp Lys Gly Lys Leu Ser Leu Gln Asp Val Ala Glu Leu Ile
            120                 125                 130

cgg gcc aga gcc tgc cag agg gtg gtg gtc atg gtg ggg gcc ggc atc    546
Arg Ala Arg Ala Cys Gln Arg Val Val Val Met Val Gly Ala Gly Ile
        135                 140                 145

agc aca ccc agt ggc att cca gac ttc aga tcg ccg ggg agt ggc ctg    594
Ser Thr Pro Ser Gly Ile Pro Asp Phe Arg Ser Pro Gly Ser Gly Leu
    150                 155                 160

tac agc aac ctc cag cag tac gat ctc ccg tac ccc gag gcc att ttt    642
Tyr Ser Asn Leu Gln Gln Tyr Asp Leu Pro Tyr Pro Glu Ala Ile Phe
165                 170                 175                 180

gaa ctc cca ttc ttc ttt cac aac ccc aag ccc ttt ttc act ttg gcc    690
Glu Leu Pro Phe Phe Phe His Asn Pro Lys Pro Phe Phe Thr Leu Ala
                185                 190                 195

aag gag ctg tac cct gga aac tac aag ccc aac gtc act cac tac ttt    738
Lys Glu Leu Tyr Pro Gly Asn Tyr Lys Pro Asn Val Thr His Tyr Phe
            200                 205                 210

ctc cgg ctg ctt cat gac aag ggg ctg ctt ctg cgg ctc tac acg cag    786
Leu Arg Leu Leu His Asp Lys Gly Leu Leu Leu Arg Leu Tyr Thr Gln
        215                 220                 225
```

```
aac atc gat ggg ctt gag aga gtg tcg ggc atc cct gcc tca aag ctg    834
Asn Ile Asp Gly Leu Glu Arg Val Ser Gly Ile Pro Ala Ser Lys Leu
    230                 235                 240

gtt gaa gct cat gga acc ttt gcc tct gcc acc tgc aca gtc tgc caa    882
Val Glu Ala His Gly Thr Phe Ala Ser Ala Thr Cys Thr Val Cys Gln
245                 250                 255                 260

aga ccc ttc cca ggg gag gac att cgg gct gac gtg atg gca gac agg    930
Arg Pro Phe Pro Gly Glu Asp Ile Arg Ala Asp Val Met Ala Asp Arg
                265                 270                 275

gtt ccc cgc tgc ccg gtc tgc acc ggc gtt gtg aag ccc gac att gtg    978
Val Pro Arg Cys Pro Val Cys Thr Gly Val Val Lys Pro Asp Ile Val
            280                 285                 290

ttc ttt ggg gag ccg ctg ccc cag agg ttc ttg ctg cat gtg gtt gat    1026
Phe Phe Gly Glu Pro Leu Pro Gln Arg Phe Leu Leu His Val Val Asp
        295                 300                 305

ttc ccc atg gca gat ctg ctg ctc atc ctt ggg acc tcc ctg gag gtg    1074
Phe Pro Met Ala Asp Leu Leu Leu Ile Leu Gly Thr Ser Leu Glu Val
    310                 315                 320

gag cct ttt gcc agc ttg acc gag gcc gtg cgg agc tca gtt ccc cga    1122
Glu Pro Phe Ala Ser Leu Thr Glu Ala Val Arg Ser Ser Val Pro Arg
325                 330                 335                 340

ctg ctc atc aac cgg gac ttg gtg ggg ccc ttg gct tgg cat cct cgc    1170
Leu Leu Ile Asn Arg Asp Leu Val Gly Pro Leu Ala Trp His Pro Arg
                345                 350                 355

agc agg gac gtg gcc cag ctg ggg gac gtg gtt cac ggc gtg gaa agc    1218
Ser Arg Asp Val Ala Gln Leu Gly Asp Val Val His Gly Val Glu Ser
            360                 365                 370

cta gtg gag ctt ctg ggc tgg aca gaa gag atg cgg gac ctt gtg cag    1266
Leu Val Glu Leu Leu Gly Trp Thr Glu Glu Met Arg Asp Leu Val Gln
        375                 380                 385

cgg gaa act ggg aag ctt gat gga cca gac aaa taggatgatg gctgccccca 1319
Arg Glu Thr Gly Lys Leu Asp Gly Pro Asp Lys
    390                 395

cacaataaat ggtaacatag gagacatcca catcccaatt ctgacaagac ctcatgcctg 1379

aagacagctt gggcaggtga aaccagaata tgtgaactga gtggacaccc gaggctgcca 1439

ctggaatgtc ttctcaggcc atgagctgca gtgactggta gggctgtgtt tacagtcagg 1499

gccaccccgt cacatataca aaggagctgc ctgcctgttt gctgtgttga actcttcact 1559

ctgctgaagc tcctaatgga aaaagctttc ttctgactgt gaccctcttg aactgaatca 1619

gaccaactgg aatcccagac cgagtctgct ttctgtgcct agttgaacgg caagctcggc 1679

atctgttggt tacaagatcc agacttgggc cgagcggtcc ccagccctct tcatgttccg 1739

aagtgtagtc ttgaggccct ggtgccgcac ttctagcatg ttggtctcct ttagtggggc 1799
```

tatttttaat gagagaaaat ctgttctttc cagcatgaaa tacatttagt ctcctcaaag 1859

ggaaaaaaaa aaaaaaaaaa aaaaaaaaaa aaaaaaaaaa aaaaaaaaaa aaaaaaaaaa 1919

aaaaaaaa 1927

<210> 8
<211> 399
<212> PRT
<213> Homo sapiens

<400> 8

```
Met Ala Phe Trp Gly Trp Arg Ala Ala Ala Ala Leu Arg Leu Trp Gly
1               5                   10                  15

Arg Val Val Glu Arg Val Glu Ala Gly Gly Gly Val Gly Pro Phe Gln
            20                  25                  30

Ala Cys Gly Cys Arg Leu Val Leu Gly Gly Arg Asp Asp Val Ser Ala
        35                  40                  45

Gly Leu Arg Gly Ser His Gly Ala Arg Gly Glu Pro Leu Asp Pro Ala
    50                  55                  60

Arg Pro Leu Gln Arg Pro Pro Arg Pro Glu Val Pro Arg Ala Phe Arg
65                  70                  75                  80

Arg Gln Pro Arg Ala Ala Ala Pro Ser Phe Phe Phe Ser Ser Ile Lys
                85                  90                  95

Gly Gly Arg Arg Ser Ile Ser Phe Ser Val Gly Ala Ser Ser Val Val
            100                 105                 110

Gly Ser Gly Gly Ser Ser Asp Lys Gly Lys Leu Ser Leu Gln Asp Val
        115                 120                 125

Ala Glu Leu Ile Arg Ala Arg Ala Cys Gln Arg Val Val Val Met Val
    130                 135                 140

Gly Ala Gly Ile Ser Thr Pro Ser Gly Ile Pro Asp Phe Arg Ser Pro
145                 150                 155                 160

Gly Ser Gly Leu Tyr Ser Asn Leu Gln Gln Tyr Asp Leu Pro Tyr Pro
                165                 170                 175

Glu Ala Ile Phe Glu Leu Pro Phe Phe Phe His Asn Pro Lys Pro Phe
            180                 185                 190

Phe Thr Leu Ala Lys Glu Leu Tyr Pro Gly Asn Tyr Lys Pro Asn Val
        195                 200                 205

Thr His Tyr Phe Leu Arg Leu Leu His Asp Lys Gly Leu Leu Leu Arg
    210                 215                 220

Leu Tyr Thr Gln Asn Ile Asp Gly Leu Glu Arg Val Ser Gly Ile Pro
225                 230                 235                 240
```

```
Ala Ser Lys Leu Val Glu Ala His Gly Thr Phe Ala Ser Ala Thr Cys
                245                 250                 255

Thr Val Cys Gln Arg Pro Phe Pro Gly Glu Asp Ile Arg Ala Asp Val
            260                 265                 270

Met Ala Asp Arg Val Pro Arg Cys Pro Val Cys Thr Gly Val Val Lys
        275                 280                 285

Pro Asp Ile Val Phe Phe Gly Glu Pro Leu Pro Gln Arg Phe Leu Leu
    290                 295                 300

His Val Val Asp Phe Pro Met Ala Asp Leu Leu Leu Ile Leu Gly Thr
305                 310                 315                 320

Ser Leu Glu Val Glu Pro Phe Ala Ser Leu Thr Glu Ala Val Arg Ser
                325                 330                 335

Ser Val Pro Arg Leu Leu Ile Asn Arg Asp Leu Val Gly Pro Leu Ala
            340                 345                 350

Trp His Pro Arg Ser Arg Asp Val Ala Gln Leu Gly Asp Val Val His
        355                 360                 365

Gly Val Glu Ser Leu Val Glu Leu Leu Gly Trp Thr Glu Glu Met Arg
    370                 375                 380

Asp Leu Val Gln Arg Glu Thr Gly Lys Leu Asp Gly Pro Asp Lys
385                 390                 395
```

<210> 9
<211> 1174
<212> DNA
<213> Homo sapiens

<220>
<221> CDS
<222> (21)..(962)

<400> 9

```
gtccgtagag ctgtgagaga atg aag atg agc ttt gcg ttg act ttc agg tca 53
                      Met Lys Met Ser Phe Ala Leu Thr Phe Arg Ser
                      1               5                   10

gca aaa ggc cgt tgg atc gca aac ccc agc cag ccg tgc tcg aaa gcc   101
Ala Lys Gly Arg Trp Ile Ala Asn Pro Ser Gln Pro Cys Ser Lys Ala
            15                  20                  25

tcc att ggg tta ttt gtg cca gca agt cct cct ctg gac cct gag aag   149
Ser Ile Gly Leu Phe Val Pro Ala Ser Pro Pro Leu Asp Pro Glu Lys
        30                  35                  40

gtc aaa gag tta cag cgc ttc atc acc ctt tcc aag aga ctc ctt gtg   197
Val Lys Glu Leu Gln Arg Phe Ile Thr Leu Ser Lys Arg Leu Leu Val
    45                  50                  55
```

```
atg act ggg gca gga atc tcc acc gaa tcg ggg ata cca gac tac agg    245
Met Thr Gly Ala Gly Ile Ser Thr Glu Ser Gly Ile Pro Asp Tyr Arg
 60                 65                  70                  75

tca gaa aaa gtg ggg ctt tat gcc cgc act gac cgc agg ccc atc cag    293
Ser Glu Lys Val Gly Leu Tyr Ala Arg Thr Asp Arg Arg Pro Ile Gln
                80                  85                  90

cat ggt gat ttt gtc cgg agt gcc cca atc cgc cag cgg tac tgg gcg    341
His Gly Asp Phe Val Arg Ser Ala Pro Ile Arg Gln Arg Tyr Trp Ala
            95                 100                 105

aga aac ttc gta ggc tgg cct caa ttc tcc tcc cac cag cct aac cct    389
Arg Asn Phe Val Gly Trp Pro Gln Phe Ser Ser His Gln Pro Asn Pro
        110                 115                 120

gca cac tgg gct ttg agc acc tgg gag aaa ctc gga aag ctg tac tgg    437
Ala His Trp Ala Leu Ser Thr Trp Glu Lys Leu Gly Lys Leu Tyr Trp
    125                 130                 135

ttg gtg acc caa aat gtg gat gct ttg cac acc aag gcg ggg agt cgg    485
Leu Val Thr Gln Asn Val Asp Ala Leu His Thr Lys Ala Gly Ser Arg
140                 145                 150                 155

cgc ctg aca gag ctc cac gga tgc atg gac agg gtc ctg tgc ttg gat    533
Arg Leu Thr Glu Leu His Gly Cys Met Asp Arg Val Leu Cys Leu Asp
                160                 165                 170

tgt ggg gaa cag act ccc cgg ggg gtg ctg caa gag cgt ttc caa gtc    581
Cys Gly Glu Gln Thr Pro Arg Gly Val Leu Gln Glu Arg Phe Gln Val
            175                 180                 185

ctg aac ccc acc tgg agt gct gag gcc cat ggc ctg gct cct gat ggt    629
Leu Asn Pro Thr Trp Ser Ala Glu Ala His Gly Leu Ala Pro Asp Gly
        190                 195                 200

gac gtc ttt ctc tca gag gag caa gtc cgg agc ttt cag gtc cca acc    677
Asp Val Phe Leu Ser Glu Glu Gln Val Arg Ser Phe Gln Val Pro Thr
    205                 210                 215

tgc gtt caa tgt gga ggc cat ctg aaa cca gat gtc gtt ttc ttc ggg    725
Cys Val Gln Cys Gly Gly His Leu Lys Pro Asp Val Val Phe Phe Gly
220                 225                 230                 235

gac aca gtg aac cct gac aag gtt gat ttt gtg cac aag cgt gta aaa    773
Asp Thr Val Asn Pro Asp Lys Val Asp Phe Val His Lys Arg Val Lys
                240                 245                 250

gaa gcc gac tcc ctc ttg gtg gtg gga tca tcc ttg cag gta tac tct    821
Glu Ala Asp Ser Leu Leu Val Val Gly Ser Ser Leu Gln Val Tyr Ser
            255                 260                 265

ggt tac agg ttt atc ctc act gcc tgg gag aag aag ctc ccg att gca    869
Gly Tyr Arg Phe Ile Leu Thr Ala Trp Glu Lys Lys Leu Pro Ile Ala
        270                 275                 280

ata ctg aac att ggg ccc aca cgg tcg gat gac ttg gcg tgt ctg aaa    917
Ile Leu Asn Ile Gly Pro Thr Arg Ser Asp Asp Leu Ala Cys Leu Lys
    285                 290                 295
```

```
ctg aat tct cgt tgt gga gag ttg ctg cct ttg ata gac cca tgc       962
Leu Asn Ser Arg Cys Gly Glu Leu Leu Pro Leu Ile Asp Pro Cys
300                 305                 310

tgaccacagc ctgatattcc agaacctgga acagggactt tcacttgaat cttgctgcta 1022

aatgtaaatg ccttctcaaa tgacagattc cagttcccat tcaacagagt agggtgcact 1082

gacaaagtat agaaggttct aggtatctta atgtgtggat attcttaatt aaaactcatt 1142

ttttttaaat aaaaaattgt tcagctttaa aa                               1174
```

<210> 10
<211> 314
<212> PRT
<213> Homo sapiens

<400> 10

```
Met Lys Met Ser Phe Ala Leu Thr Phe Arg Ser Ala Lys Gly Arg Trp
1               5                   10                  15

Ile Ala Asn Pro Ser Gln Pro Cys Ser Lys Ala Ser Ile Gly Leu Phe
            20                  25                  30

Val Pro Ala Ser Pro Pro Leu Asp Pro Glu Lys Val Lys Glu Leu Gln
        35                  40                  45

Arg Phe Ile Thr Leu Ser Lys Arg Leu Leu Val Met Thr Gly Ala Gly
    50                  55                  60

Ile Ser Thr Glu Ser Gly Ile Pro Asp Tyr Arg Ser Glu Lys Val Gly
65                  70                  75                  80

Leu Tyr Ala Arg Thr Asp Arg Arg Pro Ile Gln His Gly Asp Phe Val
                85                  90                  95

Arg Ser Ala Pro Ile Arg Gln Arg Tyr Trp Ala Arg Asn Phe Val Gly
            100                 105                 110

Trp Pro Gln Phe Ser Ser His Gln Pro Asn Pro Ala His Trp Ala Leu
        115                 120                 125

Ser Thr Trp Glu Lys Leu Gly Lys Leu Tyr Trp Leu Val Thr Gln Asn
    130                 135                 140

Val Asp Ala Leu His Thr Lys Ala Gly Ser Arg Arg Leu Thr Glu Leu
145                 150                 155                 160

His Gly Cys Met Asp Arg Val Leu Cys Leu Asp Cys Gly Glu Gln Thr
                165                 170                 175

Pro Arg Gly Val Leu Gln Glu Arg Phe Gln Val Leu Asn Pro Thr Trp
            180                 185                 190

Ser Ala Glu Ala His Gly Leu Ala Pro Asp Gly Asp Val Phe Leu Ser
        195                 200                 205
```

```
Glu Glu Gln Val Arg Ser Phe Gln Val Pro Thr Cys Val Gln Cys Gly
    210                 215                 220

Gly His Leu Lys Pro Asp Val Val Phe Phe Gly Asp Thr Val Asn Pro
225                 230                 235                 240

Asp Lys Val Asp Phe Val His Lys Arg Val Lys Glu Ala Asp Ser Leu
                245                 250                 255

Leu Val Val Gly Ser Ser Leu Gln Val Tyr Ser Gly Tyr Arg Phe Ile
            260                 265                 270

Leu Thr Ala Trp Glu Lys Lys Leu Pro Ile Ala Ile Leu Asn Ile Gly
        275                 280                 285

Pro Thr Arg Ser Asp Asp Leu Ala Cys Leu Lys Leu Asn Ser Arg Cys
    290                 295                 300

Gly Glu Leu Leu Pro Leu Ile Asp Pro Cys
305                 310
```

<210> 11
<211> 2350
<212> DNA
<213> Homo sapiens

<220>
<221> CDS
<222> (219)..(1115)

<400> 11

```
attcgggggc gcgagctgcc ccagtaaatg gaaatgtttt ctaacatata aaaacctaca 60

gaagaagaaa ataattttct ggatcaaatt agaagtctgt attatattga tgtctccaga 120

ttcaaatata ttagaaagca gccgtggaga caaccatctt cattttgggc gaaataacta 180

aagcccgcct caagcattag aactacagac aaaccctg atg cga cct ctc cag att 236
                                         Met Arg Pro Leu Gln Ile
                                         1               5

gtc cca agt cga ttg att tcc cag cta tat tgt ggc ctg aag cct cca    284
Val Pro Ser Arg Leu Ile Ser Gln Leu Tyr Cys Gly Leu Lys Pro Pro
            10                  15                  20

gcg tcc aca cga aac cag att tgc ctg aaa atg gct cgg cca agt tca    332
Ala Ser Thr Arg Asn Gln Ile Cys Leu Lys Met Ala Arg Pro Ser Ser
        25                  30                  35

agt atg gca gat ttt cga aag ttt ttt gca aaa gca aag cac ata gtc    380
Ser Met Ala Asp Phe Arg Lys Phe Phe Ala Lys Ala Lys His Ile Val
    40                  45                  50

atc atc tca gga gct ggt gtt agt gca gaa agt ggt gtt ccg acc ttc    428
Ile Ile Ser Gly Ala Gly Val Ser Ala Glu Ser Gly Val Pro Thr Phe
55                  60                  65                  70
```

```
aga gga gct gga ggt tat tgg aga aaa tgg caa gcc cag gac ctg gcg    476
Arg Gly Ala Gly Gly Tyr Trp Arg Lys Trp Gln Ala Gln Asp Leu Ala
                75                  80                  85

act ccc ctg gcc ttt gcc cac aac ccg tcc cgg gtg tgg gag ttc tac    524
Thr Pro Leu Ala Phe Ala His Asn Pro Ser Arg Val Trp Glu Phe Tyr
            90                  95                  100

cac tac cgg cgg gag gtc atg ggg agc aag gag ccc aac gcc ggg cac    572
His Tyr Arg Arg Glu Val Met Gly Ser Lys Glu Pro Asn Ala Gly His
        105                 110                 115

cgc gcc ata gcc gag tgt gag acc cgg ctg ggc aag cag ggc cgg cga    620
Arg Ala Ile Ala Glu Cys Glu Thr Arg Leu Gly Lys Gln Gly Arg Arg
    120                 125                 130

gtc gtg gtc atc acc cag aac atc gat gag ctg cac cgc aag gct ggc    668
Val Val Val Ile Thr Gln Asn Ile Asp Glu Leu His Arg Lys Ala Gly
135                 140                 145                 150

acc aag aac ctt ctg gag atc cat ggt agc tta ttt aaa act cga tgt    716
Thr Lys Asn Leu Leu Glu Ile His Gly Ser Leu Phe Lys Thr Arg Cys
                155                 160                 165

acc tct tgt gga gtt gtg gct gag aat tac aag agt cca att tgt cca    764
Thr Ser Cys Gly Val Val Ala Glu Asn Tyr Lys Ser Pro Ile Cys Pro
            170                 175                 180

gct tta tca gga aaa ggt gct cca gaa cct gga act caa gat gcc agc    812
Ala Leu Ser Gly Lys Gly Ala Pro Glu Pro Gly Thr Gln Asp Ala Ser
        185                 190                 195

atc cca gtt gag aaa ctt ccc cgg tgt gaa gag gca ggc tgc ggg ggc    860
Ile Pro Val Glu Lys Leu Pro Arg Cys Glu Glu Ala Gly Cys Gly Gly
    200                 205                 210

ttg ctg cga cct cac gtc gtg tgg ttt gga gaa aac ctg gat cct gcc    908
Leu Leu Arg Pro His Val Val Trp Phe Gly Glu Asn Leu Asp Pro Ala
215                 220                 225                 230

att ctg gag gag gtt gac aga gag ctc gcc cac tgt gat tta tgt cta    956
Ile Leu Glu Glu Val Asp Arg Glu Leu Ala His Cys Asp Leu Cys Leu
                235                 240                 245

gtg gtg ggc act tcc tct gtg gtg tac cca gca gcc atg ttt gcc ccc    1004
Val Val Gly Thr Ser Ser Val Val Tyr Pro Ala Ala Met Phe Ala Pro
            250                 255                 260

cag gtg gct gcc agg ggc gtg cca gtg gct gaa ttt aac acg gag acc    1052
Gln Val Ala Ala Arg Gly Val Pro Val Ala Glu Phe Asn Thr Glu Thr
        265                 270                 275

acc cca gct acg aac aga ttc agt cat ttg atc tcc atc tca tct cta    1100
Thr Pro Ala Thr Asn Arg Phe Ser His Leu Ile Ser Ile Ser Ser Leu
    280                 285                 290

att att ata aag aat taaaacaagt catcattgta gaaaagcaag aaaatgcaga    1155
Ile Ile Ile Lys Asn
295
```

tagagaaaaa gaagaaaata aaactggagt atttccacaa cccaagttta gagttggccc 1215
ccacctccca tgccatggac tgagcagcag gggcccagca tcccttggat atggtggctg 1275
tgtcttcatg tgaaagaaac tgaacttggt ggtttttcct gccagttcag gagagattct 1335
tggcatgtaa tatatatcac tgctcaagtc aagcctccta aaaccacaga cctgtttcag 1395
ctgctacttc agccaaaatt cttcagcttc atattgtctt gaaaacctat gattgtctct 1455
aacaaacagg ctacttgcta gttagaaatt cttatcaatt tggcaagcta cttatcaacc 1515
agactgacca caagaactgt catctcatca atgaaggagt aactgatcaa tgaagccagc 1575
aatgcttttt tcttggcatc atcaaagctg acatttagaa gagatgctgg tgatagtcat 1635
ctcatcctac tcaatttttc aaaggcagaa accaaccctg gagcaattga gaggactgtt 1695
taaacacaga gcttaacaat ggcagaattg tatatctcgt gcttaacaga ttttggttga 1755
actttaccct aggtcagggg tcagcaaact actgcctgtg ggccaaattt gcccaccacc 1815
tgtatctgta aataaggttt cattggaaca cagctgtggc catatgtttg tatattgtgt 1875
gtggctgctt ttgcattagg atgacagagg tgaatagttg caacagagac tggctggtct 1935
gcaaagccta aaatatgtcc tgtgtggccc tttacagaaa aagttttcta acccctgctc 1995
taggttacgg agaaaaaaaa atggaataat gttctctgct acttttaacc tgattttctt 2055
tgtacctaaa taggcagcta gaatgctgcc tatattttaa taaggatttg gatctcacaa 2115
gacaccttag gcctacacaa gttgttcaga ttctttgccc cagttctaat ctagtgacaa 2175
aggcatagaa ttctcctccc acaggaatgt atttctattt tcaaggtgtt aattagttcc 2235
agttttggtt ttgtcgtttt ccccatgtcc gatgcttata ttggatgatt tctgataaac 2295
ctgactattc caataaaccc taggcatttt tgaatttaaa aaaaaaaaaa aaaaa 2350

<210> 12
<211> 299
<212> PRT
<213> Homo sapiens

<400> 12

Met Arg Pro Leu Gln Ile Val Pro Ser Arg Leu Ile Ser Gln Leu Tyr
1 5 10 15

Cys Gly Leu Lys Pro Pro Ala Ser Thr Arg Asn Gln Ile Cys Leu Lys
20 25 30

Met Ala Arg Pro Ser Ser Ser Met Ala Asp Phe Arg Lys Phe Phe Ala
35 40 45

Lys Ala Lys His Ile Val Ile Ile Ser Gly Ala Gly Val Ser Ala Glu
50 55 60

Ser Gly Val Pro Thr Phe Arg Gly Ala Gly Gly Tyr Trp Arg Lys Trp
65 70 75 80

Gln Ala Gln Asp Leu Ala Thr Pro Leu Ala Phe Ala His Asn Pro Ser
85 90 95

Arg Val Trp Glu Phe Tyr His Tyr Arg Arg Glu Val Met Gly Ser Lys
100 105 110

Glu Pro Asn Ala Gly His Arg Ala Ile Ala Glu Cys Glu Thr Arg Leu
115 120 125

Gly Lys Gln Gly Arg Arg Val Val Val Ile Thr Gln Asn Ile Asp Glu
130 135 140

Leu His Arg Lys Ala Gly Thr Lys Asn Leu Leu Glu Ile His Gly Ser
145 150 155 160

Leu Phe Lys Thr Arg Cys Thr Ser Cys Gly Val Val Ala Glu Asn Tyr
165 170 175

Lys Ser Pro Ile Cys Pro Ala Leu Ser Gly Lys Gly Ala Pro Glu Pro
180 185 190

Gly Thr Gln Asp Ala Ser Ile Pro Val Glu Lys Leu Pro Arg Cys Glu
195 200 205

Glu Ala Gly Cys Gly Gly Leu Leu Arg Pro His Val Val Trp Phe Gly
210 215 220

Glu Asn Leu Asp Pro Ala Ile Leu Glu Glu Val Asp Arg Glu Leu Ala
225 230 235 240

His Cys Asp Leu Cys Leu Val Val Gly Thr Ser Ser Val Val Tyr Pro
245 250 255

Ala Ala Met Phe Ala Pro Gln Val Ala Ala Arg Gly Val Pro Val Ala
260 265 270

Glu Phe Asn Thr Glu Thr Thr Pro Ala Thr Asn Arg Phe Ser His Leu
275 280 285

Ile Ser Ile Ser Ser Leu Ile Ile Ile Lys Asn
290 295

<210> 13
<211> 1670
<212> DNA
<213> Homo sapiens

<220>
<221> CDS
<222> (297)..(1226)

<400> 13

ccggagcgcg gtcgggacac agcgcctcta ggagaaagcc tggaaggcgc tccgggggta 60

```
cccagagctc ttagcgggcc ggcagcatgt gcggggccca agtaaatgga aatgttttct 120

aacatataaa aacctacaga agaagaaaat aattttctgg atcaaattag aagtctgtat 180

tatattgatg tctccagatt caaatatatt agaaagcagc cgtggagaca accatcttca 240

ttttgggaga aataactaaa gcccgcctca agcattagaa ctacagacaa accctg atg 299
                                                              Met
                                                              1

cga cct ctc cag att gtc cca agt cga ttg att tcc cag cta tat tgt   347
Arg Pro Leu Gln Ile Val Pro Ser Arg Leu Ile Ser Gln Leu Tyr Cys
            5                   10                  15

ggc ctg aag cct cca gcg tcc aca cga aac cag att tgc ctg aaa atg   395
Gly Leu Lys Pro Pro Ala Ser Thr Arg Asn Gln Ile Cys Leu Lys Met
        20                  25                  30

gct cgg cca agt tca agt atg gca gat ttt cga aag ttt ttt gca aaa   443
Ala Arg Pro Ser Ser Ser Met Ala Asp Phe Arg Lys Phe Phe Ala Lys
    35                  40                  45

gca aag cac ata gtc atc atc tca gga gct ggt gtt agt gca gaa agt   491
Ala Lys His Ile Val Ile Ile Ser Gly Ala Gly Val Ser Ala Glu Ser
50                  55                  60                  65

ggt gtt ccg acc ttc aga gga gct gga ggt tat tgg aga aaa tgg caa   539
Gly Val Pro Thr Phe Arg Gly Ala Gly Gly Tyr Trp Arg Lys Trp Gln
                70                  75                  80

gcc cag gac ctg gcg act ccc ctg gcc ttt gcc cac aac ccg tcc cgg   587
Ala Gln Asp Leu Ala Thr Pro Leu Ala Phe Ala His Asn Pro Ser Arg
            85                  90                  95

gtg tgg gag ttc tac cac tac cgg cgg gag gtc atg ggg agc aag gag   635
Val Trp Glu Phe Tyr His Tyr Arg Arg Glu Val Met Gly Ser Lys Glu
        100                 105                 110

ccc aac gcc ggg cac cgc gcc ata gcc gag tgt gag acc cgg ctg ggc   683
Pro Asn Ala Gly His Arg Ala Ile Ala Glu Cys Glu Thr Arg Leu Gly
    115                 120                 125

aag cag ggc cgg cga gtc gtg gtc atc acc cag aac atc gat gag ctg   731
Lys Gln Gly Arg Arg Val Val Val Ile Thr Gln Asn Ile Asp Glu Leu
130                 135                 140                 145

cac cgc aag gct ggc acc aag aac ctt ctg gag atc cat ggt agc tta   779
His Arg Lys Ala Gly Thr Lys Asn Leu Leu Glu Ile His Gly Ser Leu
                150                 155                 160

ttt aaa act cga tgt acc tct tgt gga gtt gtg gct gag aat tac aag   827
Phe Lys Thr Arg Cys Thr Ser Cys Gly Val Val Ala Glu Asn Tyr Lys
            165                 170                 175

agt cca att tgt cca gct tta tca gga aaa ggt gct cca gaa cct gga   875
Ser Pro Ile Cys Pro Ala Leu Ser Gly Lys Gly Ala Pro Glu Pro Gly
        180                 185                 190
```

```
act caa gat gcc agc atc cca gtt gag aaa ctt ccc cgg tgt gaa gag   923
Thr Gln Asp Ala Ser Ile Pro Val Glu Lys Leu Pro Arg Cys Glu Glu
    195                 200                 205

gca ggc tgc ggg ggc ttg ctg cga cct cac gtc gtg tgg ttt gga gaa   971
Ala Gly Cys Gly Gly Leu Leu Arg Pro His Val Val Trp Phe Gly Glu
210                 215                 220                 225

aac ctg gat cct gcc att ctg gag gag gtt gac aga gag ctc gcc cac   1019
Asn Leu Asp Pro Ala Ile Leu Glu Glu Val Asp Arg Glu Leu Ala His
                230                 235                 240

tgt gat tta tgt cta gtg gtg ggc act tcc tct gtg gtg tac cca gca   1067
Cys Asp Leu Cys Leu Val Val Gly Thr Ser Ser Val Val Tyr Pro Ala
            245                 250                 255

gcc atg ttt gcc ccc cag gtg gct gcc agg ggc gtg cca gtg gct gaa   1115
Ala Met Phe Ala Pro Gln Val Ala Ala Arg Gly Val Pro Val Ala Glu
        260                 265                 270

ttt aac acg gag acc acc cca gct acg aac aga ttc agg ttt cat ttc   1163
Phe Asn Thr Glu Thr Thr Pro Ala Thr Asn Arg Phe Arg Phe His Phe
    275                 280                 285

cag gga ccc tgt gga acg act ctt cct gaa gcc ctt gcc tgt cat gaa   1211
Gln Gly Pro Cys Gly Thr Thr Leu Pro Glu Ala Leu Ala Cys His Glu
290                 295                 300                 305

aat gaa act gtt tct taagtgtcct ggggaagaaa gaaattacag tatatctaag   1266
Asn Glu Thr Val Ser
                310

aactaggcca cacgcagagg agaaatggtc ttatgggtgg tgagctgagt actgaacaat 1326

ctaaaaatag cctctgattc cctcgctgga atccaacctg ttgataagtg atgggggttt 1386

agaagtagca aagagcaccc acattcaaaa gtcacagaac tggaaagtta attcatatta 1446

tttggtttga actgaaacgt gaggtatctt tgatgtgtat ggttggttat tgggagggaa 1506

aaatttgta aattagattg tctaaaaaaa atagttattc tgattatatt tttgttatct 1566

gggcaaagta gaagtcaagg ggtaaaaacc ctactattct gatttttgca caagttttag 1626

tggaaaataa aatcacactc tacagtaaaa aaaaaaaaaa aaaa                 1670
```

<210> 14
<211> 310
<212> PRT
<213> Homo sapiens

<400> 14

```
Met Arg Pro Leu Gln Ile Val Pro Ser Arg Leu Ile Ser Gln Leu Tyr
1               5                   10                  15

Cys Gly Leu Lys Pro Pro Ala Ser Thr Arg Asn Gln Ile Cys Leu Lys
            20                  25                  30
```

```
Met Ala Arg Pro Ser Ser Ser Met Ala Asp Phe Arg Lys Phe Phe Ala
        35                  40                  45

Lys Ala Lys His Ile Val Ile Ile Ser Gly Ala Gly Val Ser Ala Glu
    50                  55                  60

Ser Gly Val Pro Thr Phe Arg Gly Ala Gly Gly Tyr Trp Arg Lys Trp
65                  70                  75                  80

Gln Ala Gln Asp Leu Ala Thr Pro Leu Ala Phe Ala His Asn Pro Ser
                85                  90                  95

Arg Val Trp Glu Phe Tyr His Tyr Arg Arg Glu Val Met Gly Ser Lys
            100                 105                 110

Glu Pro Asn Ala Gly His Arg Ala Ile Ala Glu Cys Glu Thr Arg Leu
        115                 120                 125

Gly Lys Gln Gly Arg Arg Val Val Val Ile Thr Gln Asn Ile Asp Glu
    130                 135                 140

Leu His Arg Lys Ala Gly Thr Lys Asn Leu Leu Glu Ile His Gly Ser
145                 150                 155                 160

Leu Phe Lys Thr Arg Cys Thr Ser Cys Gly Val Val Ala Glu Asn Tyr
                165                 170                 175

Lys Ser Pro Ile Cys Pro Ala Leu Ser Gly Lys Gly Ala Pro Glu Pro
            180                 185                 190

Gly Thr Gln Asp Ala Ser Ile Pro Val Glu Lys Leu Pro Arg Cys Glu
        195                 200                 205

Glu Ala Gly Cys Gly Gly Leu Leu Arg Pro His Val Val Trp Phe Gly
    210                 215                 220

Glu Asn Leu Asp Pro Ala Ile Leu Glu Glu Val Asp Arg Glu Leu Ala
225                 230                 235                 240

His Cys Asp Leu Cys Leu Val Val Gly Thr Ser Ser Val Val Tyr Pro
                245                 250                 255

Ala Ala Met Phe Ala Pro Gln Val Ala Ala Arg Gly Val Pro Val Ala
            260                 265                 270

Glu Phe Asn Thr Glu Thr Thr Pro Ala Thr Asn Arg Phe Arg Phe His
        275                 280                 285

Phe Gln Gly Pro Cys Gly Thr Thr Leu Pro Glu Ala Leu Ala Cys His
    290                 295                 300

Glu Asn Glu Thr Val Ser
305                 310
```

<210> 15
<211> 1638
<212> DNA
<213> Homo sapiens

<220>
<221> CDS
<222> (61)..(1125)

<400> 15

```
gcttccggcg gaagcggcct caacaaggga aactttattg ttcccgtggg gcagtcgagg 60

atg tcg gtg aat tac gcg gcg ggg ctg tcg ccg tac gcg gac aag ggc   108
Met Ser Val Asn Tyr Ala Ala Gly Leu Ser Pro Tyr Ala Asp Lys Gly
 1               5                  10                  15

aag tgc ggc ctc ccg gag atc ttc gac ccc ccg gag gag ctg gag cgg   156
Lys Cys Gly Leu Pro Glu Ile Phe Asp Pro Pro Glu Glu Leu Glu Arg
            20                  25                  30

aag gtg tgg gaa ctg gcg agg ctg gtc tgg cag tct tcc agt gtg gtg   204
Lys Val Trp Glu Leu Ala Arg Leu Val Trp Gln Ser Ser Ser Val Val
        35                  40                  45

ttc cac acg ggt gcc ggc atc agc act gcc tct ggc atc ccc gac ttc   252
Phe His Thr Gly Ala Gly Ile Ser Thr Ala Ser Gly Ile Pro Asp Phe
    50                  55                  60

agg ggt ccc cac gga gtc tgg acc atg gag gag cga ggt ctg gcc ccc   300
Arg Gly Pro His Gly Val Trp Thr Met Glu Glu Arg Gly Leu Ala Pro
65                  70                  75                  80

aag ttc gac acc acc ttt gag agc gcg cgg ccc acg cag acc cac atg   348
Lys Phe Asp Thr Thr Phe Glu Ser Ala Arg Pro Thr Gln Thr His Met
                85                  90                  95

gcg ctg gtg cag ctg gag cgc gtg ggc ctc ctc cgc ttc ctg gtc agc   396
Ala Leu Val Gln Leu Glu Arg Val Gly Leu Leu Arg Phe Leu Val Ser
            100                 105                 110

cag aac gtg gac ggg ctc cat gtg cgc tca ggc ttc ccc agg gac aaa   444
Gln Asn Val Asp Gly Leu His Val Arg Ser Gly Phe Pro Arg Asp Lys
        115                 120                 125

ctg gca gag ctc cac ggg aac atg ttt gtg gaa gaa tgt gcc aag tgt   492
Leu Ala Glu Leu His Gly Asn Met Phe Val Glu Glu Cys Ala Lys Cys
    130                 135                 140

aag acg cag tac gtc cga gac aca gtc gtg ggc acc atg ggc ctg aag   540
Lys Thr Gln Tyr Val Arg Asp Thr Val Val Gly Thr Met Gly Leu Lys
145                 150                 155                 160

gcc acg ggc cgg ctc tgc acc gtg gct aag gca agg ggg ctg cga gcc   588
Ala Thr Gly Arg Leu Cys Thr Val Ala Lys Ala Arg Gly Leu Arg Ala
                165                 170                 175

tgc agg gga gag ctg agg gac acc atc cta gac tgg gag gac tcc ctg   636
Cys Arg Gly Glu Leu Arg Asp Thr Ile Leu Asp Trp Glu Asp Ser Leu
            180                 185                 190

ccc gac cgg gac ctg gca ctc gcc gat gag gcc agc agg aac gcc gac   684
Pro Asp Arg Asp Leu Ala Leu Ala Asp Glu Ala Ser Arg Asn Ala Asp
        195                 200                 205
```

```
ctg tcc atc acg ctg ggt aca tcg ctg cag atc cgg ccc agc ggg aac     732
Leu Ser Ile Thr Leu Gly Thr Ser Leu Gln Ile Arg Pro Ser Gly Asn
    210                 215                 220

ctg ccg ctg gct acc aag cgc cgg gga ggc cgc ctg gtc atc gtc aac     780
Leu Pro Leu Ala Thr Lys Arg Arg Gly Gly Arg Leu Val Ile Val Asn
225                 230                 235                 240

ctg cag ccc acc aag cac gac cgc cat gct gac ctc cgc atc cat ggc     828
Leu Gln Pro Thr Lys His Asp Arg His Ala Asp Leu Arg Ile His Gly
                245                 250                 255

tac gtt gac gag gtc atg acc cgg ctc atg gag cac ctg ggg ctg gag     876
Tyr Val Asp Glu Val Met Thr Arg Leu Met Glu His Leu Gly Leu Glu
            260                 265                 270

atc ccc gcc tgg gac ggc ccc cgt gtg ctg gag agg gcg ctg cca ccc     924
Ile Pro Ala Trp Asp Gly Pro Arg Val Leu Glu Arg Ala Leu Pro Pro
        275                 280                 285

ctg ccc cgc ccg ccc acc ccc aag ctg gag ccc aag gag gaa tct ccc     972
Leu Pro Arg Pro Pro Thr Pro Lys Leu Glu Pro Lys Glu Glu Ser Pro
    290                 295                 300

acc cgg atc aac ggc tct atc ccc gcc ggc ccc aag cag gag ccc tgc     1020
Thr Arg Ile Asn Gly Ser Ile Pro Ala Gly Pro Lys Gln Glu Pro Cys
305                 310                 315                 320

gcc cag cac aac ggc tca gag ccc gcc agc ccc aaa cgg gag cgg ccc     1068
Ala Gln His Asn Gly Ser Glu Pro Ala Ser Pro Lys Arg Glu Arg Pro
                325                 330                 335

acc agc cct gcc ccc cac aga ccc ccc aaa agg gtg aag gcc aag gcg     1116
Thr Ser Pro Ala Pro His Arg Pro Pro Lys Arg Val Lys Ala Lys Ala
            340                 345                 350

gtc ccc agc tgaccagggt gcttggggag ggtggggctt tttgtagaaa             1165
Val Pro Ser
        355

ctgtggattc tttttctctc gtggtctcac tttgttactt gtttctgtcc ccgggagcct 1225

cagggctctg agagctgtgc tccaggccag gggttacacc tgccctccgt ggtccctccc 1285

tgggctccag gggcctctgg tgcggttccg ggaagaagcc acaccccaga ggtgacagct 1345

gagcccctgc cacaccccag cctctgactt gctgtgttgt ccagaggtga ggctgggccc 1405

tccctggtct ccagcttaaa caggagtgaa ctccctctgt ccccagggcc tcccttctgg 1465

gccccctaca gcccacccta cccctcctcc atgggccctg caggagggga gacccacctt 1525

gaagtggggg atcagtagag gcttgcactg cctttggggc tggagggaga cgtgggtcca 1585

ccaggcttct ggaaaagtcc tcaatgcaat aaaaacaatt tctttcttgc aaa         1638
```

<210> 16
<211> 355
<212> PRT
<213> Homo sapiens

<400> 16

```
Met Ser Val Asn Tyr Ala Ala Gly Leu Ser Pro Tyr Ala Asp Lys Gly
1               5                   10                  15

Lys Cys Gly Leu Pro Glu Ile Phe Asp Pro Pro Glu Glu Leu Glu Arg
            20                  25                  30

Lys Val Trp Glu Leu Ala Arg Leu Val Trp Gln Ser Ser Ser Val Val
        35                  40                  45

Phe His Thr Gly Ala Gly Ile Ser Thr Ala Ser Gly Ile Pro Asp Phe
    50                  55                  60

Arg Gly Pro His Gly Val Trp Thr Met Glu Glu Arg Gly Leu Ala Pro
65                  70                  75                  80

Lys Phe Asp Thr Thr Phe Glu Ser Ala Arg Pro Thr Gln Thr His Met
                85                  90                  95

Ala Leu Val Gln Leu Glu Arg Val Gly Leu Leu Arg Phe Leu Val Ser
            100                 105                 110

Gln Asn Val Asp Gly Leu His Val Arg Ser Gly Phe Pro Arg Asp Lys
        115                 120                 125

Leu Ala Glu Leu His Gly Asn Met Phe Val Glu Glu Cys Ala Lys Cys
    130                 135                 140

Lys Thr Gln Tyr Val Arg Asp Thr Val Val Gly Thr Met Gly Leu Lys
145                 150                 155                 160

Ala Thr Gly Arg Leu Cys Thr Val Ala Lys Ala Arg Gly Leu Arg Ala
                165                 170                 175

Cys Arg Gly Glu Leu Arg Asp Thr Ile Leu Asp Trp Glu Asp Ser Leu
            180                 185                 190

Pro Asp Arg Asp Leu Ala Leu Ala Asp Glu Ala Ser Arg Asn Ala Asp
        195                 200                 205

Leu Ser Ile Thr Leu Gly Thr Ser Leu Gln Ile Arg Pro Ser Gly Asn
    210                 215                 220

Leu Pro Leu Ala Thr Lys Arg Arg Gly Gly Arg Leu Val Ile Val Asn
225                 230                 235                 240

Leu Gln Pro Thr Lys His Asp Arg His Ala Asp Leu Arg Ile His Gly
                245                 250                 255

Tyr Val Asp Glu Val Met Thr Arg Leu Met Glu His Leu Gly Leu Glu
            260                 265                 270

Ile Pro Ala Trp Asp Gly Pro Arg Val Leu Glu Arg Ala Leu Pro Pro
        275                 280                 285
```

```
Leu Pro Arg Pro Pro Thr Pro Lys Leu Glu Pro Lys Glu Glu Ser Pro
    290                 295                 300

Thr Arg Ile Asn Gly Ser Ile Pro Ala Gly Pro Lys Gln Glu Pro Cys
305                 310                 315                 320

Ala Gln His Asn Gly Ser Glu Pro Ala Ser Pro Lys Arg Glu Arg Pro
                325                 330                 335

Thr Ser Pro Ala Pro His Arg Pro Pro Lys Arg Val Lys Ala Lys Ala
            340                 345                 350

Val Pro Ser
        355
```

<210> 17
<211> 1718
<212> DNA
<213> Homo sapiens

<220>
<221> CDS
<222> (34)..(1233)

<400> 17

```
gcggaagcgg aagagcaggt ctccagggga gcg atg gca gcc ggg ggt ctg agc   54
                                     Met Ala Ala Gly Gly Leu Ser
                                     1               5

cgc tcc gag cgc aaa gcg gcg gag cgg gtc cgg agg ttg cgg gag gag    102
Arg Ser Glu Arg Lys Ala Ala Glu Arg Val Arg Arg Leu Arg Glu Glu
        10                  15                  20

cag cag agg gag cgc ctc cgc cag gtg tcg cgc atc ctg agg aag gcg    150
Gln Gln Arg Glu Arg Leu Arg Gln Val Ser Arg Ile Leu Arg Lys Ala
    25                  30                  35

gcg gcg gag cgc agc gcc gag gag ggc cgg ctg ctg gcc gag agc gcg    198
Ala Ala Glu Arg Ser Ala Glu Glu Gly Arg Leu Leu Ala Glu Ser Ala
40                  45                  50                  55

gac ctg gta acg gag ctg cag ggc cgg agc cgg cgg cgc gag ggc ctg    246
Asp Leu Val Thr Glu Leu Gln Gly Arg Ser Arg Arg Arg Glu Gly Leu
                60                  65                  70

aag cgg cgg cag gag gag gtg tgc gac gac ccg gag gag ctg cgg ggg    294
Lys Arg Arg Gln Glu Glu Val Cys Asp Asp Pro Glu Glu Leu Arg Gly
            75                  80                  85

aag gtc cgg gag ctg gcc agc gcc gtc cgg aac gcc aaa tac ttg gtc    342
Lys Val Arg Glu Leu Ala Ser Ala Val Arg Asn Ala Lys Tyr Leu Val
        90                  95                  100

gtc tac aca ggc gcg gga atc agc acg gca gcg tct atc cca gac tac    390
Val Tyr Thr Gly Ala Gly Ile Ser Thr Ala Ala Ser Ile Pro Asp Tyr
    105                 110                 115
```

```
cgg ggc cct aat gga gtg tgg aca ctg ctt cag aaa ggg aga agc gtt    438
Arg Gly Pro Asn Gly Val Trp Thr Leu Leu Gln Lys Gly Arg Ser Val
120                 125                 130                 135

agt gct gcc gac ctg agc gag gcc gag cca acc ctc acc cac atg agc    486
Ser Ala Ala Asp Leu Ser Glu Ala Glu Pro Thr Leu Thr His Met Ser
                140                 145                 150

atc acc cgt ctg cat gag cag aag ctg gtg cag cat gtg gtg tct cag    534
Ile Thr Arg Leu His Glu Gln Lys Leu Val Gln His Val Val Ser Gln
            155                 160                 165

aac tgt gac ggg ctc cac ctg agg agt ggg ctg ccg cgc acg gcc atc    582
Asn Cys Asp Gly Leu His Leu Arg Ser Gly Leu Pro Arg Thr Ala Ile
        170                 175                 180

tcc gag ctc cac ggg aac atg tac att gaa gtc tgt acc tcc tgc gtt    630
Ser Glu Leu His Gly Asn Met Tyr Ile Glu Val Cys Thr Ser Cys Val
    185                 190                 195

ccc aac agg gag tac gtg cgg gtg ttc gat gtg acg gag cgc act gcc    678
Pro Asn Arg Glu Tyr Val Arg Val Phe Asp Val Thr Glu Arg Thr Ala
200                 205                 210                 215

ctc cac aga cac cag aca ggc cgg acc tgc cac aag tgt ggg acc cag    726
Leu His Arg His Gln Thr Gly Arg Thr Cys His Lys Cys Gly Thr Gln
                220                 225                 230

ctg cgg gac acc att gtg cac ttt ggg gag agg ggg acg ttg ggg cag    774
Leu Arg Asp Thr Ile Val His Phe Gly Glu Arg Gly Thr Leu Gly Gln
            235                 240                 245

cct ctg aac tgg gaa gcg gcg acc gag gct gcc agc aga gca gac acc    822
Pro Leu Asn Trp Glu Ala Ala Thr Glu Ala Ala Ser Arg Ala Asp Thr
        250                 255                 260

atc ctg tgt cta ggg tcc agc ctg aag gtt cta aag aag tac cca cgc    870
Ile Leu Cys Leu Gly Ser Ser Leu Lys Val Leu Lys Lys Tyr Pro Arg
    265                 270                 275

ctc tgg tgc atg acc aag ccc cct agc cgg cgg ccg aag ctt tac atc    918
Leu Trp Cys Met Thr Lys Pro Pro Ser Arg Arg Pro Lys Leu Tyr Ile
280                 285                 290                 295

gtg aac ctg cag tgg acc ccg aag gat gac tgg gct gcc ctg aag cta    966
Val Asn Leu Gln Trp Thr Pro Lys Asp Asp Trp Ala Ala Leu Lys Leu
                300                 305                 310

cat ggg aag tgt gat gac gtc atg cgg ctc ctc atg gcc gag ctg ggc    1014
His Gly Lys Cys Asp Asp Val Met Arg Leu Leu Met Ala Glu Leu Gly
            315                 320                 325

ttg gag atc ccc gcc tat agc agg tgg cag gat ccc att ttc tca ctg    1062
Leu Glu Ile Pro Ala Tyr Ser Arg Trp Gln Asp Pro Ile Phe Ser Leu
        330                 335                 340

gcg act ccc ctg cgt gct ggt gaa gaa ggc agc cac agt cgg aag tcg    1110
Ala Thr Pro Leu Arg Ala Gly Glu Glu Gly Ser His Ser Arg Lys Ser
    345                 350                 355
```

```
ctg tgc aga agc aga gag gag gcc ccg cct ggg gac cgg ggt gca ccg   1158
Leu Cys Arg Ser Arg Glu Glu Ala Pro Pro Gly Asp Arg Gly Ala Pro
360                 365                 370                 375

ctt agc tcg gcc ccc atc cta ggg ggc tgg ttt ggc agg ggc tgc aca   1206
Leu Ser Ser Ala Pro Ile Leu Gly Gly Trp Phe Gly Arg Gly Cys Thr
                380                 385                 390

aaa cgc aca aaa agg aag aaa gtg acg taatcacgtg ctcgatgaag         1253
Lys Arg Thr Lys Arg Lys Lys Val Thr
            395                 400

aacagttggc actttgcaga tggccagtgt cacggtgaag gctgggttgc ccccacgggt 1313

ctagggagaa cgaactcttt ggggatgaca ttttcaccgt gacattttta gccatttgtc 1373

cttgaggaag ccccttgcac tgctgcggtt gtaccctgat acggcctggc catcgaggac 1433

acctgcccat ccggcctctg tgtcaagagg tggcagccgc acctttctgt gagaacggaa 1493

ctcgggttat ttcagccccg gcctgcagag tggaagcgcc cagcggcctt tcctcgctca 1553

ccaggccagt ctcagggcct caccgtattt ctactactac ttaatgaaaa agtgtgaact 1613

ttatagaatc ctctctgtac tggatgtgcg gcagaggggt ggctccgagc ctcggctcta 1673

tgcagacctt tttatttcta ttaaacgttt ctgcactggc aaaaa                 1718
```

<210> 18
<211> 400
<212> PRT
<213> Homo sapiens

<400> 18

```
Met Ala Ala Gly Gly Leu Ser Arg Ser Glu Arg Lys Ala Ala Glu Arg
1               5                   10                  15

Val Arg Arg Leu Arg Glu Glu Gln Gln Arg Glu Arg Leu Arg Gln Val
            20                  25                  30

Ser Arg Ile Leu Arg Lys Ala Ala Ala Glu Arg Ser Ala Glu Glu Gly
        35                  40                  45

Arg Leu Leu Ala Glu Ser Ala Asp Leu Val Thr Glu Leu Gln Gly Arg
    50                  55                  60

Ser Arg Arg Arg Glu Gly Leu Lys Arg Arg Gln Glu Glu Val Cys Asp
65                  70                  75                  80

Asp Pro Glu Glu Leu Arg Gly Lys Val Arg Glu Leu Ala Ser Ala Val
                85                  90                  95

Arg Asn Ala Lys Tyr Leu Val Val Tyr Thr Gly Ala Gly Ile Ser Thr
            100                 105                 110

Ala Ala Ser Ile Pro Asp Tyr Arg Gly Pro Asn Gly Val Trp Thr Leu
        115                 120                 125
```

```
Leu Gln Lys Gly Arg Ser Val Ser Ala Ala Asp Leu Ser Glu Ala Glu
    130                 135                 140

Pro Thr Leu Thr His Met Ser Ile Thr Arg Leu His Glu Gln Lys Leu
145                 150                 155                 160

Val Gln His Val Val Ser Gln Asn Cys Asp Gly Leu His Leu Arg Ser
                165                 170                 175

Gly Leu Pro Arg Thr Ala Ile Ser Glu Leu His Gly Asn Met Tyr Ile
            180                 185                 190

Glu Val Cys Thr Ser Cys Val Pro Asn Arg Glu Tyr Val Arg Val Phe
        195                 200                 205

Asp Val Thr Glu Arg Thr Ala Leu His Arg His Gln Thr Gly Arg Thr
    210                 215                 220

Cys His Lys Cys Gly Thr Gln Leu Arg Asp Thr Ile Val His Phe Gly
225                 230                 235                 240

Glu Arg Gly Thr Leu Gly Gln Pro Leu Asn Trp Glu Ala Ala Thr Glu
                245                 250                 255

Ala Ala Ser Arg Ala Asp Thr Ile Leu Cys Leu Gly Ser Ser Leu Lys
            260                 265                 270

Val Leu Lys Lys Tyr Pro Arg Leu Trp Cys Met Thr Lys Pro Pro Ser
        275                 280                 285

Arg Arg Pro Lys Leu Tyr Ile Val Asn Leu Gln Trp Thr Pro Lys Asp
    290                 295                 300

Asp Trp Ala Ala Leu Lys Leu His Gly Lys Cys Asp Asp Val Met Arg
305                 310                 315                 320

Leu Leu Met Ala Glu Leu Gly Leu Glu Ile Pro Ala Tyr Ser Arg Trp
                325                 330                 335

Gln Asp Pro Ile Phe Ser Leu Ala Thr Pro Leu Arg Ala Gly Glu Glu
            340                 345                 350

Gly Ser His Ser Arg Lys Ser Leu Cys Arg Ser Arg Glu Glu Ala Pro
        355                 360                 365

Pro Gly Asp Arg Gly Ala Pro Leu Ser Ser Ala Pro Ile Leu Gly Gly
    370                 375                 380

Trp Phe Gly Arg Gly Cys Thr Lys Arg Thr Lys Arg Lys Lys Val Thr
385                 390                 395                 400
```

<210> 19
<211> 6
<212> PRT
<213> Homo sapiens

<220>
<221> MOD_RES
<222> (1)
<223> Lys(Ac)

<220>

<221> MOD_RES
<222> (6)
<223> Lys(Ac)

<400> 19

Lys Gln Thr Ala Arg Lys
1 5

<210> 20
<211> 6
<212> PRT
<213> Homo sapiens

<220>
<221> MOD_RES
<222> (1)
<223> Lys(Ac)

<220>
<221> MOD_RES
<222> (6)
<223> Lys(Ac)

<400> 20

Lys Ser Thr Gly Gly Lys
1 5

<210> 21
<211> 6
<212> PRT
<213> Homo sapiens

<220>
<221> MOD_RES
<222> (1)
<223> Lys(Ac)

<220>
<221> MOD_RES
<222> (2)
<223> Ser(PO3)

<220>
<221> MOD_RES
<222> (6)
<223> Lys(Ac)

<400> 21

Lys Ser Thr Gly Gly Lys
1 5

<210> 22
<211> 5
<212> PRT
<213> Homo sapiens

<220>
<221> MOD_RES
<222> (1)
<223> Lys(Ac)

<220>
<221> MOD_RES
<222> (5)
<223> Lys(Ac)

<400> 22

```
Lys Ala Pro Arg Lys
  1               5
```

<210> 23
<211> 5
<212> PRT
<213> Homo sapiens

<220>
<221> MOD_RES
<222> (5)
<223> Lys(Ac)

<400> 23

```
Ser Gly Arg Gly Lys
  1               5
```

<210> 24
<211> 5
<212> PRT
<213> Homo sapiens

<220>
<221> MOD_RES
<222> (5)
<223> Lys(Ac)

<400> 24

```
Lys Gly Gly Ala Lys
  1               5
```

<210> 25
<211> 5
<212> PRT
<213> Homo sapiens

<220>
<221> MOD_RES
<222> (1)
<223> Lys(Ac)

<220>
<221> MOD_RES
<222> (5)

<223> Lys(Ac)

<400> 25

Lys Gly Gly Ala Lys
1 5

<210> 26
<211> 4
<212> PRT
<213> Homo sapiens

<220>
<221> MOD_RES
<222> (4)
<223> Lys(Ac)

<400> 26

Gln Pro Lys Lys
1

<210> 27
<211> 4
<212> PRT
<213> Homo sapiens

<220>
<221> MOD_RES
<222> (1)
<223> Lys(Ac)

<220>
<221> MOD_RES
<222> (4)
<223> Lys(Ac)

<400> 27

Lys Ser Lys Lys
1

<210> 28
<211> 4
<212> PRT
<213> Homo sapiens

<220>
<221> MOD_RES
<222> (4)
<223> Lys(Ac)

<400> 28

Arg His Lys Lys
1

<210> 29

<211> 4
<212> PRT
<213> Homo sapiens

<220>
<221> MOD_RES
<222> (3)
<223> Lys(Ac)

<220>
<221> MOD_RES
<222> (4)
<223> Lys(Ac)

<400> 29

```
Arg His Lys Lys
1
```

<210> 30
<211> 369
<212> PRT
<213> Homo sapiens

<400> 30

```
Met Pro Leu Ala Glu Cys Pro Ser Cys Arg Cys Leu Ser Ser Phe Arg
1               5                   10                  15

Ser Val Asp Phe Leu Arg Asn Leu Phe Ser Gln Thr Leu Ser Leu Gly
            20                  25                  30

Ser Gln Lys Glu Arg Leu Leu Asp Glu Leu Thr Leu Glu Gly Val Ala
        35                  40                  45

Arg Tyr Met Gln Ser Glu Arg Cys Arg Arg Val Ile Cys Leu Val Gly
    50                  55                  60

Ala Gly Ile Ser Thr Ser Ala Gly Ile Pro Asp Phe Arg Ser Pro Ser
65                  70                  75                  80

Thr Gly Leu Tyr Asp Asn Leu Glu Lys Tyr His Leu Pro Tyr Pro Glu
                85                  90                  95

Ala Ile Phe Glu Ile Ser Tyr Phe Lys Lys His Pro Glu Pro Phe Phe
            100                 105                 110

Ala Leu Ala Lys Glu Leu Tyr Pro Gly Gln Phe Lys Pro Thr Ile Cys
        115                 120                 125

His Tyr Phe Met Arg Leu Leu Lys Asp Lys Gly Leu Leu Leu Arg Cys
    130                 135                 140
```

Tyr Thr Gln Asn Ile Asp Thr Leu Glu Arg Ile Ala Gly Leu Glu Gln
145 150 155 160

Glu Asp Leu Val Glu Ala His Gly Thr Phe Tyr Thr Ser His Cys Val
165 170 175

Ser Ala Ser Cys Arg His Glu Tyr Pro Leu Ser Trp Met Lys Glu Lys
180 185 190

Ile Phe Ser Glu Val Thr Leu Lys Cys Glu Asp Cys Gln Ser Leu Val
195 200 205

Lys Pro Asp Ile Val Phe Phe Gly Glu Ser Leu Pro Ala Arg Phe Phe
210 215 220

Ser Cys Met Gln Ser Asp Phe Leu Lys Val Asp Leu Leu Leu Val Met
225 230 235 240

Gly Thr Ser Leu Gln Val Gln Pro Phe Ala Ser Leu Ile Ser Lys Ala
245 250 255

Pro Leu Ser Thr Pro Arg Leu Leu Ile Asn Lys Glu Lys Ala Gly Gln
260 265 270

Ser Asp Pro Phe Leu Gly Met Ile Met Gly Leu Gly Gly Gly Met Asp
275 280 285

Phe Asp Ser Lys Lys Ala Tyr Arg Asp Val Ala Trp Leu Gly Glu Cys
290 295 300

Asp Gln Gly Cys Leu Ala Leu Ala Glu Leu Leu Gly Trp Lys Lys Glu
305 310 315 320

Leu Glu Asp Leu Val Arg Arg Glu His Ala Ser Ile Asp Ala Gln Ser
325 330 335

Gly Ala Gly Val Pro Asn Pro Ser Thr Ser Ala Ser Pro Lys Lys Ser
340 345 350

Pro Pro Pro Ala Lys Asp Glu Ala Arg Thr Thr Glu Arg Glu Lys Pro
355 360 365

Gln

<210> 31
<211> 562
<212> PRT
<213> Saccharomyces cerevisiae

<400> 31

Met Thr Ile Pro His Met Lys Tyr Ala Val Ser Lys Thr Ser Glu Asn
1 5 10 15

Lys Val Ser Asn Thr Val Ser Pro Thr Gln Asp Lys Asp Ala Ile Arg
20 25 30

Lys Gln Pro Asp Asp Ile Ile Asn Asn Asp Glu Pro Ser His Lys Lys
35 40 45

Ile Lys Val Ala Gln Pro Asp Ser Leu Arg Glu Thr Asn Thr Thr Asp
50 55 60

Pro Leu Gly His Thr Lys Ala Ala Leu Gly Glu Val Ala Ser Met Glu
65 70 75 80

Leu Lys Pro Thr Asn Asp Met Asp Pro Leu Ala Val Ser Ala Ala Ser
85 90 95

Val Val Ser Met Ser Asn Asp Val Leu Lys Pro Glu Thr Pro Lys Gly
100 105 110

Pro Ile Ile Ile Ser Lys Asn Pro Ser Asn Gly Ile Phe Tyr Gly Pro
115 120 125

Ser Phe Thr Lys Arg Glu Ser Leu Asn Ala Arg Met Phe Leu Lys Tyr
130 135 140

Tyr Gly Ala His Lys Phe Leu Asp Thr Tyr Leu Pro Glu Asp Leu Asn
145 150 155 160

Ser Leu Tyr Ile Tyr Tyr Leu Ile Lys Leu Leu Gly Phe Glu Val Lys
165 170 175

Asp Gln Ala Leu Ile Gly Thr Ile Asn Ser Ile Val His Ile Asn Ser
180 185 190

Gln Glu Arg Val Gln Asp Leu Gly Ser Ala Ile Ser Val Thr Asn Val
195 200 205

Glu Asp Pro Leu Ala Lys Lys Gln Thr Val Arg Leu Ile Lys Asp Leu
210 215 220

Gln Arg Ala Ile Asn Lys Val Leu Cys Thr Arg Leu Arg Leu Ser Asn
225 230 235 240

Phe Phe Thr Ile Asp His Phe Ile Gln Lys Leu His Thr Ala Arg Lys
245 250 255

Ile Leu Val Leu Thr Gly Ala Gly Val Ser Thr Ser Leu Gly Ile Pro
260 265 270

Asp Phe Arg Ser Ser Glu Gly Phe Tyr Ser Lys Ile Lys His Leu Gly
275 280 285

Leu Asp Asp Pro Gln Asp Val Phe Asn Tyr Asn Ile Phe Met His Asp
290 295 300

Pro Ser Val Phe Tyr Asn Ile Ala Asn Met Val Leu Pro Pro Glu Lys
305 310 315 320

Ile Tyr Ser Pro Leu His Ser Phe Ile Lys Met Leu Gln Met Lys Gly
325 330 335

Lys Leu Leu Arg Asn Tyr Thr Gln Asn Ile Asp Asn Leu Glu Ser Tyr
340 345 350

```
Ala Gly Ile Ser Thr Asp Lys Leu Val Gln Cys His Gly Ser Phe Ala
        355                 360                 365

Thr Ala Thr Cys Val Thr Cys His Trp Asn Leu Pro Gly Glu Arg Ile
    370                 375                 380

Phe Asn Lys Ile Arg Asn Leu Glu Leu Pro Leu Cys Pro Tyr Cys Tyr
385                 390                 395                 400

Lys Lys Arg Arg Glu Tyr Phe Pro Glu Gly Tyr Asn Asn Lys Val Gly
                405                 410                 415

Val Ala Ala Ser Gln Gly Ser Met Ser Glu Arg Pro Pro Tyr Ile Leu
            420                 425                 430

Asn Ser Tyr Gly Val Leu Lys Pro Asp Ile Thr Phe Phe Gly Glu Ala
        435                 440                 445

Leu Pro Asn Lys Phe His Lys Ser Ile Arg Glu Asp Ile Leu Glu Cys
    450                 455                 460

Asp Leu Leu Ile Cys Ile Gly Thr Ser Leu Lys Val Ala Pro Val Ser
465                 470                 475                 480

Glu Ile Val Asn Met Val Pro Ser His Val Pro Gln Val Leu Ile Asn
                485                 490                 495

Arg Asp Pro Val Lys His Ala Glu Phe Asp Leu Ser Leu Leu Gly Tyr
            500                 505                 510

Cys Asp Asp Ile Ala Ala Met Val Ala Gln Lys Cys Gly Trp Thr Ile
        515                 520                 525

Pro His Lys Lys Trp Asn Asp Leu Lys Asn Lys Asn Phe Lys Cys Gln
    530                 535                 540

Glu Lys Asp Lys Gly Val Tyr Val Val Thr Ser Asp Glu His Pro Lys
545                 550                 555                 560

Thr Leu
```